# EUROPEAN PATENT APPLICATION

(11) **EP 4 617 262 A1**
(43) Date of publication of application: **17.09.2025**
(21) Application number: 23888683.2
(22) Date of filing: 07.11.2023
(51) Int. Cl.: C07D 239/95, A61K 31/517, A61K 31/519, A61P 3/00, A61P 43/00, C07D 401/12, C07D 405/12, C07D 405/14, C07D 409/12, C07D 413/12, C07D 413/14, C07D 417/12, C07D 471/04

(54) **NITROGEN-CONTAINING HETEROCYCLIC COMPOUND**

(30) Priority: 07.11.2022 JP 2022178401
(71) Applicant: Kyoto University, Kyoto-shi, Kyoto 606-8501 (JP)
(72) Inventor: HAGIWARA, Masatoshi, Kyoto 606-8501 (JP); AWAYA, Tomonari, Kyoto 606-8501 (JP); AJIRO, Masahiko, Kyoto 606-8501 (JP); GOTANDA, Kentoku, Tokyo 103-8426 (JP); TAKEMOTO, Naohiro, Tokyo 103-8426 (JP); NOJI, Toshiharu, Tokyo 103-8426 (JP)
(74) Representative: Dehns
(86) International application number: PCT/JP2023/039979
(87) International publication number: WO 2024/101336

(57) **Abstract**

An aspect of the present disclosure provides a compound capable of suppressing aberrant splicing that contributes to the onset or progression of a disease, or a pharmaceutically acceptable salt thereof. An aspect of the present disclosure relates to a compound represented by Formula (I) below or a pharmaceutically acceptable salt thereof.

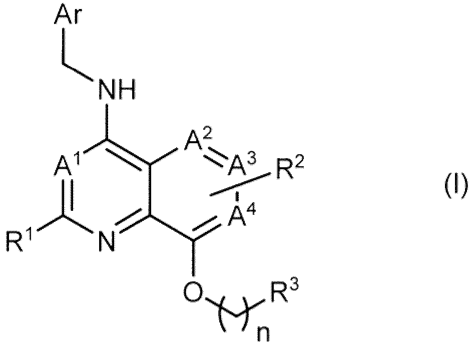

## Description

### Technical Field

The present disclosure relates to a compound capable of suppressing aberrant splicing that contributes to the onset or progression of a disease, or a pharmaceutically acceptable salt thereof.

### Background Art

RNA splicing refers to the process of removing introns from post-transcriptional pre-mRNA and joining exons together. In this process, exons are not only constitutively spliced, but the recognition of particular exon sequences or intron sequences may also be regulated in a fixed proportion depending on the tissue or cell lineage.

In addition, even in an originally intron region, a genetic mutation can sometimes create a new splicing regulatory region, resulting in part of the intron region being recognized as an exon and causing aberrant splicing. This aberrant splicing may lead to various diseases.

Fabry disease is a genetic disorder, some cases of which are caused by aberrant splicing. In addition to symptomatic treatment, enzyme replacement therapy using recombinant α-galactosidase A (GLA) enzyme protein has been developed as a treatment method for Fabry disease. However, there is no treatment that acts on the gene itself, which is the root cause.

Aberrant splicing is known to occur in many other genetic diseases, and there is a need to develop new compounds that can suppress aberrant splicing.

The present inventors have identified splicing-controlling compounds (e.g., 2-chloro-6-(2-furylmethyl)purine), as disclosed in Patent Document 1, for example, for diseases caused by aberrant splicing, including cardiac Fabry disease.

### Prior Art Documents

### Patent Document

Patent Document 1: WO 2018/151326

### Disclosure of Invention

### Problem to be Solved by the Invention

The present disclosure provides a compound capable of suppressing aberrant splicing that contributes to the onset or progression of a disease, or a pharmaceutically acceptable salt thereof.

### Means for Solving Problem

An aspect of the present disclosure relates to a compound represented by Formula (I) below or a pharmaceutically acceptable salt thereof: 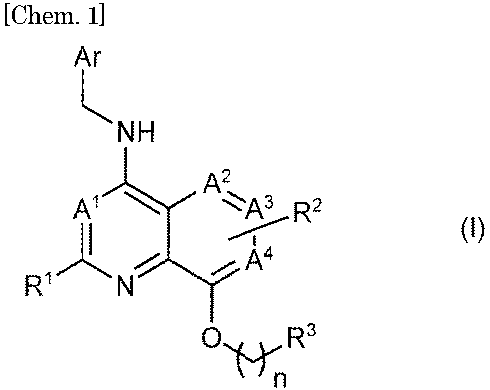 wherein, in Formula (I),
A¹, A², A³, and A⁴ each independently represent CH or N,
Ar represents a 6- to 12-membered aryl group, or a 5- or 6-membered heteroaryl group having 1 to 3 heteroatoms selected from the group consisting of nitrogen, oxygen, and sulfur atoms in the ring, wherein the 6- to 12-membered aryl group and the 5- or 6-membered heteroaryl group may be substituted with one or more halogen atoms,
n is 1 or 2,
R¹ represents a halogen atom,
R² represents a hydrogen atom, a halogen atom, or a C₁-C₄ alkoxy group, and
R³ represents a hydrogen atom, a C₃-C₆ cycloalkyl group, or a 4- to 6-membered heterocyclyl group having 1 to 3 heteroatoms selected from the group consisting of nitrogen, oxygen, and sulfur atoms in the ring,
wherein the C₃-C₆ cycloalkyl group and the 4- to 6-membered heterocyclyl group of R³ may have one or two oxo groups (=O) on the ring and/or the C₃-C₆ cycloalkyl group and the 4- to 6-membered heterocyclyl group may be substituted with one or two substituents selected from Group A below:
   Group A:
   a halogen atom, a hydroxy group, a C₁-C₄ alkyl group that may be substituted with a halogen atom, a C₁-C₆ hydroxyalkyl group that may be substituted with a halogen atom, a C₁-C₂ alkoxy group that may be substituted with a halogen atom, a C₂-C₆ alkoxyalkyl group that may be substituted with a halogen atom, a C₃-C₆ alkoxyalkoxyalkyl group that may be substituted with a halogen atom, a C₁-C₂ alkylsulfonyl group that may be substituted with a halogen atom, an acetyl group (-C(O)CH₃), a cyclopropylmethyl group, a phenyl group that may be substituted with a halogen atom, a phenyl C₁₋C₃ alkyl group that may be substituted with a halogen atom, a 3- to 5-membered heterocyclyl group having one or two oxygen atoms, a 3- to 5-membered heterocyclyl-C₁₋C₃ alkyl group having one or two oxygen atoms, an amino-oxo C₁₋C₃ alkyl group, a C₂-C₄ alkynyl group that may be substituted with a halogen atom, an amino C₂-C₆ (alkoxyalkyl group) that may be protected, a C₁-C₃ alkylaminocarbonyl group, a 5-membered heteroaryl C₁₋C₃ alkyl group, a C₂-C₄ alkylene group (linked to the same carbon), a C₁-C₄ alkylenedioxy group (linked to the same carbon), and a C₁-C₄ alkyleneoxy-C₁-C₄ alkyl group (linked to the same carbon).

An aspect of the present disclosure relates to a compound represented by Formula (II) below or a pharmaceutically acceptable salt thereof: 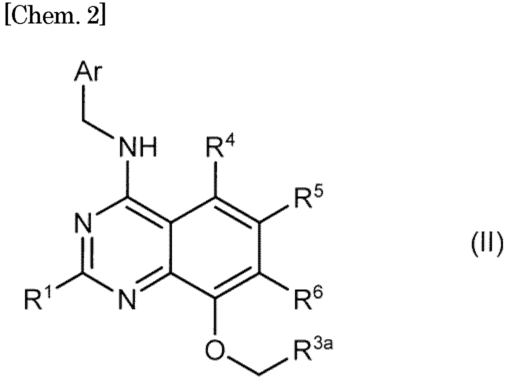 wherein, in Formula (II),
Ar represents a furyl group, a phenyl group, a thienyl group, a pyridyl group, a pyrimidinyl group, a thiazolyl group, an oxazolyl group, an isoxazolyl group, an isothiazolyl group, or an oxadiazolyl group, wherein the furyl group, the phenyl group, and the pyridyl group may be substituted with one fluorine atom,
R¹ represents a halogen atom,
R⁴, R⁵, and R⁶ each independently represent a hydrogen atom, a halogen atom, or a methoxy group, wherein at least two of R⁴, R⁵, and R⁶ represent hydrogen atoms, and
R^{3a} represents a hydrogen atom or any group selected from Group C below:
   Group C: 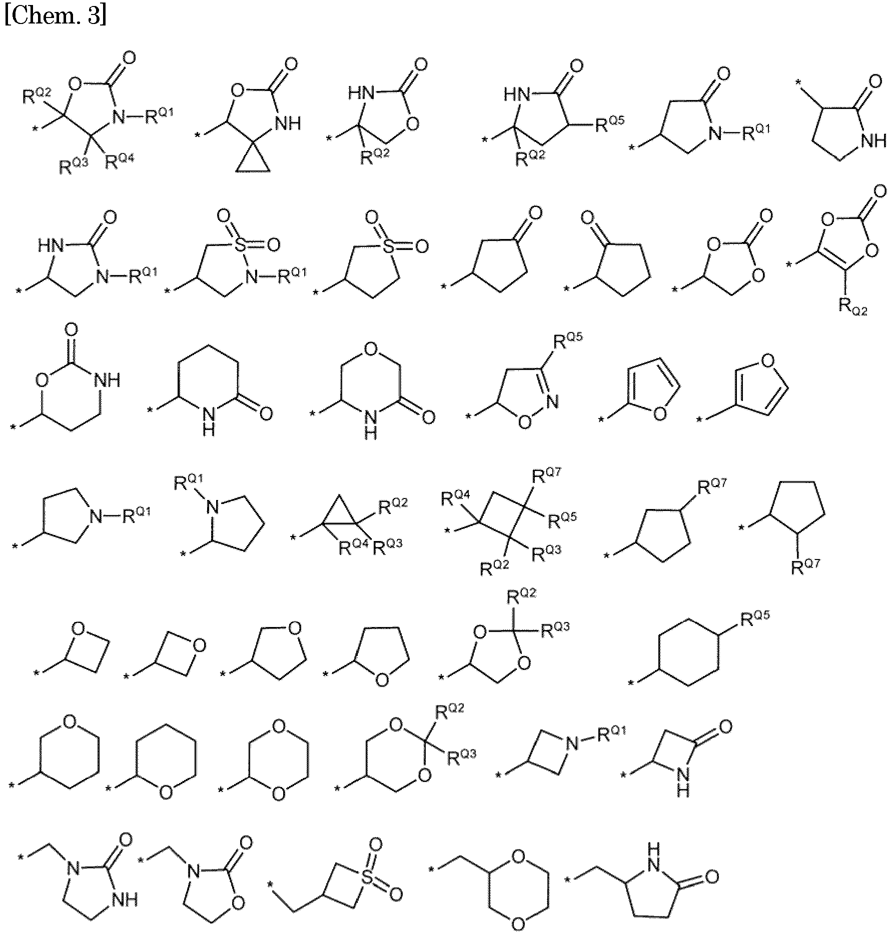
wherein R^{Q1} represents a hydrogen atom, a fluorine atom, a methyl group, an ethyl group, an isopropyl group, a fluoroethyl group, a difluoroethyl group, a hydroxyethyl group, a 2-hydroxy-2-methylpropyl group, a methoxyethyl group (-C₂H₄OCH₃), a methoxyethoxyethyl group (-C₂H₄OC₂H₄OCH₃), a methylsulfonyl group (-S(O)₂CH₃), an acetyl group (-C(O)CH₃), a cyclopropylmethyl group, a phenyl group, a benzyl group, an oxetanyl group, an oxetanylmethyl group, a 2-amino-2-oxoethyl group (-CH₂C(O)NH₂), a propargyl group (-CH₂C≡CH), a tert-butoxycarbonyl-2-aminoethoxyethyl group (-C₂H₄OC₂H₄NHC(O)O(CH₃)₃), an ethylaminocarbonyl group (-C(O)NHCH₂CH₃), or an oxadiazolylmethyl group,
R^{Q2}, R^{Q3}, and R^{Q4} each independently represent a hydrogen atom, a fluorine atom, a hydroxy group, a methyl group, or a trifluoromethyl group,
R^{Q5} and R^{Q6} each independently represent a hydrogen atom, a fluorine atom, a hydroxy group, a methyl group, a methoxy group (-OCH₃), a difluoromethoxy group (-OCHF₂), or a methoxymethyl group, and
R^{Q7} represents a hydrogen atom, a fluorine atom, a methyl group, or an ethyl group, or alternatively an ethylenedioxy group (linked to the same carbon) or a methyleneoxymethyl group (linked to the same carbon).

An aspect of the present disclosure relates to a compound represented by Formula (III) below or a pharmaceutically acceptable salt thereof: 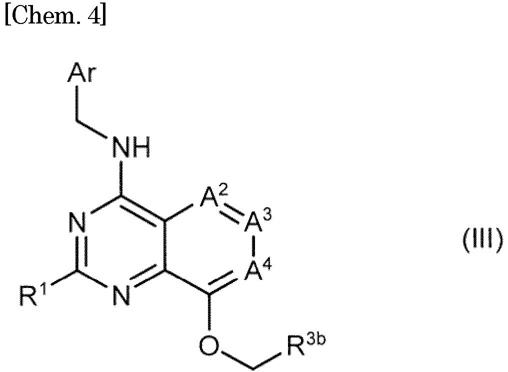 wherein, in Formula (III),
Ar represents a furyl group or a phenyl group,
R¹ represents a chlorine atom,
A², A³, and A⁴ each independently represent CH or N and any one of A², A³, and A⁴ represents N, and
R^{3b} represents a hydrogen atom or any group selected from Group D below:
   Group D:

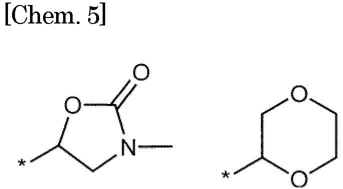

An aspect of the present disclosure relates to a compound represented by Formula (IV) below or a pharmaceutically acceptable salt thereof: 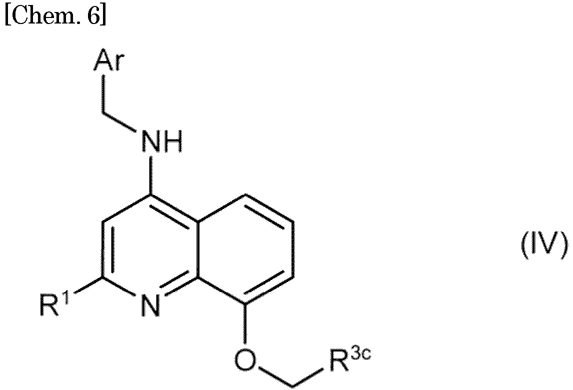 wherein, in Formula (IV),
Ar represents a furyl group or a phenyl group,
R¹ represents a chlorine atom,
R^{3c} represents a hydrogen atom or any group selected from Group E below:
   Group E: 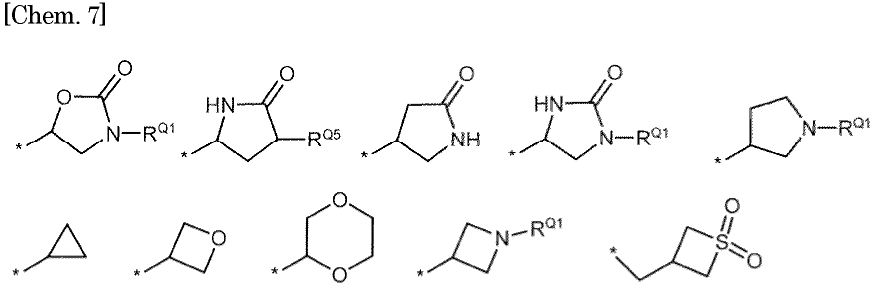
wherein R^{Q1} represents a hydrogen atom, a methyl group, an acetyl group, a methylsulfonyl group, a fluoroethyl group, or a hydroxyethyl group, and
R^{Q5} represents a hydrogen atom or a methyl group.

In an aspect, the present disclosure relates to a compound represented by Formula (V) below or a pharmaceutically acceptable salt thereof: 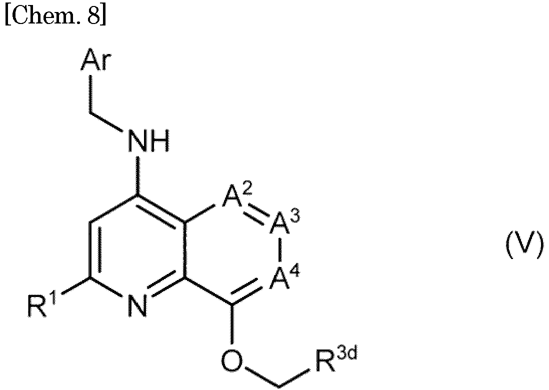 wherein, in Formula (V),
Ar represents a furyl group or a phenyl group,
R¹ represents a chlorine atom,
A², A³, and A⁴ each independently represent CH or N and any one of A², A³, and A⁴ represents N, and
R^{3d} represents a hydrogen atom or any group selected from Group F below:
   Group F: 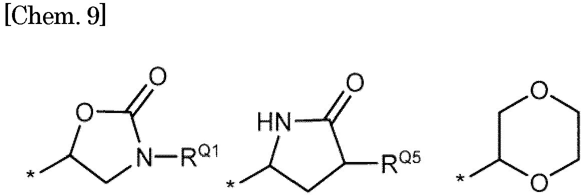
wherein R^{Q1} represents a hydrogen atom, a methyl group, a fluoroethyl group, or a hydroxyethyl group, and
R^{Q5} represents a hydrogen atom or a methyl group.

Another aspect of the present disclosure relates to a pharmaceutical composition containing, as an active ingredient, the compound of the present disclosure or pharmaceutically acceptable salt thereof.

Another aspect of the present disclosure relates to a pharmaceutical composition for treating a genetic disease caused by aberrant splicing, the pharmaceutical composition containing, as an active ingredient, the compound of the present disclosure or pharmaceutically acceptable salt thereof.

Another aspect of the present disclosure relates to a pharmaceutical composition for treating a genetic disease caused by aberrant splicing, the pharmaceutical composition containing, as an active ingredient, the compound of the present disclosure or pharmaceutically acceptable salt thereof.

Another aspect of the present disclosure relates to a method for treating a genetic disease caused by aberrant splicing, the method including administering the compound of the present disclosure or pharmaceutically acceptable salt thereof to a subject.

Another aspect of the present disclosure relates to use of the compound of the present disclosure or pharmaceutically acceptable salt thereof in production of a pharmaceutical composition for use in treatment of a genetic disease caused by aberrant splicing.

### Effects of the Invention

The present disclosure can provide a compound capable of suppressing aberrant splicing that contributes to the onset or progression of a disease, or a pharmaceutically acceptable salt thereof.

### Brief Description of Drawings

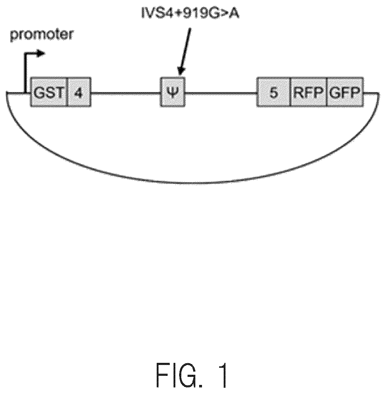

[FIG. 1] FIG. 1 shows a schematic diagram of a GLA splicing reporter vector. Normal splicing of exon 4 and exon 5 results in the expression of a green fluorescent protein GFP as a GST (glutathione S-transferase) fusion protein. However, when disease-type splicing of exon 4, a pseudoexon (Ψ), and exon 5 occurs, the frame is shifted by one base during translation, resulting in the expression of a red fluorescent protein RFP.

### Description of the Invention

The term "halogen atom" as used in the present disclosure refers to a fluorine atom, a chlorine atom, a bromine atom, or an iodine atom, preferably a fluorine atom or a chlorine atom, and more preferably a chlorine atom.

The phrase "may be substituted with a halogen atom" as used in the present disclosure means either being unsubstituted or having one or two or more hydrogen atoms replaced with a halogen atom.

The term "C₁-C₄ alkyl group" as used in the present disclosure refers to a linear or branched alkyl group having 1 to 4 carbon atoms. In one or more embodiments, examples of the C₁-C₄ alkyl group include a methyl group, an ethyl group, an n-propyl group, an isopropyl group, an n-butyl group, a sec-butyl group, a tert-butyl group, and the like.

The term "C₁-C₄ alkyl group that may be substituted with a halogen atom" as used in the present disclosure may include a group in which one, two, or three hydrogen atoms of a "C₁-C₄ alkyl group" are replaced with a halogen atom. In one or more embodiments, examples of the "C₁-C₄ alkyl group that may be substituted with a halogen atom" include a fluoromethyl group, a difluoromethyl group, a trifluoromethyl group, a chloromethyl group, a dichloromethyl group, a trichloromethyl group, a 1-fluoroethyl group, a 2-fluoroethyl group, a difluoroethyl group, a 2,2,2-trifluoroethyl group, a 1,2-difluoropropyl group, and the like.

The term "C₁-C₆ hydroxyalkyl group" as used in the present disclosure refers to a group in which one or two hydrogen atoms of a linear or branched alkyl group having 1 to 6 carbon atoms are replaced with a hydroxy group. In one or more embodiments, examples of the C₁-C₆ hydroxyalkyl group include a hydroxymethyl group, a 1-hydroxyethyl group, a 2-hydroxyethyl group, a 1-hydroxypropyl group, a 2-hydroxypropyl group, a 1-hydroxyisopropyl group, a 1-hydroxybutyl group, a 2-hydroxybutyl group, a 2-hydroxyisobutyl group, a 1-hydroxypentyl group, a 2-hydroxypentyl group, a 1-hydroxyhexyl group, a 1,2-hydroxyethyl group, and the like.

The term "C₁-C₄ alkylene group" as used in the present disclosure refers to a linear or branched alkylene group having 1 to 4 carbon atoms. In one or more embodiments, examples of the C₁-C₄ alkylene group include a methylene group, an ethylene group (-(CH₂)₂-), a trimethylene group (-(CH₂)₃-), a tetramethylene group, a methylmethylene group (-CH(CH₃)-), methylethylene groups (-CH(CH₃)CH₂- and - CH₂CH(CH₃)-), and the like.

The term "C₂-C₄ alkylene group (linked to the same carbon)" as used in the present disclosure may be a C₃-C₅ cycloalkyl group formed by two bonds of a C₂-C₄ alkylene group linked to the same carbon atom.

The term "C₁-C₄ alkylenedioxy group" as used in the present disclosure refers to a linear or branched alkylene group having 1 to 4 carbon atoms and two oxygen atoms (-O-). In one or more embodiments, examples of the C₁-C₄ alkylenedioxy group include a methylenedioxy group (-O-CH₂-O-), an ethylenedioxy group (-O-CH₂=CH₂-O-), and the like. The term "C₁-C₄ alkylenedioxy group (linked to the same carbon)" as used in the present disclosure may be a C₂-C₄ cycloalkylenedioxy group formed by two bonds of a C₁-C₄ alkylenedioxy group linked to the same carbon atom.

In one or more embodiments, examples of a C₁-C₄ alkyleneoxy-C₁-C₄ alkyl group of the present disclosure include a methyleneoxymethyl group (-CH₂-O-CH₂-) and the like. The term "C₁-C₄ alkyleneoxy-C₁-C₄ alkyl group (linked to the same carbon)" as used in the present disclosure may be a C₂-C₄ cycloalkylenedioxy group formed by two bonds of a C₁-C₄ alkyleneoxy-C₁-C₄ alkyl group linked to the same carbon atom.

The term "C₁-C₄ alkoxy group" as used in the present disclosure refers to a group in which an alkyl group having 1 to 4 carbon atoms is linked to an oxygen atom. In one or more embodiments, examples of the C₁-C₄ alkoxy group include a methoxy group, an ethoxy group, a propoxy group, a butoxy group, a t-butoxy group, and the like.

The term "C₁-C₂ alkoxy group" as used in the present disclosure refers to a group in which an alkyl group having 1 or 2 carbon atoms is linked to an oxygen atom. In one or more embodiments, examples of the C₁-C₂ alkoxy group include a methoxy group (-OCH₃), an ethoxy group, and the like.

The term "C₁-C₂ alkoxy group that may be substituted with a halogen atom" as used in the present disclosure includes a group in which one, two, or three hydrogen atoms of a "C₁-C₂ alkoxy group" are replaced with a halogen atom. In one or more embodiments, examples of the C₁-C₂ alkoxy group that may be substituted with a halogen atom include a fluoromethoxy group, a difluoromethoxy group (-OCHF₂), a trifluoromethoxy group, a chloromethoxy group, a dichloromethoxy group, a 1-fluoroethoxy group, a 1-chloroethoxy group, a 2-fluoroethoxy group, and the like.

The term "C₂-C₆ alkoxyalkyl group" as used in the present disclosure refers to an alkoxyalkyl group having 2 to 6 carbon atoms in which one hydrogen atom of an alkyl group is replaced with an alkoxy group. In one or more embodiments, examples of the C₂-C₆ alkoxyalkyl group include a C₁-C₃ alkoxy C₁-C₃ alkyl group and the like. In one or more embodiments, examples of the C₂-C₆ alkoxyalkyl group include a methoxymethyl group (-CH₂OCH₃), an ethoxymethyl group, an n-propoxymethyl group, an isopropoxymethyl group, a methoxyethyl group (-C₂H₄OCH₃), an ethoxyethyl group, a propoxyethyl group, an isopropoxyethyl group, and the like.

The term "C₂-C₆ alkoxyalkyl group that may be substituted with a halogen atom" as used in the present disclosure includes a group in which one, two, or three hydrogen atoms of a "C₂-C₆ alkoxyalkyl group" are replaced with a halogen atom. In one or more embodiments, examples of the C₂-C₆ alkoxyalkyl group that may be substituted with a halogen atom include a halogenated C₁-C₃ alkoxy C₁-C₃ alkyl group and the like. In one or more embodiments, examples of the C₂-C₆ alkoxyalkyl group that may be substituted with a halogen atom include a fluoromethoxymethyl group, a difluoromethoxymethyl group, a trifluoromethoxymethyl group, a fluoromethoxyethyl group, a difluoromethoxyethyl group, a trifluoromethoxyethyl group, a fluoroethoxyethyl group, a difluoroethoxyethyl group, a trifluoroethoxyethyl group, and the like.

The term "C₃-C₆ alkoxyalkoxyalkyl group" as used in the present disclosure refers to an alkoxyalkoxyalkyl group having 3 to 6 carbon atoms in which one hydrogen atom of an alkoxyalkyl group is replaced with an alkoxy group. In one or more embodiments, examples of the C₃-C₆ alkoxyalkoxyalkyl group include a C₁-C₂ alkoxy C₁-C₂ alkoxy C₁-C₂ alkyl group and the like. In one or more embodiments, examples of the C₃-C₆ alkoxyalkoxyalkyl group include a methoxymethoxymethyl group, a methoxyethoxymethyl group, a methoxyethoxyethyl group (-C₂H₄OC₂H₄OCH₃), and the like.

The term "C₃-C₆ alkoxyalkoxyalkyl group that may be substituted with a halogen atom" as used in the present disclosure includes a group in which one, two, or three hydrogen atoms of a "C₃-C₆ alkoxyalkoxyalkyl group" are replaced with a halogen atom. In one or more embodiments, examples of the C₃-C₆ alkoxyalkoxyalkyl group that may be substituted with a halogen atom include a halogenated C₁-C₂ alkoxy C₁-C₂ alkoxy C₁-C₂ alkyl group and the like. In one or more embodiments, examples of the C₃-C₆ alkoxyalkoxyalkyl group that may be substituted with a halogen atom include a fluoromethoxymethoxymethyl group, a difluoromethoxymethoxymethyl group, a trifluoromethoxymethoxymethyl group, a fluoromethoxyethoxymethyl group, a difluoromethoxyethoxymethyl group, a trifluoromethoxyethoxymethyl group, a fluoromethoxyethoxyethyl group, a difluoromethoxyethoxyethyl group, a trifluoromethoxyethoxyethyl group, and the like.

The term "C₁-C₂ alkylsulfonyl group" as used in the present disclosure refers to a group in which an alkyl group having 1 or 2 carbon atoms is linked to a sulfur atom of a sulfonyl group. In one or more embodiments, examples of the C₁-C₂ alkylsulfonyl group include a methylsulfonyl group (-S(O)₂CH₃), an ethylsulfonyl group, and the like.

The term "C₁-C₂ alkylsulfonyl group that may be substituted with a halogen atom" as used in the present disclosure includes a group in which one, two, or three hydrogen atoms of a "C₁-C₂ alkylsulfonyl group" are replaced with a halogen atom. In one or more embodiments, examples of the C₁-C₂ alkylsulfonyl group that may be substituted with a halogen atom include a fluoromethylsulfonyl group, a difluoromethylsulfonyl group, a trifluoromethylsulfonyl group, a fluoroethylsulfonyl group, a difluoroethylsulfonyl group, a trifluoroethylsulfonyl group, and the like.

The term "phenyl C₁-C₃ alkyl group" as used in the present disclosure refers to a group in which one hydrogen atom of a phenyl group is replaced with an alkyl group having 1 to 3 carbon atoms. In one or more embodiments, examples of the phenyl C₁-C₃ alkyl group include a benzyl group, a phenylethyl group, a phenylpropyl group, and the like.

The term "phenyl C₁-C₃ alkyl group that may be substituted with a halogen atom" as used in the present disclosure includes a group in which one, two, or three hydrogen atoms of a "C₁-C₃ alkyl group" are replaced with a halogen atom. In one or more embodiments, examples of the phenyl C₁-C₃ alkyl group that may be substituted with a halogen atom include a fluorobenzyl group, a difluorobenzyl group, a trifluorobenzyl group, a fluorophenylethyl group, a difluorophenylethyl group, a trifluorophenylethyl group, a fluorophenylpropyl group, a diphenylpropyl group, a trifluorophenylpropyl group, and the like.

The term "amino-oxo C₁-C₃ alkyl group" as used in the present disclosure refers to a group in which one hydrogen atom of a C₁-C₃ alkyl group is replaced with an amino-oxo group. In one or more embodiments, examples of the amino-oxo C₁-C₃ alkyl group include a 2-amino-2-oxoethyl group (-CH₂C(O)NH₂) and the like.

The term "C₂-C₄ alkynyl group" as used in the present disclosure refers to an alkyl group having at least one carbon-carbon triple bond (-C=C-) in the molecule. In one or more embodiments, examples of the C₂-C₄ alkynyl group include -C=CH, - C≡CCH₃, -C≡CCH₂CH₃, a propargyl group (-CH₂C≡CH), and the like.

The term "C₂-C₄ alkynyl group that may be substituted with a halogen atom" as used in the present disclosure includes a group in which at least one hydrogen atom of a "C₂-C₄ alkynyl group" is replaced with a halogen atom. In one or more embodiments, examples of the C₂-C₄ alkynyl group that may be substituted with a halogen atom include -C=CF, -C≡CCF₃, -C≡CCH₂CF₃, -CH₂C≡CF, and the like.

The term "C₂-C₆ (alkoxyalkyl group) that may be protected" as used in the present disclosure refers to a C₂-C₆ alkoxyalkyl group in which one hydrogen atom of the C₂-C₆ alkoxyalkyl group may be protected by a protecting group and a C₂-C₆ alkoxyalkyl group in which no hydrogen atom is protected by a protecting group. In one or more embodiments, examples of the protecting group include a tert-butoxycarbonyl (Boc) amino group and the like. In one or more embodiments, examples of the C₂-C₆ (alkoxyalkyl group) that may be protected include a tert-butoxycarbonyl-2-aminoethoxyethyl group (-C₂H₄OC₂H₄NHC(O)O(CH₃)₃) and the like.

The term "C₁-C₃ alkylaminocarbonyl group" as used in the present disclosure refers to a group in which one hydrogen atom of a C₁-C₃ alkyl group is replaced with an aminocarbonyl group. In one or more embodiments, examples of the C₁-C₃ alkylaminocarbonyl group include a methylaminocarbonyl group, an ethylaminocarbonyl group (-C(O)NHCH₂CH₃), and the like.

The term "6- to 12-membered aryl group" as used in the present disclosure refers to monocyclic and polycyclic aromatic hydrocarbon ring systems having 6 to 12 carbon atoms. In one or more embodiments, examples of the 6- to 12-membered aryl group include aromatic hydrocarbon groups having 6 to 12 carbon atoms. In one or more embodiments, examples of the 6- to 12-membered aryl group include a phenyl group, a 1-naphthyl group, a 2-naphthyl group, an acenaphthyl group, an azulenyl group, and the like.

The term "C₃-C₆ cycloalkyl group" as used in the present disclosure refers to cyclic alkyl groups (saturated hydrocarbon groups (rings)) having 3 to 6 carbon atoms. In one or more embodiments, examples of the C₃-C₆ cycloalkyl group include a cyclopropyl group, a cyclobutyl group, a cyclopentyl group, a cyclohexyl group, and the like.

The term "4- to 6-membered heterocyclyl group" as used in the present disclosure refers to a saturated or unsaturated monocyclic or polycyclic 4- to 6-membered cycloalkyl group having 1 to 3 heteroatoms selected from the group consisting of nitrogen, oxygen, and sulfur atoms in the ring. In one or more embodiments, the 4- to 6-membered heterocyclyl group has one oxygen atom, one sulfur atom, one nitrogen atom, one oxygen atom and one nitrogen atom, one sulfur atom and one nitrogen atom, two oxygen atoms, or two nitrogen atoms.

In one or more embodiments, examples of a heterocyclyl group include an oxiranyl group, a thiaranyl group, an aziridinyl group, an oxetanyl group, a thietanyl group, an azetidinyl group, a tetrahydrofuryl group, a tetrahydrothiophenyl group, a pyrrolidinyl group, a tetrahydropyranyl group, a pyranyl group, a tetrahydrothiopyranyl group, a thiopyranyl group, a piperidinyl group, an imidazolidinyl group, a 1,4-dioxanyl group, a 1,3-dioxolanyl group, a 1,3-dioxolyl group, a 1,4-oxathianyl group, a morpholinyl group, a thiomorpholinyl group, a 1,4-dithianyl group, a piperazinyl group, a 1,4-azathianyl group, an oxepanyl group, a thiepanyl group, an azepanyl group, an isothizolidinyl group, a 1,4-dioxepanyl group, a 1,4-oxathiepanyl group, a 1,4-oxaazepanyl group, a 1,4-dithiepanyl group, a 1,4-thieazepanyl group, a 1,4-azaphosphinanyl group, a 1,4-diazepanyl group, a 1,2-tetrahydrothiazin-2-yl group, a 1,3-tetrahydrothiazin-3-yl group, a tetrahydrothiadiazinyl group, a 1,2-tetrahydrodiazin-2-yl group, a 1,3-tetrahydrodiazin-1-yl group, a tetrahydroazepinyl group, a chromanyl group, a chromenyl group, an oxazolidinyl group, an isoxazolidinyl group, a 1,3-oxazolidin-3-yl group, an oxazinyl group, an isothiazolidinyl group, a 1,3-thiazolidin-3-yl group, a 1,2-pyrazolidin-2-yl group, a 1,3-pyrazolidin-1-yl group, a 7-oxa-1-aza-spiro[4.4]nonanyl group, a 3-azabicyclo[3.1.0]hexanyl group, an indolinyl group, a dihydroindolinyl group, an octahydro-1H-indolyl group, an octahydro-2H-pyrido[1,2-a]pyrazinyl group, a 3-azabicyclo[4.1.0]heptanyl group, a 3,4-dihydro-2H-pyranyl group, a 1,2,3,4-tetrahydropyridyl group, a 1,2,5,6-tetrahydropyridyl group, a tetrahydro-1H-benzo[d]azepinyl group, and the like.

The 4- to 6-membered heterocyclyl group may have one or two oxo groups (=O) on the ring. In one or more embodiments, examples of the 4- to 6-membered heterocyclyl group having an oxo group on the ring include a 2-oxooxazolidinyl group, a 2-oxooxazinyl group, a 3-oxomorpholinyl group, a 2-oxoazetidinyl group, a 2-oxopyrrolidinyl group, a 2-oxopiperidinyl group, a 2-oxoimidazolidinyl group, a 2-oxo-1,3-dioxolanyl group, a 2-oxo-1,3-dioxolyl group, a 1,1-dioxo-thietanyl group, a 1,1-dioxo-tetrahydrothiophenyl group, a 1,1-dioxo-isothizolidinyl group, and the like.

The term "3- to 5-membered heterocyclyl group having one or two oxygen atoms" as used in the present disclosure refers to a saturated or unsaturated monocyclic 3- to 5-membered cycloalkyl group having an oxygen atom as a heteroatom. In one or more embodiments, examples of the 3- to 5-membered heterocyclyl group having one or two oxygen atoms include an oxetanyl group, an oxazolyl group, a tetrahydrofuryl group, a furyl group, a tetrahydropyranyl group, a dioxanyl group, a dioxolanyl group, and the like.

The term "3- to 5-membered heterocyclyl-C₁-C₃ alkyl group having one or two oxygen atoms" as used in the present disclosure refers to a group in which one hydrogen atom of a 3- to 5-membered heterocyclyl group having one or two oxygen atoms as a heteroatom or heteroatoms is replaced with a C₁-C₃ alkyl group. In one or more embodiments, examples of the 3- to 5-membered heterocyclyl-C₁-C₃ alkyl group having one or two oxygen atoms include an oxetanylmethyl group, an oxadiazolylmethyl group, and the like.

The term "5- or 6-membered heteroaryl group" as used in the present disclosure refers to a 5- or 6-membered heteroaryl group having one, two, or three heteroatoms selected from the group consisting of nitrogen, oxygen, and sulfur atoms in the ring. In one or more embodiments, examples of the 5-membered heteroaryl group include a furyl group, a thienyl group, a pyrrolyl group, an oxazolyl group, an isoxazolyl group, a thiazolyl group, an isothiazolyl group, an oxadiazolyl group, and the like. In one or more embodiments, examples of the 6-membered heteroaryl group include a diazinylpyridyl group, a pyridyl group, a pyrimidinyl group, and the like.

The term "5-membered heteroaryl C₁-C₃ alkyl group" as used in the present disclosure refers to a group in which one hydrogen atom of a 5-membered heteroaryl group is replaced with a C₁-C₃ alkyl group. In one or more embodiments, examples of the 5-membered heteroaryl C₁-C₃ alkyl group include an oxadiazolylmethyl group and the like.

### Compound Represented by Formula (I)

Suitable substituents or examples of a compound represented by Formula (I) of the present disclosure will be described below.

In one or more embodiments, A¹ represents N or CH, and one or none of A², A³, and A⁴ represents N, with the other two or three representing CH. In one or more embodiments, A¹ represents N, and A², A³, and A⁴ represent CH; A¹ represents N, and any one of A², A³, and A⁴ represents N; all of A¹, A², A³, and A⁴ represent CH; or A¹ represents CH, and any one of A², A³, and A⁴ represents N.

In one or more embodiments, Ar represents a phenyl group that may be substituted with one halogen atom or a 5- or 6-membered heteroaryl group that may be substituted with one halogen atom. In one or more embodiments, examples of the 5-membered heteroaryl group include a furyl group, a thienyl group, a pyrrolyl group, an oxazolyl group, an isoxazolyl group, a thiazolyl group, an isothiazolyl group, an oxadiazolyl group, and the like. In one or more embodiments, examples of the 6-membered heteroaryl group include a pyridyl group, a pyrimidyl group, and the like. In one or more embodiments, examples of Ar include a phenyl group, a fluorophenyl group, a furyl group, a fluorofuryl group, a thienyl group, a pyrrolyl group, an oxazolyl group, an isoxazolyl group, a thiazolyl group, an isothiazolyl group, an oxadiazolyl group, a pyridyl group, a fluoropyridyl group, a pyrimidyl group, and the like.

In one or more embodiments, R¹ represents a chlorine atom or a bromine atom.

In one or more embodiments, R² represents a hydrogen atom, a fluorine atom, a chlorine atom, a bromine atom, or a C₁-C₂ alkoxy group.

In one or more embodiments, the heterocyclyl group of R³ has one oxygen atom, one sulfur atom, one nitrogen atom, one oxygen atom and one nitrogen atom, one sulfur atom and one nitrogen atom, two oxygen atoms, or two nitrogen atoms.

In one or more embodiments, examples of the heterocyclyl group of R³ include an oxetanyl group, a tetrahydrofuryl group, a furyl group, a tetrahydropyranyl group, a dioxolanyl group, a dioxanyl group, and the like.

In one or more embodiments, examples of the heterocyclyl group having an oxo group on the ring, of R³ include a 2-oxooxazolidinyl group, a 2-oxooxazinyl group, a 3-oxomorpholinyl group, a 2-oxoazetidinyl group, a 2-oxopyrrolidinyl group, a 2-oxopiperidinyl group, a 2-oxoimidazolidinyl group, a 2-oxo-1,3-dioxolanyl group, a 2-oxo-1,3-dioxolyl group, a 1,1-dioxo-thietanyl group, a 1,1-dioxo-tetrahydrothiophenyl group, a 1,1-dioxo-isothizolidinyl group, and the like.

In one or more embodiments, examples of the heterocyclyl group having no oxo group as a substituent on the ring, of R³ include an oxazolyl group, an azetidinyl group, a pyrrolidinyl group, a dioxolanyl group, a dioxanyl group, a tetrahydrofuryl group, a pyranyl group, a furyl group, an oxetanyl group, and the like.

In one or more embodiments, the substituent in R³ is selected from Group B below:
Group B:
a fluorine atom; a hydroxy group; a methyl group, an ethyl group, and an isopropyl group; a fluoroethyl group, a difluoroethyl group, and a trifluoromethyl group; a hydroxyethyl group and a 2-hydroxy-2-methylpropyl group; a methoxy group (-OCH₃) and a difluoromethoxy group (-OCHF₂); a methoxymethyl group (-CH₂OCH₃) and a methoxyethyl group (-C₂H₄OCH₃); a methoxyethoxyethyl group (-C₂H₄OC₂H₄OCH₃); a methylsulfonyl group (-S(O)₂CH₃); an acetyl group (-C(O)CH₃); a cyclopropylmethyl group; a phenyl group; a benzyl group; an oxetanyl group; an oxetanylmethyl group; a 2-amino-2-oxoethyl group (-CH₂C(O)NH₂); a propargyl group; a tert-butoxycarbonyl-2-aminoethoxyethyl group (-C₂H₄OC₂H₄NHC(O)O(CH₃)₃); an ethylaminocarbonyl group (-C(O)NHCH₂CH₃); an oxadiazolylmethyl group; an ethylene group (linked to the same carbon); a methylenedioxy group (linked to the same carbon) and an ethylenedioxy group (linked to the same carbon); and a methyleneoxymethyl group (linked to the same carbon).

In the present disclosure, suitable examples of the compound represented by Formula (I) include, in one or more embodiments, compounds represented by Formula (II), (III), (IV), or (V) or pharmaceutically acceptable salts thereof.

### Compound Represented by Formula (II)

Another aspect of the present disclosure relates to a compound represented by Formula (II) below or a pharmaceutically acceptable salt thereof: 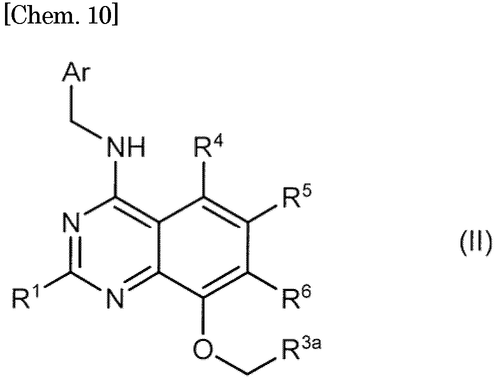 wherein, in Formula (II),
Ar represents a furyl group, a phenyl group, a thienyl group, a pyridyl group, a pyrimidinyl group, a thiazolyl group, an oxazolyl group, an isoxazolyl group, an isothiazolyl group, or an oxadiazolyl group, wherein the furyl group, the phenyl group, and the pyridyl group may be substituted with one fluorine atom,
R¹ represents a halogen atom,
R⁴, R⁵, and R⁶ each independently represent a hydrogen atom, a halogen atom, or a methoxy group, wherein at least two of R⁴, R⁵, and R⁶ represent hydrogen atoms, and
R^{3a} represents a hydrogen atom or any group selected from Group C below:
   Group C: 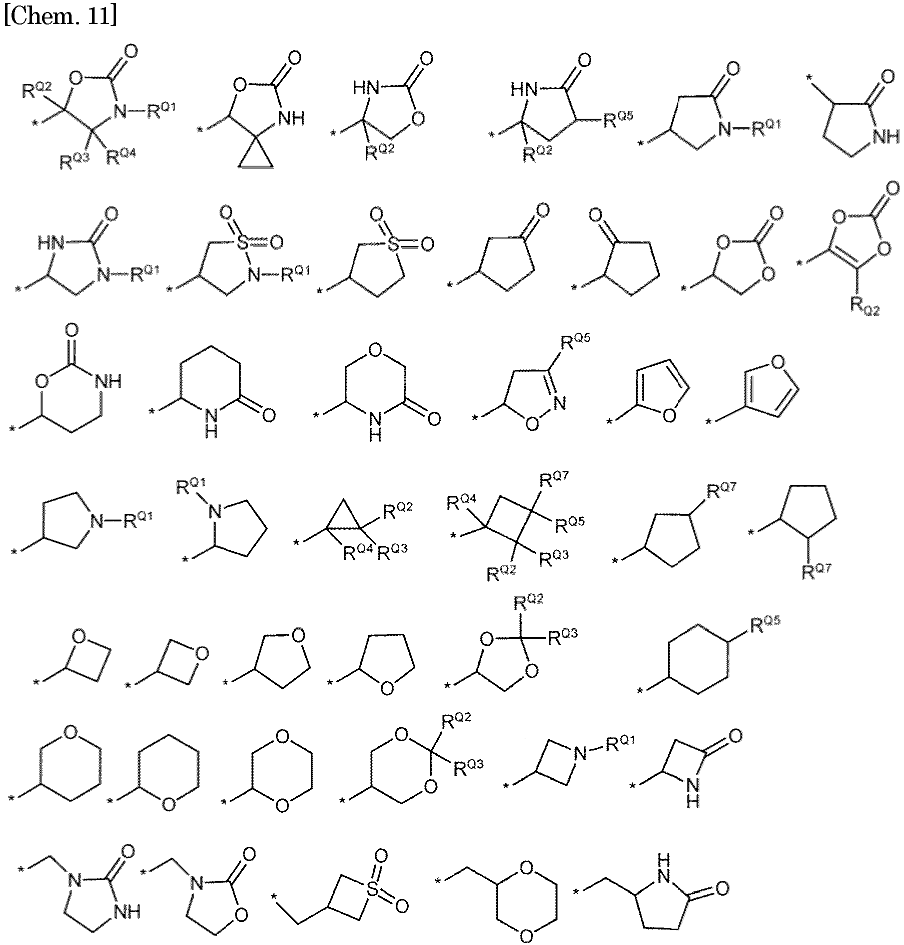
wherein R^{Q1} represents a hydrogen atom, a fluorine atom, a methyl group, an ethyl group, an isopropyl group, a fluoroethyl group, a difluoroethyl group, a hydroxyethyl group, a 2-hydroxy-2-methylpropyl group, a methoxyethyl group (-C₂H₄OCH₃), a methoxyethoxyethyl group (-C₂H₄OC₂H₄OCH₃), a methylsulfonyl group (-S(O)₂CH₃), an acetyl group (-C(O)CH₃), a cyclopropylmethyl group, a phenyl group, a benzyl group, an oxetanyl group, an oxetanylmethyl group, a 2-amino-2-oxoethyl group (-CH₂C(O)NH₂), a propargyl group (-CH₂C≡CH), a tert-butoxycarbonyl-2-aminoethoxyethyl group (-C₂H₄OC₂H₄NHC(O)O(CH₃)₃), an ethylaminocarbonyl group (-C(O)NHCH₂CH₃), or an oxadiazolylmethyl group,
R^{Q2}, R^{Q3}, and R^{Q4} each independently represent a hydrogen atom, a fluorine atom, a hydroxy group, a methyl group, or a trifluoromethyl group,
R^{Q5} and R^{Q6} each independently represent a hydrogen atom, a fluorine atom, a hydroxy group, a methyl group, a methoxy group (-OCH₃), a difluoromethoxy group (-OCHF₂), or a methoxymethyl group, and
R^{Q7} represents a hydrogen atom, a fluorine atom, a methyl group, or an ethyl group, or alternatively an ethylenedioxy group (linked to the same carbon) or a methyleneoxymethyl group (linked to the same carbon).

### Compound Represented by Formula (III)

Another aspect of the present disclosure relates to a compound represented by Formula (III) below or a pharmaceutically acceptable salt thereof: 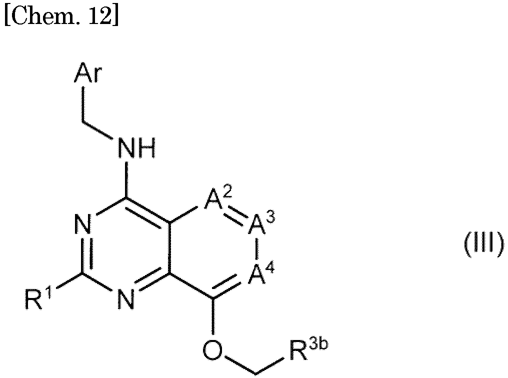 wherein, in Formula (III),
Ar represents a furyl group or a phenyl group,
R¹ represents a chlorine atom,
A², A³, and A⁴ each independently represent CH or N and any one of A², A³, and A⁴ represents N, and
R^{3b} represents a hydrogen atom or any group selected from Group D below:
   Group D:

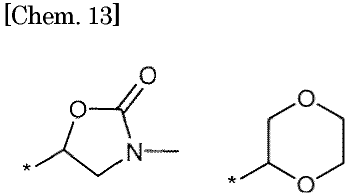

### Compound Represented by Formula (IV)

Another aspect of the present disclosure relates to a compound represented by Formula (IV) below or a pharmaceutically acceptable salt thereof: 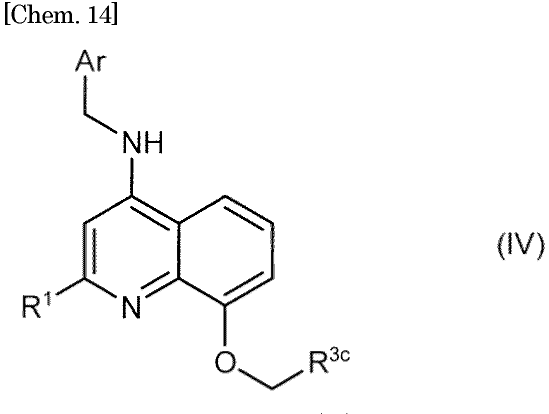 wherein, in Formula (IV),
Ar represents a furyl group or a phenyl group,
R¹ represents a chlorine atom,
R^{3c} represents a hydrogen atom or any group selected from Group E below:
   Group E: 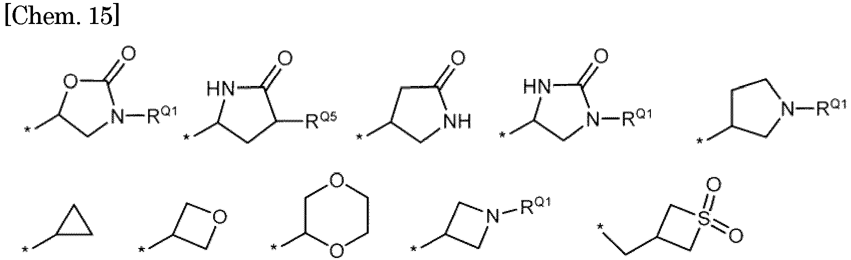
wherein R^{Q1} represents a hydrogen atom, a methyl group, an acetyl group, a methylsulfonyl group, a fluoroethyl group, or a hydroxyethyl group, and
R^{Q5} represents a hydrogen atom or a methyl group.

### Compound Represented by Formula (V)

Another aspect of the present disclosure relates to a compound represented by Formula (V) below or a pharmaceutically acceptable salt thereof: 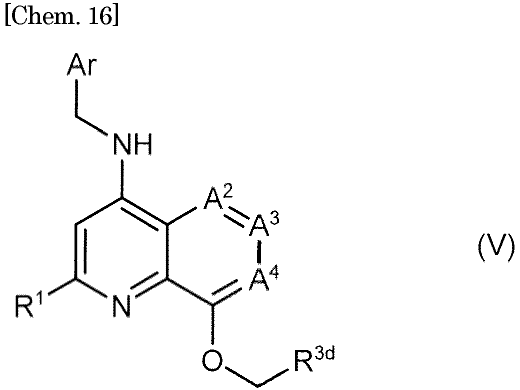 wherein, in Formula (V),
Ar represents a furyl group or a phenyl group,
A², A³, and A⁴ each independently represent CH or N and any one of A², A³, and A⁴ represents N, and
R¹ represents a chlorine atom,
R^{3d} represents a hydrogen atom or any group selected from Group F below:
   Group F: 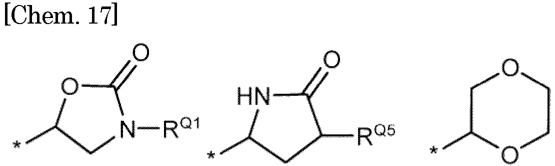
wherein R^{Q1} represents a hydrogen atom, a methyl group, a fluoroethyl group, or a hydroxyethyl group, and
R^{Q5} represents a hydrogen atom or a methyl group.

In one or more embodiments, it is preferable that, in the compound represented by Formula (V), A² and A³ represent CH, and A⁴ represents N. In one or more embodiments, it is preferable that, in the compound represented by Formula (V), R^{3d} represents a group shown below:

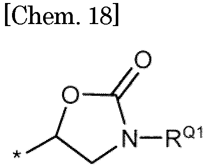

In one or more embodiments, it is preferable that the compound of the present disclosure is a compound or a pharmaceutically acceptable salt thereof, the compound being any one compound selected from the group consisting of:
2-chloro-N-(furan-2-ylmethyl)-8-methoxyquinazolin-4-amine,
2-chloro-N-(furan-2-ylmethyl)-8-methoxyquinolin-4-amine,
2-chloro-7-fluoro-N-(furan-2-ylmethyl)-8-methoxyquinazolin-4-amine,
2 -chloro-5-fluoro-N-(furan-2-ylmethyl)-8-methoxyquinazolin-4-amine,
2-bromo-N-(furan-2-ylmethyl)-8-methoxyquinazolin-4-amine,
2-chloro-N-(furan-2-ylmethyl)-8-methoxypyrido[3,4-d]pyrimidin-4-amine,
2-chloro-N-(furan-2-ylmethyl)-8-methoxypyrido[4,3-d]pyrimidin-4-amine,
2-chloro-N-(furan-2-ylmethyl)-8-methoxypyrido[3,2-d]pyrimidin-4-amine,
2 -chloro-6-fluoro-N-(furan-2-ylmethyl)-8-methoxyquinazolin-4-amine,
2-chloro-N-(furan-2-ylmethyl)-6,8-dimethoxyquinazolin-4-amine,
2-chloro-N-(furan-2-ylmethyl)-7,8-dimethoxyquinazolin-4-amine,
2-chloro-8-methoxy-N-(thiophen-2-ylmethyl)quinazolin-4-amine,
2-chloro-8-methoxy-N-(1,3-thiazol-2-ylmethyl)quinazolin-4-amine,
2-chloro-8-methoxy-N-(1,3-oxazol-2-ylmethyl)quinazolin-4-amine,
2-chloro-8-methoxy-N-(thiophen-3-ylmethyl)quinazolin-4-amine,
2-chloro-8-methoxy-N-(pyridin-4-ylmethyl)quinazolin-4-amine,
2-chloro-N-(2-fluorobenzyl)-8-methoxyquinazolin-4-amine,
2-chloro-8-(1,4-dioxan-2-ylmethoxy)-N-(furan-2-ylmethyl)quinazolin-4-amine,
5-[({2-chloro-4-[(furan-2-ylmethyl)amino]quinazolin-8-yl}oxy)methyl]-1,3-oxazolidin-2-one,
(5R)-5-[({2-chloro-4-[(furan-2-ylmethyl)amino]quinazolin-8-yl}oxy)methyl]-1,3-oxazolidin-2-one,
(5S)-5-[({2-chloro-4-[(furan-2-ylmethyl)amino]quinazolin-8-yl}oxy)methyl]-1,3-oxazolidin-2-one,
1-{3-[({2-chloro-4-[(furan-2-ylmethyl)amino]quinazolin-8-yl}oxy)methyl]pyrrolidin-1-yl}ethanone,
2-chloro-N-(furan-2-ylmethyl)-8-{[1-(methylsulfonyl)pyrrolidin-3-yl]methoxy}quinazolin-4-amine,
2-chloro-8-[(2,2-dimethyl-1,3-dioxan-5-yl)methoxy]-N-(furan-2-ylmethyl)quinazolin-4-amine,
5-[({2-chloro-4-[(furan-2-ylmethyl)amino]quinazolin-8-yl}oxy)methyl]pyrrolidin-2-one,
2-chloro-8-[(2,2-difluorocyclobutyl)methoxy]-N-(furan-2-ylmethyl)quinazolin-4-amine,
2-chloro-8-(1,4-dioxaspiro[4.4]nonan-7-ylmethoxy)-N-(furan-2-ylmethyl)quinazolin-4-amine,
3-[({2-chloro-4-[(furan-2-ylmethyl)amino]quinazolin-8-yl}oxy)methyl]cyclopentanone,
2-chloro-8-(1,4-dioxaspiro[4.4]nonan-6-ylmethoxy)-N-(furan-2-ylmethyl)quinazolin-4-amine,
2-[({2-chloro-4-[(furan-2-ylmethyl)amino]quinazolin-8-yl}oxy)methyl]cyclopentanone,
4-[({2-chloro-4-[(furan-2-ylmethyl)amino]quinazolin-8-yl}oxy)methyl]pyrrolidin-2-one,
3-[({2-chloro-4-[(furan-2-ylmethyl)amino]quinazolin-8-yl}oxy)methyl]cyclobutanol,
2-chloro-8-{[3-(difluoromethoxy)cyclobutyl]methoxy}-N-(furan-2-ylmethyl)quinazolin-4-amine,
5-[({2,6-dichloro-4-[(furan-2-ylmethyl)amino]quinazolin-8-yl}oxy)methyl]-3-methyl-1,3-oxazolidin-2-one,
(3S,5R)-5-[({2,6-dichloro-4-[(furan-2-ylmethyl)amino]quinazolin-8-yl}oxy)methyl]-3-methylpyrrolidin-2-one,
5-({[4-(benzylamino)-2,6-dichloroquinazolin-8-yl]oxy}methyl)-3-methyl-1,3-oxazolidin-2-one,
5-[({2,7-dichloro-4-[(furan-2-ylmethyl)amino]quinazolin-8-yl}oxy)methyl]-3-methyl-1,3-oxazolidin-2-one,
2-chloro-8-(1,4-dioxan-2-ylmethoxy)-N-(furan-2-ylmethyl)quinolin-4-amine,
5-[({2-chloro-4-[(furan-2-ylmethyl)amino]quinolin-8-yl}oxy)methyl]-1,3-oxazolidin-2-one,
5-({[4-(benzylamino)-2-chloroquinazolin-8-yl]oxy}methyl)-3-methyl-1,3-oxazolidin-2-one,
5-({[4-(benzylamino)-2-chloroquinazolin-8-yl]oxy}methyl)-3-(2-fluoroethyl)-1,3-oxazolidin-2-one,
5-({[4-(benzylamino)-2-chloroquinazolin-8-yl]oxy}methyl)-3-(2-hydroxyethyl)-1,3-oxazolidin-2-one,
5-({[4-(benzylamino)-2-chloroquinolin-8-yl]oxy}methyl)-3-methyl-1,3-oxazolidin-2-one,
2-chloro-N-(furan-2-ylmethyl)-8-(tetrahydrofuran-3-ylmethoxy)quinazolin-4-amine,
2-chloro-N-(furan-2-ylmethyl)-8-(tetrahydro-2H-pyran-3-ylmethoxy)quinazolin-4-amine,
2-chloro-8-[(1-fluorocyclopropyl)methoxy]-N-(furan-2-ylmethyl)quinazolin-4-amine,
1-[2-({2-chloro-4-[(furan-2-ylmethyl)amino]quinazolin-8-yl}oxy)ethyl]imidazolidin-2-one,
2-chloro-8-[(1,1-dioxidotetrahydrothiophen-3-yl)methoxy]-N-(furan-2-ylmethyl)quinazolin-4-amine,
7-[({2-chloro-4-[(furan-2-ylmethyl)amino]quinazolin-8-yl}oxy)methyl]-6-oxa-4-azaspiro[2.4]heptan-5-one,
(3S,5R)-5-[({2-chloro-4-[(furan-2-ylmethyl)amino]quinazolin-8-yl)oxy)methyl]-3-methylpyrrolidin-2-one,
2-chloro-N-(furan-2-ylmethyl)-8-[(2-methyl-1,1-dioxido-1,2-thiazolidin-4-yl)methoxy] quinazolin-4-amine,
2-chloro-8-(furan-2-ylmethoxy)-N-(furan-2-ylmethyl)quinazolin-4-amine,
3-[2-({2-chloro-4-[(furan-2-ylmethyl)amino]quinazolin-8-yl}oxy)ethyl]-1,3-oxazolidin-2-one,
4-[({2-chloro-4-[(furan-2-ylmethyl)amino]quinazolin-8-yl}oxy)methyl]-1,3-dioxolan-2-one,
2-chloro-8-[2-(1,4-dioxan-2-yl)ethoxy]-N-(furan-2-ylmethyl)quinazolin-4-amine,
2-chloro-N-(furan-2-ylmethyl)-8-[(3-methoxy-4,5-dihydro-1,2-oxazol-5-yl)methoxy] quinazolin-4-amine,
5-[({2-chloro-4-[(furan-2-ylmethyl)amino]quinazolin-8-yl}oxy)methyl]morpholin-3-one,
1-benzyl-4-[({2-chloro-4-[(furan-2-ylmethyl)amino]quinazolin-8-yl}oxy)methyl]imidazolidin-2-one,
2-chloro-N-(furan-2-ylmethyl)-8-(2-oxaspiro[3.3]heptan-6-ylmethoxy)quinazolin-4-amine,
2-chloro-8-(cyclopentylmethoxy)-N-(furan-2-ylmethyl)quinazolin-4-amine,
1-{3-[({2-chloro-4-[(furan-2-ylmethyl)amino]quinazolin-8-yl}oxy)methyl]azetidin-1-yl}ethanone,
2-chloro-8-(furan-3-ylmethoxy)-N-(furan-2-ylmethyl)quinazolin-4-amine,
2-chloro-N-(furan-2-ylmethyl)-8-{[1-(trifluoromethyl)cyclobutyl]methoxy}quinazolin-4-amine,
2-chloro-8-(cyclohexylmethoxy)-N-(furan-2-ylmethyl)quinazolin-4-amine,
2-chloro-N-(furan-2-ylmethyl)-8-(tetrahydrofuran-2-ylmethoxy)quinazolin-4-amine,
1-{2-[({2-chloro-4-[(furan-2-ylmethyl)amino]quinazolin-8-yl}oxy)methyl]pyrrolidin-1-yl}ethanone,
4-[({2-chloro-4-[(furan-2-ylmethyl)amino]quinazolin-8-yl}oxy)methyl]-1,3-oxazolidin-2-one,
3-benzyl-5-[({2-chloro-4-[(furan-2-ylmethyl)aminolquinazolin-8-yl}oxy)methyl]-1,3-oxazolidin-2-one,
5-[({2-chloro-4-[(furan-2-ylmethyl)amino]quinazolin-8-yl}oxy)methyl]-3-phenyl-1,3-oxazolidin-2-one,
(3S,5R)-5-({[4-(benzylamino)-2-chloroquinazolin-8-yl]oxy}methyl)-3-methylpyrrolidin-2-one,
(3R,5R)-5-({[4-(benzylamino)-2-chloroquinazolin-8-yl]oxy}methyl)-3-methylpyrrolidin-2-one,
4-({[4-(benzylamino)-2-chloroquinazolin-8-yl]oxy}methyl)pyrrolidin-2-one,
5-[({2-chloro-4-[(furan-2-ylmethyl)amino]quinazolin-8-yl}oxy)methyl]-4,4-dimethyl-1,3-oxazolidin-2-one,
6-[({2-chloro-4-[(furan-2-ylmethyl)amino]quinazolin-8-yl}oxy)methyl]-1,3-oxazinan-2-one,
4-[({2-chloro-4-[(furan-2-ylmethyl)aminolquinazolin-8-yl}oxy)methyl]-1-methylpyrrolidin-2-one,
4-[({2-chloro-4-[(furan-2-ylmethyl)amino]quinazolin-8-yl}oxy)methyl]-5-methyl-1,3-dioxol-2-one,
6-[({2-chloro-4-[(furan-2-ylmethyl)amino]quinazolin-8-yl}oxy)methyl]piperidin-2-one,
(5R)-5-[({2-chloro-4-[(furan-2-ylmethyl)amino] quinazolin-8-yl}oxy)methyl]pyrrolidin-2-one,
(69)-5-[({2-chloro-4-[(furan-2-ylmethyl)amino] quinazolin-8-yl}oxy)methyl]pyrrolidin-2-one,
4-[({2-chloro-4-[(furan-2-ylmethy)amino]quinazolin-8-yl}oxy)methyl]azetidin-2-one,
5-[({2-chloro-4-[(furan-2-ylmethyl)amino]quinazolin-8-yl}oxy)methyl]-5-methylpyrrolidin-2-one,
4-[({2-chloro-4-[(furan-2-ylmethyl)amino]quinazolin-8-yl}oxy)methyl]-4-methyl-1,3-oxazolidin-2-one,
5-[({2-chloro-4-[(furan-2-ylmethyl)amino]quinazolin-8-yl}oxy)methyl]-3-methyl-1,3-oxazolidin-2-one,
5-({[4-(benzylamino)-2-chloroquinazolin-8-yl]oxy}methyl)-1,3-oxazolidin-2-one,
5-[({2-chloro-4-[(furan-2-ylmethyl)amino]quinolin-8-yl}oxy)methyl]pyrrolidin-2-one,
5-[({2-chloro-4-[(furan-2-ylmethyl)amino]quinolin-8-yl}oxy)methyl]-3-(2-hydroxyethyl)-1,3-oxazolidin-2-one,
5-[({2-chloro-4-[(furan-2-ylmethyl)amino]quinolin-8-yl}oxy)methyl]-3-(2-fluoroethyl)- 1,3-oxazolidin-2-one,
1-{3-[({2-chloro-4-[(furan-2-ylmethyl)amino]quinolin-8-yl]oxy)methyl]azetidin-1-yl}ethanone,
4-[({2-chloro-4-[(furan-2-ylmethyl)amino]quinolin-8-yl}oxy)methyl]pyrrolidin-2-one,
2-chloro-N-(furan-2-ylmethyl)-8-{[1-(methylsulfonyl)azetidin-3-yl]methoxy}quinolin-4-amine,
(3S,5R)-5-[({2-chloro-4-[(furan-2-ylmethyl)amino]quinolin-8-yl}oxy)methyl]-3-methylpyrrolidin-2-one,
2-chloro-N-(furan-2-ylmethyl)-8-{[1-(methylsulfonyl)pyrrolidin-3-yl]methoxy}quinolin-4-amine,
1-{3-[({2-chloro-4-[(furan-2-ylmethyl)amino]quinolin-8-yl}oxy)methyl]pyrrolidin-1-yl}ethanone,
(5R)-5-[({2-chloro-4-[(furan-2-ylmethyl)amino] quinolin-8-yl}oxy)methyl]-1,3-oxazolidin-2-one,
(5S)-5-[({2-chloro-4-[(furan-2-ylmethyl)amino]quinolin-8-yl}oxy)methyl]-1,3-oxazolidin-2-one,
(5R)-5-[({2-chloro-4-[(furan-2-ylmethyl)amino]quinolin-8-yl}oxy)methyl]pyrrolidin-2-one,
(69)-5-[({2-chloro-4-[(furan-2-ylmethyl) amino] quinolin-8-yl}oxy)methyl]pyrrolidin-2-one,
5-({[4-(benzylamino)-2-chloroquinolin-8-yl]oxy}methyl)-1,3-oxazolidin-2-one,
5-({{4-(benzylamino)-2-chloroquinolin-8-yl]oxy}methyl)-3-(2-hydroxyethyl)-1,3-oxazolidin-2-one,
5-({[4-(benzylamino)-2-chloroquinolin-8-yl]oxy}methyl)-3-(2-fluoroethyl)-1,3-oxazolidin-2-one,
(3S,5R)-5-({[4-(benzylamino)-2-chloroquinolin-8-yl]oxy}methyl)-3-methylpyrrolidin-2-one,
4-({[4-(benzylamino)-2-chloroquinolin-8-yl]oxy}methyl)pyrrolidin-2-one,
2-chloro-8-(1,4-dioxan-2-ylmethoxy)-N-(furan-2-ylmethyl)- 1,5-naphthyridin-4-amine,
5-[({6-chloro-8-[(furan-2-ylmethyl)amino]-1,5-naphthyridin-4-yl}oxy)methyl]-1,3-oxazolidin-2-one,
5-[({6-chloro-8-[(furan-2-ylmethyl)amino]-1,5-naphthyridin-4-yl}oxy)methyl]-3-methyl-1,3-oxazolidin-2-one,
(3S,5R)-5-[({6-chloro-8-[(furan-2-ylmethyl)amino]-1,5-naphthyridin-4-yl}oxy)methyl]-3-methylpyrrolidin-2-one,
5-[({6-chloro-8-[(furan-2-ylmethyl)amino]-1,5-naphthyridin-4-yl}oxy)methyl]-3-(2-hydroxyethyl)-1,3-oxazolidin-2-one,
5-[({6-chloro-8-[(furan-2-ylmethyl)amino]-1,5-naphthyridin-4-yl}oxy)methyl]-3-(2-fluoroethyl)-1,3-oxazolidin-2-one,
5-({[8-(benzylamino)-6-chloro-1,5-naphthyridin-4-yl]oxy}methyl)-1,3-oxazolidin-2-one,
5-({[8-(benzylamino)-6-chloro-1,5-naphthyridin-4-yl]oxy}methyl)-3-methyl-1,3-oxazolidin-2-one,
(3S,5R)-5-({[8-(benzylamino)-6-chloro-1,5-naphthyridin-4-yl]oxy}methyl)-3-methylpyrrolidin-2-one,
5-({[8-(benzylamino)-6-chloro-1,5-naphthyridin-4-yl]oxy}methyl)-3-(2-hydroxyethyl)-1,3-oxazolidin-2-one,
5-({[8-(benzylamino)-6-chloro-1,5-naphthyridin-4-yl]oxy}methyl)-3-(2-fluoroethyl)- 1,3-oxazolidin-2-one,
5-({[4-(benzylamino)-2-chloropyrido[3,2-d]pyrimidin-8-yl]oxy}methyl)-3-methyl-1,3-oxazolidin-2-one,
(5R)-5-[({2-chloro-4-[(furan-2-ylmethyl)amino]quinazolin-8-yl}oxy)methyl]-3-(methoxymethy)pyrrolidin-2-one,
3-[({2-chloro-4-[(furan-2-ylmethyl)amino]quinazolin-8-yl}oxy)methyl]pyrrolidin-2-one,
4-[({2-chloro-4-[(furan-2-ylmethyl)amino]quinazolin-8-yl}oxy)methyl]-1-methylimidazolidin-2-one,
5-[2-({2-chloro-4-[(furan-2-ylmethyl)amino]quinazolin-8-yl}oxy)ethyl]pyrrolidin-2-one,
2-chloro-8-[(2,2-dimethyl-1,3-dioxolan-4-yl)methoxy]-N-(furan-2-ylmethyl)quinazolin-4-amine,
trans-4-[({2-chloro-4-[(furan-2-ylmethyl)amino]quinazolin-8-yl}oxy)methyl]cyclohexanol,
4-[({2-chloro-4-[(furan-2-ylmethyl)amino]quinolin-8-yl}oxy)methyl]-1-methylimidazolidin-2-one,
2-chloro-8-(cyclopropylmethoxy)-N-(furan-2-ylmethyl)quinazolin-4-amine,
2-chloro-N-(furan-2-ylmethyl)-8-(oxetan-3-ylmethoxy)quinazolin-4-amine,
2-chloro-8-(1,3-dioxan-5-ylmethoxy)-N-(furan-2-ylmethyl)quinazolin-4-amine,
2-chloro-8-(cyclobutylmethoxy)-N-(furan-2-ylmethyl)quinazolin-4-amine,
2-chloro-8-[(2S)-1,4-dioxan-2-ylmethoxy]-N-(furan-2-ylmethyl)quinazolin-4-amine,
2-chloro-8-[(2S)-1,4-dioxan-2-ylmethoxy]-7-fluoro-N-(furan-2-ylmethyl)quinazolin-4-amine,
2-chloro-N-(furan-2-ylmethyl)-8-(oxetan-3-ylmethoxy)quinolin-4-amine,
2-chloro-8-[(2,2-difluorocyclopropyl)methoxy]-N-(furan-2-ylmethyl)quinazolin-4-amine,
2-chloro-8-[(3,3-difluorocyclobutyl)methoxy]-N-(furan-2-ylmethyl)quinazolin-4-amine,
2-chloro-N-(furan-2-ylmethyl)-8-[(1-methylcyclopropyl)methoxy]quinazolin-4-amine,
2-chloro-N-(furan-2-ylmethyl)-8-(tetrahydro-2H-pyran-2-ylmethoxy)quinazolin-4-amine,
2-chloro-N-(furan-2-ylmethyl)-8-(oxetan-2-ylmethoxy)quinazolin-4-amine,
2-chloro-8-[2-(1,1-dioxidothiethan-3-yl)ethoxy]-N-(furan-2-ylmethyl)quinazolin-4-amine,
2-chloro-8-[(2R)-1,4-dioxan-2-ylmethoxy]-N-(furan-2-ylmethyl) quinazolin-4-amine,
5-[({2-chloro-4-[(furan-2-ylmethyl)amino]quinazolin-8-yl}oxy)methyl]-5-methyl-1,3-oxazolidin-2-one,
2-chloro-8-[(2R)-1,4-dioxan-2-ylmethoxy]-7-fluoro-N-(furan-2-ylmethyl)quinazolin-4-amine,
2-chloro-8-(cyclopropylmethoxy)-7-fluoro-N-(furan-2-ylmethyl) quinazolin-4-amine,
2-chloro-8-(cyclobutylmethoxy)-7-fluoro-N-(furan-2-ylmethylquinazolin-4-amine,
2-chloro-7-fluoro-N-(furan-2-ylmethyl)-8-(oxetan-3-ylmethoxy)quinazolin-4-amine,
2-chloro-8-(cyclopropylmethoxy)-N-(furan-2-ylmethyl)quinolin-4-amine,
2-chloro-8-[2-(1,1-dioxidothiethan-3-yl)ethoxy]-N-(furan-2-ylmethyl)quinolin-4-amine,
2-chloro-8-[(2,2-difluorocyclopropyl)methoxyl-N-(thiophen-2-ylmethyl)quinazolin-4-amine,
2-chloro-8-(1,4-dioxan-2-ylmethoxy)-N-(thiophen-2-ylmethyl)quinazolin-4-amine,
2-chloro-8-[(2,2-difluorocyclopropyl)methoxy]-N-(thiophen-3-ylmethyl)quinazolin-4-amine,
2-chloro-8-(1,4-dioxan-2-ylmethoxy)-N-(thiophen-3-ylmethyl)quinazolin-4-amine,
5-{[(2-chloro-4-{[(4-fluorofuran-2-yl)methyl]amino}quinazolin-8-yl)oxy]methyl}-1,3-oxazolidin-2-one,
2-chloro-8-[(2,2-difluorocyclopropyl)methoxy]-N-(1,2-thiazol-5-ylmethyl)quinazolin-4-amine,
2-chloro-8-[(2,2-difluorocyclopropyl)methoxy]-N-(1,3-oxazol-4-ylmethyl)quinazolin-4-amine,
2-chloro-8-(1,4-dioxan-2-ylmethoxy)-N-(1,2-oxazol-3-ylmethyl)quinazolin-4-amine,
5-[({2-chloro-4-[(1,3-oxazol-2-ylmethyl)amino]quinazolin-8-yl}oxy)methyl]-1,3-oxazolidin-2-one,
2-chloro-8-(1,4-dioxan-2-ylmethoxy)-N-(1,3-oxazol-2-ylmethyl)quinazolin-4-amine,
5-[({2-chloro-4-[(thiophen-2-ylmethyl)amino]quinazolin-8-yl}oxy)methyl]-1,3-oxazolidin-2-one,
5-[({2-chloro-4-[(thiophen-3-ylmethyl)amino]quinazolin-8-yl}oxy)methyl]-1,3-oxazolidin-2-one,
2-chloro-8-[(2,2-difluorocyclopropyl)methoxy]-N-(1,3-thiazol-2-ylmethyl)quinazolin-4-amine,
2-chloro-8-(1,4-dioxan-2-ylmethoxy)-N-(1,3-thiazol-2-ylmethyl)quinazolin-4-amine,
5-[({2-chloro-4-[(1,3-thiazol-2-ylmethyl)amino]quinazolin-8-yl}oxy)methyl]-1,3-oxazolidin-2-one,
2-chloro-8-[(2,2-difluorocyclopropyl)methoxy]-N-(1,3-oxazol-2-ylmethyl)quinazolin-4-amine,
2-chloro-8-[(2,2-difluorocyclopropyl)methoxy]-N-(pyridin-4-ylmethyl)quinazolin-4-amine,
5-[({2-chloro-4-[(pyridin-4-ylmethyl)amino]quinazolin-8-yl}oxy)methyl]-1,3-oxazolidin-2-one,
5-[({2-chloro-4-[(pyrimidin-4-ylmethyl)amino]quinazolin-8-yl}oxy)methyl]-1,3-oxazolidin-2-one,
5-[({2-chloro-4-[(pyridin-2-ylmethyl)amino]quinazolin-8-yl}oxy)methyl]-1,3-oxazolidin-2-one,
5-{[(2-chloro-4-{[(3-fluoropyridin-4-yl)methyl]amino}quinazolin-8-yl)oxy]methyl}-1,3-oxazolidin-2-one,
5-[({2-chloro-4-[(2-fluorobenzyl)amino]quinazolin-8-yl}oxy)methyl]-1,3-oxazolidin-2-one,
5-[({2-chloro-4-[(3-fluorobenzyl)amino]quinazolin-8-yl}oxy)methyl]-1,3-oxazolidin-2-one,
2-chloro-8-(1,4-dioxan-2-ylmethoxy)-N-(1,2,4-oxadiazol-3-ylmethyl) quinazolin-4-amine,
5-[({2-chloro-4-[(1,2-thiazol-5-ylmethyl)amino]quinazolin-8-yl}oxy)methyl]-1,3-oxazolidin-2-one,
5-[({2-chloro-4-[(4-fluorobenzyl)amino]quinazolin-8-yl}oxy)methyl]-1,3-oxazolidin-2-one,
5-[({2-chloro-4-[(furan-2-ylmethyl)amino]-1,7-naphthyridin-8-yl}oxy)methyl]-3-methyl-1,3-oxazolidin-2-one,
(5S)-5-[({2-chloro-4-[(furan-2-ylmethyl)amino]-1,7-naphthyridin-8-yl}oxy)methyl]-3-methyl-1,3-oxazolidin-2-one,
(5R)-5-[({2-chloro-4-[(furan-2-ylmethyl)amino]- 1, 7-naphthyridin-8-yl}oxy)methyl]-3-methyl- 1,3-oxazolidin-2-one,
5-({[4-(benzylamino)-2-chloro-1,7-naphthyridin-8-yl]oxy}methyl)-1,3-oxazolidin-2-one,
5-[({2-chloro-4-[(furan-2-ylmethyl)amino]-1,7-naphthyridin-8-yl}oxy)methyl]-3-(2-fluoroethyl)-1,3-oxazolidin-2-one,
5-[({2-chloro-4-[(furan-2-ylmethyl)amino]-1,7-naphthyridin-8-yl}oxy)methyl]-3-(2-hydroxyethyl)-1,3-oxazolidin-2-one,
5-({[4-(benzylamino)-2-chloro-1,7-naphthyridin-8-yl]oxy}methyl)-3-(2-fluoroethyl)- 1,3-oxazolidin-2-one,
5-({[4-(benzylamino)-2-chloro-1,7-naphthyridin-8-yl]oxy}methyl)-3-methyl-1,3-oxazolidin-2-one,
5-({[4-(benzylamino)-2-chloro-1,7-naphthyridin-8-yl]oxy}methyl)-3-(2-hydroxyethyl)-1,3-oxazolidin-2-one,
2-chloro-8-(1,4-dioxan-2-ylmethoxy)-N-(furan-2-ylmethyl)pyrido[3,2-d]pyrimidin-4-amine,
2-chloro-8-(1,4-dioxan-2-ylmethoxy)-N-(furan-2-ylmethyl)pyrido[3,4-d]pyrimidin-4-amine,
5-[({2-chloro-4-[(furan-2-ylmethyl)amino]pyrido[3,4-d]pyrimidin-8-yl}oxy)methyl]-3-methyl-1,3-oxazolidin-2-one,
5-({[4-(benzylamino)-2-chloropyrido[3,4-d]pyrimidin-8-yl]oxy}methyl)-3-methyl-1,3-oxazolidin-2-one,
5-[({2-chloro-4-[(furan-2-ylmethyl)amino]pyrido[4,3-d]pyrimidin-8-yl}oxy)methyl]-3-methyl-1,3-oxazolidin-2-one,
5-({[4-(benzylamino)-2-chloropyrido[4,3-d]pyrimidin-8-yl]oxy}methyl)-3-methyl-1,3-oxazolidin-2-one,
5-({[4-(benzylamino)-2-chloro-1,6-naphthyridin-8-yl]oxy}methyl)-1,3-oxazolidin-2-one,
5-({[4-(benzylamino)-2-chloro-1,6-naphthyridin-8-yl]oxy}methyl)-3-methyl-1,3-oxazolidin-2-one,
5-[({2-chloro-4-[(furan-2-ylmethyl)amino]quinazolin-8-yl}oxy)methyl]-3-ethyl-1,3-oxazolidin-2-one,
5-[({2-chloro-4-[(furan-2-ylmethyl)amino]quinazolin-8-yl}oxy)methyl]-3-(propan-2-yl)-1,3-oxazolidin-2-one,
5-[({2-chloro-4-[(furan-2-ylmethyl)amino]quinazolin-8-yl}oxy)methyl]-3-(2-fluoroethyl)- 1,3-oxazolidin-2-one,
5-[({2-chloro-4-[(furan-2-ylmethyl)amino]quinazolin-8-yl}oxy)methyl]-3-(cyclopropylmethyl)-1,3-oxazolidin-2-one,
5-[({2-chloro-4-[(furan-2-ylmethyl)amino]quinazolin-8-yl}oxy)methyl]-3-(2,2-difluoroethyl)-1,3-oxazolidin-2-one,
5-[({2-chloro-4-[(furan-2-ylmethyl)amino]quinazolin-8-yl}oxy)methyl]-3-(2-hydroxyethyl)-1,3-oxazolidin-2-one,
5-[({2-chloro-4-[(furan-2-ylmethyl)amino]quinazolin-8-yl}oxy)methyl]-3-(oxetan-3-yl)-1,3-oxazolidin-2-one,
5-[({2-chloro-4-[(furan-2-ylmethyl)amino]quinazolin-8-yl}oxy)methyl]-3-(2-hydroxy-2-methylpropyl)-1,3-oxazolidin-2-one,
5-[({2-chloro-4-[(furan-2-ylmethyl)amino]quinazolin-8-yl}oxy)methyl]-N-ethyl-2-oxo-1,3-oxazolidine-3-carboxamide,
4-[({2-chloro-4-[(furan-2-ylmethyl)amino]quinazolin-8-yl}oxy)methyl]-1-(2-hydroxyethyl)pyrrolidin-2-one,
4-[({2-chloro-4-[(furan-2-ylmethyl)amino]quinazolin-8-yl}oxy)methyl]-1-(2-fluoroethyl)pyrrolidin-2-one,
5-[({2-chloro-4-[(furan-2-ylmethyl)amino]quinazolin-8-yl}oxy)methyl]-3-(prop-2-yn-1-yl)-1,3-oxazolidin-2-one,
tert-butyl[2-(2-{5-[({2-chloro-4-[(furan-2-ylmethyl)amino]quinazolin-8-yl}oxy)methyl]-2-oxo-1,3-oxazolidin-3-yl}ethoxy)ethyl]carbamate,
5-[({2-chloro-4-[(furan-2-ylmethyl)amino]quinazolin-8-yl}oxy)methyl]-3-[2-(2-methoxyethoxy)ethyl]-1,3-oxazolidin-2-one,
5-[({2-chloro-4-[(furan-2-ylmethyl)amino]quinazolin-8-yl}oxy)methyl]-3-(oxetan-3-ylmethyl)-1,3-oxazolidin-2-one,
5-[({2-chloro-4-[(furan-2-ylmethyl)amino]quinazolin-8-yl}oxy)methyl]-3-(1,2,4-oxadiazol-5-ylmethyl)- 1,3-oxazolidin-2-one,
2-{5-[({2-chloro-4-[(furan-2-ylmethyl)amino]quinazolin-8-yl}oxy)methyl]-2-oxo-1,3-oxazolidin-3-yl}acetamide, and
5-[({2-chloro-4-[(furan-2-ylmethyl)amino]quinazolin-8-yl}oxy)methyl]-3-(2-methoxyethyl)-1,3-oxazolidin-2-one.

The term "pharmaceutically acceptable salt" as used in the present disclosure refers to a salt that can be used as a medicine, and may be a basic salt, an acid salt, or the like in one or more embodiments.

In one or more embodiments, examples of the "basic salt" include alkali metal salts, such as sodium salts, potassium salts, and lithium salts; alkaline earth metal salts, such as magnesium salts and calcium salts; organic base salts, such as N-methylmorpholine salts, triethylamine salts, tributylamine salts, diisopropylethylamine salts, dicyclohexylamine salts, N-methylpiperidine salts, pyridine salts, 4-pyrrolidinopyridine salts, and picoline salts; amino acid salts, such as glycine salts, lysine salts, arginine salts, ornithine salts, glutamates, and aspartates; and the like. Alkali metal salts are preferable.

In one or more embodiments, examples of the "acid salt" include hydrogen halide salts, such as hydrofluorides, hydrochlorides, hydrobromides, and hydroiodides; inorganic acid salts, such as nitrates, perchlorates, sulfates, and phosphates; lower alkane sulfonates, such as methanesulfonates, trifluoromethanesulfonates, and ethanesulfonates; arylsulfonates, such as benzenesulfonates and p-toluenesulfonates; organic acid salts, such as acetates, malates, fumarates, succinates, citrates, ascorbates, tartrates, oxalates, and maleates; amino acid salts, such as glycine salts, lysine salts, arginine salts, ornithine salts, glutamates, and aspartates; and the like. Hydrogen halide salts (in particular, hydrochlorides) are preferable.

A pharmaceutically acceptable salt of the compound of the present disclosure may encompass a hydrate. Also, the term "salt of a compound" as used in the present disclosure may encompass a solvate that may be formed as a result of the compound absorbing another certain type of solvent.

The compound of the present disclosure, pharmaceutically acceptable salt thereof, or solvate thereof may exist as various isomers, such as geometric isomers (cis- and trans-isomers, etc.), optical isomers (enantiomers) (tautomers, rotamers, D-isomers and L-isomers, etc.), and diastereomers, depending on the kind and the combination of substituents. Unless otherwise specified, the compound of the present disclosure should be construed as encompassing all such isomers, stereoisomers, and mixtures of these isomers and stereoisomers in any ratio. Mixtures of these isomers can be separated using a known dividing means.

The compound of the present disclosure also encompasses a labeled compound, that is, a compound in which one or two or more atoms of the compound are replaced with an isotope (e.g., ²H, ³H, ¹³C, ¹⁴C, ³⁵S, etc.).

A compound that is converted into a compound represented by Formula (I), (II), (III), (IV), or (V), which is an active ingredient of the pharmaceutical composition of the invention, through a reaction with an enzyme, gastric acid, or the like under *in* vivo physiological conditions, or in other words, a compound that is enzymatically oxidized, reduced, or hydrolyzed, for example, to a compound represented by Formula (I), (II), (III), (IV), or (V), or a compound that is hydrolyzed, for example, by gastric acid or the like to a compound represented by Formula (I), (II), (III), (IV), or (V), is encompassed by the present disclosure as a "medicinally acceptable prodrug compound". In one or more embodiments, a prodrug is a compound having a group that can be converted into an amino group, a hydroxy group, a carboxyl group, or the like of a compound by hydrolysis or under physiological conditions, and groups that form such prodrugs are described in Prog. Med, Vol. 5, pp. 2157-2161, 1985, for example.

When the compound represented by Formula (I), (II), (III), (IV), or (V) contains an amino group, compounds in which the amino group is acylated, alkylated, or phosphorylated (e.g., compounds in which the amino group is eicosanoylated, alanylated, pentylaminocarbonylated, (5-methyl-2-oxo-1,3-dioxolen-4-yl)methoxycarbonylated, tetrahydrofurylated, pyrrolidylmethylated, pivaloyloxymethylated, or tert-butylated) can be presented as examples of the prodrug. When the compound represented by Formula (I), (II), (III), (IV), or (V) contains a hydroxy group, compounds in which the hydroxy group is acylated, alkylated, phosphorylated, or a borylated (e.g., compounds in which the hydroxy group is acetylated, palmitoylated, propanoylated, pivaloylated, succinylated, fumarylated, alanylated, or dimethylaminomethylcarbonylated) can be presented as examples of the prodrug. When the compound represented by Formula (I), (II), (III), (IV), or (V) contains a carboxy group, compounds in which the carboxy group is esterified or amidated (e.g., compounds in which the carboxy group is ethyl esterified, phenyl esterified, carboxymethyl esterified, dimethylaminomethyl esterified, pivaloyloxymethyl esterified, ethoxycarbonyloxyethyl esterified, or methylamidated) can be presented as examples of the prodrug.

### Production Methods

Hereinafter, representative methods for producing a compound represented by Formula (I), (II), (III), (IV), or (V) will be described. In one or more embodiments, the compound of the present disclosure can be produced using any one of the following methods A to N. Note that the production methods described below are merely examples, and the present disclosure should not be construed as being limited to these methods.

Compounds produced using the methods A to N below may be isolated and purified as non-solvates, salts thereof, or various solvates such as hydrates. The salts can be produced using ordinary methods. In one or more embodiments, examples of the salts include hydrochlorides, sulfates, and the like, as well as organic amine salts, sodium salts, potassium salts, and the like.

There are no particular limitations on solvents used in the reactions in steps of the methods A to N described below, as long as they partially dissolve the starting materials without inhibiting the reactions. In one or more embodiments, examples of the solvents include aliphatic hydrocarbons, aromatic hydrocarbons, halogenated hydrocarbons, ethers, ketones, esters, nitriles, carboxylic acids, alcohols, amides, sulfoxides, water, mixtures thereof, and the like.

In one or more embodiments, examples of the aliphatic hydrocarbons include n-hexane, n-pentane, petroleum ether, cyclohexane, and the like.

In one or more embodiments, examples of the aromatic hydrocarbons include benzene, toluene, xylene, and the like.

In one or more embodiments, examples of the halogenated hydrocarbons include dichloromethane (DCM, methylene chloride), chloroform, carbon tetrachloride, dichloroethane, chlorobenzene, dichlorobenzene, and the like.

In one or more embodiments, examples of the ethers include diethyl ether, diisopropyl ether, tetrahydrofuran (THF), dioxane, dimethoxyethane, diethylene glycol dimethyl ether, and the like.

In one or more embodiments, examples of the ketones include acetone, methyl ethyl ketone, methyl isobutyl ketone, cyclohexanone, and the like.

In one or more embodiments, examples of the esters include ethyl acetate, propyl acetate, butyl acetate, and the like.

In one or more embodiments, examples of the nitriles include acetonitrile, propionitrile, butyronitrile, isobutyronitrile, and the like.

In one or more embodiments, examples of the carboxylic acids include acetic acid, propionic acid, and the like.

In one or more embodiments, examples of the alcohols include methanol, ethanol, 1-propanol, 2-propanol, 1-butanol, 2-butanol, 2-methyl-1-propanol, 2-methyl-2-propanol, and the like.

In one or more embodiments, examples of the amides include formamide, N,N-dimethylformamide (DMF), N,N-dimethylacetamide, N-methylpyrrolidone (NMP), hexamethylphosphorotriamide, and the like.

In one or more embodiments, examples of the sulfoxides include dimethyl sulfoxide (DMSO), tetrahydrothiophene-1,1-dioxide, and the like.

There are no particular limitations on bases used in the reactions in steps of the methods A to N described below, as long as they do not inhibit the reactions. In one or more embodiments, examples of the bases include alkali metal carbonates, alkali metal bicarbonates, alkali metal hydroxides, alkaline earth metal hydroxides, alkali metal hydrides, alkali metal amides, alkali metal alkoxides, organic amines, and the like.

In one or more embodiments, examples of the alkali metal carbonates include lithium carbonate, sodium carbonate, potassium carbonate, cesium carbonate, and the like.

In one or more embodiments, examples of the alkali metal bicarbonates include lithium bicarbonate, sodium bicarbonate, potassium bicarbonate, and the like.

In one or more embodiments, examples of the alkali metal hydroxides include lithium hydroxide, sodium hydroxide, potassium hydroxide, and the like.

In one or more embodiments, examples of the alkaline earth metal hydroxides include calcium hydroxide, barium hydroxide, and the like.

In one or more embodiments, examples of the alkali metal hydrides include lithium hydride, sodium hydride, potassium hydride, and the like.

In one or more embodiments, examples of the alkali metal amides include lithium amide, sodium amide, potassium amide, and the like.

In one or more embodiments, examples of the organic amines include triethylamine (TEA), tributylamine, N,N-diisopropylethylamine (DIPEA), 1-methylpiperidine, 4-methylmorpholine, 4-ethylmorpholine, pyridine, picoline, 4-dimethylaminopyridine, 4-pyrrolidinopyridine, 2,6-di-tert-butyl-4-methylpyridine, quinoline, N,N-dimethylaniline, N,N-diethylaniline, 1,5-diazabicyclo[4.3.0]-5-nonene (DBN), 1,4-diazabicyclo[2.2.2]octane (DABCO), 1,8-diazabicyclo[5.4.0]-7-undecene (DBU), imidazole, and the like.

### Method A

The method A is an example of a method for producing a compound represented by Formula (II). The method A includes steps A-1, or A-2 and A-3, to A-7 or A-8 below. In the following intermediates and compound, Ar, A⁴, and R² are as previously described regarding Formula (II), R^{3a'} represents -(CH₂)ₙ-R^{3a}, and R^{3a} and n are as previously described regarding Formula (II). 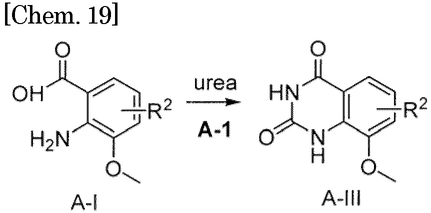
(1) Step A-1 is a step of obtaining an intermediate A-III from an intermediate A-I. The reaction conditions for step A-1 are as follows.
   Reaction Conditions
   Solvent: Acetic acid
   Reaction temperature: Room temperature to 150°C
   Reaction time: 30 minutes to 24 hours 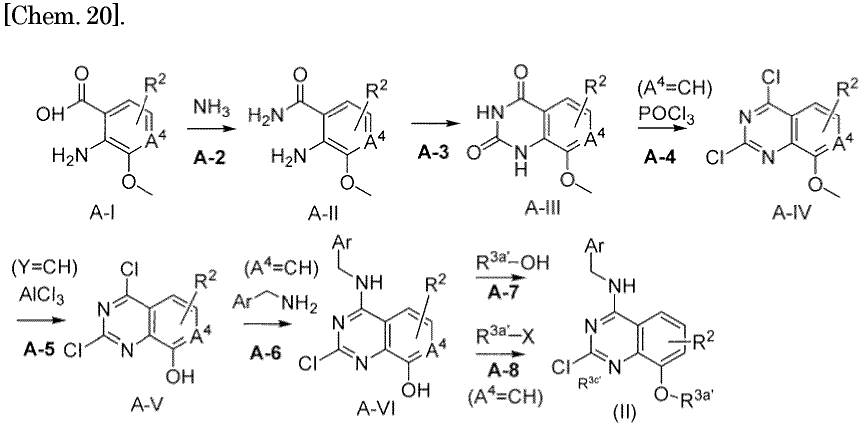
(2) Step A-2 is a step of obtaining an intermediate A-II from the intermediate A-I. The reaction conditions for step A-2 are as follows.
   Reaction Conditions
   Solvent: Tetrahydrofuran
   Condensing agent: 1,1'-Carbonyldiimidazole
   Reaction temperature: 0°C to 80°C
   Reaction time: 30 minutes to 24 hours
(3) Step A-3 is a step of obtaining an intermediate A-III from the intermediate A-II. Step A-3 can be performed, for example, under the following reaction conditions a or b.
   Reaction Conditions a
      Solvent: Dimethylformamide
      Base: Potassium carbonate
      Reactant: 1,1'-Carbonyldiimidazole
      Reaction temperature: Room temperature to 120°C
      Reaction time: 30 minutes to 24 hours
   Reaction Conditions b
      Solvent: Tetrahydrofuran
      Base: Lithium bis(trimethylsilyl)amide
      Reactants: Triphosgene
      Reaction temperature: 0°C to 60°C
      Reaction time: 30 minutes to 24 hours
(4) Step A-4 is a step of obtaining an intermediate A-IV from the intermediate A-III. Step A-4 can be performed, for example, under the following reaction conditions (when A⁴ = CH).
   Reaction Conditions
   Base: N,N-Diethylaniline
   Reaction temperature: Room temperature to 120°C
   Reaction time: 30 minutes to 24 hours
(5) Step A-5 is a step of obtaining an intermediate A-V from the intermediate A-IV Step A-5 can be performed, for example, under the following reaction conditions (when A⁴ = CH).
   Reaction Conditions
   Solvent: Dichloromethane
   Reaction temperature: 0°C to 40°C
   Reaction time: 30 minutes to 24 hours
(6) Step A-6 is a step of obtaining an intermediate A-VI from the intermediate A-V Step A-6 can be performed, for example, under the following reaction conditions (when A⁴ = CH).
   Reaction Conditions
   Solvent: Acetonitrile
   Base: Triethylamine
   Reaction temperature: Room temperature to 80°C
   Reaction time: 30 minutes to 24 hours
(7) Step A-7 is a step of obtaining a compound represented by Formula (II) from the intermediate A-VI. The reaction conditions for step A-7 are as follows (when A⁴ = CH in the intermediate A-VI).
   Reaction Conditions
   Solvent: Toluene
   Mitsunobu reagent: Cyanomethylenetributylphosphorane
   Reaction temperature: Room temperature to 110°C
   Reaction time: 30 minutes to 24 hours
(8) Step A-8 is a step of obtaining a compound represented by Formula (II) from the intermediate A-VI. Step A-8 can be performed, for example, under the following reaction conditions (when A⁴ = CH in the intermediate A-VI).
   Reaction Conditions
   Solvent: Dimethylformamide
   Base: Potassium carbonate
   Reaction temperature: Room temperature to 100°C
   Reaction time: 30 minutes to 24 hours

### Method B

The method B is an example of a method for producing an intermediate B-IV of a compound represented by Formula (II). In the following intermediates, Ar and R² are as previously described regarding Formula (II). 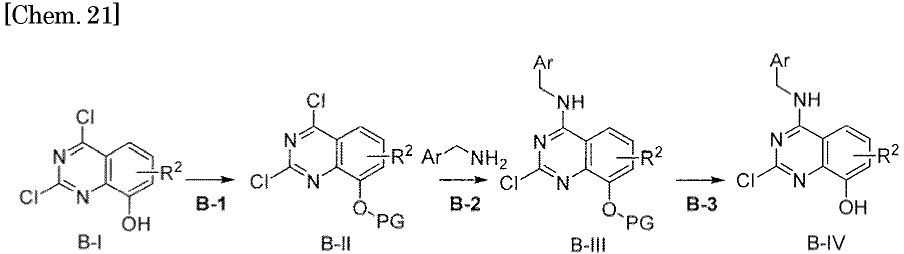
(1) Step B-1 is a step of obtaining an intermediate B-II from an intermediate B-I. The reaction conditions for step B-1 are as follows.
   Reaction Conditions
   Protecting group (PG): tert-Butyldimethylsilyl group
   Protecting reagent: tert-Butyldimethylsilyl trifluoromethanesulfonate
   Solvent: Dichloromethane
   Base: 2,6-Lutidine
   Reaction temperature: 0°C to room temperature
   Reaction time: 30 minutes to 24 hours
(2) Step B-2 is a step of obtaining an intermediate B-III from the intermediate B-II. The reaction conditions for step B-2 are as follows.
   Reaction Conditions
   Solvent: Acetonitrile
   Base: Triethylamine
   Reaction temperature: Room temperature to 80°C
(3) Step B-3 is a step of obtaining an intermediate B-IV by deprotecting the intermediate B-III. The reaction conditions for step B-3 are as follows.
   Reaction Conditions
   Reagent: Tetrabutylammonium fluoride
   Solvent: Tetrahydrofuran
   Reaction temperature: 0°C to 80°C
   Reaction time: 30 minutes to 24 hours

### Method C

The method C is an example of a method for producing a compound represented by Formula (II). In the following intermediates and compound, R², R^{3a}, and n are as previously described regarding Formula (II), and R^{3a'} represents -(CH₂)ₙ-R^{3a}. 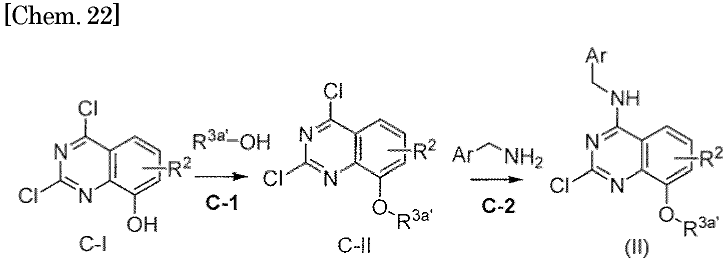
(1) Step C-1 is a step of obtaining an intermediate C-II from an intermediate C-I. The reaction conditions for step C-1 are as follows.
   Reaction Conditions
   Solvent: Toluene
   Reaction reagents: Bis(2-methoxymethyl)azodicarboxylate and triphenylphosphine
   Reaction temperature: Room temperature to 110°C
   Reaction time: 30 minutes to 24 hours
(2) Step C-2 is a step of obtaining a compound represented by Formula (II) from the intermediate C-II. The reaction conditions for step C-2 are as follows.
   Reaction Conditions
   Solvent: Acetonitrile
   Base: Triethylamine
   Reaction temperature: Room temperature to 80°C
   Reaction time: 30 minutes to 24 hours

### Method D

The method D is an example of a method for producing a compound represented by Formula (IV). In the following intermediates and compound, Ar and R² are as previously described regarding Formula (IV), R^{3c'} represents -(CH₂)ₙ-R^{3c}, and R^{3c} and n are as previously described regarding Formula (IV). X represents a halogen atom, such as a chlorine atom. 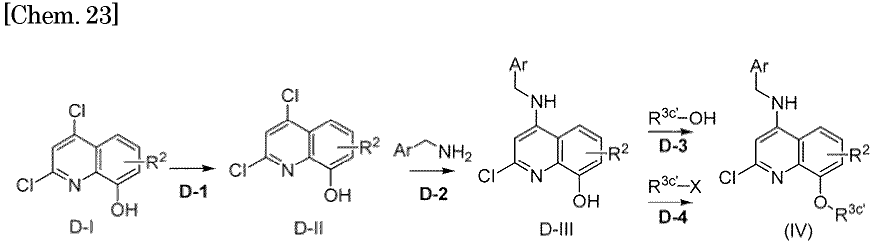
(1) Step D-1 is a step of obtaining an intermediate D-II from an intermediate D-I. The reaction conditions for step D-1 are as follows.
   Reaction Conditions
   Reagent: Boron tribromide
   Solvent: Dichloromethane
   Reaction temperature: 0°C to 40°C
   Reaction time: 30 minutes to 24 hours
(2) Step D-2 is a step of obtaining an intermediate D-III from the intermediate D-II. The reaction conditions for step D-2 are as follows.
   Reaction Conditions
   Solvent: N-Methylpyrrolidone
   Base: N,N-Diisopropylethylamine
   Reaction temperature: Room temperature to 120°C
   Reaction time: 30 minutes to 24 hours
(3) Step D-3 is a step of obtaining a compound represented by Formula (IV) from the intermediate D-III. The reaction conditions for step D-3 are as follows.
   Reaction Conditions
   Solvent: Toluene
   Mitsunobu reagent: Cyanomethylenetributylphosphorane
   Reaction temperature: Room temperature to 110°C
   Reaction time: 30 minutes to 24 hours
(4) Step D-4 is a step of obtaining a compound represented by Formula (IV) from the intermediate D-III. The reaction conditions for step D-4 are as follows.
   Reaction Conditions
   Solvent: Dimethylformamide
   Base: Potassium carbonate
   Reaction temperature: Room temperature to 100°C
   Reaction time: 30 minutes to 24 hours

### Method E

The method E is an example of a method for producing an intermediate E-III of a compound represented by Formula (III). In the following intermediates, Ar is as previously described regarding Formula (III). 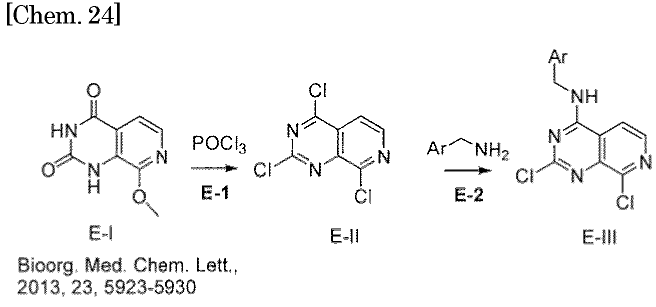
(1) Step E-1 is a step of obtaining an intermediate E-II from an intermediate E-I. The reaction conditions for step E-1 are as follows.
   Reaction Conditions
   Base: N,N-Diethylaniline
   Reaction temperature: Room temperature to 120°C
(2) Step E-2 is a step of obtaining an intermediate E-III from the intermediate E-II. The reaction conditions for step E-2 are as follows.
   Reaction Conditions
   Solvent: Acetonitrile
   Base: Triethylamine
   Reaction temperature: Room temperature to 80°C
   Reaction time: 30 minutes to 24 hours

### Method F

The method F is an example of a method for producing a compound represented by Formula (V). In the following intermediates and compound, Ar is as previously described regarding Formula (V), R^{3d'} represents -(CH₂)ₙ-R^{3d}, and R^{3d} and n are as previously described regarding Formula (V). 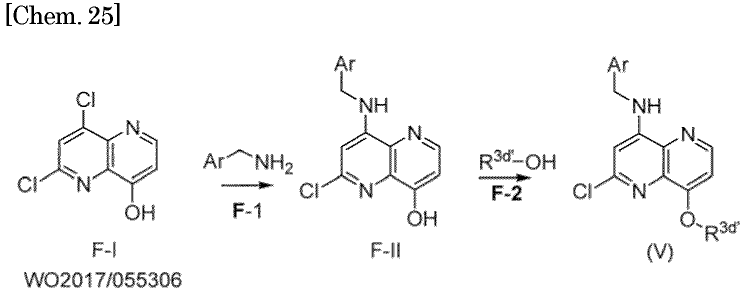
(1) Step F-1 is a step of obtaining an intermediate F-II from an intermediate F-I. The reaction conditions for step F-1 are as follows.
   Reaction Conditions
   Solvent: N-Methylpyrrolidone
   Base: N,N-Diisopropylethylamine
   Reaction temperature: Room temperature to 120°C
   Reaction time: 30 minutes to 24 hours
(2) Step F-2 is a step of obtaining a compound represented by Formula (V) from the intermediate F-II. The reaction conditions for step F-2 are as follows.
   Reaction Conditions
   Solvent: Toluene
   Mitsunobu reagent: Cyanomethylenetributylphosphorane
   Reaction temperature: Room temperature to 110°C
   Reaction time: 30 minutes to 24 hours

### Method G

The method G is an example of a method for producing an intermediate of a compound represented by Formula (V). In the following intermediates, Ar is as previously described regarding Formula (V). 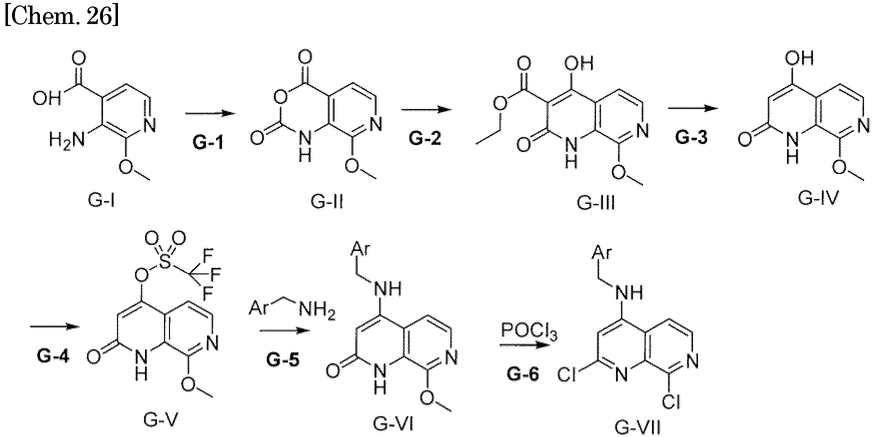
(1) Step G-1 is a step of obtaining an intermediate G-II from an intermediate G-I. The reaction conditions for step G-1 are as follows.
   Reaction Conditions
   Solvent: Tetrahydrofuran
   Reaction reagent: Triphosgene
   Reaction temperature: 0°C to 80°C
   Reaction time: 30 minutes to 24 hours
(2) Step G-2 is a step of obtaining an intermediate G-III from the intermediate G-II. The reaction conditions for step G-2 are as follows.
   Reaction Conditions
   Solvent: Dimethylformamide
   Reaction reagent: Diethyl malonate
   Base: Sodium hydride
   Reaction temperature: Room temperature to 80°C
   Reaction time: 30 minutes to 24 hours
(3) Step G-3 is a step of obtaining an intermediate G-IV from the intermediate G-III. The reaction conditions for step G-3 are as follows.
   Reaction Conditions
   Solvent: Water
   Base: Potassium hydroxide
   Reaction temperature: Room temperature to 120°C
   Reaction time: 30 minutes to 24 hours
(4) Step G-4 is a step of obtaining an intermediate G-V from the intermediate G-IV The reaction conditions for step G-4 are as follows.
   Reaction Conditions
   Solvent: Tetrahydrofuran
   Reaction reagent: N-Phenylbis(trifluoromethanesulfonimide)
   Base: Triethylamine
   Reaction temperature: Room temperature to 80°C
   Reaction time: 30 minutes to 24 hours
(5) Step G-5 is a step of obtaining an intermediate G-VI from the intermediate G-V The reaction conditions for step G-5 are as follows.
   Reaction Conditions
   Solvent: Acetonitrile
   Reaction temperature: Room temperature to 80°C
   Reaction time: 30 minutes to 24 hours
(6) Step G-6 is a step of obtaining an intermediate G-VII from the intermediate G-VI. The reaction conditions for step G-6 are as follows.
   Reaction Conditions
   Base: N,N-Diethylaniline
   Reaction temperature: Room temperature to 110°C
   Reaction time: 30 minutes to 24 hours

### Method H

The method H is an example of a method for producing an intermediate of a compound represented by Formula (III). In the following intermediates, Ar is as previously described regarding Formula (III). 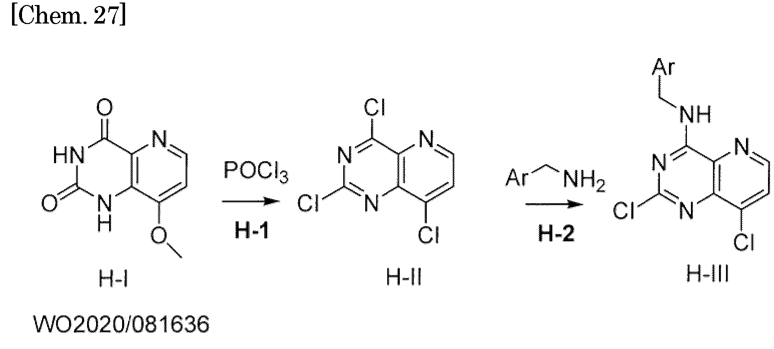
(1) Step H-1 is a step of obtaining an intermediate H-II from an intermediate H-I. The reaction conditions for step H-1 are as follows.
   Reaction Conditions
   Base: N,N-Diisopropylethylamine
   Reaction temperature: Room temperature to 120°C
   Reaction time: 30 minutes to 24 hours
(2) Step H-2 is a step of obtaining an intermediate H-III from the intermediate H-II. The reaction conditions for step H-2 are as follows.
   Reaction Conditions
   Solvent: Acetonitrile
   Base: Triethylamine
   Reaction temperature: Room temperature to 80°C
   Reaction time: 30 minutes to 24 hours

### Method I

The method I is an example of a method for producing a compound represented by Formula (I). In the following intermediate and compound, Ar, A¹, A², and A⁴ are as previously described regarding Formula (I), R^{3'} represents -(CH₂)ₙ-R³, and R³ and n are as previously described regarding Formula (I).

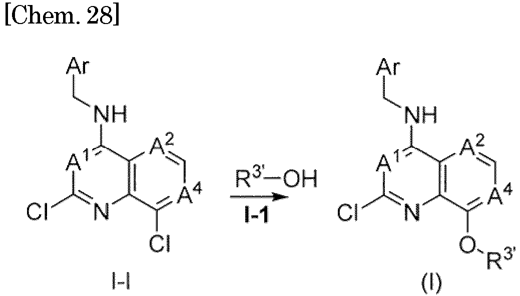

Step I-1 is a step of obtaining a compound represented by Formula (I) from an intermediate I-I. The reaction conditions for step I-1 are as follows.

### Reaction Conditions

Solvent: Tetrahydrofuran
Base: Sodium hydride
Reaction temperature: Room temperature to 80°C
Reaction time: 30 minutes to 24 hours

### Method J

The method J is an example of a method for producing an intermediate of a compound represented by Formula (III). In the following intermediates, Ar is as previously described regarding Formula (III). 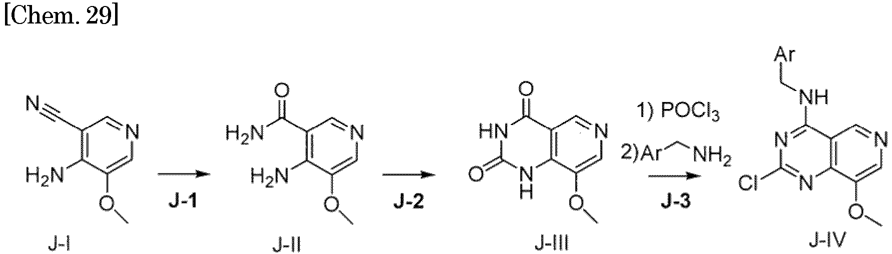
(1) Step J-1 is a step of obtaining an intermediate J-II from an intermediate J-I. The reaction conditions for step J-1 are as follows.
   Reaction Conditions
   Solvent: Dimethyl sulfoxide
   Reaction reagent: Hydrogen peroxide solution
   Base: Potassium carbonate
   Reaction temperature: 0°C to 80°C
   Reaction time: 30 minutes to 24 hours
(2) Step J-2 is a step of obtaining an intermediate J-III from the intermediate J-II. The reaction conditions for step J-2 are as follows.
   Reaction Conditions
   Solvent: 1,4-Dioxane
   Reaction reagent: Triphosgene
   Base: Lithium bis(trimethylsilyl)amide
   Reaction temperature: 0°C to room temperature
   Reaction time: 30 minutes to 24 hours
(3) Steps J-3_1) and J-3_2) are steps of obtaining an intermediate J-IV from the intermediate J-III. Step J-3_1) can be performed, for example, under the following reaction conditions 1). Step J-3_2) can be performed, for example, under the following reaction conditions 2).
   Reaction Conditions 1)
      Base: N,N-Diisopropylethylamine
      Reaction temperature: Room temperature to 120°C
      Reaction time: 30 minutes to 24 hours
   Reaction Conditions 2)
      Solvent: Tetrahydrofuran
      Base: N,N-Diisopropylethylamine
      Reaction temperature: Room temperature to 80°C
      Reaction time: 30 minutes to 24 hours

### Method K

The method K is an example of a method for producing a compound represented by Formula (III). In the following intermediates and compound, Ar is as previously described regarding Formula (III). R^{3b'} represents -(CH₂)ₙ-R^{3b}, and R^{3b} and n are as previously described regarding Formula (III). 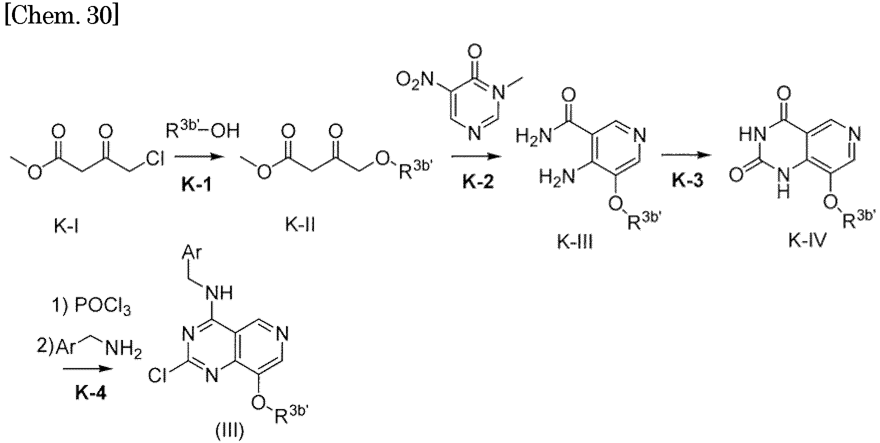
(1) Step K-1 is a step of obtaining an intermediate K-II from an intermediate K-I. The reaction conditions for step K-1 are as follows.
   Reaction Conditions
   Solvent: Tetrahydrofuran
   Base: Sodium hydride
   Reaction temperature: 0°C to 80°C
   Reaction time: 30 minutes to 24 hours
(2) Step K-2 is a step of obtaining an intermediate K-III from the intermediate K-II. The reaction conditions for step K-2 are as follows.
   Reaction Conditions
   Solvent: Methanol
   Reaction reagent: Ammonium acetate
   Reaction temperature: Room temperature to 80°C
   Reaction time: 30 minutes to 24 hours
(3) Step K-3 is a step of obtaining an intermediate K-IV from the intermediate K-III. The reaction conditions for step K-3 are as follows.
   Reaction Conditions
   Solvent: Tetrahydrofuran
   Reaction reagent: Trichloroacetyl isocyanate
   Reaction temperature: Room temperature to 80°C
   Reaction time: 30 minutes to 24 hours
(4) Steps K-4_1) and K-4_2) are steps of obtaining a compound represented by Formula (III) from the intermediate K-IV. Step K-4_1) can be performed, for example, under the following reaction conditions 1). Step K-4_2) can be performed, for example, under the following reaction conditions 2).
   Reaction Conditions 1)
      Base: N,N-Diisopropylethylamine
      Reaction temperature: Room temperature to 120°C
      Reaction time: 30 minutes to 24 hours
   Reaction Conditions 2)
      Solvent: Tetrahydrofuran
      Base: N,N-Diisopropylethylamine
      Reaction temperature: Room temperature to 80°C
      Reaction time: 30 minutes to 24 hours

### Method L

The method L is an example of a method for producing an intermediate of a compound represented by Formula (III). In the following intermediates, Ar is as previously described regarding Formula (III).

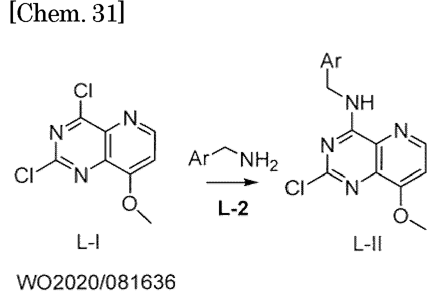

Step L-1 is a step of obtaining an intermediate L-II from an intermediate L-I. The reaction conditions for step L-1 are as follows.
Reaction Conditions
Solvent: Tetrahydrofuran
Base: N,N-Diisopropylethylamine
Reaction temperature: Room temperature to 80°C
Reaction time: 30 minutes to 24 hours

### Method M

The method M is an example of a method for producing a compound represented by Formula (V). In the following intermediates and compound, Ar is as previously described regarding Formula (V), R^{3d'} represents -(CH₂)ₙ-R^{3d}, and R^{3d} and n are as previously described regarding Formula (V). 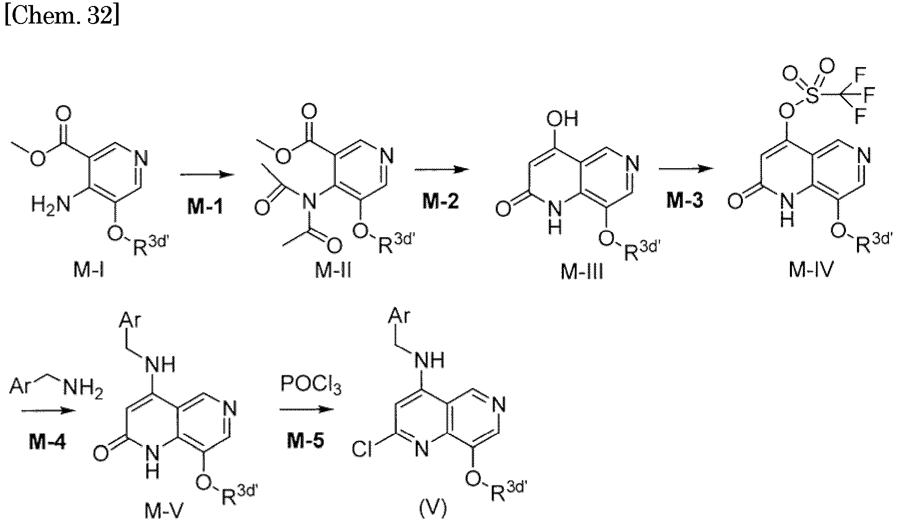
(1) Step M-1 is a step of obtaining an intermediate M-II from an intermediate M-I. The reaction conditions for step M-1 are as follows.
   Reaction Conditions
   Solvent: Acetic anhydride
   Base: Pyridine
   Reaction temperature: Room temperature to 100°C
   Reaction time: 30 minutes to 24 hours
(2) Step M-2 is a step of obtaining an intermediate M-III from the intermediate M-II. The reaction conditions for step M-2 are as follows.
   Reaction Conditions
   Solvent: Tetrahydrofuran
   Base: Potassium bis(trimethylsilyl)amide
   Reaction temperature: -78°C to 60°C
   Reaction time: 30 minutes to 48 hours
(3) Step M-3 is a step of obtaining an intermediate M-IV from the intermediate M-III. The reaction conditions for step M-3 are as follows.
   Reaction Conditions
   Solvent: Tetrahydrofuran
   Reaction reagent: N-Phenylbis(trifluoromethanesulfonimide)
   Base: Triethylamine
   Reaction temperature: Room temperature to 80°C
   Reaction time: 30 minutes to 24 hours
(4) Step M-4 is a step of obtaining an intermediate M-V from the intermediate M-IV The reaction conditions for step M-4 are as follows.
   Reaction Conditions
   Solvent: 1,4-Dioxane
   Reaction temperature: Room temperature to 100°C
   Reaction time: 30 minutes to 24 hours
(5) Step M-5 is a step of obtaining a compound represented by Formula (V) from the intermediate M-V The reaction conditions for step M-5 are as follows.
   Reaction Conditions
   Solvent: 1,4-Dioxane
   Base: N,N-Diethylaniline
   Reaction temperature: Room temperature to 110°C
   Reaction time: 30 minutes to 24 hours

### Method N

The method N is an example of a method for producing a compound represented by Formula (I'). The compound represented by Formula (I') is an example of a compound represented by Formula (I). In the following intermediate and compound, Ar, A¹, A², A³, and A⁴ are as previously described regarding Formula (I), R^{Q1} is as previously described regarding Formula (II), and W represents an oxygen atom, -NH-, or -CH-.

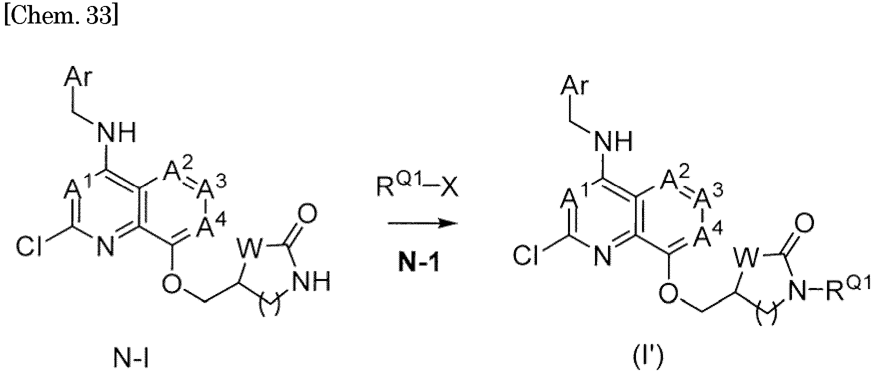

Step N- 1 is a step of obtaining a compound represented by Formula (I') from an intermediate N-I. The reaction conditions for step N-1 are as follows.
Reaction Conditions
Solvents: Tetrahydrofuran and dimethylformamide
Base: tert-Butoxypotassium
Reaction temperature: 0°C to 80°C
Reaction time: 30 minutes to 24 hours

In one or more embodiments, the compound of the present disclosure or pharmaceutically acceptable salt thereof has an effect capable of suppressing aberrant splicing that contributes to the onset or progression of a genetic disease. In one or more embodiments, the compound of the present disclosure or pharmaceutically acceptable salt thereof can be used to treat a genetic disease caused by aberrant splicing.

The term "genetic disease caused by aberrant splicing" as used in the present disclosure encompasses, in one or more embodiments, genetic diseases caused by aberrant splicing due to exon skipping type mutations, splice-site selection type mutations, intron retention type mutations, pseudoexon type mutations, and the like. An exon skipping type mutation refers to a splicing mutation in which a normally recognized exon is no longer recognized (skipping occurs) due to a mutation in that exon or a neighboring intron sequence. A splice-site selection mutation refers to a splicing mutation in which a plurality of 5' splice sites or 3' splice sites are generated due to a mutation in a splicing regulatory sequence in an exon region or an intron region. An intron retention type mutation refers to a splicing mutation in which recognition of an intron region is incomplete due to a mutation in an exon or intron region around a 5' splice site or a 3' splice site, inducing intron retention. A pseudoexon type mutation refers to a splicing mutation in which a sequence originally from an intron region is recognized as an exon due to a mutation.

In one or more embodiments, examples of the genetic disease caused by aberrant splicing include Fabry disease and the like. Fabry disease is a disease in which glycolipids such as globotriaosylceramide (Gb3) accumulate in cell lysosomes due to a deficiency of the GLA enzyme, one of the lysosomal hydrolases, resulting in various symptoms involving various organs, including the heart and other circulatory organs, the kidneys, and the like.

A single nucleotide substitution (IVS4+9 19G>A mutation) in the fourth intron of the α-galactosidase (GLA) gene has been reported as a cause of the cardiac variant Fabry disease. The IVS4+9 19G>A mutation causes alternative splicing in the transcription of the GLA gene, resulting in a deficiency of the GLA enzyme in lysosomes. It has been reported that many cardiovascular abnormalities and the like have been confirmed in adult Taiwanese people with this IVS4+9 19G>A mutation. In addition, it has been reported that in Taiwanese newborn screening studies for GLA enzyme activity, about 70% to 80% of newborns with low GLA enzyme activity in plasma and Fabry disease-causing mutations had the IVS4+919G>A mutation (Lin H-Y, et al., Circ Cardiovasc Genet 2(5): 450-456, 2009; Hwu W-L et al., Hum Mutat 30(10): 1397-1405, 2009).

In one or more embodiments, aberrant splicing that is a cause of Fabry disease is due to a mutation in the gene to be spliced. In one or more additional embodiments, the aberrant splicing is splicing (aberrant splicing due to a pseudoexon type mutation) occurring in the pre-mRNA of a mutant GLA gene having the IVS4+9 19G>A mutation.

### Pharmaceutical Composition

Another aspect of the present disclosure relates to a pharmaceutical composition containing, as an active ingredient, the compound of the present disclosure or pharmaceutically acceptable salt thereof. In one or more embodiments, the pharmaceutical composition of the present disclosure can be used to treat a genetic disease caused by aberrant splicing.

Yet another aspect of the present disclosure relates to a pharmaceutical composition for treating a genetic disease caused by aberrant splicing, the pharmaceutical composition containing, as an active ingredient, the compound of the present disclosure or pharmaceutically acceptable salt thereof.

Yet another aspect of the present disclosure relates to a pharmaceutical composition for treating a genetic disease caused by aberrant splicing, the pharmaceutical composition containing, as an active ingredient, a compound represented by Formula (I), (II), (III), (IV), or (V) above or a pharmaceutically acceptable salt thereof.

In one or more embodiments, the pharmaceutical composition of the present disclosure contains the compound of the present disclosure or a pharmaceutically acceptable salt thereof and may additionally contain a pharmaceutically acceptable carrier, preservative, diluent, or excipient, or other medicinally acceptable ingredients.

In one or more embodiments, the content ratio of the compound of the present disclosure or pharmaceutically acceptable salt thereof, which is an active ingredient, in the pharmaceutical composition of the present disclosure can be appropriately determined based on the dosage form, administration method, carrier, and the like. In one or more embodiments, the pharmaceutical composition of the present disclosure can be produced according to a routine method by adding the compound of the present disclosure in a proportion of 0.01% to 100% (w/w), or 0.1% to 95% (w/w), relative to the total formulation amount.

In one or more embodiments, the pharmaceutical composition of the present disclosure can be formed in a dosage form that is suitable for the form of administration, by applying a well-known drug preparation technique. In one or more embodiments, examples of the form of administration include oral administration, parenteral administration, and the like. In one or more embodiments, examples of formulations for oral administration include those having dosage forms such as tablets, capsules, granules, powders, pills, troches, syrups, liquid medicines (e.g., solutions and suspensions), and the like. In one or more embodiments, examples of formulations for parenteral administration include injectables, aerosols, and the like. In one or more embodiments, these formulations can be produced using a well-known method with additives such as an excipient, a lubricant, a binder, a disintegrant, a stabilizing agent, a corrigent, and a diluent.

In one or more embodiments, examples of the excipient include starches such as starch, potato starch, and corn starch, lactose, crystalline cellulose, calcium hydrogen phosphate, and the like. In one or more embodiments, examples of the lubricant include ethyl cellulose, hydroxypropyl cellulose, hydroxypropyl methylcellulose, shellac, talc, carnauba wax, paraffin, and the like. In one or more embodiments, examples of the binder include polyvinylpyrrolidone, macrogol, and compounds that are similar to those given as examples of the excipient. In one or more embodiments, examples of the disintegrant include compounds that are similar to those given as examples of the excipient and chemically-modified starches and celluloses, such as croscarmellose sodium, sodium carboxymethyl starch, and crosslinked polyvinylpyrrolidone. In one or more embodiments, examples of the stabilizing agent include para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. In one or more embodiments, examples of the corrigent include sweeteners, acidulants, flavors, and the like that are commonly used.

In one or more embodiments, to produce a liquid medicine for oral administration, ethanol, phenol, chlorocresol, purified water, distilled water, or the like can be used as a solvent. Also, a surfactant, a preservative, an isotonic agent, a pH regulator, an emulsifying agent, or the like can be used as needed. In one or more embodiments, the liquid medicine for oral administration may additionally contain a solubilizing agent, a wetting agent, a suspending agent, a sweetening agent, a flavoring agent, an aromatic agent, or a preservative.

An injectable for parenteral administration may be a sterile aqueous or non-aqueous liquid medicine, a suspension, or an emulsion. In one or more embodiments, distilled water or physiological saline may be used as an aqueous solvent for an injectable. In one or more embodiments, vegetable oil, an alcohol, or polysorbate 80 (pharmacopeia name) may be used as a non-aqueous solvent for an injectable. Examples of the vegetable oil include propylene glycol, polyethylene glycol, olive oil, and the like. Examples of the alcohol include ethanol and the like. In one or more embodiments, the injectable may additionally contain an isotonic agent, a preservative, a wetting agent, an emulsifying agent, a dispersing agent, a stabilizing agent, or a solubilizing agent. In one or more embodiments, these formulations may be sterilized by filtration through a bacteria-retaining filter, the addition of a bactericide, or irradiation with radiation. Alternatively, compositions obtained by dissolving or suspending a sterile solid composition in sterile water or a solvent for injection before use can also be used as these formulations.

The method of use of the pharmaceutical composition according to the present disclosure may vary depending on the symptoms, age, administration method, and the like. As a method of use, in one or more embodiments, the pharmaceutical composition can be intermittently or continuously administered orally, percutaneously, submucosally, subcutaneously, intramuscularly, intravascularly, intracerebrally, or intra-abdominally such that the concentration of the above compound, which is an active component, in the body is any value within a range from 100 pM to 1 mM. In a non-limiting embodiment, when the pharmaceutical composition is orally administered to a subject (an adult in the case of a human), a daily dose of 0.01 mg/kg body weight to 2000 mg/kg body weight, 0.1 mg/kg body weight to 500 mg/kg body weight, or 0.1 mg/kg body weight to 100 mg/kg body weight, in terms of the compound represented by Formula (I), may be administered to the subject at once or over multiple times depending on the symptoms. In a non-limiting embodiment, when the pharmaceutical composition is intravenously administered to a subject (an adult in the case of a human), a daily dose of 0.001 mg/kg body weight to 50 mg/kg body weight, or 0.01 mg/kg body weight to 50 mg/kg body weight may be administered to the subject at once or over multiple times depending on the symptoms.

### Treatment Method

Another aspect of the present disclosure relates to a method for treating a genetic disease caused by aberrant splicing, the method including administering the compound of the present disclosure or pharmaceutically acceptable salt thereof to a subject.

Another aspect of the present disclosure relates to use of the compound of the present disclosure or pharmaceutically acceptable salt thereof in the production of a pharmaceutical composition for use in treatment of a genetic disease caused by aberrant splicing.

The present disclosure may relate to one or more non-limiting embodiments below.
[1] A compound represented by Formula (I) below or a pharmaceutically acceptable salt thereof: 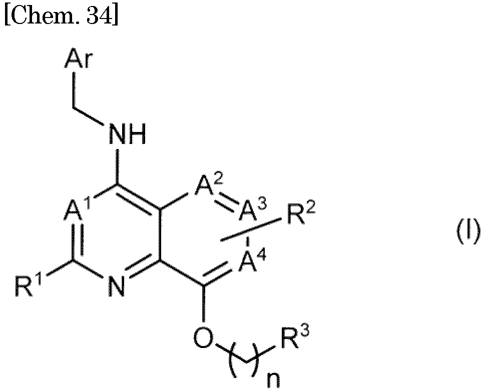 wherein, in Formula (I),
   A¹, A², A³, and A⁴ each independently represent CH or N,
   Ar represents a 6- to 12-membered aryl group, or a 5- or 6-membered heteroaryl group having 1 to 3 heteroatoms selected from the group consisting of nitrogen, oxygen, and sulfur atoms in the ring, wherein the 6- to 12-membered aryl group and the 5- or 6-membered heteroaryl group may be substituted with one or more halogen atoms,
   n is 1 or 2,
   R¹ represents a halogen atom,
   R² represents a hydrogen atom, a halogen atom, or a C₁₋C₄ alkoxy group, and
   R³ represents a hydrogen atom, a C₃-C₆ cycloalkyl group, or a 4- to 6-membered heterocyclyl group having 1 to 3 heteroatoms selected from the group consisting of nitrogen, oxygen, and sulfur atoms in the ring,
   wherein the C₃-C₆ cycloalkyl group and the 4- to 6-membered heterocyclyl group of R³ may have one or two oxo groups (=O) on the ring and/or may be substituted with one or two substituents selected from Group A below:
      Group A:
      a halogen atom,
      a hydroxy group,
      a C₁-C₄ alkyl group that may be substituted with a halogen atom,
      a C₁-C₆ hydroxyalkyl group that may be substituted with a halogen atom,
      a C₁-C₂ alkoxy group that may be substituted with a halogen atom,
      a C₂-C₆ alkoxyalkyl group that may be substituted with a halogen atom,
      a C₃-C₆ alkoxyalkoxyalkyl group that may be substituted with a halogen atom,
      a C₁-C₂ alkylsulfonyl group that may be substituted with a halogen atom,
      an acetyl group (-C(O)CH₃),
      a cyclopropylmethyl group,
      a phenyl group that may be substituted with a halogen atom,
      a phenyl C₁-C₃ alkyl group that may be substituted with a halogen atom,
      a 3- to 5-membered heterocyclyl group having one or two oxygen atoms,
      a 3- to 5-membered heterocyclyl-C₁-C₃ alkyl group having one or two oxygen atoms,
      an amino-oxo C₁-C₃ alkyl group,
      a C₂-C₄ alkynyl group that may be substituted with a halogen atom,
      an amino C₂-C₆ (alkoxyalkyl group) that may be protected,
      a C₁-C₃ alkylaminocarbonyl group,
      a 5-membered heteroaryl C₁-C₃ alkyl group,
      a C₂-C₄ alkylene group (linked to the same carbon),
      a C₁-C₄ alkylenedioxy group (linked to the same carbon), and
      a C₁-C₄ alkyleneoxy-C₁-C₄ alkyl group (linked to the same carbon).
[2] The compound according to clause [1] or pharmaceutically acceptable salt thereof,
   wherein the substituent in the C₃-C₆ cycloalkyl group and the 4- to 6-membered heterocyclyl group of R³ is selected from Group B below:
   Group B:
   a fluorine atom;
   a hydroxy group;
   a methyl group, an ethyl group, and an isopropyl group;
   a fluoroethyl group, a difluoroethyl group, and a trifluoromethyl group;
   a hydroxyethyl group and a 2-hydroxy-2-methylpropyl group;
   a methoxy group (-OCH₃) and a difluoromethoxy group (-OCHF₂);
   a methoxymethyl group (-CH₂OCH₃) and a methoxyethyl group (-C₂H₄OCH₃);
   a methoxyethoxyethyl group (-C₂H₄OC₂H₄OCH₃);
   a methylsulfonyl group (-S(O)₂CH₃);
   an acetyl group (-C(O)CH₃);
   a cyclopropylmethyl group;
   a phenyl group;
   a benzyl group;
   an oxetanyl group;
   an oxetanylmethyl group;
   a 2-amino-2-oxoethyl group (-CH₂C(O)NH);
   a propargyl group;
   a tert-butoxycarbonyl-2-aminoethoxyethyl group (-C₂H₄OC₂H₄NHC(O)O(CH₃)₃);
   an ethylaminocarbonyl group (-C(O)NHCH₂CH₃);
   an oxadiazolylmethyl group;
   an ethylene group (linked to the same carbon);
   a methylenedioxy group (linked to the same carbon) and an ethylenedioxy group (linked to the same carbon); and
   a methyleneoxymethyl group (linked to the same carbon).
[3] The compound according to clause [1] or [2] or pharmaceutically acceptable salt thereof,
   wherein A¹ represents N or CH, and one or none of A², A³, and A⁴ represents N.
[4] The compound according to any one of clauses [1] to [3] or pharmaceutically acceptable salt thereof,
   wherein Ar represents a phenyl group that may be substituted with one halogen atom or a 5- or 6-membered heteroaryl group that may be substituted with one halogen atom,
   the 5-membered heteroaryl group is selected from the group consisting of a furyl group, a thienyl group, a pyrrolyl group, an oxazolyl group, a thiazolyl group, an isothiazolyl group, and an oxadiazolyl group, and
   the 6-membered heteroaryl group is a pyridyl group or a pyrimidyl group.
[5] The compound according to any one of clauses [1] to [4] or pharmaceutically acceptable salt thereof,
   wherein the halogen atom is fluorine or chlorine.
[6] The compound according to any one of clauses [1] to [5] or pharmaceutically acceptable salt thereof,
   wherein R¹ represents a chlorine atom or a bromine atom.
[7] The compound according to any one of clauses [1] to [6] or pharmaceutically acceptable salt thereof,
   wherein R² is selected from the group consisting of a hydrogen atom, a fluorine atom, a chlorine atom, a bromine atom, and a C₁-C₂ alkoxy group.
[8] The compound according to any one of clauses [1] to [7] or pharmaceutically acceptable salt thereof,
   wherein the heterocyclyl group of R³ has one oxygen atom, one sulfur atom, one nitrogen atom, one oxygen atom and one nitrogen atom, one sulfur atom and one nitrogen atom, two oxygen atoms, or two nitrogen atoms.
[9] The compound according to any one of clauses [1] to [8] or pharmaceutically acceptable salt thereof,
   wherein the heterocyclyl group of R³ is an oxetanyl group, a tetrahydrofuryl group, a furyl group, a tetrahydropyranyl group, a dioxolanyl group, and a dioxanyl group.
[10] The compound according to any one of clauses [1] to [9] or pharmaceutically acceptable salt thereof,
   wherein the heterocyclyl group having an oxo group on the ring, of R³ is selected from the group consisting of a 2-oxooxazolidinyl group, a 2-oxooxazinyl group, a 3-oxomorpholinyl group, a 2-oxoazetidinyl group, a 2-oxopyrrolidinyl group, a 2-oxopiperidinyl group, a 2-oxoimidazolidinyl group, a 2-oxo-1,3-dioxolanyl group, a 2-oxo-1,3-dioxolyl group, a 1,1-dioxo-thietanyl group, a 1,1-dioxo-tetrahydrothiophenyl group, and a 1,1-dioxo-isothizolidinyl group.
[11] The compound according to any one of clauses [1] to [10] or pharmaceutically acceptable salt thereof,
   wherein the heterocyclyl group having no oxo group as a substituent on the ring, of R³ is selected from the group consisting of an oxazolyl group, an azetidinyl group, a pyrrolidinyl group, a dioxolanyl group, a dioxanyl group, a tetrahydrofuryl group, a tetrahydropyranyl group, a furyl group, and an oxetanyl group.
[12] A compound represented by Formula (II) below or a pharmaceutically acceptable salt thereof: 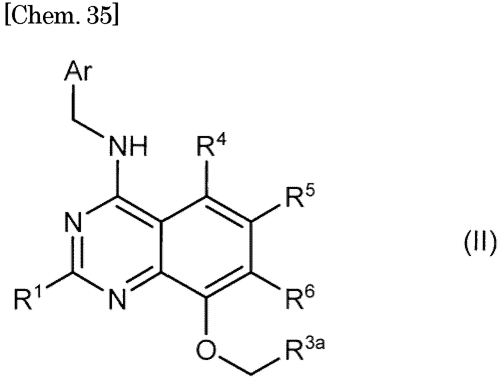 wherein, in Formula (II),
   Ar represents a furyl group, a phenyl group, a thienyl group, a pyridyl group, a pyrimidinyl group, a thiazolyl group, an oxazolyl group, an isoxazolyl group, an isothiazolyl group, or an oxadiazolyl group, wherein the furyl group, the phenyl group, and the pyridyl group may be substituted with one fluorine atom,
   R¹ represents a halogen atom,
   R⁴, R⁵, and R⁶ each independently represent a hydrogen atom, a halogen atom, or a methoxy group, wherein at least two of R⁴, R⁵, and R⁶ represent hydrogen atoms, and
   R^{3a} represents a hydrogen atom or any group selected from Group C below:

      Group C: 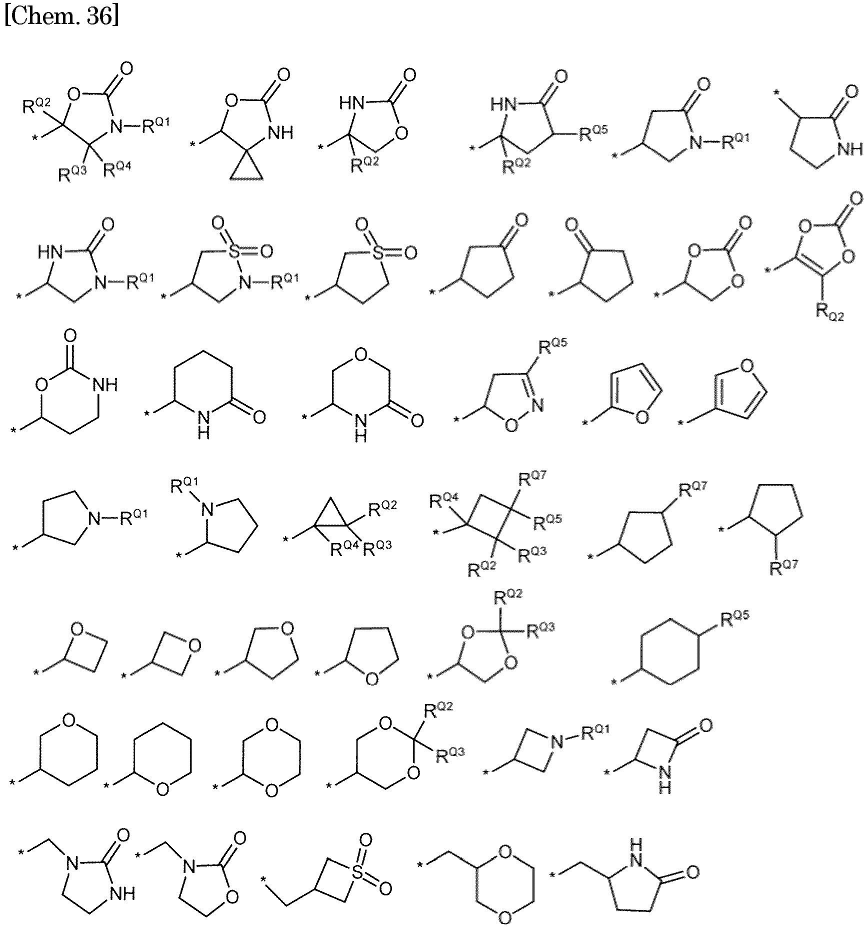
   wherein R^{Q1} represents a hydrogen atom, a fluorine atom, a methyl group, an ethyl group, an isopropyl group, a fluoroethyl group, a difluoroethyl group, a hydroxyethyl group, a 2-hydroxy-2-methylpropyl group, a methoxyethyl group (-C₂H₄OCH₃), a methoxyethoxyethyl group (-C₂H₄OC₂H₄OCH₃), a methylsulfonyl group (-S(O)₂CH₃), an acetyl group (-C(O)CH₃), a cyclopropylmethyl group, a phenyl group, a benzyl group, an oxetanyl group, an oxetanylmethyl group, a 2-amino-2-oxoethyl group (-CH₂C(O)NH₂), a propargyl group (-CH₂C≡CH), a tert-butoxycarbonyl-2-aminoethoxyethyl group (-C₂H₄OC₂H₄NHC(O)O(CH₃)₃), an ethylaminocarbonyl group (-C(O)NHCH₂CH₃), or an oxadiazolylmethyl group,
   R^{Q2}, R^{Q3}, and R^{Q4} each independently represent a hydrogen atom, a fluorine atom, a hydroxy group, a methyl group, or a trifluoromethyl group,
   R^{Q5} and R^{Q6} each independently represent a hydrogen atom, a fluorine atom, a hydroxy group, a methyl group, a methoxy group (-OCH₃), a difluoromethoxy group (-OCHF₂), or a methoxymethyl group, and
   R^{Q7} represents a hydrogen atom, a fluorine atom, a methyl group, or an ethyl group, or alternatively an ethylenedioxy group (linked to the same carbon) or a methyleneoxymethyl group (linked to the same carbon).
[13] A compound represented by Formula (III) below or a pharmaceutically acceptable salt thereof: 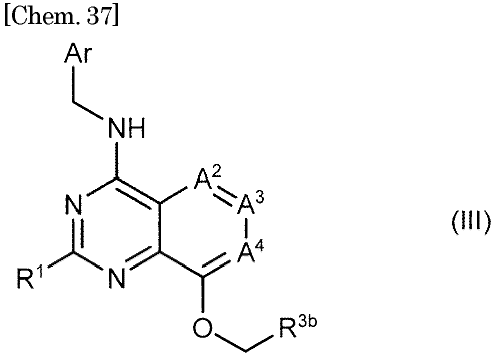 wherein, in Formula (III),
   Ar represents a furyl group or a phenyl group,
   R¹ represents a chlorine atom,
   A², A³, and A⁴ each independently represent CH or N and any one of A², A³, and A⁴ represents N, and
   R^{3b} represents a hydrogen atom or any group selected from Group D below:
      Group D: 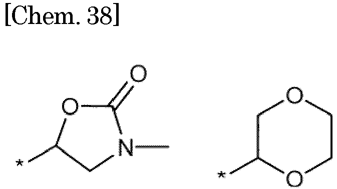
[14] A compound represented by Formula (IV) below or a pharmaceutically acceptable salt thereof: 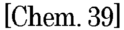 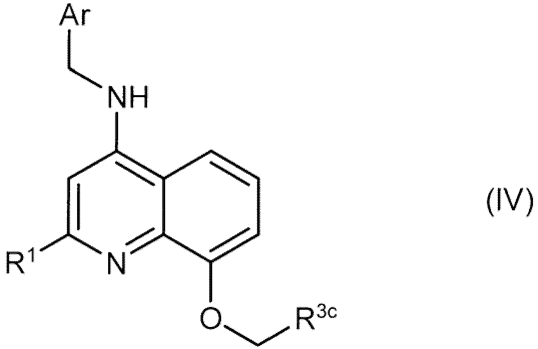 wherein, in Formula (IV),
   Ar represents a furyl group or a phenyl group,
   R¹ represents a chlorine atom,
   R^{3c} represents a hydrogen atom or any group selected from Group E below:
      Group E: 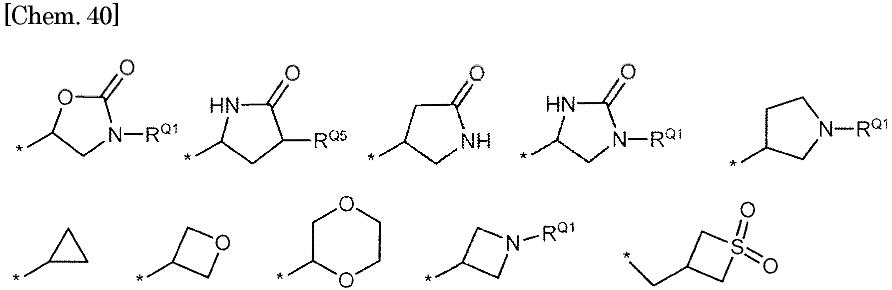
   wherein R^{Q1} represents a hydrogen atom, a methyl group, a methylsulfonyl group (-S(O)₂CH₃), an acetyl group (-C(O)CH₃), a fluoroethyl group, or a hydroxyethyl group, and
   R^{Q5} represents a hydrogen atom or a methyl group.
[15] A compound represented by Formula (V) below or a pharmaceutically acceptable salt thereof: 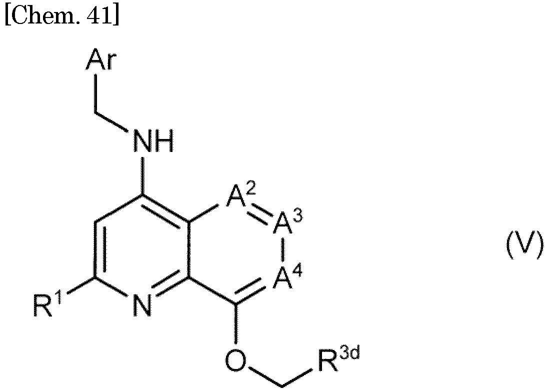 wherein, in Formula (V),
   Ar represents a furyl group or a phenyl group,
   R¹ represents a chlorine atom,
   A², A³, and A⁴ each independently represent CH or N and any one of A², A³, and A⁴ represents N, and
   R^{3d} represents a hydrogen atom or any group selected from Group F below:
      Group F: 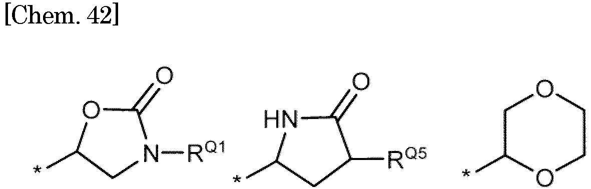
   wherein R^{Q1} represents a hydrogen atom, a methyl group, a fluoroethyl group, or a hydroxyethyl group, and
   R^{Q5} represents a hydrogen atom or a methyl group.
[16] A compound or a pharmaceutically acceptable salt thereof, the compound being any one compound selected from the group consisting of:
   2-chloro-N-(furan-2-ylmethyl)-8-methoxyquinazolin-4-amine,
   2-chloro-N-(furan-2-ylmethyl)-8-methoxyquinolin-4-amine,
   2-chloro-7-fluoro-N-(furan-2-ylmethyl)-8-methoxyquinazolin-4-amine,
   2-chloro-5-fluoro-N-(furan-2-ylmethyl)-8-methoxyquinazolin-4-amine,
   2-bromo-N-(furan-2-ylmethyl)-8-methoxyquinazolin-4-amine,
   2-chloro-N-(furan-2-ylmethyl)-8-methoxypyrido[3,4-d]pyrimidin-4-amine,
   2-chloro-N-(furan-2-ylmethyl)-8-methoxypyrido[4,3-d]pyrimidin-4-amine,
   2-chloro-N-(furan-2-ylmethyl)-8-methoxypyrido[3,2-d]pyrimidin-4-amine,
   2-chloro-6-fluoro-N-(furan-2-ylmethyl)-8-methoxyquinazolin-4-amine,
   2-chloro-N-(furan-2-ylmethyl)-6,8-dimethoxyquinazolin-4-amine,
   2-chloro-N-(furan-2-ylmethyl)-7,8-dimethoxyquinazolin-4-amine,
   2-chloro-8-methoxy-N-(thiophen-2-ylmethyl)quinazolin-4-amine,
   2-chloro-8-methoxy-N-(1,3-thiazol-2-ylmethyl)quinazolin-4-amine,
   2-chloro-8-methoxy-N-(1,3-oxazol-2-ylmethyl)quinazolin-4-amine,
   2-chloro-8-methoxy-N-(thiophen-3-ylmethyl)quinazolin-4-amine,
   2-chloro-8-methoxy-N-(pyridin-4-ylmethyl)quinazolin-4-amine,
   2-chloro-N-(2-fluorobenzyl)-8-methoxyquinazolin-4-amine,
   2-chloro-8-(1,4-dioxan-2-ylmethoxy)-N-(furan-2-ylmethyl)quinazolin-4-amine,
   5-[({2-chloro-4-[(furan-2-ylmethyl)amino]quinazolin-8-yl}oxy)methyl]-1,3-oxazolidin-2-one,
   (5R)-5-[({2-chloro-4-[(furan-2-ylmethyl)amino]quinazolin-8-yl}oxy)methyl]-1,3-oxazolidin-2-one,
   (5S)-5-[({2-chloro-4-[(furan-2-ylmethyl)amino]quinazolin-8-yl}oxy)methyl]-1,3-oxazolidin-2-one,
   1-{3-[({2-chloro-4-[(furan-2-ylmethyl)amino]quinazolin-8-yl}oxy)methyl]pyrrolidin-1-yl}ethanone,
   2-chloro-N-(furan-2-ylmethyl)-8-{[1-(methylsulfonyl)pyrrolidin-3-yl]methoxy}quinazolin-4-amine,
   2-chloro-8-[(2,2-dimethyl-1,3-dioxan-5-yl)methoxy]-N-(furan-2-ylmethyl)quinazolin-4-amine,
   5-[({2-chloro-4-[(furan-2-ylmethyl)amino]quinazolin-8-yl}oxy)methyl]pyrrolidin-2-one,
   2-chloro-8-[(2,2-difluorocyclobuty)methoxy]-N-(furan-2-ylmethyl)quinazolin-4-amine,
   2-chloro-8-(1,4-dioxaspiro[4.4]nonan-7-ylmethoxy)-N-(furan-2-ylmethyl)quinazolin-4-amine,
   3-[({2-chloro-4-[(furan-2-ylmethyl)amino]quinazolin-8-yl}oxy)methyl]cyclopentanone,
   2-chloro-8-(1,4-dioxaspiro[4.4]nonan-6-ylmethoxy)-N-(furan-2-ylmethyl)quinazolin-4-amine,
   2-[({2-chloro-4-[(furan-2-ylmethyl)amino]quinazolin-8-yl}oxy)methyl]cyclopentanone,
   4-[({2-chloro-4-[(furan-2-ylmethyl)amino]quinazolin-8-yl}oxy)methyl]pyrrolidin-2-one,
   3-[({2-chloro-4-[(furan-2-ylmethyl)amino]quinazolin-8-yl}oxy)methyl]cyclobutanol,
   2-chloro-8-{[3-(difluoromethoxy)cyclobutyl]methoxy}-N-(furan-2-ylmethyl)quinazolin-4-amine,
   5-[({2,6-dichloro-4-[(furan-2-ylmethyl)amino]quinazolin-8-yl}oxy)methyl]-3-methyl-1,3-oxazolidin-2-one,
   (3S,5R)-5-[({2,6-dichloro-4-[(furan-2-ylmethyl)amino]quinazolin-8-yl}oxy)methyl]-3-methylpyrrolidin-2-one,
   5-({[4-(benzylamino)-2,6-dichloroquinazolin-8-yl]oxy}methyl)-3-methyl-1,3-oxazolidin-2-one,
   5-[({2,7-dichloro-4-[(furan-2-ylmethyl)amino]quinazolin-8-yl}oxy)methyl]-3-methyl-1,3-oxazolidin-2-one,
   2-chloro-8-(1,4-dioxan-2-ylmethoxy)-N-(furan-2-ylmethyl)quinolin-4-amine,
   5-[({2-chloro-4-[(furan-2-ylmethyl)amino]quinolin-8-yl}oxy)methyl]-1,3-oxazolidin-2-one,
   5-({[4-(benzylamino)-2-chloroquinazolin-8-yl]oxy}methyl)-3-methyl-1,3-oxazolidin-2-one,
   5-({[4-(benzylamino)-2-chloroquinazolin-8-yl]oxy}methyl)-3-(2-fluoroethyl)-1,3-oxazolidin-2-one,
   5-({[4-(benzylamino)-2-chloroquinazolin-8-yl]oxy}methyl)-3-(2-hydroxyethyl)-1,3-oxazolidin-2-one,
   5-({[4-(benzylamino)-2-chloroquinolin-8-yl]oxy}methyl)-3-methyl-1,3-oxazolidin-2-one,
   2-chloro-N-(furan-2-ylmethyl)-8-(tetrahydrofuran-3-ylmethoxy)quinazolin-4-amine,
   2-chloro-N-(furan-2-ylmethyl)-8-(tetrahydro-2H-pyran-3-ylmethoxy)quinazolin-4-amine,
   2-chloro-8-[(1-fluorocyclopropyl)methoxy]-N-(furan-2-ylmethyl)quinazolin-4-amine,
   1-[2-({2-chloro-4-[(furan-2-ylmethyl)amino]quinazolin-8-yl}oxy)ethyl]imidazolidin-2-one,
   2-chloro-8-[(1,1-dioxidotetrahydrothiophen-3-yl)methoxy]-N-(furan-2-ylmethyl)quinazolin-4-amine,
   7-[({2-chloro-4-[(furan-2-ylmethyl)amino]quinazolin-8-yl}oxy)methyl]-6-oxa-4-azaspiro[2.4]heptan-5-one,
   (3S,5R)-5-[({2-chloro-4-[(furan-2-ylmethyl)amino]quinazolin-8-yl}oxy)methyl]-3-methylpyrrolidin-2-one,
   2-chloro-N-(furan-2-ylmethyl)-8-[(2-methyl-1,1-dioxido-1,2-thiazolidin-4-yl)methoxy] quinazolin-4-amine,
   2-chloro-8-(furan-2-ylmethoxy)-N-(furan-2-ylmethyl)quinazolin-4-amine,
   3-[2-({2-chloro-4-[(furan-2-ylmethyl)amino]quinazolin-8-yl}oxy)ethyl]-1,3-oxazolidin-2-one,
   4-[({2-chloro-4-[(furan-2-ylmethyl)amino]quinazolin-8-yl}oxy)methyl]-1,3-dioxolan-2-one,
   2-chloro-8-[2-(1,4-dioxan-2-yl)ethoxy]-N-(furan-2-ylmethyl)quinazolin-4-amine,
   2-chloro-N-(furan-2-ylmethyl)-8-[(3-methoxy-4,5-dihydro-1,2-oxazol-5-yl)methoxy] quinazolin-4-amine,
   5-[({2-chloro-4-[(furan-2-ylmethyl)amino]quinazolin-8-yl}oxy)methyl]morpholin-3-one,
   1-benzyl-4-[({2-chloro-4-[(furan-2-ylmethyl)amino]quinazolin-8-yl}oxy)methyl]imidazolidin-2-one,
   2-chloro-N-(furan-2-ylmethyl)-8-(2-oxaspiro[3.3]heptan-6-ylmethoxy)quinazolin-4-amine,
   2-chloro-8-(cyclopentylmethoxy)-N-(furan-2-ylmethyl)quinazolin-4-amine,
   1-{3-[({2-chloro-4-[(furan-2-ylmethyl)amino]quinazolin-8-yl}oxy)methyl]azetidin-1-yl}ethanone,
   2-chloro-8-(furan -3-ylmethoxy)-N-(furan-2-ylmethyl)quinazolin-4-amine,
   2-chloro-N-(furan-2-ylmethyl)-8-{[1-(trifluoromethyl)cyclobutyl]methoxy}quinazolin-4-amine,
   2-chloro-8-(cyclohexylmethoxy)-N-(furan-2-ylmethyl)quinazolin-4-amine,
   2-chloro-N-(furan-2-ylmethyl)-8-(tetrahydrofuran-2-ylmethoxy)quinazolin-4-amine,
   1-{2-[({2-chloro-4-[(furan-2-ylmethyl)amino]quinazolin-8-yl}oxy)methyl]pyrrolidin-1-yl}ethanone,
   4-[({2-chloro-4-[(furan-2-ylmethyl)amino]quinazolin-8-yl}oxy)methyl]-1,3-oxazolidin-2-one,
   3-benzyl-5-[({2-chloro-4-[(furan-2-ylmethyl)amino]quinazolin-8-yl}oxy)methyl]-1,3-oxazolidin-2-one,
   5-[({2-chloro-4-[(furan-2-ylmethyl)amino]quinazolin-8-yl}oxy)methyl]-3-phenyl-1,3-oxazolidin-2-one,
   (3S,5R)-5-({[4-(benzylamino)-2-chloroquinazolin-8-yl]oxy}methyl)-3-methylpyrrolidin-2-one,
   (3R,5R)-5-({[4-(benzylamino)-2-chloroquinazolin-8-yl]oxy}methyl)-3-methylpyrrolidin-2-one,
   4-({[4-(benzylamino)-2-chloroquinazolin-8-yl]oxy}methyl)pyrrolidin-2-one,
   5-[({2-chloro-4-[(furan-2-ylmethylamino]quinazolin-8-yl}oxy)methyl]-4,4-dimethyl-1,3-oxazolidin-2-one,
   6-[({2-chloro-4-[(furan-2-ylmethyl)amino]quinazolin-8-yl}oxy)methyl]-1,3-oxazinan-2-one,
   4-[({2-chloro-4-[(furan-2-ylmethyl)amino]quinazolin-8-yl}oxy)methyl]-1-methylpyrrolidin-2-one,
   4-[({2-chloro-4-[(furan-2-ylmethyl)amino]quinazolin-8-yl}oxy)methyl]-5-methyl-1,3-dioxol-2-one,
   6-[({2-chloro-4-[(furan-2-ylmethyl)amino]quinazolin-8-yl}oxy)methyl]piperidin-2-one,
   (5R)-5-[({2-chloro-4-[(furan-2-ylmethyl)amino] quinazolin-8-yl}oxy)methyl]pyrrolidin-2-one,
   (69)-5-[({2-chloro-4-[(furan-2-ylmethyl)amino] quinazolin-8-yl}oxy)methyl]pyrrolidin-2-one,
   4-[({2-chloro-4-[(furan-2-ylmethyl)amino]quinazolin-8-yl}oxy)methyl]azetidin-2-one,
   5-[({2-chloro-4-[(furan-2-ylmethyl)amino]quinazolin-8-yl}oxy)methyl]-5-methylpyrrolidin-2-one,
   4-[({2-chloro-4-[(furan-2-ylmethyl)amino]quinazolin-8-yl}oxy)methyl]-4-methyl-1,3-oxazolidin-2-one,
   5-[({2-chloro-4-[(furan-2-ylmethyl)amino]quinazolin-8-yl}oxy)methyl]-3-methyl-1,3-oxazolidin-2-one,
   5-({[4-(benzylamino)-2-chloroquinazolin-8-yl]oxy}methyl)-1,3-oxazolidin-2-one,
   5-[({2-chloro-4-[(furan-2-ylmethyl)amino]quinolin-8-yl}oxy)methyl]pyrrolidin-2-one,
   5-[({2-chloro-4-[(furan-2-ylmethyl)amino]quinolin-8-yl}oxy)methyl]-3-(2-hydroxyethyl)-1,3-oxazolidin-2-one,
   5-[({2-chloro-4-[(furan-2-ylmethyl)amino]quinolin-8-yl}oxy)methyl]-3-(2-fluoroethyl)- 1,3-oxazolidin-2-one,
   1-{3-[({2-chloro-4-[(furan-2-ylmethyl)amino]quinolin-8-yl}oxy)methyl]azetidin-1-yl}ethanone,
   4-[({2-chloro-4-[(furan-2-ylmethyl)amino]quinolin-8-yl}oxy)methyl]pyrrolidin-2-one,
   2-chloro-N-(furan-2-ylmethyl)-8-{[1-(methylsulfonyl)azetidin-3-yl]methoxy}quinolin-4-amine,
   (3S,5R)-5-[({2-chloro-4-[(furan-2-ylmethyl)amino]quinolin-8-yl}oxy)methyl]-3-methylpyrrolidin-2-one,
   2-chloro-N-(furan-2-ylmethyl)-8-{[1-(methylsulfonyl)pyrrolidin-3-yl]methoxy}quinolin-4-amine,
   1-{3-[({2-chloro-4-[(furan-2-ylmethyl)amino]quinolin-8-yl}oxy)methyl]pyrrolidin-1-yl}ethanone,
   (5R)-5-[({2-chloro-4-[(furan-2-ylmethyl)amino] quinolin-8-yl}oxy)methyl]-1,3-oxazolidin-2-one,
   (5S)-5-[({2-chloro-4-[(furan-2-ylmethyl)amino]quinolin-8-yl}oxy)methyl]-1,3-oxazolidin-2-one,
   (5R)-5-[({2-chloro-4-[(furan-2-ylmethyl)amino]quinolin-8-yl}oxy)methyl]pyrrolidin-2-one,
   (5S)-5-[({2-chloro-4-[(furan-2-ylmethyl)amino]quinolin-8-yl}oxy)methyl]pyrrolidin-2-one,
   5-({[4-(benzylamino)-2-chloroquinolin-8-yl]oxy}methyl)-1,3-oxazolidin-2-one,
   5-({[4-(benzylamino)-2-chloroquinolin-8-yl]oxy}methyl)-3-(2-hydroxyethyl)-1,3-oxazolidin-2-one,
   5-({[4-(benzylamino)-2-chloroquinolin-8-yl]oxy}methyl)-3-(2-fluoroethyl)-1,3-oxazolidin-2-one,
   (3S,5R)-5-({[4-(benzylamino)-2-chloroquinolin-8-yl]oxy}methyl)-3-methylpyrrolidin-2-one,
   4-({[4-(benzylamino)-2-chloroquinolin-8-yl]oxy}methyl)pyrrolidin-2-one,
   2-chloro-8-(1,4-dioxan-2-ylmethoxy)-N-(furan-2-ylmethyl)- 1,5-naphthyridin-4-amine,
   5-[({6-chloro-8-[(furan-2-ylmethyl)amino]-1,5-naphthyridin-4-yl}oxy)methyl]-1,3-oxazolidin-2-one,
   5-[({6-chloro-8-[(furan-2-ylmethyl)amino]-1,5-naphthyridin-4-yl}oxy)methyl]-3-methyl-1,3-oxazolidin-2-one,
   (3S,5R)-5-[({6-chloro-8-[(furan-2-ylmethyl)amino]-1,5-naphthyridin-4-yl}oxy)methyl]-3-methylpyrrolidin-2-one,
   5-[({6-chloro-8-[(furan-2-ylmethyl)amino]-1,5-naphthyridin-4-yl}oxy)methyl]-3-(2-hydroxyethyl)-1,3-oxazolidin-2-one,
   5-[({6-chloro-8-[(furan-2-ylmethyl)amino]-1,5-naphthyridin-4-yl}oxy)methyl]-3-(2-fluoroethyl)-1,3-oxazolidin-2-one,
   5-({[8-(benzylamino)-6-chloro-1,5-naphthyridin-4-yl]oxy}methyl)-1,3-oxazolidin-2-one,
   5-({[8-(benzylamino)-6-chloro-1,5-naphthyridin-4-yl]oxy}methyl)-3-methyl-1,3-oxazolidin-2-one,
   (3S,5R)-5-({[8-(benzylamino)-6-chloro-1,5-naphthyridin-4-yl]oxy}methyl)-3-methylpyrrolidin-2-one,
   5-({[8-(benzylamino)-6-chloro-1,5-naphthyridin-4-yl]oxy}methyl)-3-(2-hydroxyethyl)-1,3-oxazolidin-2-one,
   5-({[8-(benzylamino)-6-chloro-1,5-naphthyridin-4-yl]oxy}methyl)-3-(2-fluoroethyl)- 1,3-oxazolidin-2-one,
   5-({[4-(benzylamino)-2-chloropyrido[3,2-d]pyrimidin-8-yl]oxy}methyl)-3-methyl-1,3-oxazolidin-2-one,
   (5R)-5-[({2-chloro-4-[(furan-2-ylmethyl)amino]quinazolin-8-yl}oxy)methyl]-3-(methoxymethyl)pyrrolidin-2-one,
   3-[({2-chloro-4-[(furan-2-ylmethyl)amino]quinazolin-8-yl}oxy)methyl]pyrrolidin-2-one,
   4-[({2-chloro-4-[(furan-2-ylmethyl)amino]quinazolin-8-yl}oxy)methyl]-1-methylimidazolidin-2-one,
   5-[2-({2-chloro-4-[(furan-2-ylmethyl)amino]quinazolin-8-yl}oxy)ethyl]pyrrolidin-2-one,
   2-chloro-8-[(2,2-dimethyl-1,3-dioxolan-4-yl)methoxy]-N-(furan-2-ylmethyl)quinazolin-4-amine,
   trans-4-[({2-chloro-4-[(furan-2-ylmethyl)amino]quinazolin-8-yl}oxy)methyl]cyclohexanol,
   4-[({2-chloro-4-[(furan-2-ylmethyl)amino]quinolin-8-yl}oxy)methyl]-1-methylimidazolidin-2-one,
   2-chloro-8-(cyclopropylmethoxy)-N-(furan-2-ylmethyl)quinazolin-4-amine,
   2-chloro-N-(furan-2-ylmethyl)-8-(oxetan-3-ylmethoxy)quinazolin-4-amine,
   2-chloro-8-(1,3-dioxan-5-ylmethoxy)-N-(furan-2-ylmethyl)quinazolin-4-amine,
   2-chloro-8-(cyclobutylmethoxy)-N-(furan-2-ylmethyl)quinazolin-4-amine,
   2-chloro-8-[(2S)-1,4-dioxan-2-ylmethoxy]-N-(furan-2-ylmethyl)quinazolin-4-amine,
   2-chloro-8-[(2S)-1,4-dioxan-2-ylmethoxy]-7-fluoro-N-(furan-2-ylmethyl)quinazolin-4-amine,
   2-chloro-N-(furan-2-ylmethyl)-8-(oxetan-3-ylmethoxy)quinolin-4-amine,
   2-chloro-8-[(2,2-difluorocyclopropyl)methoxy]-N-(furan-2-ylmethyl)quinazolin-4-amine,
   2-chloro-8-[(3,3-difluorocyclobutyl)methoxy]-N-(furan-2-ylmethyl)quinazolin-4-amine,
   2-chloro-N-(furan-2-ylmethyl)-8-[(1-methylcyclopropyl)methoxy]quinazolin-4-amine,
   2-chloro-N-(furan-2-ylmethyl)-8-(tetrahydro-2H-pyran-2-ylmethoxy)quinazolin-4-amine,
   2-chloro-N-(furan-2-ylmethyl)-8-(oxetan-2-ylmethoxy)quinazolin-4-amine,
   2-chloro-8-[2-(1,1-dioxidothiethan-3-yl)ethoxy]-N-(furan-2-ylmethyl)quinazolin-4-amine,
   2-chloro-8-[(2R)-1,4-dioxan-2-ylmethoxy]-N-(furan-2-ylmethyl) quinazolin-4-amine,
   5-[({2-chloro-4-[(furan-2-ylmethyl)amino]quinazolin-8-yl}oxy)methyl]-5-methyl-1,3-oxazolidin-2-one,
   2-chloro-8-[(2R)-1,4-dioxan-2-ylmethoxy]-7-fluoro-N-(furan-2-ylmethyl)quinazolin-4-amine,
   2-chloro-8-(cyclopropylmethoxy)-7-fluoro-N-(furan-2-ylmethyl) quinazolin-4-amine,
   2-chloro-8-(cyclobutylmethoxy)-7-fluoro-N-(furan-2-ylmethyl)quinazolin-4-amine,
   2-chloro-7-fluoro-N-(furan-2-ylmethyl)-8-(oxetan-3-ylmethoxy)quinazolin-4-amine,
   2-chloro-8-(cyclopropylmethoxy)-N-(furan-2-ylmethyl)quinolin-4-amine,
   2-chloro-8-[2-(1,1-dioxidothiethan-3-yl)ethoxy]-N-(furan-2-ylmethyl)quinolin-4-amine,
   2-chloro-8-[(2,2-difluorocyclopropyl)methoxy]-N-(thiophen-2-ylmethyl)quinazolin-4-amine,
   2-chloro-8-(1,4-dioxan-2-ylmethoxy)-N-(thiophen-2-ylmethyl)quinazolin-4-amine,
   2-chloro-8-[(2,2-difluorocyclopropyl)methoxy]-N-(thiophen-3-ylmethyl)quinazolin-4-amine,
   2-chloro-8-(1,4-dioxan-2-ylmethoxy)-N-(thiophen-3-ylmethyl)quinazolin-4-amine,
   5-{[(2-chloro-4-{[(4-fluorofuran-2-yl)methyl]amino}quinazolin-8-y)oxy]methyl}-1,3-oxazolidin-2-one,
   2-chloro-8-[(2,2-difluorocyclopropylmethoxy]-N-(1,2-thiazol-5-ylmethyl)quinazolin-4-amine,
   2-chloro-8-[(2,2-difluorocyclopropyl)methoxy]-N-(1,3-oxazol-4-ylmethyl)quinazolin-4-amine,
   2-chloro-8-(1,4-dioxan-2-ylmethoxy)-N-(1,2-oxazol-3-ylmethyl)quinazolin-4-amine,
   5-[({2-chloro-4-[(1,3-oxazol-2-ylmethyl)amino]quinazolin-8-yl}oxy)methyl]-1,3-oxazolidin-2-one,
   2-chloro-8-(1,4-dioxan-2-ylmethoxy)-N-(1,3-oxazol-2-ylmethyl)quinazolin-4-amine,
   5-[({2-chloro-4-[(thiophen-2-ylmethyl)amino]quinazolin-8-yl}oxy)methyl]-1,3-oxazolidin-2-one,
   5-[({2-chloro-4-[(thiophen-3-ylmethyl)amino]quinazolin-8-yl}oxy)methyl]-1,3-oxazolidin-2-one,
   2-chloro-8-[(2,2-difluorocyclopropyl)methoxy]-N-(1,3-thiazol-2-ylmethyl)quinazolin-4-amine,
   2-chloro-8-(1,4-dioxan-2-ylmethoxy)-N-(1,3-thiazol-2-ylmethyl)quinazolin-4-amine,
   5-[({2-chloro-4-[(1,3-thiazol-2-ylmethyl)amino]quinazolin-8-yl}oxy)methyl]-1,3-oxazolidin-2-one,
   2-chloro-8-[(2,2-difluorocyclopropyl)methoxy]-N-(1,3-oxazol-2-ylmethyl)quinazolin-4-amine,
   2-chloro-8-[(2,2-difluorocyclopropyl)methoxy]-N-(pyridin-4-ylmethyl)quinazolin-4-amine,
   5-[({2-chloro-4-[(pyridin-4-ylmethyl)amino]quinazolin-8-yl}oxy)methyl]-1,3-oxazolidin-2-one,
   5-[({2-chloro-4-[(pyrimidin-4-ylmethyl)amino]quinazolin-8-yl}oxy)methyl]-1,3-oxazolidin-2-one,
   5-[({2-chloro-4-[(pyridin-2-ylmethyl)amino]quinazolin-8-yl}oxy)methyl]-1,3-oxazolidin-2-one,
   5-{[(2-chloro-4-{[(3-fluoropyridin-4-yl)methyl]amino}quinazolin-8-yl)oxy]methyl}-1,3-oxazolidin-2-one,
   5-[({2-chloro-4-[(2-fluorobenzyl)amino]quinazolin-8-yl}oxy)methyl]-1,3-oxazolidin-2-one,
   5-[({2-chloro-4-[(3-fluorobenzyl)amino]quinazolin-8-yl}oxy)methyl]-1,3-oxazolidin-2-one,
   2-chloro-8-(1,4-dioxan-2-ylmethoxy)-N-(1,2,4-oxadiazol-3-ylmethyl) quinazolin-4-amine,
   5-[({2-chloro-4-[(1,2-thiazol-5-ylmethyl)amino]quinazolin-8-yl}oxy)methyl]-1,3-oxazolidin-2-one,
   5-[({2-chloro-4-[(4-fluorobenzyl)amino]quinazolin-8-yl}oxy)methyl]-1,3-oxazolidin-2-one,
   5-[({2-chloro-4-[(furan-2-ylmethyl)amino]-1,7-naphthyridin-8-yl}oxy)methyl]-3-methyl-1,3-oxazolidin-2-one,
   (5S)-5-[({2-chloro-4-[(furan-2-ylmethyl)amino]-1,7-naphthyridin-8-yl}oxy)methyl]-3-methyl-1,3-oxazolidin-2-one,
   (5R)-5-[({2-chloro-4-[(furan-2-ylmethyl)amino]- 1, 7-naphthyridin-8-yl}oxy)methyl]-3-methyl- 1,3-oxazolidin-2-one,
   5-({[4-(benzylamino)-2-chloro-1,7-naphthyridin-8-yl]oxy}methyl)-1,3-oxazolidin-2-one,
   5-[({2-chloro-4-[(furan-2-ylmethyl)amino]-1,7-naphthyridin-8-yl}oxy)methyl]-3-(2-fluoroethyl)-1,3-oxazolidin-2-one,
   5-[({2-chloro-4-[(furan-2-ylmethyl)amino]-1,7-naphthyridin-8-yl}oxy)methyl]-3-(2-hydroxyethyl)-1,3-oxazolidin-2-one,
   5-({[4-(benzylamino)-2-chloro-1,7-naphthyridin-8-yl]oxy}methyl)-3-(2-fluoroethyl)- 1,3-oxazolidin-2-one,
   5-({[4-(benzylamino)-2-chloro-1,7-naphthyridin-8-yl]oxy}methyl)-3-methyl-1,3-oxazolidin-2-one,
   5-({[4-(benzylamino)-2-chloro-1,7-naphthyridin-8-yl]oxy}methyl)-3-(2-hydroxyethyl)-1,3-oxazolidin-2-one,
   2-chloro-8-(1,4-dioxan-2-ylmethoxy)-N-(furan-2-ylmethyl)pyrido[3,2-d]pyrimidin-4-amine,
   2-chloro-8-(1,4-dioxan-2-ylmethoxy)-N-(furan-2-ylmethyl)pyrido[3,4-d]pyrimidin-4-amine,
   5-[({2-chloro-4-[(furan-2-ylmethyl)amino]pyrido[3,4-d]pyrimidin-8-yl}oxy)methyl]-3-methyl-1,3-oxazolidin-2-one,
   5-({[4-(benzylamino)-2-chloropyrido[3,4-d]pyrimidin-8-yl]oxy}methyl)-3-methyl-1,3-oxazolidin-2-one,
   5-[({2-chloro-4-[(furan-2-ylmethyl)amino]pyrido[4,3-d]pyrimidin-8-yl}oxy)methyl]-3-methyl-1,3-oxazolidin-2-one,
   5-({[4-(benzylamino)-2-chloropyrido[4,3-d]pyrimidin-8-yl]oxy}methyl)-3-methyl-1,3-oxazolidin-2-one,
   5-({[4-(benzylamino)-2-chloro-1,6-naphthyridin-8-yl]oxy}methyl)-1,3-oxazolidin-2-one,
   5-({[4-(benzylamino)-2-chloro-1,6-naphthyridin-8-yl]oxy}methyl)-3-methyl-1,3-oxazolidin-2-one,
   5-[({2-chloro-4-[(furan-2-ylmethyl)amino]quinazolin-8-yl}oxy)methyl]-3-ethyl-1,3-oxazolidin-2-one,
   5-[({2-chloro-4-[(furan-2-ylmethyl)amino]quinazolin-8-yl}oxy)methyl]-3-(propan-2-yl)-1,3-oxazolidin-2-one,
   5-[({2-chloro-4-[(furan-2-ylmethyl)amino]quinazolin-8-yl}oxy)methyl]-3-(2-fluoroethyl)- 1,3-oxazolidin-2-one,
   5-[({2-chloro-4-[(furan-2-ylmethyl)amino]quinazolin-8-yl}oxy)methyl]-3-(cyclopropylmethyl)-1,3-oxazolidin-2-one,
   5-[({2-chloro-4-[(furan-2-ylmethyl)amino]quinazolin-8-yl}oxy)methyl]-3-(2,2-difluoroethyl)-1,3-oxazolidin-2-one,
   5-[({2-chloro-4-[(furan-2-ylmethyl)amino]quinazolin-8-yl}oxy)methyl]-3-(2-hydroxyethyl)-1,3-oxazolidin-2-one,
   5-[({2-chloro-4-[(furan-2-ylmethyl)amino]quinazolin-8-yl}oxy)methyl]-3-(oxetan-3-yl)-1,3-oxazolidin-2-one,
   5-[({2-chloro-4-[(furan-2-ylmethyl)amino]quinazolin-8-yl}oxy)methyl]-3-(2-hydroxy-2-methylpropyl)-1,3-oxazolidin-2-one,
   5-[({2-chloro-4-[(furan-2-ylmethyl)amino]quinazolin-8-yl}oxy)methyl]-N-ethyl-2-oxo-1,3-oxazolidine-3-carboxamide,
   4-[({2-chloro-4-[(furan-2-ylmethyl)amino]quinazolin-8-yl}oxy)methyl]-1-(2-hydroxyethyl)pyrrolidin-2-one,
   4-[({2-chloro-4-[(furan-2-ylmethyl)amino]quinazolin-8-yl}oxy)methyl]-1-(2-fluoroethyl)pyrrolidin-2-one,
   5-[({2-chloro-4-[(furan-2-ylmethyl)amino]quinazolin-8-yl}oxy)methyl]-3-(prop-2-yn-1-yl)-1,3-oxazolidin-2-one,
   tert-butyl[2-(2-{5-[({2-chloro-4-[(furan-2-ylmethyl)amino]quinazolin-8-yl}oxy)methyl]-2-oxo-1,3-oxazolidin-3-yl}ethoxy)ethyl]carbamate,
   5-[({2-chloro-4-[(furan-2-ylmethyl)amino]quinazolin-8-yl}oxy)methyl]-3-[2-(2-methoxyethoxy)ethyl]-1,3-oxazolidin-2-one,
   5-[({2-chloro-4-[(furan-2-ylmethyl)amino]quinazolin-8-yl}oxy)methyl]-3-(oxetan-3-ylmethyl)-1,3-oxazolidin-2-one,
   5-[({2-chloro-4-[(furan-2-ylmethyl)amino]quinazolin-8-yl}oxy)methyl]-3-(1,2,4-oxadiazol-5-ylmethyl)-1,3-oxazolidin-2-one,
   2-{5-[({2-chloro-4-[(furan-2-ylmethyl)amino]quinazolin-8-yl}oxy)methyl]-2-oxo-1,3-oxazolidin-3-yl}acetamide, and
   5-[({2-chloro-4-[(furan-2-ylmethyl)amino]quinazolin-8-yl}oxy)methyl]-3-(2-methoxyethyl)-1,3-oxazolidin-2-one.
[17] A pharmaceutical composition containing, as an active ingredient, the compound according to any one of clauses [1] to [16] or pharmaceutically acceptable salt thereof.
[18] A pharmaceutical composition for treating a genetic disease caused by aberrant splicing, the pharmaceutical composition containing, as an active ingredient, the compound according to any one of clauses [1] to [16] or pharmaceutically acceptable salt thereof.
[19] The pharmaceutical composition according to clause [18],
   wherein the aberrant splicing is caused by an IVS4+9 19G>A mutation in the α-galactosidase gene.
[20] The pharmaceutical composition according to clause [18] or [19],
   wherein the genetic disease caused by aberrant splicing is Fabry disease.
[21] A method for treating a genetic disease caused by aberrant splicing, the method comprising:
   administering the compound according to any one of clauses [1] to [16] or pharmaceutically acceptable salt thereof to a subject.
[22] The method according to clause [21],
   wherein the genetic disease caused by aberrant splicing is Fabry disease.
[23] Use of the compound according to any one of clauses [1] to [16] or pharmaceutically acceptable salt thereof in production of a pharmaceutical composition for use in treatment of a genetic disease caused by aberrant splicing.
[24] The use according to clause [23],
   wherein the genetic disease caused by aberrant splicing is Fabry disease.

### Examples

Hereinafter, the present disclosure will be described in further detail using examples. However, the scope of the present disclosure is not limited to the following examples, and these examples should not be construed in any way as limiting. Reagents, solvents, and starting materials not specifically described herein are readily available from commercial sources.

Proton nuclear magnetic resonance spectra (1H-NMR) were measured using a 400 MHz NMR spectrometer manufactured by JEOL Ltd., a 400 MHz NMR spectrometer manufactured by Varian, or a 400 MHz NMR spectrometer manufactured by Bruker. The notation of the spectral data indicates significant peaks, showing the chemical shift (expressed as relative ppm (δ) using tetramethylsilane as the reference material), the number of protons, and the signal multiplicity (expressed as follows: s: singlet; d: doublet; t: triplet; q: quartet; quint: quintet; m: multiplet; br: broad; br s: broad singlet; etc.). Also, when clearly identifiable, the spin-spin coupling constant was expressed as a J value (in Hz).

Mass spectra (MS m/z) were measured using electrospray ionization (ESI) or atmospheric pressure chemical ionization (APCI). Mass spectral data indicate maximum ionization peaks, which in most cases coincide with maximum UV absorption peaks, after passage through reversed-phase high-performance liquid chromatography columns (Agilent system; columns: Develosil Combi-RP-5, 2.0 × 50 mm, Cadenza CD-C18, 3.0 × 75mm, or ZORBAX SB-C18, 1.8 µm, 2.1 × 50 mm; and solvents: either a 0.1% formic acid-containing acetonitrile/water system or a 0.01% trifluoroacetic acid-containing acetonitrile/water system).

Silica gel column chromatography was performed using a commercially available packed column and an automated preparative purification apparatus (e.g., SP1 manufactured by Biotage, EPCLC-W-Prep 2XY manufactured by Yamazen Corporation, or Purif-α2 manufactured by Shoko Science Co., Ltd.), and only multiple solvent species used as mobile phases are described. Elution was performed under observation by thin layer chromatography (TLC). The TLC plates used were Silica gel 60 F254 or 60 NH2 F254S manufactured by Merck, NH2 Silica Gel 60 F254 plates manufactured by Wako Pure Chemical Industries, Ltd., or CHROMATOREX NH TLC manufactured by Fuji Silysia Chemical Ltd. The developing solvents used were the mobile phases used in the column chromatography. As the detection methods, a UV detector or a color reagent was used. Note that "silica gel column chromatography (NH)" in the following Examples refers to silica gel whose surface is chemically modified with a functional group having an amino group (e.g., Purif-Pack (registered trademark, Shoko Scientific) EX and NH series, etc.).

### (Example 1) 2-Chloro-N-(furan-2-ylmethyl)-8-methoxyquinazolin-4-amine

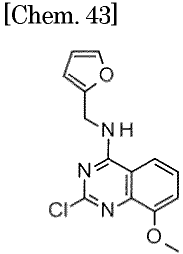

Furfurylamine (44.0 µL, 0.468 mmol) and triethylamine (75.0 µL, 0.534 mmol) were added to a suspension (1.5 mL) of 2,4-dichloro-8-methoxyquinazoline (102 mg, 0.445 mmol) in acetonitrile, followed by stirring at 60°C for 7 hours. After the reaction solution was allowed to cool to room temperature, water (6 mL) was added to the reaction solution, and the solid was collected by filtration. The resulting crudely purified product was washed with water and then dried to obtain the title compound (120 mg, 93% yield) as a white solid.

### (Example 2) 2-Chloro-N-(furan-2-ylmethyl)-8-methoxyquinolin-4-amine

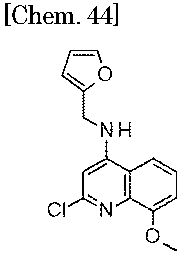

The same operation as in Example 1 was performed using 2,4-dichloro-8-methoxyquinoline (214 mg, 0.938 mmol) disclosed in J. Mol. Structure, 2017, 1131, 51-72, furfurylamine (96.0 µL, 1.03 mmol), and NMP (1.5 mL) instead of DMF as the solvent. Thus, the title compound (82.1 mg, 30% yield) was obtained as a pale brown solid.

### (Example 3) 2-Chloro-7-fluoro-N-(furan-2-ylmethyl)-8-methoxyquinazolin-4-amine

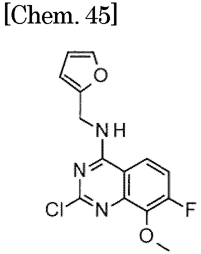

### (Example 3-1) 7-Fluoro-8-methoxyquinazoline-2,4(1H,3H)-dione

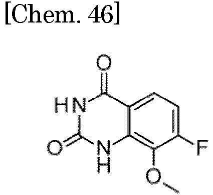

A mixture of 2-amino-3-methoxy-4-fluorobenzoic acid (400 mg, 2.16 mmol), urea (1.00 g, 21.6 mmol), and acetic acid (3 mL) was stirred at 150°C for 0.5 hours. After the reaction solution was allowed to cool to room temperature, water (50 mL) was added to form a suspension. Then, the solid was collected by filtration, washed with water, and then dried to obtain the title compound (320 mg, 70% yield) as a white solid.

### (Example 3-2) 2,4-Dichloro-7-fluoro-8-methoxyquinazoline

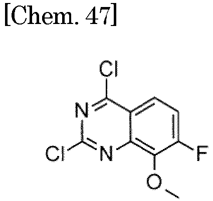

A mixture of 7-fluoro-8-methoxyquinazoline-2,4(1H,3H)-dione (320 mg, 1.53 mmol) obtained in Example 3-1, phosphoryl chloride (4 mL), and N,N-diethylaniline (370 µL, 2.28 mmol) was stirred at 100°C for 3 hours. The reaction solution was allowed to cool to room temperature and then concentrated under reduced pressure. The resulting residue was purified by silica gel column chromatography (ethyl acetate/hexane = 0/100 to 10/90) to obtain the title compound (280 mg, 74% yield) as a white solid.

### (Example 3-3) 2-Chloro-7-fluoro-N-(furan-2-ylmethyl)-8-methoxyquinazolin-4-amine

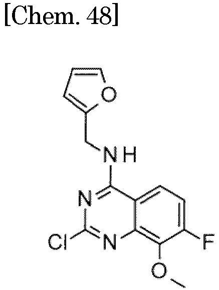

Furfurylamine (0.120 mL, 11.2 mmol) and N,N-diisopropylamine (300 µL, 1.70 mmol) were added to a suspension (4 mL) of 2,4-dichloro-7-fluoro-8-methoxyquinazoline (280 mg, 1.134 mmol) obtained in Example 3-2 in NMP, followed by stirring at 120°C for 1 hour using a microwave. Water (10 mL) was added to the reaction solution, followed by extraction with ethyl acetate. Then, the organic layer was washed with a saturated saline solution, dried over anhydrous sodium sulfate, and then concentrated under reduced pressure. The resulting residue was purified by silica gel column chromatography (ethyl acetate/hexane = 0/100 to 100/0) to obtain the title compound (226 mg, 65% yield) as a white solid.

### (Example 4) 2-Chloro-5-fluoro-N-(furan-2-ylmethyl)-8-methoxyquinazolin-4-amine

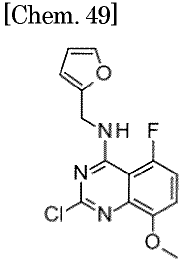

### (Example 4-1) 2-Amino-6-fluoro-3-methoxybenzamide

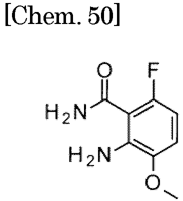

Hydrazine monohydrate was added to a suspension of 6-fluoro-3-methoxy-2-nitrobenzonitrile (3.93 g, 22.0 mmol) disclosed in WO 2009/060209, iron(II) phthalocyanine (62.9 mg, 0.111 mmol), and iron(II) sulfate heptahydrate (29.1 mg, 0.105 mmol) in ethanol (60 mL)/water (60 mL), followed by stirring at 120°C for 8.5 hours. After the reaction solution was allowed to cool to room temperature, a saturated saline solution (100 mL) was added to the reaction solution, followed by extraction with ethyl acetate. Then, the organic layer was washed with a saturated saline solution, dried over magnesium sulfate, and then concentrated under reduced pressure. The resulting residue was purified by silica gel column chromatography (ethyl acetate/hexane = 0/100 to 40/60) to obtain the title compound (2.04 g, 55% yield) as a pale yellow solid.

### (Example 4-2) 5-Fluoro-8-methoxyquinazoline-2,4(1H,3H)-dione

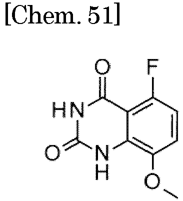

Potassium carbonate (3.39 g, 24.5 mmol) and 1,1'-carbonyldiimidazole (3.66 g, 22.6 mmol) were added to a solution (30 mL) of 2-amino-6-fluoro-3-methoxybenzamide (2.04 g, 11.1 mmol) obtained in Example 4-1 in DMF, followed by stirring at 90°C for 4 hours. Water (200 mL) was added to the reaction solution, and the mixture was washed with ethyl acetate. Then, the aqueous layer was neutralized with 2M hydrochloric acid while being stirred under ice-cooling. The precipitated solid was collected by filtration. The resulting solid was washed with water and then dried to obtain the title compound (1.67 g, 72% yield) as a white solid.

### (Example 4-3) 2,4-Dichloro-5-fluoro-8-methoxyquinazoline

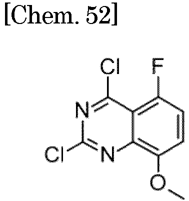

A mixture of 5-fluoro-8-methoxyquinazoline-2,4(1H,3H)-dione (156 mg, 0.742 mmol) obtained in Example 4-2, phosphoryl chloride (855 mg, 5.58 mmol), and N,N-diethylaniline (238 µL, 1.48 mmol) was stirred at 100°C for 8 hours. During the course of the reaction, N,N-diethylaniline was added at 3 hours (60.0 µL, 0.371 mmol) and 5.5 hours (25.0 µL, 0.148 mmol) after the start of the reaction. The reaction solution was allowed to cool to room temperature and then concentrated under reduced pressure. Toluene was added to the resulting residue, and the mixture was concentrated again under reduced pressure. Chloroform (3 mL) was added to the resulting residue, and a saturated aqueous solution of sodium bicarbonate was added in small portions to the resulting suspension under stirring until the pH reached 5. After extraction with chloroform, the mixture was concentrated under reduced pressure. The resulting residue was purified by silica gel column chromatography (ethyl acetate/hexane = 1/100 to 20/80) to obtain the title compound (155 mg, 85% yield) as a pale yellow solid.

### (Example 4-4) 2-Chloro-5-fluoro-N-(furan-2-ylmethyl)-8-methoxyquinazolin-4-amine

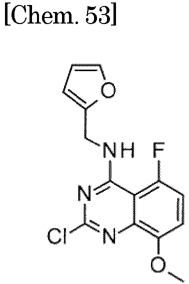

Furfurylamine (32.0 µL, 0.347 mmol) and triethylamine (61.0 µL, 0.434 mmol) were added to a solution of 2,4-dichloro-5-fluoro-8-methoxyquinazoline (71.4 mg, 0.289 mmol) obtained in Example 4-3 in acetonitrile (1.5 mL), followed by stirring at 60°C for 2 hours. After the reaction solution was allowed to cool to room temperature, water (4 mL) was added, and the insoluble material was collected by filtration, washed with water, and dried to obtain a crudely purified product. The resulting crudely purified product was purified by silica gel column chromatography (ethyl acetate/hexane = 0/100 to 35/65) to obtain the title compound (62.6 mg, 70% yield) as a white solid.

### (Example 5) 2-Bromo-N-(furan-2-ylmethyl)-8-methoxyquinazolin-4-amine

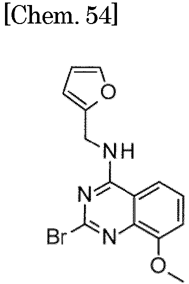

Furfurylamine (64.0 µL, 0.692 mmol) and N,N-diisopropylethylamine (160 µL, 0.943 mmol) were added to a solution (10 mL) of 2,4-dibromo-8-methoxyquinazoline (200 mg, 0.629 mmol) in NMP, followed by stirring at 120°C for 0.5 hours. After the reaction solution was allowed to cool to room temperature, water was added, followed by extraction with ethyl acetate. The organic layer was washed with a saturated saline solution, then dried over anhydrous sodium sulfate, and concentrated under reduced pressure. The resulting residue was purified by silica gel column chromatography (ethyl acetate/hexane = 0/100 to 100/0) to obtain the title compound (168 mg, 80% yield) as a white solid.

### (Example 6) 2-Chloro-N-(furan-2-ylmethyl)-8-methoxypyrido[3,4-d]pyrimidin-4-amine

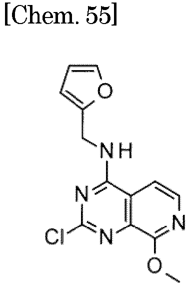

Phosphoryl chloride (194 µL, 2.09 mmol) was added to a solution (5 mL) of 8-methoxypyrido[3,4-d]pyrimidine-2,4(1H,3H)-dione (200 mg, 1.04 mmol) disclosed in Bioorg. Med. Chem. Lett., 2013, 23, 5923-5930 and N,N-diisopropylethylamine (404 µL, 2.32 mmol) in toluene, followed by stirring at 80°C for 2 hours. The reaction solution was concentrated under reduced pressure to obtain a yellow oily substance. THF (5 mL) was added thereto to form a solution. Then, furfurylamine (114 µL, 1.24 mmol) was added to the solution, followed by stirring at room temperature for 3 hours. The reaction solution was concentrated, and the resulting residue was purified by preparative HPLC (column: Phenomenex Synergi C18, 150 × 25 mm × 10 µm; elution solvent: 0.225% aqueous formic acid/acetonitrile = 79/21 to 49/51) to obtain the title compound (25.1 mg, 8% yield) as a yellow solid.

### (Example 7) 2-Chloro-N-(furan-2-ylmethyl)-8-methoxypyrido[4,3-d]pyrimidin-4-amine

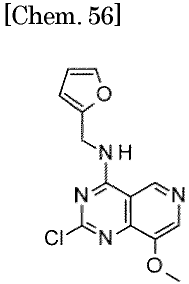

### (Example 7-1) 4-Amino-5-methoxypyridine-3-carboxamide

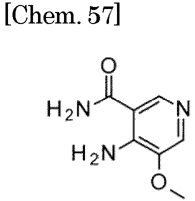

A 30% hydrogen peroxide solution (10 mL, 104 mmol) and potassium carbonate (1.70 g, 12.3 mmol) were added to a solution (10 mL) of 4-amino-5-methoxypyridine-3-carbonitrile (900 mg, 6.03 mmol) in DMSO under ice-cooling. Then, the mixture was stirred at room temperature for 12 hours. Water (50 mL) was added to the reaction solution, followed by extraction with ethyl acetate. Then, the organic layer was dried over anhydrous sodium sulfate and concentrated under reduced pressure. The remaining aqueous layer was neutralized with 3M hydrochloric acid and concentrated under reduced pressure. Methanol was added to the resulting white solid, and the solid was removed by filtration. Then, the methanol was concentrated under reduced pressure. The residue obtained by concentrating the organic layer was combined with the residue obtained by concentrating the methanol. The mixture was then purified by silica gel column chromatography (methanol/DCM = 1/40 to 1/10) to obtain the title compound (720 mg, 72% yield) as a white solid.

### (Example 7-2) 8-Methoxypyrido[4,3-d]pyrimidine-2,4(1H,3H)-dione

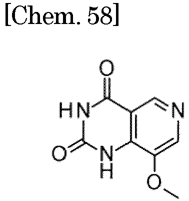

1M lithium bis(trimethylsilyDamide-THF (8.40 mL, 8.40 mmol) was added, under ice-cooling, to a solution (10 mL) of 4-amino-5-methoxypyridine-3-carboxamide (480 mg, 2.87 mmol) obtained in Example 7-1 in 1,4-dioxane, followed by stirring at 15°C for 1 hour. Then, a solution (3 mL) of triphosgene (1.20 g, 4.04 mmol) in 1,4-dioxane was added to the reaction solution under ice-cooling, followed by stirring at 0°C to 15°C for 12 hours. The reaction solution was concentrated under reduced pressure, and the resulting residue was purified by preparative HPLC (column: Shim-pack C18, 150 × 25 mm × 10 µm; elution solvent: 0.1% aqueous trifluoroacetic acid/acetonitrile = 99/1 to 75/25) to obtain the title compound (125 mg, 23% yield) as a white solid.

### (Example 7-3) 2-Chloro-N-(furan-2-ylmethyl)-8-methoxypyrido[4,3-d]pyrimidin-4-amine

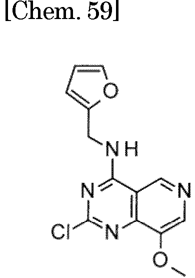

A mixture of 8-methoxypyrido[4,3-d]pyrimidine-2,4(1H,3H)-dione (100 mg, 0.518 mmol) obtained in Example 7-2, phosphoryl chloride (10.0 mL, 108 mmol), and N,N-diisopropylethylamine (0.900 mL, 5.17 mmol) was stirred at 100°C for 1 hour. The reaction solution was concentrated under reduced pressure. Then, the operation of adding toluene to the resulting residue and concentrating the mixture under reduced pressure was repeated three times. Furfurylamine (51.9 µL, 0.566 mmol) and N,N-diisopropylethylamine (333 µL, 1.91 mmol) were added to a solution (5 mL) of the resulting residue in THF, followed by stirring at 15°C for 1 hour. The reaction solution was then concentrated under reduced pressure, water (10 mL) was added to the residue, followed by extraction with ethyl acetate. Then, the organic layer was washed with a saturated saline solution, dried over anhydrous sodium oxalate, and then concentrated under reduced pressure. The resulting residue was purified by preparative HPLC (column: Phenomenex Synergi C18, 150 × 25 mm × 10 µm; elution solvent: 0.225% aqueous formic acid/acetonitrile = 79/21 to 49/51) to obtain the title compound (28.0 mg, 19% yield) as a yellow solid.

### (Example 8) 2-Chloro-N-(furan-2-ylmethyl)-8-methoxypyrido[3,2-d]pyrimidin-4-amine

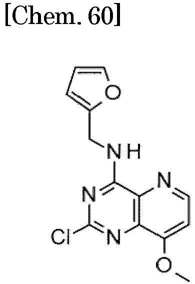

Furfurylamine (56.6 µL, 0.618 mmol) and N,N-diisopropylethylamine (189 µL, 1.08 mmol) were added to a solution (5 mL) of 2,4-dichloro-8-methoxy-pyrido[3,2-d]pyrimidine (119 mg, 0.517 mmol) disclosed in WO 2020/081636 in THF, followed by stirring at room temperature for 4 hours. The reaction solution was filtered, and the filtrate was then concentrated under reduced pressure. The resulting residue was purified by preparative HPLC (column: Phenomenex Synergi C18, 150 × 25 mm × 10 µm; elution solvent: 0.225% aqueous formic acid/acetonitrile = 62/38 to 32/68) to obtain the title compound (30.3 mg, 20% yield) as a yellow solid.

Structural formulae and physical scientific data of the compounds described in Examples 1 to 8 are shown below.

**[Table 1]**

| Example | Structural formulae | Data |
|---|---|---|
| 1 | 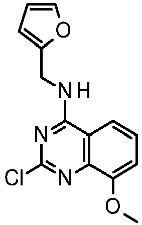 | 1H-NMR (DMSO-D6) δ: 3.90 (3H, s), 4.72 (2H, d, J = 6.1 Hz), 6.35 (1H, d, J = 3.6 Hz), 6.41-6.43 (1H, m), 7.31 (1H, d, J = 7.9 Hz), 7.46 (1H, t, J = 7.9 Hz), 7.61 (1H, br s), 7.80 (1H, d, J = 8.5 Hz), 9.11 (1H, t, J = 6.1 Hz). |
| | | MS (m/z): 290 (M+H)⁺. |
| 2 | 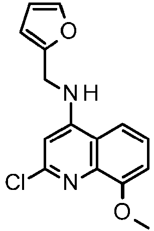 | 1H-NMR (DMSO-D6) δ: 3.89 (3H, s), 4.54 (2H, d, J = 5.5 Hz), 6.39 (1H, d, J = 3.0 Hz), 6.41 (1H, dd, J = 3.0, 1.8 Hz), 6.55 (1H, s), 7.15 (1H, d, J = 7.3 Hz), 7.39 (1H, t, J = 8.2 Hz), 7.60-7.62 (1H, m), 7.75 (1H, d, J = 7.9 Hz), 7.98 (1H, t, J = 5.5 Hz). |
| | | MS (m/z): 289 (M+H)⁺. |
| 3-1 | 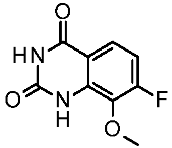 | 1H-NMR (DMSO-D6) δ: 3.87 (3H, d, J = 1.2 Hz), 7.10 (1H, dd, J = 11.0, 8.6 Hz), 7.66 (1H, dd, J = 9.2, 5.5 Hz), 11.00 (1H, s), 11.38 (1H, s). |
| 3-2 | 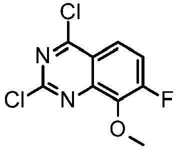 | 1H-NMR (DMSO-D6) δ: 4.12 (3H, d, J = 1.2 Hz), 7.88 (1H, dd, J = 11.0, 9.5 Hz), 8.10 (1H, dd, J = 9.5, 5.2 Hz). |
| 3-3 | 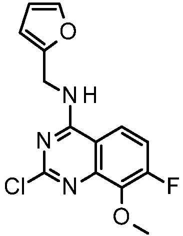 | 1H-NMR (DMSO-D6) δ: 4.00 (3H, s), 4.73 (2H, d, J = 5.5 Hz), 6.37 (1H, dd, J = 3.1, 1.2 Hz), 6.43 (1H, dd, J = 3.1, 1.8 Hz), 7.52 (1H, dd, J = 11.0, 9.2 Hz), 7.62 (1H, d, J = 1.8 Hz), 8.08 (1H, dd, J = 9.2, 5.5 Hz), 9.28 (1H, t, J = 5.5 Hz). |
| | | MS (m/z): 308 (M+H)⁺. |
| 4-1 | 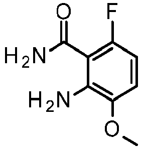 | 1H-NMR (DMSO-D6) δ: 3.77 (3H, s), 5.88 (2H, br s), 6.31 (1H, dd, J = 11.2, 9.1 Hz), 6.82 (1H, dd, J = 9.1, 4.9 Hz), 7.47 (1H, br s), 7.58 (1H, br s). |
| 4-2 | 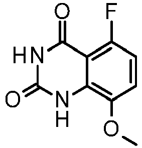 | 1H-NMR (DMSO-D6) δ: 3.84 (3H, s), 6.89 (1H, dd, J = 11.2, 8.8 Hz), 7.25 (1H, dd, J = 8.8, 4.3 Hz), 10.58 (1H, s), 11.29 (1H, s). |
| 4-3 | 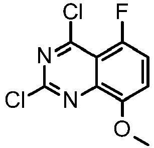 | 1H-NMR (DMSO-D6) δ: 3.99 (3H, s), 7.58 (1H, dd, J = 8.8, 4.3 Hz), 7.65 (1H, dd, J = 11.2, 8.8 Hz). |
| 4-4 | 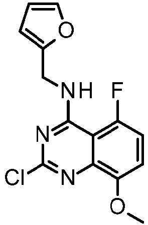 | 1H-NMR (DMSO-D6) δ: 3.88 (3H, s), 4.73 (2H, d, J = 6.1 Hz), 6.33 (1H, d, J = 3.6 Hz), 6.41 (1H, dd, J = 3.0, 1.8 Hz), 7.29-7.33 (2H, m), 7.59 (1H, br s), 8.46-8.51 (1H, m). |
| | | MS (m/z): 308 (M+H)⁺. |
| 5 | 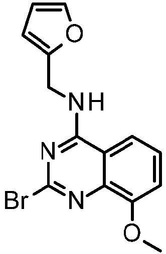 | 1H-NMR (DMSO-D6) δ: 3.90 (3H, s), 4.71 (2H, d, J = 6.7 Hz), 6.35-6.36 (1H, m), 6.41-6.43 (1H, m), 7.30 (1H, dd, J = 8.3, 1.2 Hz), 7.47 (1H, td, J = 8.3, 3.7 Hz), 7.61 (1H, d, J = 1.8 Hz), 7.78-7.82 (1H, m), 9.11 (1H, br s). |
| | | MS (m/z): 334 (M), 336 (M+2). |
| 6 | 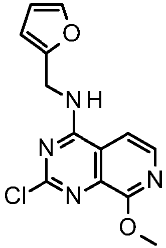 | 1H-NMR (DMSO-D6) δ: 4.00 (3H, s), 4.73 (2H, d, J = 5.5 Hz), 6.39 (1H, d, J = 3.2 Hz), 6.43 (1H, dd, J = 3.2, 1.8 Hz), 7.62-7.63 (1H, m), 7.71 (1H, d, J = 6.1 Hz), 8.12 (1H, d, J = 6.1 Hz), 9.37 (1H, t, J = 5.5 Hz). |
| | | MS (m/z): 291 (M+H)⁺. |
| 7-1 | 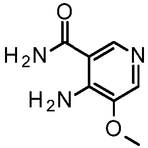 | 1H-NMR (DMSO-D6) δ: 3.85 (3H, s), 6.99 (2H, br s), 7.29 (1H, br s), 7.91 (2H, s), 8.33 (1H, s). |
| 7-2 | 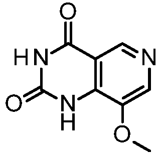 | 1H-NMR (DMSO-D6) δ: 3.97 (3H, s), 8.41 (1H, s), 8.63 (1H, s), 11.22 (1H, br s), 11.55 (1H, br s). |
| 7-3 | 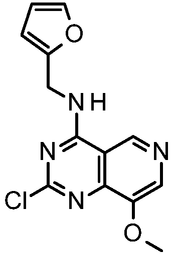 | 1H-NMR (DMSO-D6) δ: 3.99 (3H, s), 4.76 (1H, d, J = 5.5 Hz), 6.41 (1H, d, J = 3.0 Hz), 6.43-6.45 (1H, m), 7.63 (2H, d, J = 1.8 Hz), 8.49 (1H, s), 9.21 (1H, s), 9.67 (1H, t, J = 5.5 Hz). |
| | | MS (m/z): 291 (M+H)⁺. |
| 8 | 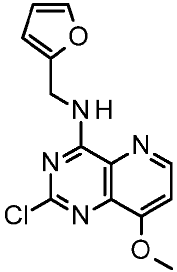 | 1H-NMR (DMSO-D6) δ: 4.00 (3H, s), 4.68 (2H, d, J = 6.1 Hz), 6.31 (1H, d, J = 2.4 Hz), 6.40 (1H, dd, J = 3.0, 2.4 Hz), 7.36 (1H, d, J = 5.5 Hz), 7.58-7.59 (1H, m), 8.64 (1H, d, J = 5.5 Hz), 9.28 (1H, t, J = 6.1 Hz). |
| | | MS (m/z): 291 (M+H)⁺. |

Compounds of Examples 9 to 17 shown below were produced in accordance with the steps described in Examples 1 to 4 above.

**[Table 2]**

| Example | Structural formulae | Data |
|---|---|---|
| 9-1 | 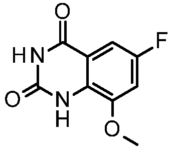 | 1H-NMR (DMSO-D6) δ: 3.90 (3H, s), 7.15 (1H, dd, J =8.6, 3.1 Hz), 7.29 (1H, dd, J= 10.4, 3.1 Hz), 10.64 (1H, s), 11.43 (1H, s). |
| 9-2 | 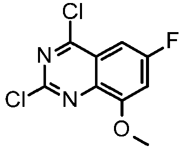 | 1H-NMR (DMSO-D6) δ: 4.05 (3H, s), 7.56 (1H, dd, J =8.6, 2.5 Hz), 7.64 (1H, dd, J = 11.0, 2.5 Hz). |
| 9-3 | 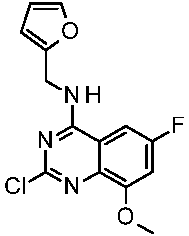 | 1H-NMR (DMSO-D6) δ: 3.93 (3H, s), 4.71 (2H, d, J = 5.5 Hz), 6.38 (1H, d, J = 4.3 Hz), 6.43 (1H, dd, J = 3.1, 1.8 Hz), 7.29 (1H, dd, J = 11.0, 3.1 Hz), 7.62 (1H, t, J= 1.5 Hz), 7.67 (1H, dd, J = 9.8, 3.1 Hz), 9.03 (1H, t, J = 5.5 Hz). |
| | | MS (m/z): 308 (M+H)⁺. |
| 10 | 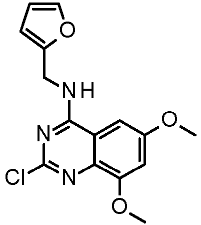 | 1H-NMR (DMSO-D6) δ: 3.86 (3H, s), 3.88 (3H, s), 4.72 (2H, d, J= 4.9 Hz), 6.37 (1H, dd, J = 3.1, 1.2 Hz), 6.43 (1H, dd, J = 3.1, 1.8 Hz), 6.90 (1H, d, J = 2.5 Hz), 7.25 (1H, d, J = 2.5 Hz), 7.62 (1H, dd, J = 1.8, 0.6 Hz), 8.91 (1H, t, J = 4.9 Hz). |
| | | MS (m/z): 320 (M+H)⁺. |
| 11-1 | 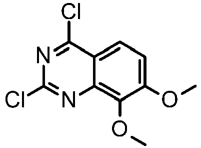 | 1H-NMR (DMSO-D6) δ: 3.93 (3H, s), 4.06 (3H, s), 7.81 (1H, d, J = 9.7 Hz), 8.09 (1H, d, J = 9.7 Hz). |
| 11-2 | 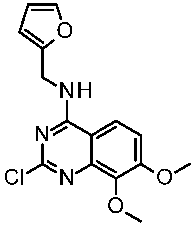 | 1H-NMR (DMSO-D6) δ: 3.88 (3H, s), 3.94 (3H, s), 4.71 (2H, d, J = 5.5 Hz), 6.35 (1H, d, J = 3.6 Hz), 6.42 (1H, dd, J = 3.0, 1.8 Hz), 7.40 (1H, d, J = 9.1 Hz), 7.61 (1H, d, J = 1.8 Hz), 8.06 (1H, d, J = 9.1 Hz), 9.09 (1H, t, J = 5.5 Hz). |
| | | MS (m/z): 320 (M+H)⁺. |
| 12 | 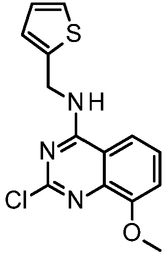 | 1H-NMR (DMSO-D6) δ: 3.90 (3H, s), 4.87 (2H, d, J = 5.8 Hz), 6.98 (1H, dd, J = 4.9, 3.6 Hz), 7.10 (1H, d, J = 3.6 Hz), 7.31 (1H, d, J = 7.9 Hz), 7.40 (1H, t, J = 2.4 Hz), 7.47 (1H, t, J = 8.2 Hz), 7.76 (1H, d, J = 8.5 Hz), 9.28 (1H, t, J = 5.8 Hz). |
| | | MS (m/z): 306 (M+H)⁺. |
| 13 | 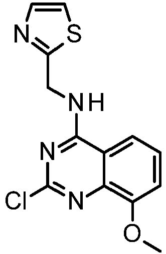 | 1H-NMR (DMSO-D6) δ: 3.91 (3H, s), 5.00 (2H, br d, J = 5.5 Hz), 7.34 (1H, d, J = 7.9 Hz), 7.49-7.53 (1H, m), 7.63-7.65 (1H, m), 7.75-7.76 (1H, m), 7.80 (1H, d, J = 8.5 Hz), 9.50 (1H, br t, J = 5.5 Hz). |
| | | MS (m/z): 307 (M+H)⁺. |
| 14 | 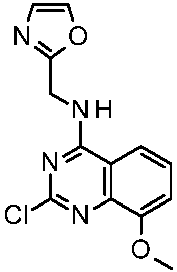 | 1H-NMR (DMSO-D6) δ: 3.91 (3H, s), 4.84 (2H, d, J = 5.5 Hz), 7.17 (1H, s), 7.34 (1H, d, J = 7.3 Hz), 7.50 (1H, t, J = 8.5 Hz), 7.81 (1H, dd, J = 8.5, 1.2 Hz), 8.07 (1H, s), 9.28 (1H, t, J = 5.5 Hz). |
| | | MS (m/z): 291 (M+H)⁺. |
| 15 | 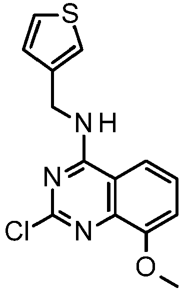 | 1H-NMR (DMSO-D6) δ: 3.90 (3H, s), 4.72 (2H, d, J = 5.5 Hz), 7.14 (1H, dd, J= 5.5, 1.2 Hz), 7.30 (1H, d, J = 7.3 Hz), 7.38 (1H, br s), 7.46 (1H, t, J = 8.2 Hz), 7.50 (1H, dd, J = 4.9, 3.0 Hz), 7.80 (1H, d, J = 7.3 Hz), 9.12 (1H, t, J = 5.5 Hz). |
| | | MS (m/z): 306 (M+H)⁺. |
| 16 | 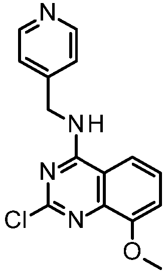 | 1H-NMR (DMSO-D6) δ: 3.90 (3H, s), 4.75 (2H, d, J = 6.0 Hz), 7.32-7.34 (3H, m), 7.50 (1H, t, J = 8.0 Hz), 7.82 (1H, d, J = 7.6 Hz), 8.49-8.51 (2H, m), 9.21 (1H, t, J = 6.0 Hz). |
| | | MS (m/z): 301 (M+H)⁺. |
| 17 | 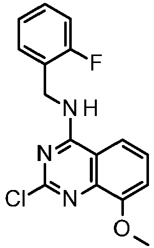 | 1H-NMR (DMSO-D6) δ: 3.90 (3H, s), 4.77 (2H, d, J = 5.5 Hz), 7.14-7.24 (2H, m), 7.31-7.41 (3H, m), 7.48 (1H, t, J = 8.2 Hz), 7.85 (1H, dd, J = 8.5, 1.2 Hz), 9.14 (1H, t, J = 5.5 Hz). |
| | | MS (m/z): 318 (M+H)⁺. |

### (Example 18) 2-Chloro-8-(1,4-dioxan-2-ylmethoxy)-N-(furan-2-ylmethyl)quinazolin-4-amine

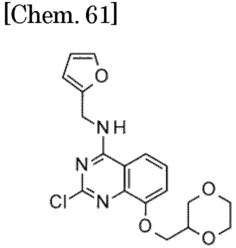

### (Example 18-1) 2,4-Dichloroquinazolin-8-ol

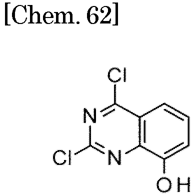

Under ice-cooling, a solution (90 mL) of 2,4-dichloro-8-methoxyquinazoline (8.71 g, 38.0 mmol) in dichloromethane was added to a suspension (120 mL) of aluminum chloride (30.4 mg, 228 mmol) in dichloromethane, and the mixture was stirred for 30 minutes. Then, the mixture was stirred at room temperature for 6 hours. The reaction solution was poured into ice in a beaker and quenched. Then, concentrated hydrochloric acid (20 mL) was added, and the mixture was stirred. The reaction solution was extracted with dichloromethane. The organic layer was washed with a saturated saline solution, then dried over anhydrous sodium sulfate, and concentrated under reduced pressure. The resulting residue was purified by silica gel column chromatography (ethyl acetate/hexane = 10% to 20%), and the resulting crudely purified product was then purified again by column chromatography (ethyl acetate/hexane = 2% to 15%) to obtain the title compound (5.02 g, 62% yield) as a pale yellow solid.

### (Example 18-2) 2-Chloro-4-[(furan-2-ylmethyl)amino]quinazolin-8-ol

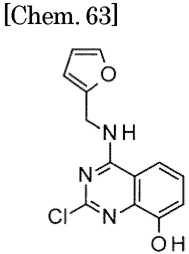

Furfurylamine (0.478 mL, 5.17 mmol) and triethylamine (1.30 mL, 9.39 mmol) were added to a mixture of 2,4-dichloroquinazolin-8-ol (1.01 g, 4.70 mmol) obtained in Example 18-1 and acetonitrile (25 mL), followed by stirring at 60°C for 2 hours. After the reaction solution was allowed to cool to room temperature, water was added, followed by extraction with ethyl acetate. The organic layer was washed with a saturated saline solution and then concentrated under reduced pressure. The resulting residue was purified by silica gel column chromatography (ethyl acetate/hexane = 0/100 to 25/75) to obtain the title compound (1.14 g, 88% yield) as a yellow solid.

### (Example 18-3) 2-Chloro-8-(1,4-dioxan-2-ylmethoxy)-N-(furan-2-ylmethyl)quinazolin-4-amine

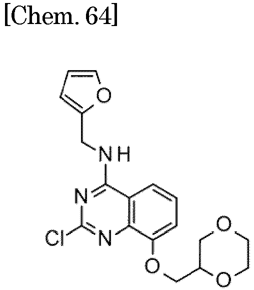

(1,4-Dioxan-2-yl)methanol (86.4 µL, 0.816 mmol) and cyanomethylenetributylphosphorane (429 µL, 1.63 mmol) were added to a solution (5 mL) of 2-chloro-4-[(furan-2-ylmethyl)amino]quinazolin-8-ol (150 mg, 0.544 mmol) obtained in Example 18-2 in toluene, followed by stirring at 80°C for 2 hours. The reaction solution was allowed to cool to room temperature and then concentrated under reduced pressure. The resulting residue was purified by silica gel column chromatography (ethyl acetate/hexane = 34/66 to 66/34) to obtain the title compound (87.1 mg, 43% yield) as a white solid.

### (Example 19) 5-[({2-Chloro-4-[(furan-2-ylmethyl)amino]quinazolin-8-yl}oxy)methyl]-1,3-oxazolidin-2-one

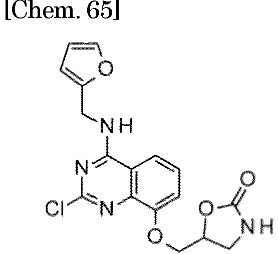

5-(Hydroxymethyl)-1,3-oxazolidin-2-one (32.5 mg, 0.277 mmol) and cyanomethylenetributylphosphorane (73.0 µL, 0.277 mmol) were added to a solution (1.5 mL) of 2-chloro-4-[(furan-2-ylmethyl)amino]quinazolin-8-ol (38.2 mg, 0.139 mmol) obtained in Example 18-2 in toluene, followed by stirring at 80°C for 4 hours. The reaction solution was allowed to cool to room temperature and then concentrated under reduced pressure. The residue was purified by silica gel column chromatography (ethyl acetate/hexane = 20/80 to 100/0) to obtain the title compound (42.4 mg, 82% yield) as a white solid.

### (Example 20) (5R)-5-[({2-Chloro-4-[(furan-2-ylmethyl)amino]quinazolin-8-yl}oxy)methyl]-1,3-oxazolidin-2-one

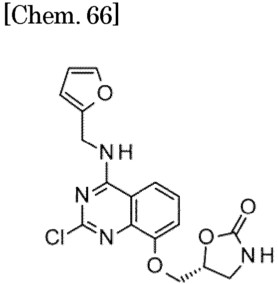

(5R)-5-(Hydroxymethyl)-1,3-oxazolidin-2-one (44.1 mg, 0.376 mmol) and cyanomethylenetributylphosphorane (123 µL, 0.470 mmol) were added to a solution (1 mL) of 2-chloro-4-[(furan-2-ylmethyl)amino]quinazolin-8-ol (66.1 mg, 0.240 mmol) obtained in Example 18-2 in toluene, followed by stirring at 80°C for 2 hours. The reaction solution was allowed to cool to room temperature and then concentrated under reduced pressure. The residue was purified by silica gel column chromatography (ethyl acetate/hexane = 20/80 to 100/0). The obtained solid was suspended in a solvent mixture of ethyl acetate/hexane (50/50) to form a suspension, from which the solid was then collected by filtration and dried to obtain the title compound (48.0 mg, 55% yield) as a white solid.

### (Example 21) (5S)-5-[({2-Chloro-4-[(furan-2-ylmethyl)amino]quinazolin-8-yl}oxy)methyl]-1,3-oxazolidin-2-one

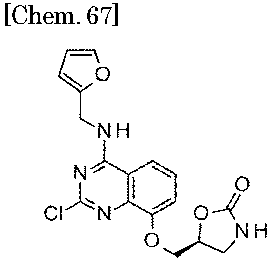

(5S)-5-(Hydroxymethyl)-1,3-oxazolidin-2-one (46.5 mg, 0.397 mmol) and cyanomethylenetributylphosphorane (126 µL, 0.480 mmol) were added to a solution (1 mL) of 2-chloro-4-[(furan-2-ylmethyl)amino]quinazolin-8-ol (66.1 mg, 0.240 mmol) obtained in Example 18-2 in toluene, followed by stirring at 80°C for 2 hours. The reaction solution was allowed to cool to room temperature and then concentrated under reduced pressure. The residue was purified by silica gel column chromatography (ethyl acetate/hexane = 20/80 to 100/0). The obtained solid was suspended in a solvent mixture of ethyl acetate/hexane (50/50) to form a suspension, from which the solid was then collected by filtration and dried to obtain the title compound (59.5 mg, 66% yield) as a white solid.

### (Example 22) 1-{3-[({2-Chloro-4-[(furan-2-ylmethyl)amino]quinazolin-8-yl}oxy)methyl]pyrrolidin-1-yl}ethanone

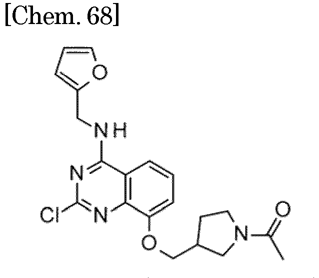

1-[3-(Hydroxymethyl)pyrrolidin-1-yl]ethan-1-one (46.2 mg, 0.323 mmol and cyanomethylenetributylphosphorane (113 µL, 0.430 mmol) were added to a solution (1 mL) of 2-chloro-4-[(furan-2-ylmethyl)amino]quinazolin-8-ol (59.3 mg, 0.215 mmol) obtained in Example 18-2 in toluene, followed by stirring at 80°C for 1 hour. The reaction solution was allowed to cool to room temperature and then concentrated under reduced pressure. The residue was purified by silica gel column chromatography (methanol/ethyl acetate = 0/100 to 10/90) to obtain the title compound (84.4 mg, 98% yield) as a yellow solid.

### (Example 23) 2-Chloro-N-(furan-2-ylmethyl)-8-{[1-(methylsulfonyl)pyrrolidin-3-yl]methoxy}quinazolin-4-amine

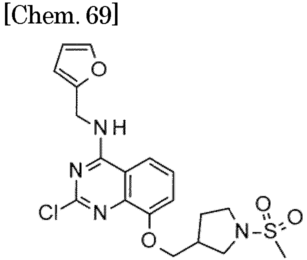

(1-Methylsulfonylpyrrolidin-3-yl)methanol (40.9 mg, 0.228 mmol) and cyanomethylenetributylphosphorane (58.0 µL, 0.222 mmol) were added to a solution (1 mL) of 2-chloro-4-[(furan-2-ylmethyl)amino]quinazolin-8-ol (40.8 mg, 0.148 mmol) obtained in Example 18-2 in toluene, followed by stirring at 80°C for 1 hour. The reaction solution was allowed to cool to room temperature and then concentrated under reduced pressure. The residue was crudely purified by silica gel column chromatography (NH, ethyl acetate/hexane = 30/70 to 75/25), and the resulting crudely purified product was then purified by silica gel column chromatography (ethyl acetate/hexane = 30/70 to 75/25) to obtain the title compound (50.7 mg, 78% yield) as a yellow solid.

### (Example 24) 2-Chloro-8-[(2,2-dimethyl-1,3-dioxan-5-yl)methoxy]-N-(furan-2-ylmethyl)quinazolin-4-amine

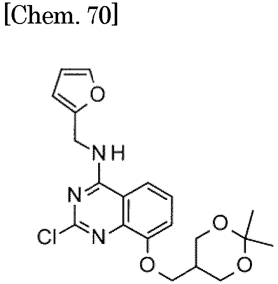

(2,2-Dimethyl-1,3-dioxan-5-yl)methanol (16.1 mg, 0.110 mmol) and cyanomethylenetributylphosphorane (46.0 µL, 0.162 mmol) were added to a solution (0.5 mL) of 2-chloro-4-[(furan-2-ylmethyl)amino]quinazolin-8-ol (22.3 mg, 0.0809 mmol) obtained in Example 18-2 in toluene, followed by stirring at 80°C for 4.5 hours. The reaction solution was allowed to cool to room temperature and then concentrated under reduced pressure. The residue was purified by silica gel column chromatography (ethyl acetate/hexane = 0/100 to 40/60) to obtain the title compound (32.7 mg, 100% yield) as a pale yellow solid.

### (Example 25) 5-[({2-Chloro-4-[(furan-2-ylmethyl)amino]quinazolin-8-yl}oxy)methyl]pyrrolidin-2-one

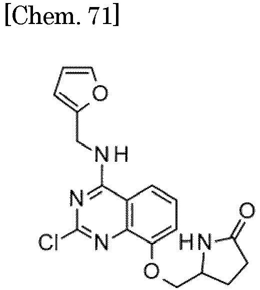

5-(Hydroxymethyl)pyrrolidin-2-one (33.0 mg, 0.287 mmol) and cyanomethylenetributylphosphorane (96.0 µL, 0.365 mmol) were added to a solution (1 mL) of 2-chloro-4-[(furan-2-ylmethyl)amino]quinazolin-8-ol (50.3 mg, 0.182 mmol) obtained in Example 18-2 in toluene, followed by stirring at 80°C for 1.5 hours. After the reaction solution was allowed to cool to room temperature, the precipitated solid was collected by filtration. The obtained solid was washed with a solvent mixture of ethyl acetate/hexane (50/50) to obtain the title compound (55.4 mg, 81% yield) as a pale brown solid.

### (Example 26) 2-Chloro-8-[(2,2-difluorocyclobutyl)methoxy]-N-(furan-2-ylmethyl)quinazolin-4-amine

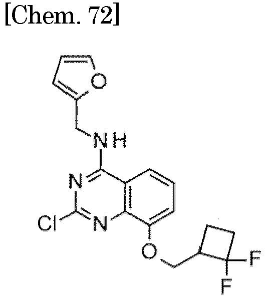

(2,2-Difluorocyclobutyl)methanol (20.0 mg, 0.160 mmol) and cyanomethylenetributylphosphorane (76.0 µL, 0.290 mmol) were added to a solution (1 mL) of 2-chloro-4-[(furan-2-ylmethyl)amino]quinazolin-8-ol (40.0 mg, 0.145 mmol) obtained in Example 18-2 in toluene, followed by stirring at 80°C for 0.5 hours. The reaction solution was allowed to cool to room temperature and then concentrated under reduced pressure. The residue was purified by silica gel column chromatography (ethyl acetate/hexane = 0/100 to 30/70) to obtain the title compound (51.0 mg, 93% yield) as a white solid.

### (Example 27) 2-Chloro-8-(1,4-dioxaspiro[4.4]nonan-7-ylmethoxy)-N-(furan-2-ylmethyl)quinazolin-4-amine

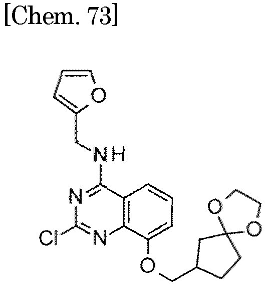

1,4-Dioxaspiro[4.4]nonan-8-ylmethanol (90.0 mg, 0.569 mmol) and cyanomethylenetributylphosphorane (220 mg, 0.912 mmol) were added to a solution (5 mL) of 2-chloro-4-[(furan-2-ylmethyl)amino]quinazolin-8-ol (100 mg, 0.363 mmol) obtained in Example 18-2 in toluene, followed by stirring at 80°C for 12 hours. The reaction solution was allowed to cool to room temperature and then concentrated under reduced pressure. The resulting residue was purified by preparative HPLC (column: Waters XBridge, 150 × 25 mm × 5 µm; elution solvent: 0.05% aqueous ammonia/acetonitrile = 57/43 to 27/73) to obtain the title compound (97.0 mg, 64% yield) as a white solid.

### (Example 28) 3-[({2-Chloro-4-[(furan-2-ylmethyl)amino]quinazolin-8-yl}oxy)methyl]cyclopentanone

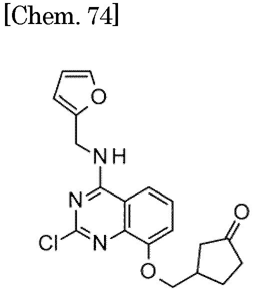

p-Toluenesulfonic acid (32.0 mg, 0.186 mmol) was added to a solution of 2-chloro-8-(1,4-dioxaspiro[4.4lnonan-7-ylmethoxy)-N-(furan-2-ylmethyl) quinazolin-4-amine (75.0 mg, 0.180 mmol) obtained in Example 27 in acetone (5 mL)/water (2 mL), followed by stirring at room temperature for 1 hour. After the acetone was distilled off under reduced pressure, water (20 mL) was added to the residue, followed by extraction with ethyl acetate. The organic layer was then washed with a saturated saline solution, dried over anhydrous sodium sulfate, and then concentrated under reduced pressure. The resulting residue was crudely purified by preparative HPLC (column: Shim-pack C18, 150 × 25 mm × 10 µm; elution solvent: 0.225% aqueous formic acid/acetonitrile = 67/33 to 37/63), followed by preparative HPLC purification (column: Waters XBridge, 150 × 25 mm × 5 µm; elution solvent: 0.05% aqueous ammonia/acetonitrile = 70/30 to 43/57). Thus, the title compound (41.1 mg, 61% yield) was obtained as a yellow solid.

### (Example 29) 2-Chloro-8-(1,4-dioxaspiro[4.4]nonan-6-ylmethoxy)-N-(furan-2-ylmethyl)quinazolin-4-amine

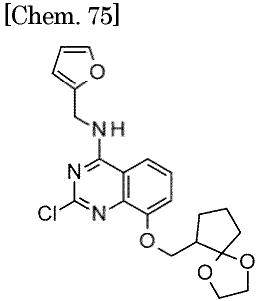

A solution (5 mL) of 2-chloro-4-[(furan-2-ylmethyl)amino]quinazolin-8-ol (150 mg, 0.544 mmol) obtained in Example 18-2 in toluene was subjected to the same operation as in Example 18-3 using 1,4-dioxaspiro[4,4]nonan-9-ylmethanol (150 mg, 0.948 mmol). Thus, the title compound (170 mg, 75% yield) was obtained as a yellow solid.

### (Example 30) 2-[({2-Chloro-4-[(furan-2-ylmethyl)amino]quinazolin-8-yl}oxy)methyl]cyclopentanone

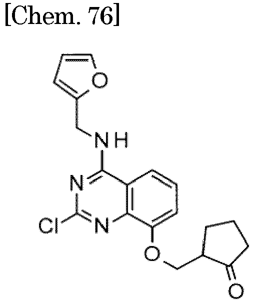

p-Toluenesulfonic acid monohydrate (45.0 mg, 0.237 mmol) was added to a solution of 2-chloro-8-(1,4-dioxaspiro[4.4]nonan-6-ylmethoxy)-N-(furan-2-ylmethyl)quinazolin-4-amine (90.0 mg, 0.216 mmol) obtained in Example 29 in acetone (5 mL)/water (2 mL), followed by stirring at room temperature for 1 hour. After the acetone was distilled off under reduced pressure, water (20 mL) was added to the residue, followed by extraction with ethyl acetate. The organic layer was then washed with a saturated saline solution, dried over anhydrous sodium sulfate, and then concentrated under reduced pressure. The resulting residue was purified by preparative HPLC (column: Phenomenex Synergi C18, 150 × 25 mm × 10 µm; elution solvent: 0.225% aqueous formic acid/acetonitrile = 62/38 to 32/68) to obtain the title compound (50.3 mg, 60% yield) as a white solid.

### (Example 31) 4-[({2-Chloro-4-[(furan-2-ylmethyl)amino]quinazolin-8-yl}oxy)methyl]pyrrolidin-2-one

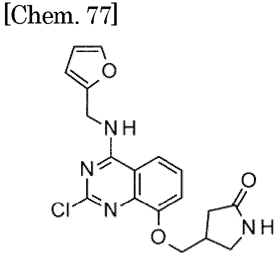

The same operation as in Example 18-3 was performed using 2-chloro-4-[(furan-2-ylmethyl)amino]quinazolin-8-ol (37.1 mg, 0.135 mmol) obtained in Example 18-2 and 4-(hydroxymethyl)pyrrolidin-2-one (31.5 mg, 0.269 mmol). Thus, the title compound (42.0 mg, 84% yield) was obtained as a pale white solid.

### (Example 32) 3-[({2-Chloro-4-[(furan-2-ylmethyl)amino]quinazolin-8-yl}oxy)methyl]cyclobutanol

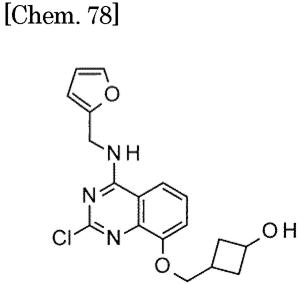

The same operation as in Example 18-3 was performed using 2-chloro-4-[(furan-2-ylmethyl)amino]quinazolin-8-o1 (102 mg, 0.369 mmol) obtained in Example 18-2 and 3-(hydroxymethylcyclobutan-1-ol (38.4 mg, 0.376 mmol). Thus, the title compound (22.5 mg, 17% yield) was obtained as a yellow solid.

### (Example 33) 2-Chloro-8-{[3-(difluoromethoxy)cyclobutyl]methoxy}-N-(furan-2-ylmethyl) quinazolin-4-amine

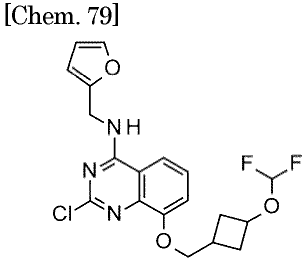

To a solution (1 mL) of 3-[({2-chloro-4-[(furan-2-ylmethyl)amino]quinazolin-8-yl}oxy)methyl]cyclobutanol (16.0 mg, 0.0445 mmol) obtained in Example 32 and copper(I) iodide (1.70 mg, 0.00889 mmol) in acetonitrile, a solution (0.5 mL) of 2,2-difluoro-2-(fluorosulfonyl)acetic acid (7.00 µL, 0.0667 mmol) in acetonitrile was added dropwise at 50°C in small portions, followed by stirring at 50°C for 1.5 hours. The reaction solution was concentrated under reduced pressure. The resulting residue was then purified by silica gel column chromatography (ethyl acetate/hexane = 10/90 to 35/65) to obtain the title compound (6.10 mg, 33% yield) as a yellow solid.

### (Example 34) 5-[({2,6-Dichloro-4-[(furan-2-ylmethyl)amino]quinazolin-8-yl}oxy)methyl]-3-methyl-1,3-oxazolidin-2-one

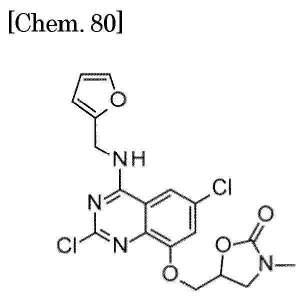

### (Example 34-1) 2-Amino-5-chloro-3-methoxybenzamide

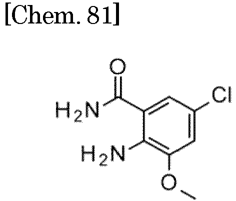

1,1'-Carbonyldiimidazole (2.82 g, 17.4 mmol) was added to a solution (60 mL) of 2-amino-5-chloro-3-methoxybenzoic acid (2.92 g, 14.5 mmol) in THF under ice-cooling, followed by stirring at room temperature for 3 hours. Then, 28% aqueous ammonia (4.84 mL, 72.4 mmol) was added to the reaction solution, followed by stirring for 3 hours. The reaction solution was concentrated under reduced pressure. Water (50 mL) was added to the resulting residue, and the solid was collected by filtration. Diethyl ether was added to the obtained solid, and slurry purification was performed. Thus, the title compound (2.33 g, 80% yield) was obtained as a pale white solid.

### (Example 34-2) 6-Chloro-8-methoxyquinazoline-2,4(1H,3H)-dione

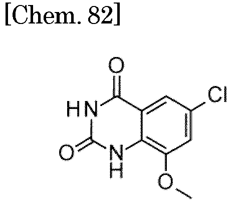

To a solution (70 mL) of 2-amino-5-chloro-3-methoxybenzamide (2.30 g, 11.5 mmol) obtained in Example 34-1 in THF, 1.11M lithium bis(trimethylsilyl)amide (26.0 mL, 28.7 mmol) was added under ice-cooling, and the mixture was stirred at the same temperature for 15 minutes. A solution (12 mL) of triphosgene (3.57 g, 12.0 mmol) in THF was then added to the reaction solution, and the mixture was stirred for 30 minutes, followed by stirring at room temperature for 2 hours. After the reaction solution was ice-cooled, water (30 mL) was added, and the organic solvent was concentrated under reduced pressure. The solid was then collected by filtration, washed with water, and dried to obtain a crudely purified product. A solvent mixture of ethyl acetate/hexane (2/1) was added to the obtained crudely purified product to form a suspension, which was stirred for 1 hour. The solid was then collected by filtration. Thus, the title compound (2.12 g, 82% yield) was obtained as a light brown solid.

### (Example 34-3) 2,4,6-Trichloro-8-methoxyquinazoline

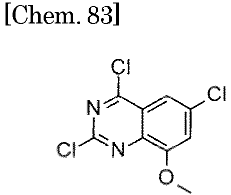

A mixture of 6-chloro-8-methoxyquinazoline-2,4(1H,3H)-dione (2.08 g, 9.18 mmol) obtained in Example 34-2, phosphoryl chloride (10.0 g, 65.2 mmol), and N,N-diethylaniline (2.95 mL, 18.4 mmol) was stirred at 100°C for 4 hours. The reaction solution was allowed to cool to room temperature. Then, the operation of adding toluene and concentrating the mixture under reduced pressure was repeated three times. Chloroform (50 mL) was added to the resulting residue, and a saturated aqueous solution of sodium bicarbonate was added under vigorous stirring until the pH reached 5 to 6, followed by extraction with chloroform. The organic layer was washed with a saturated saline solution, dried over anhydrous magnesium sulfate, and concentrated under reduced pressure. The resulting residue was purified by silica gel column chromatography (ethyl acetate/hexane = 0/100 to 18/82) to obtain the title compound (1.57 g, 65% yield) as a pale yellow solid.

### (Example 34-4) 2,4,6-Trichloroquinazolin-8-ol

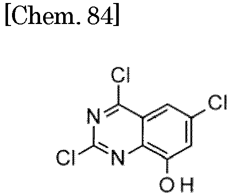

The same operation as in Example 18-1 was performed using 2,4,6-trichloro-8-methoxyquinazoline (1.57 g, 5.96 mmol) obtained in Example 34-3 and aluminum chloride (4.77 g, 35.7 mmol). Thus, the title compound (1.45 g, 98% yield) was obtained as a pale yellow solid.

### (Example 34-5) 2,6-Dichloro-4-[(furan-2-ylmethyl)amino]quinazolin-8-ol

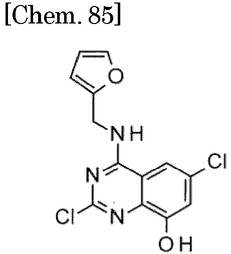

The same operation as in Example 18-2 was performed using 2,4,6-trichloroquinazolin-8-ol (509 mg, 2.04 mmol) obtained in Example 34-4 and furfurylamine (208 µL, 2.24 mmol). Thus, the title compound (512 mg, 81% yield) was obtained as a yellow solid.

### (Example 34-6) 5-[(12,6-Dichloro-4-[(furan-2-ylmethyl)amino]quinazolin-8-yl}oxy)methyl]-3-methyl-1,3-oxazolidin-2-one

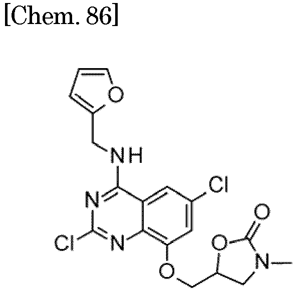

The same operation as in Example 18-3 was performed using 2,6-dichloro-4-[(furan-2-ylmethyl)amino]quinazolin-8-ol (50.8 mg, 0.164 mmol) obtained in Example 34-5 and 5-(hydroxymethyl)-3-methyl-1,3-oxazolidin-2-one (33.4 mg, 0.254 mmol). Thus, the title compound (58.3 mg, 84% yield) was obtained as a white solid.

### (Example 35) (3S,5R)-5-[({2,6-Dichloro-4-[(furan-2-ylmethyl)amino]quinazolin-8-yl}oxy)methyl]-3-methylpyrrolidin-2-one

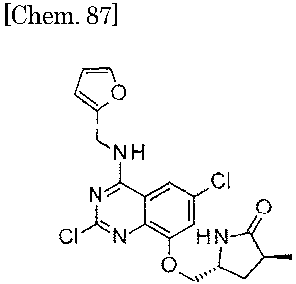

The same operation as in Example 18-3 was performed using 2,6-dichloro-4-[furan-2-ylmethyl)aminolquinazolin-8-ol (45.5 mg, 0.147 mmol) obtained in Example 34-5 and (3S,5R)-5-(hydroxymethyl)-3-methylpyrrolidin-2-one (29.3 mg, 0.227 mmol). Thus, the title compound (24.1 mg, 39% yield) was obtained as a white solid.

### (Example 36) 5-({[4-(Benzylamino)-2,6-dichloroquinazolin-8-yl]oxy}methyl)-3-methyl-1,3-oxazolidin-2-one

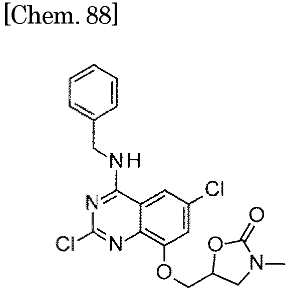

### (Example 36-1) 4-(Benzylamino)-2,6-dichloroquinazolin-8-ol

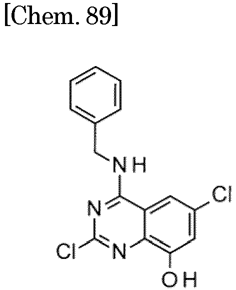

The same operation as in Example 18-2 was performed using 2,4,6-trichloroquinazolin-8-ol (240 mg, 0.962 mmol) obtained in Example 34-4 and benzylamine (116 µL, 1.06 mmol). Thus, the title compound (218 mg, 71% yield) was obtained as a pale yellow solid.

### (Example 36-2) 5-({[4-(Benzylamino)-2,6-dichloroquinazolin-8-yl]oxy}methyl)-3-methyl-1,3-oxazolidin-2-one

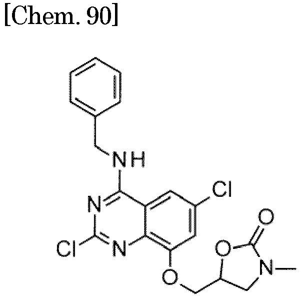

The same operation as in Example 18-3 was performed using 4-(benzylamino)-2,6-dichloroquinazolin-8-ol (53.7 mg, 0.168 mmol) obtained in Example 36-1 and 5-(hydroxymethyl)-3-methyl-1,3-oxazolidin-2-one (34.0 mg, 0.259 mmol). Thus, the title compound (58.7 mg, 81% yield) was obtained as a white solid.

### (Example 37) 5-[({2,7-Dichloro-4-[(furan-2-ylmethyl)amino]quinazolin-8-yl}oxy)methyl]-3-methyl-1,3-oxazolidin-2-one

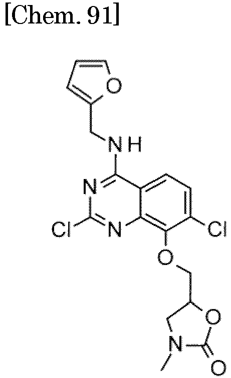

### (Example 37-1) 2-Amino-4-chloro-3-methoxybenzamide

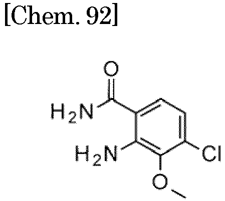

The same operation as in Example 34-1 was performed using 2-amino-4-chloro-3-methoxybenzoic acid (1.21 g, 6.00 mmol). Thus, the title compound (1.06 g, 88% yield) was obtained as a brown solid.

### (Example 37-2) 7-Chloro-8-methoxyquinazoline-2,4(1H,3H)-dione

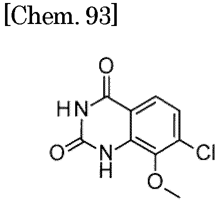

The same operation as in Example 34-2 was performed using 2-amino-4-chloro-3-methoxybenzamide (1.06 g, 5.28 mmol) obtained in Example 37-1. Thus, the title compound (0.950 g, 79% yield) was obtained as a light brown solid.

### (Example 37-3) 2,4,7-Trichloro-8-methoxyquinazoline

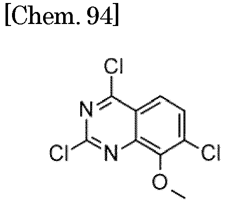

The same operation as in Example 34-3 was performed using 7-chloro-8-methoxyquinazoline-2,4(1H,3H)-dione (950 mg, 4.19 mmol) obtained in Example 37-2. Thus, the title compound (297 mg, 27% yield) was obtained as a white solid.

### (Example 37-4) 2,4,7-Trichloroquinazolin-8-ol

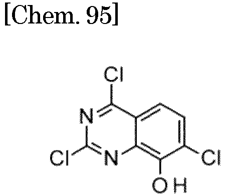

The same operation as in Example 34-4 was performed using 2,4,7-trichloro-8-methoxyquinazoline (103 mg, 0.391 mmol) obtained in Example 37-3. Thus, the title compound (98.4 mg, 100% yield) was obtained.

### (Example 37-5) 2,7-Dichloro-4-[(furan-2-ylmethyl)amino]quinazolin-8-ol

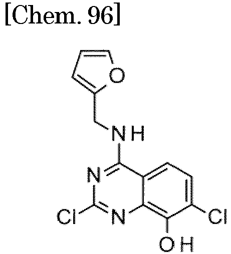

The same operation as in Example 34-5 was performed using 2,4,7-trichloroquinazolin-8-ol (97.5 mg, 0.391 mmol) obtained in Example 37-4. Thus, the title compound (103 mg, 85% yield) was obtained as a pale white solid.

### (Example 37-6) 5-[({2,7-Dichloro-4-[(furan-2-ylmethyl)amino]quinazolin-8-yl}oxy)methyl]-3-methyl-1,3-oxazolidin-2-one

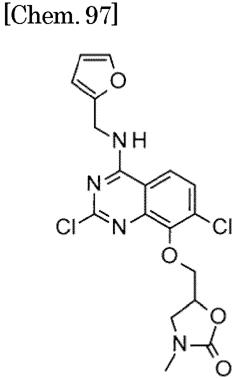

The same operation as in Example 18-3 was performed using 2,7-dichloro-4-[furan-2-ylmethyl)aminolquinazolin-8-ol (43.5 mg, 0.140 mmol) obtained in Example 37-5 and 5-(hydroxymethyl)-3-methyl-1,3-oxazolidin-2-one (27.6 mg, 0.210 mmol). Thus, the title compound (51.0 mg, 86% yield) was obtained as a white solid.

### (Example 38) 2-Chloro-8-(1,4-dioxan-2-ylmethoxy)-N-(furan-2-ylmethyl)quinolin-4-amine

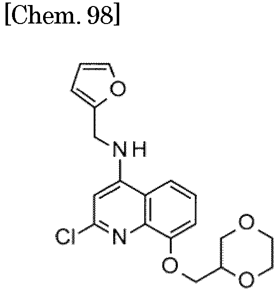

### (Example 38-1) 2,4-Dichloroquinolin-8-ol

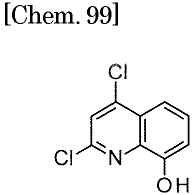

To a solution (5 mL) of 2,4-dichloro-8-methoxyquinoline (154 mg, 0.675 mmol) disclosed in J. Mol. Structure, 2017, 1131, 51-72 in DCM, 1M boron tribromide-DCM (1.35 mL, 1.35 mmol) was added under ice-cooling, and the mixture was stirred at the same temperature for 5 hours, followed by stirring at room temperature for 18 hours. The reaction solution was ice-cooled, water (2 mL) was added, and the mixture was then concentrated under reduced pressure. Water was added to the resulting residue, and the solid was collected by filtration, washed with water, and then dried to obtain the title compound (143 mg, 99% yield) as a pale yellow solid.

### (Example 38-2) 2-Chloro-4-[(furan-2-ylmethyl)amino]quinolin-8-ol

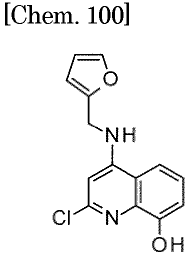

Furfurylamine (5.20 mL, 56.2 mmol) and N,N-diisopropylethylamine (12.2 mL, 70.0 mmol) were added to a solution of 2,4-dichloroquinolin-8-ol (6.01 g, 28.1 mmol) obtained in Example 38-1 in NMP (112 mL), followed by stirring at 120°C for 19 hours. After the reaction solution was allowed to cool to room temperature, water (200 mL) was added, followed by extraction with ethyl acetate. The organic layer was washed with water and then with a saturated saline solution, dried over anhydrous magnesium sulfate, and then concentrated under reduced pressure. The resulting residue was crudely purified by silica gel column chromatography (methanol/DCM = 0/100 to 10/90), followed by purification by silica gel column chromatography (ethyl acetate/hexane = 0/100 to 50/50), to obtain the title compound (4.94 g, 64% yield) as a pale yellow solid. Also, a positional isomer, 4-chloro-2-[(furan-2-ylmethyl)amino]quinolin-8-ol (1.38 g, 18% yield), of the title compound was obtained as a white solid.

### (Example 38-3) 2-Chloro-8-(1,4-dioxan-2-ylmethoxy)-N-(furan-2-ylmethyl)quinolin-4-amine

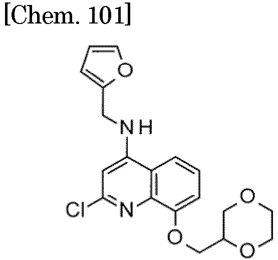

The same operation as in Example 18-3 was performed using 2-chloro-4-[(furan-2-ylmethyl)amino]quinolin-8-ol (150 mg, 0.546 mmol) obtained in Example 38-2 and (1,4-dioxan-2-yl)methanol (86.7 µL, 0.819 mmol). Thus, the title compound (167 mg, 81% yield) was obtained as a white solid.

### (Example 39) 5-[({2-Chloro-4-[(furan-2-ylmethyl)amino]quinolin-8-yl}oxy)methyl]-1,3-oxazolidin-2-one

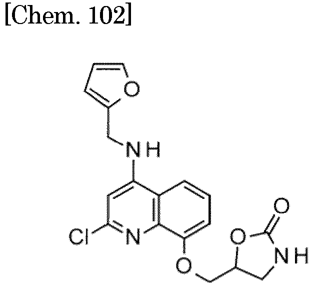

The same operation as in Example 18-3 was performed using 2-chloro-4-[furan-2-ylmethyl)amino]quinolin-8-ol (90.7 mg, 0.330 mmol) obtained in Example 38-2 and 5-(hydroxymethyl)-1,3-oxazolidin-2-one (79.1 mg, 0.675 mmol). Thus, the title compound (65.3 mg, 53% yield) was obtained as a white solid.

### (Example 40) 5-({[4-(Benzylamino)-2-chloroquinazolin-8-yl]oxy}methyl)-3-methyl-1,3-oxazolidin-2-one

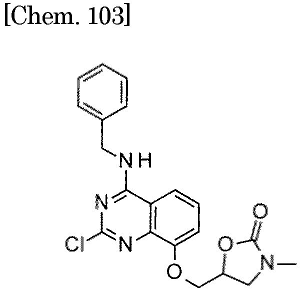

### (Example 40-1) 4-(Benzylamino)-2-chloroquinazolin-8-ol

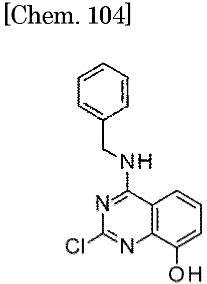

Benzylamine (83.0 µL, 0.759 mmol) and triethylamine (200 µL, 1.44 mmol) were added to a mixture of 2,4-dichloroquinazolin-8-ol (156 mg, 0.725 mmol) obtained in Example 18-1 and acetonitrile (3 mL), followed by stirring at 60°C for 1.5 hours. The reaction solution was allowed to cool to room temperature and then concentrated under reduced pressure. The resulting residue was purified by silica gel column chromatography (ethyl acetate/hexane = 0/100 to 30/70) to obtain the title compound (158 mg, 76% yield) as a white solid.

### (Example 40-2) 5-({[4-(Benzylamino)-2-chloroquinazolin-8-yl]oxy}methyl)-3-methyl-1,3-oxazolidin-2-one

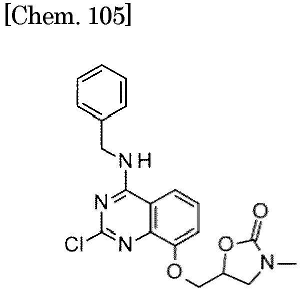

5-(Hydroxymethyl)-3-methyl-1,3-oxazolidin-2-one (103 mg, 0.785 mmol) and cyanomethylenetributylphosphorane (224 µL, 0.853 mmol) were added to a solution (2 mL) of 4-(benzylamino)-2-chloroquinazolin-8-ol (122 mg, 0.427 mmol) obtained in Example 40-1 in toluene, followed by stirring at 80°C for 1.5 hours. The reaction solution was allowed to cool to room temperature and then concentrated under reduced pressure. The resulting residue was purified by silica gel column chromatography (ethyl acetate/hexane = 0/100 to 50/50 to 100/0) to obtain the title compound (137 mg, 80% yield) as a white solid.

### (Example 41) 5-({[4-(Benzylamino)-2-chloroquinazolin-8-yl]oxy}methyl)-3-(2-fluoroethyl)-1,3-oxazolidin-2-one

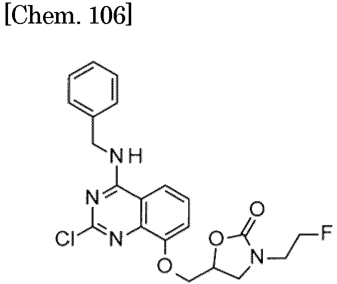

### (Example 41-1) 5-[(Benzyloxy)methyl]-3-(2-fluoroethyl)-1,3-oxazolidin-2-one

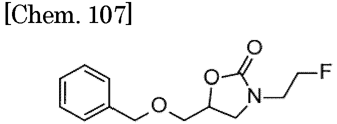

tert-Butoxypotassium (0.931 mg, 8.30 mmol), 1-bromo-2-fluoroethane (0.831 g, 6.54 mmol), and tetrabutylammonium ammonium iodide (TBAI) (0.201 g, 0.545 mmol) were added to a solution (10 mL) of 5-[(benzyloxy)methyl]-1,3-oxazolidin-2-one (1.13 g, 5.45 mmol) disclosed in WO 2008/044700 in THF, followed by stirring at 60°C for 5 hours. After the reaction solution was allowed to cool to room temperature, water (1 mL) was added, and the mixture was then concentrated under reduced pressure. Ethyl acetate (20 mL) was added to the resulting residue, the mixture was washed with water and then with a saturated saline solution, dried over magnesium sulfate, and then concentrated under reduced pressure. The resulting residue was purified by silica gel column chromatography (ethyl acetate/hexane = 0/100 to 50/50) to obtain the title compound (0.908 g, 66% yield) as a yellow oily substance.

### (Example 41-2) 3-(2-Fluoroethyl)-5-(hydroxymethyl)-1,3-oxazolidin-2-one

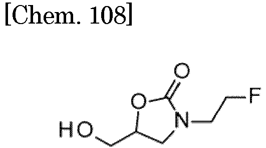

20% Palladium hydroxide on carbon (90.0 mg) was added to a solution (7 mL) of 5-[(benzyloxy)methyl]-3-(2-fluoroethyl)-1,3-oxazolidin-2-one (907 mg, 3.58 mmol) obtained in Example 41-1 in ethanol, followed by stirring in a hydrogen atmosphere for 8 hours. The reaction solution was filtered and then concentrated under reduced pressure. Toluene was added to the resulting residue, and the mixture was concentrated again under reduced pressure. Thus, the title compound (568 mg, 97% yield) was obtained as a colorless oily substance.

### (Example 41-3) 5-({[4-(Benzylamino)-2-chloroquinazolin-8-yl]oxy}methyl)-3-(2-fluoroethyl)-1,3-oxazolidin-2-one

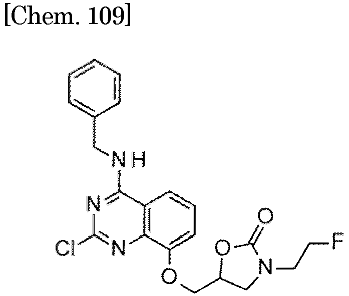

The same operation as in Example 18-3 was performed using 4-(benzylamino)-2-chloroquinazolin-8-ol (40.0 mg, 0.140 mmol) obtained in Example 40-1 and 3-(2-fluoroethyl)-5-(hydroxymethyl)-1,3-oxazolidin -2-one (40.0 mg, 0.245 mmol) obtained in Example 41-2. Thus, the title compound (46.2 mg, 77% yield) was obtained as a pale yellow solid.

### (Example 42) 5-({[4-(Benzylamino)-2-chloroquinazolin-8-yl]oxy}methyl)-3-(2-hydroxyethyl)-1,3-oxazolidin-2-one

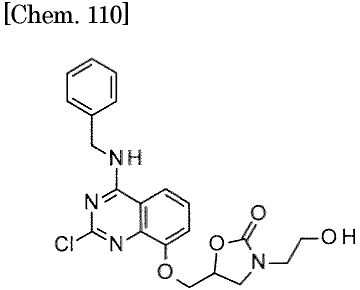

### (Example 42-1) 5-[(Benzyloxy)methyl]-3-(2-{[tert-butyl(dimethyl)silyl]oxy}ethyl)-1,3-oxazolidin-2-one

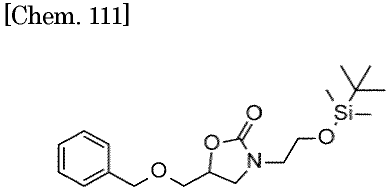

The same operation as in Example 41-1 was performed using 5-[(benzyloxy)methyl]-1,3-oxazolidin-2-one (1.13 g, 5.45 mmol) disclosed in WO 2008/044700 and (2-bromoethoxy)-tert-butyldimethylsilane (1.40 mL, 6.54 mmol). Thus, the title compound (1.26 g, 63% yield) was obtained as a yellow oily substance.

### (Example 42-2) 3-(2-{[tert- Butyl(dimethyl)silyl]oxy}ethyl)-5-(hydroxymethyl)-1,3-oxazolidin-2-one

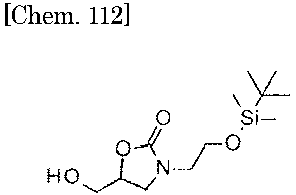

The same operation as in Example 41-2 was performed using 5-[(benzyloxy)methyl]-3-(2-{[tert-butyl(dimethyl)silyl]oxy}ethyl)-1,3-oxazolidin-2-one (1.26 g, 3.45 mmol) obtained in Example 42-1. Thus, the title compound (0.948 g, 100% yield) was obtained as a colorless oily substance.

### (Example 42-3) 5-({[4-(Benzylamino)-2-chloroquinazolin-8-yl]oxy}methyl)-3-(2-hydroxyethyl)-1,3-oxazolidin-2-one

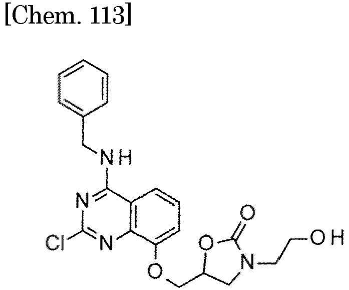

The same operation as in Example 18-3 was performed using 4-(benzylamino)-2-chloroquinazolin-8-ol (40.2 mg, 0.141 mmol) obtained in Example 40-1 and 3-(2-{[tert-butyl(dimethyl)silyl]oxy}ethyl)-5-(hydroxymethyl)-1,3-oxazolidin-2-one (59.2 mg, 0.215 mmol) obtained in Example 42-2. Thus, crude 5-({[4-(benzylamino)-2-chloroquinazolin-8-yl]oxy}methyl)-3-(2-{[tert-butyl(dimethyl)silyl]oxy}ethyl)-1,3-oxazolidin-2-one (33.3 mg) [MS (m/z): 543 (M:)⁺] was obtained as a brown oily substance. The obtained 5-({{4-(benzylamino)-2-chloroquinazolin-8-yl]oxy}methyl)-3-(2-{[tert-butyl(dimethyl)silyl]oxy}ethyl)-1,3-oxazolidin-2-one was dissolved in THF (2 mL). 1M TBAF-THF (92.0 µL, 0.0920 mmol) was added to the solution, and the mixture was stirred at room temperature for 1.5 hours. The reaction solution was concentrated under reduced pressure, and ethyl acetate was then added to form a suspension. The suspension was washed with water, and the precipitated solid was collected by filtration, washed with water, ethyl acetate, and hexane, and then dried to obtain the title compound (11.9 mg, 45% yield) as a white solid.

### (Example 43) 5-({[4-(Benzylamino)-2-chloroquinolin-8-yl]oxy}methyl)-3-methyl-1,3-oxazolidin-2-one

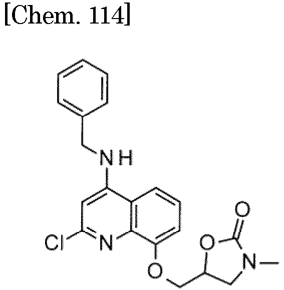

### (Example 43-1) 4-(Benzylamino)-2-chloroquinolin-8-ol

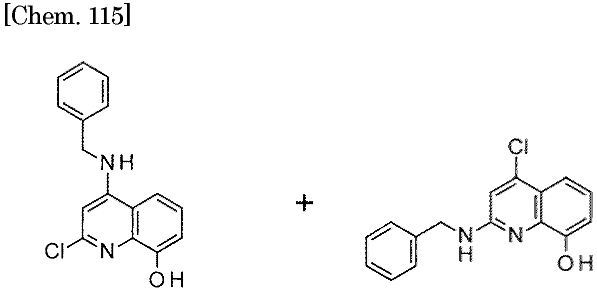

Benzylamine (306 µL, 2.80 mmol) and N,N-diisopropylethylamine (488 µL, 2.80 mmol) were added to a solution of 2,4-dichloroquinolin-8-ol (300 mg, 1.40 mmol) obtained in Example 38-1 in NMP (3.5 mL), followed by stirring at 120°C for 9 hours. After the reaction solution was allowed to cool to room temperature, water was added, followed by extraction with ethyl acetate. The organic layer was washed with a saturated saline solution, dried over anhydrous magnesium sulfate, and then concentrated under reduced pressure. The resulting residue was purified by silica gel column chromatography (ethyl acetate/hexane = 0/100 to 50/50) to obtain the title compound (268 mg, 67% yield) as a pale yellow solid. In addition, a positional isomer, 2-(benzylamino)-4-chloroquinolin-8-ol (102 mg, 26% yield), of the title compound was obtained as a pale yellow solid.

### (Example 43-2) 5-({[4-(Benzylamino)-2-chloroquinolin-8-yl]oxy}methyl)-3-methyl-1,3-oxazolidin-2-one

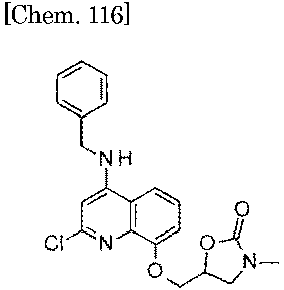

5-(Hydroxymethyl)-3-methyl-1,3-oxazolidin-2-one (100 mg, 0.763 mmol) and cyanomethylenetributylphosphorane (251 µL, 0.956 mmol) were added to a solution (2 mL) of 4-(benzylamino)-2-chloroquinolin-8-ol (136 mg, 0.478 mmol) obtained in Example 43-1 in toluene, followed by stirring at 80°C for 2 hours. The reaction solution was allowed to cool to room temperature and then concentrated under reduced pressure. The resulting residue was purified by silica gel column chromatography (ethyl acetate/hexane = 0/100 to 100/0) to obtain the title compound (107 mg, 56% yield) as a white solid.

Structural formulae and physical scientific data of the compounds described in Examples 18 to 43 are shown below.

**[Table 3]**

| Example | Structural formulae | Data |
|---|---|---|
| 18-1 | 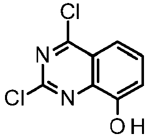 | ¹H-NMR (CDCL₃) δ: 7.45-7.47 (2H, m), 7.64 (1H, t, *J* = 7.9 Hz), 7.75 (1H, dd, *J* = 7.9, 1.2 Hz). |
| 18-2 | 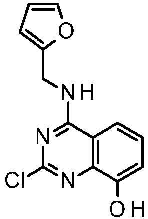 | ¹H-NMR (DMSO-D₆) δ: 4.72 (2H, d, *J* = 5.5 Hz), 6.35 (1H, d, *J* = 2.4 Hz), 6.41-6.43 (1H, m), 7.15 (1H, d, *J* = 7.9 Hz), 7.35 (1H, t, *J* = 7.9 Hz), 7.61 (1H, br s), 7.69 (1H, t, *J =* 4.3 Hz), 9.09 (1H, t, *J =* 5.5 Hz), 9.80 (1H, s). |
| 18-3 | 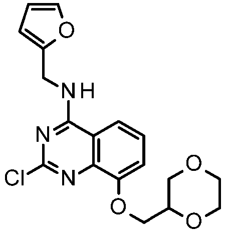 | 1H-NMR (DMSO-D6) δ: 3.44-3.54 (2H, m), 3.63-3.70 (2H, m), 3.76-3.80 (1H, m), 3.88 (1H, dd, J = 11.2, 2.4 Hz), 3.94-4.00 (1H, m), 4.05-4.13 (2H, m), 4.72 (2H, s), 6.35 (1H, dd, J = 3.2, 0.4 Hz), 6.42 (1H, dd, J = 3.2, 2.0 Hz), 7.34 (1H, d, J = 7.2 Hz), 7.44 (1H, t, J = 8.4 Hz), 7.61 (1H, dd, J = 2.0, 0.4 Hz), 7.83 (1H, dd, J = 8.4, 0.8 Hz), 9.11 (1H, br s). |
| | | MS (m/z): 376 (M+H)⁺. |
| 19 | 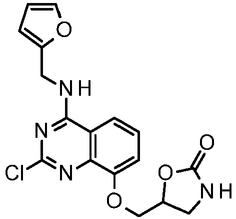 | 1H-NMR (DMSO-D6) δ: 3.39 (1H, dd, J = 8.8, 7.0 Hz), 3.65 (1H, t, J = 8.8 Hz), 4.24-4.33 (2H, m), 4.72 (2H, d, J = 5.8 Hz), 4.98-5.05 (1H, m), 6.36 (1H, br d, J = 3.0 Hz), 6.42 (1H, dd, J = 3.6, 1.8 Hz), 7.38 (1H, d, J = 7.3 Hz), 7.46 (1H, t, J = 8.2 Hz), 7.61 (1H, d, J = 1.8 Hz), 7.65 (1H, s), 7.86 (1H, d, J = 8.5 Hz), 9.13 (1H, t, J = 5.8 Hz). |
| | | MS (m/z): 375 (M+H)⁺. |
| 20 | 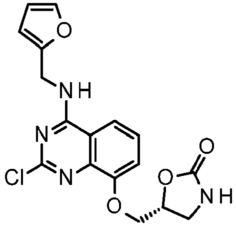 | 1H-NMR (DMSO-D6) δ: 3.39 (1H, dd, J = 9.1, 7.0 Hz), 3.65 (1H, t, J = 9.1 Hz), 4.25-4.33 (2H, m), 4.72 (2H, d, J = 3.6 Hz), 4.98-5.05 (1H, m), 6.36 (1H, br d, J = 3.6 Hz), 6.42 (1H, dd, J = 3.6, 1.8 Hz), 7.38 (1H, d, J = 7.3 Hz), 7.46 (1H, t, J = 7.9 Hz), 7.61 (1H, d, J = 1.8 Hz), 7.65 (1H, s), 7.86 (1H, d, J = 9.1 Hz), 9.14 (1H, br s). |
| | | MS (m/z): 375 (M+H)⁺. |
| 21 | 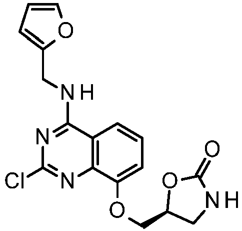 | 1H-NMR (DMSO-D6) δ: 3.39 (1H, dd, J = 8.2, 7.0 Hz), 3.65 (1H, t, J = 9.1 Hz), 4.24-4.33 (2H, m), 4.72 (2H, br s), 4.98-5.05 (1H, m), 6.36 (1H, br d, J = 3.6 Hz), 6.42 (1H, dd, J = 3.6, 1.8 Hz), 7.38 (1H, d, J = 7.3 Hz), 7.46 (1H, t, J = 7.9 Hz), 7.61 (1H, br d, J = 1.8 Hz), 7.65 (1H, s), 7.86 (1H, d, J = 7.9 Hz), 9.14 (1H, br s). |
| | | MS (m/z): 375 (M+H)⁺. |
| 22 | 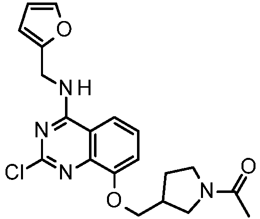 | 1H-NMR (DMSO-D6) δ: 1.70-1.95 (4H, m), 2.00-2.18 (1H, m), 2.67-2.85 (1H, m), 3.20-3.33 (1H, m), 3.41-3.55 (2H, m), 3.60-3.71 (1H, m), 4.04-4.15 (2H, m), 4.72 (2H, d, J = 6.1 Hz), 6.35 (1H, br d, J = 3.1 Hz), 6.41-6.43 (1H, m), 7.35 (1H, dd, J = 11.3, 7.6 Hz), 7.42-7.47 (1H, m), 7.61 (1H, br s), 7.80-7.84 (1H, m), 9.09-9.13 (1H, m). |
| | | MS (m/z): 401 (M+H)⁺. |
| 23 | 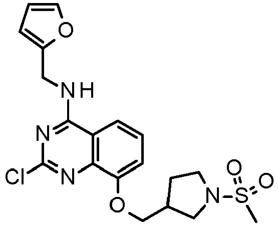 | 1H-NMR (DMSO-D6) δ: 1.79-1.88 (1H, m), 2.07-2.15 (1H, m), 2.77-2.85 (1H, m), 2.95 (3H, s), 3.17-3.22 (1H, m), 3.25-3.31 (1H, m), 3.43-3.50 (2H, m), 4.07-4.15 (2H, m), 4.72 (2H, d, J = 5.5 Hz), 6.35 (1H, d, J = 3.7 Hz), 6.42 (1H, dd, J = 3.1, 1.8 Hz), 7.35 (1H, d, J = 7.9 Hz), 7.45 (1H, t, J = 7.9 Hz), 7.61 (1H, br d, J = 1.2 Hz), 7.83 (1H, d, J = 7.9 Hz), 9.12 (1H, t, J = 5.5 Hz). |
| | | MS (m/z): 437 (M+H)⁺. |
| 24 | 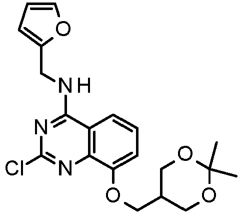 | 1H-NMR (DMSO-D6) δ: 1.33 (3H, s), 1.38 (3H, s), 2.11-2.17 (1H, br m), 3.79-3.84 (2H, m), 4.00-4.05 (2H, m), 4.19 (2H, d, J = 7.3 Hz), 4.72 (2H, d, J = 5.8 Hz), 6.35 (1H, br d, J = 3.6 Hz), 6.42 (1H, dd, J = 3.0, 1.8 Hz), 7.37 (1H, d, J = 7.9 Hz), 7.45 (1H, t, J = 7.9 Hz), 7.61 (1H, br s), 7.82 (1H, d, J = 7.9 Hz), 9.11 (1H, t, J = 5.8 Hz). |
| | | MS (m/z): 404 (M+H)⁺. |
| 25 | 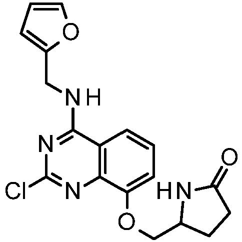 | 1H-NMR (DMSO-D6) δ: 1.90-1.98 (1H, m), 2.06-2.14 (1H, m), 2.17-2.24 (1H, m), 2.62-2.68 (1H, m), 3.93-3.98 (1H, m), 4.03 (1H, dd, J = 9.7, 4.9 Hz), 4.10 (1H, dd, J = 9.7, 4.9 Hz), 4.72 (2H, br d, J = 3.0 Hz), 6.35 (1H, br d, J = 3.0 Hz), 6.42 (1H, dd, J = 2.7, 1.4 Hz), 7.36 (1H, br d, J = 7.9 Hz), 7.45 (1H, t, J = 8.2 Hz), 7.60 (1H, br s), 7.78 (1H, br s), 7.84 (1H, d, J = 8.5 Hz), 9.12 (1H, t, J = 5.2 Hz). |
| | | MS (m/z): 373 (M+H)⁺. |
| 26 | 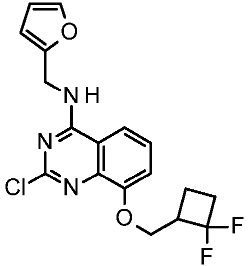 | 1H-NMR (DMSO-D6) δ: 1.60-1.69 (1H, m), 1.95-2.08 (2H, m), 2.51-2.62 (2H, m), 4.19 (1H, dd, J = 10.4, 5.5 Hz), 4.32 (1H, dd, J = 10.4, 8.6 Hz), 4.72 (2H, d, J = 5.5 Hz), 6.35 (1H, d, J = 3.7 Hz), 6.42 (1H, dd, J = 3.7, 1.8 Hz), 7.36 (1H, d, J = 8.0 Hz), 7.45 (1H, t, J = 8.0 Hz), 7.61 (1H, d, J = 1.8 Hz), 7.84 (1H, d, J = 7.4 Hz), 9.11 (1H, t, J = 5.5 Hz). |
| | | MS (m/z): 380 (M+H)⁺. |
| 27 | 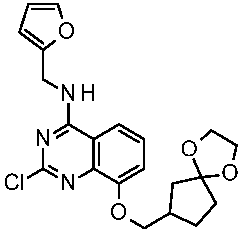 | 1H-NMR (DMSO-D6) δ: 1.48-1.55 (1H, m), 1.59-1.65 (1H, m), 1.71-1.79 (1H, m), 1.83-1.94 (2H, m), 1.98-2.04 (1H, m), 2.51-2.56 (1H, m), 3.82-3.85 (4H, m), 4.02 (2H, d, J = 6.7 Hz), 4.72 (2H, d, J = 5.5 Hz), 6.35 (1H, d, J = 3.0 Hz), 6.42 (1H, dd, J = 3.0, 1.8 Hz), 7.32 (1H, d, J = 7.9 Hz), 7.43 (1H, t, J = 7.9 Hz), 7.60-7.61 (1H, m), 7.80 (1H, d, J = 8.5 Hz), 9.08 (1H, t, J = 5.5 Hz). |
| | | MS (m/z): 416 (M+H)⁺. |
| 28 | 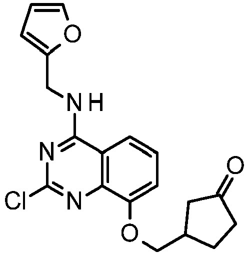 | 1H-NMR (DMSO-D6) δ: 1.75-1.85 (1H, m), 2.07-2.25 (3H, m), 2.30-2.42 (2H, m), 2.72-2.83 (1H, m), 4.14 (2H, d, J = 6.7 Hz), 4.72 (2H, d, J = 5.5 Hz), 6.35 (1H, d, J = 3.0 Hz), 6.42 (1H, dd, J = 3.0, 1.8 Hz), 7.35 (1H, dd, J = 8.2, 1.2 Hz), 7.44 (1H, t, J = 8.2 Hz), 7.60-7.61 (1H, m), 7.82 (1H, d, J = 8.5 Hz), 9.10 (1H, t, J = 5.5 Hz). |
| | | MS (m/z): 372 (M+H)⁺. |
| 29 | 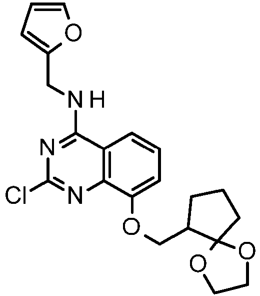 | 1H-NMR (DMSO-D6) δ: 1.48-1.82 (5H, m), 1.95-2.01 (1H, m), 2.38-2.45 (1H, m), 3.77-3.92 (3H, m), 3.96-4.12 (3H, m), 4.71 (2H, d, J = 5.2 Hz), 6.35 (1H, d, J = 2.4 Hz), 6.42 (1H, dd, J = 3.6, 1.8 Hz), 7.30 (1H, d, J = 7.3 Hz), 7.43 (1H, t, J = 8.2 Hz), 7.60-7.61 (1H, m), 7.79 (1H, d, J = 7.3 Hz), 9.08 (1H, t, J = 5.2 Hz). |
| | | MS (m/z): 416 (M+H)⁺. |
| 30 | 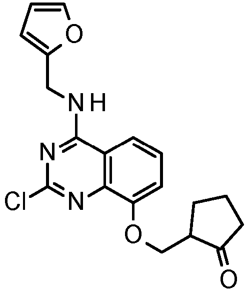 | 1H-NMR (DMSO-D6) δ: 1.83-1.97 (2H, m), 2.09-2.29 (4H, m), 2.67-2.70 (1H, m), 4.20-4.27 (2H, m), 4.72 (2H, d, J = 5.6 Hz), 6.35 (1H, d, J = 2.8 Hz), 6.42 (1H, dd, J = 3.2, 2.0 Hz), 7.32 (1H, d, J = 3.2 Hz), 7.44 (1H, t, J = 8.0 Hz), 7.60 (1H, br s), 7.82 (1H, t, J = 8.0 Hz), 9.08 (1H, t, J = 5.6 Hz). |
| | | MS (m/z): 372 (M+H)⁺. |
| 31 | 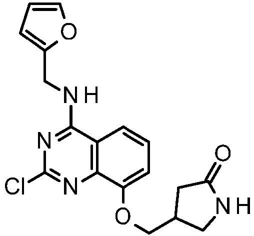 | 1H-NMR (DMSO-D6) δ: 2.08 (1H, dd, J = 16.7, 6.4 Hz), 2.36 (1H, dd, J = 16.7, 8.8 Hz), 2.90-3.00 (1H, m), 3.17 (1H, dd, J = 10.0, 5.2 Hz), 3.45 (1H, dd, J = 9.4, 8.2 Hz), 4.05-4.13 (2H, m), 4.72 (2H, d, J = 5.5 Hz), 6.35 (1H, d, J = 3.0 Hz), 6.42 (1H, dd, J = 3.0, 1.8 Hz), 7.37 (1H, d, J = 7.9 Hz), 7.45 (1H, t, J = 8.2 Hz), 7.59-7.61 (2H, m), 7.83 (1H, d, J = 7.9 Hz), 9.11 (1H, t, J = 5.5 Hz). |
| | | MS (m/z): 373 (M+H)⁺. |
| 32 | 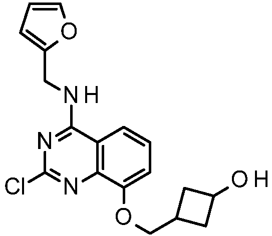 | 1H-NMR (DMSO-D6) δ: 1.60-1.71 (2H, m), 2.17-2.27 (1H, m), 2.32-2.41 (2H, m), 3.95-4.04 (1H, m), 4.06 (2H, d, J = 6.7 Hz), 4.71 (2H, d, J = 5.5 Hz), 5.07 (1H, d, J = 6.1 Hz), 6.35 (1H, d, J = 3.1 Hz), 6.42 (1H, dd, J = 3.1, 1.8 Hz), 7.30 (1H, d, J = 7.3 Hz), 7.43 (1H, t, J = 8.2 Hz), 7.60 (1H, d, J = 1.8 Hz), 7.80 (1H, d, J = 7.3 Hz), 9.08 (1H, t, J = 5.5 Hz). |
| | | MS (m/z): 360 (M+H)⁺. |
| 33 | 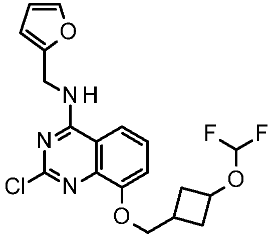 | 1H-NMR (DMSO-D6) δ: 1.93-2.04 (2H, m), 2.31-2.57 (3H, m), 4.12 (2H, d, J = 5.5 Hz), 4.50-4.58 (1H, m), 4.71 (2H, d, J = 5.5 Hz), 6.35 (1H, d, J = 3.1 Hz), 6.40-6.44 (1H, m), 6.66 (1H, t, J = 75.7 Hz), 7.31 (1H, d, J = 7.9 Hz), 7.44 (1H, t, J = 8.2 Hz), 7.61 (1H, br s), 7.81 (1H, d, J = 7.9 Hz), 9.09 (1H, t, J = 5.5 Hz). |
| | | MS (m/z): 410 (M+H)⁺. |
| 34-1 | 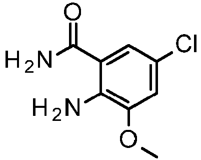 | 1H-NMR (DMSO-D6) δ: 3.81 (3H, s), 6.41 (2H, br s), 6.92 (1H, d, J = 2.4 Hz), 7.23 (1H, br s), 7.28 (1H, d, J = 2.4 Hz), 7.84 (1H, br s). |
| 34-2 | 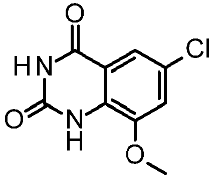 | 1H-NMR (DMSO-D6) δ: 3.90 (3H, s), 7.35 (1H, d, J = 1.8 Hz), 7.40 (1H, d, J = 1.8 Hz), 10.75 (1H, s), 11.46 (1H, s). |
| 34-3 | 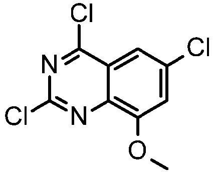 | 1H-NMR (DMSO-D6) δ: 4.05 (3H, s), 7.68 (1H, d, J = 1.8 Hz), 7.83 (1H, d, J = 1.8 Hz). |
| 34-4 | 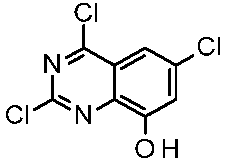 | 1H-NMR (CDCl3) δ: 7.42 (1H, d, J = 2.4 Hz), 7.56 (1H, s), 7.73 (1H, d, J = 2.4 Hz). |
| 34-5 | 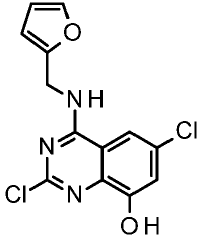 | 1H-NMR (DMSO-D6) δ: 4.70 (2H, d, J = 5.5 Hz), 6.37 (1H, d, J = 3.0 Hz), 6.43 (1H, dd, J = 3.0, 1.8 Hz), 7.14 (1H, d, J = 2.4 Hz), 7.62 (1H, d, J = 1.8 Hz), 7.84 (1H, d, J = 1.8 Hz), 9.12 (1H, t, J = 5.5 Hz), 10.53 (1H, br s). |
| 34-6 | 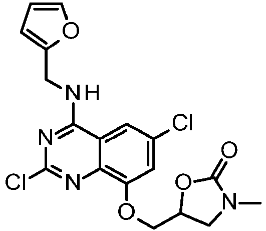 | 1H-NMR (DMSO-D6) δ: 2.80 (3H, s), 3.42 (1H, dd, J = 9.1, 6.7 Hz), 3.73 (1H, t, J = 9.1 Hz), 4.29 (1H, dd, J = 10.9, 6.7 Hz), 4.38 (1H, dd, J = 10.9, 3.0 Hz), 4.70 (2H, s), 4.91-4.97 (1H, m), 6.38 (1H, d, J = 3.6 Hz), 6.43 (1H, dd, J = 3.6, 1.8 Hz), 7.44 (1H, d, J = 1.8 Hz), 7.62 (1H, d, J = 1.2 Hz), 8.03 (1H, d, J = 1.8 Hz), 9.18 (1H, br s). |
| | | MS (m/z): 424 (M+H)⁺. |
| 35 | 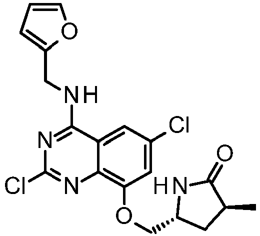 | 1H-NMR (DMSO-D6) δ: 1.13 (3H, d, J = 6.7 Hz), 1.48-1.55 (1H, m), 2.33-2.46 (2H, m), 3.90-3.96 (1H, br m), 4.06 (1H, t, J = 4.6 Hz), 4.16 (1H, dd, J = 10.0, 4.6 Hz), 4.70 (2H, d, J = 5.5 Hz), 6.38 (1H, d, J = 3.0 Hz), 6.43 (1H, dd, J = 3.0, 1.8 Hz), 7.43 (1H, d, J = 1.8 Hz), 7.62 (1H, d, J = 1.8 Hz), 7.81 (1H, s), 8.02 (1H, d, J = 1.8 Hz), 9.16 (1H, t, J = 5.5 Hz). |
| | | MS (m/z): 422 (M+H)⁺. |
| 36-1 | 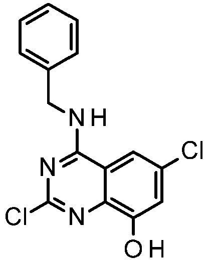 | 1H-NMR (DMSO-D6) δ: 4.72 (2H, d, J = 5.5 Hz), 7.14 (1H, d, J = 2.4 Hz), 7.25-7.29 (1H, m), 7.32-7.39 (4H, m), 7.85 (1H, d, J = 2.4 Hz), 9.20 (1H, t, J = 5.5 Hz), 10.51 (1H, br s). |
| 36-2 | 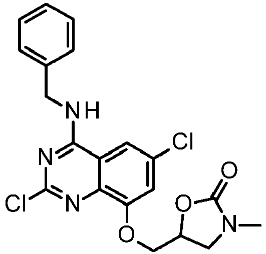 | 1H-NMR (DMSO-D6) δ: 2.80 (3H, s), 3.42 (1H, dd, J = 8.8, 6.7 Hz), 3.73 (1H, t, J = 8.8 Hz), 4.29 (1H, dd, J = 11.5, 6.7 Hz), 4.39 (1H, dd, J = 11.5, 3.3 Hz), 4.73 (2H, br s), 4.91-4.97 (1H, m), 7.25-7.29 (1H, m), 7.33-7.39 (4H, m), 7.45 (1H, d, J = 2.4 Hz), 8.05 (1H, d, J = 1.8 Hz), 9.26 (1H, br s). |
| | | MS (m/z): 434 (M+H)⁺. |
| 37-1 | 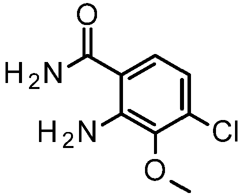 | 1H-NMR (DMSO-D6) δ: 3.70 (3H, s), 6.56 (1H, d, J = 8.5 Hz), 6.71 (2H, br s), 7.24 (1H, br s), 7.36 (1H, d, J = 8.5 Hz), 7.83 (1H, br s). |
| 37-2 | 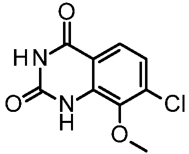 | 1H-NMR (DMSO-D6) δ: 3.80 (3H, s), 7.26 (1H, d, J = 8.5 Hz), 7.65 (1H, d, J = 8.5 Hz), 11.04 (1H, s), 11.44 (1H, s). |
| 37-3 | 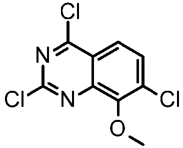 | 1H-NMR (DMSO-D6) δ: 4.10 (3H, s), 7.96 (1H, d, J = 9.1 Hz), 8.06 (1H, d, J = 9.1 Hz). |
| 37-4 | 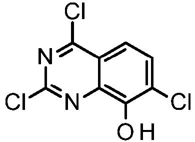 | 1H-NMR (CDCl3) δ: 7.67-7.74 (3H, m). |
| 37-5 | 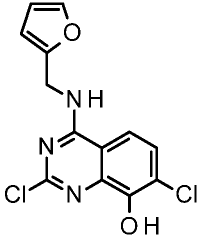 | 1H-NMR (DMSO-D6) δ: 4.72 (2H, d, J = 5.5 Hz), 6.37 (1H, d, J = 3.0 Hz), 6.42-6.43 (1H, m), 7.51 (1H, d, J = 8.5 Hz), 7.61 (1H, d, J = 1.8 Hz), 7.71 (1H, d, J = 8.5 Hz), 9.23 (1H, t, J = 5.5 Hz), 10.27 (1H, br s). |
| 37-6 | 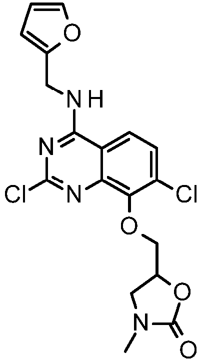 | 1H-NMR (DMSO-D6) δ: 2.78 (3H, s), 3.70 (2H, d, J = 8.5 Hz), 4.37-4.41 (1H, m), 4.50-4.55 (1H, m), 4.69-4.72 (2H, br m), 4.81-4.87 (1H, m), 6.36-6.38 (1H, br m), 6.41-6.43 (1H, br m), 7.58-7.63 (2H, br m), 8.04 (1H, br t, J = 8.8 Hz), 9.38 (1H, br s). |
| | | MS (m/z): 424 (M+H)⁺. |
| 38-1 | 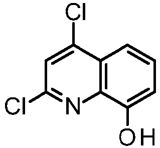 | 1H-NMR (DMSO-D6) δ: 7.24-7.29 (1H, m), 7.61-7.62 (2H, m), 7.94 (1H, s), 10.37 (1H, br s). |
| 38-2 | 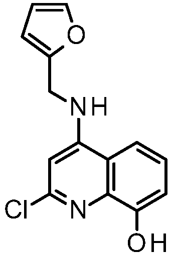 | 1H-NMR (DMSO-D6) δ: 4.55 (2H, d, J = 5.5 Hz), 6.39-6.42 (2H, m), 6.54 (1H, s), 7.02 (1H, d, J= 6.7 Hz), 7.29 (1H, t, J = 7.9 Hz), 7.61-7.64 (2H, m), 8.01 (1H, t, J = 5.5 Hz), 9.33 (1H, br s). |
| 38-2 | 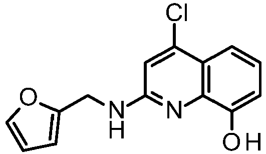 | 1H-NMR (DMSO-D6) δ: 4.78 (2H, d, J = 5.5 Hz), 6.38-6.41 (2H, m), 6.99-7.03 (2H, m), 7.14 (1H, t, J = 7.9 Hz), 7.32 (1H, d, J = 7.9 Hz), 7.60 (1H, br s), 7.64 (1H, t, J = 5.5 Hz), 8.82 (1H, s). |
| 38-3 | 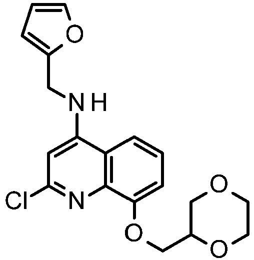 | ¹H-NMR (DMSO-D6) δ: 3.41-3.54 (2H, m), 3.63-3.70 (2H, m), 3.76-3.80 (1H, m), 3.89 (1H, dd, J = 11.5, 2.4 Hz), 3.95-4.13 (3H, m), 4.54 (2H, d, J = 5.8 Hz), 6.39 (1H, d, J = 3.0 Hz), 6.41 (1H, dd, J = 3.0, 1.8 Hz), 6.56 (1H, s), 7.18 (1H, d, J = 7.3 Hz), 7.37 (1H, t, J = 8.2 Hz), 7.61 (1H, d, J = 1.8 Hz), 7.77 (1H, d, J = 7.9 Hz), 7.98 (1H, t, J = 5.8 Hz). |
| | | MS (m/z): 375 (M+H)⁺. |
| 39 | 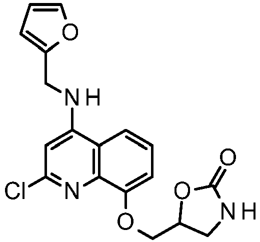 | ¹H-NMR (DMSO-D6) δ: 3.41 (1H, dd, J = 8.8, 6.6 Hz), 3.65 (1H, t, J = 8.8 Hz), 4.24-4.30 (2H, m), 4.54 (2H, d, J = 6.1 Hz), 4.98-5.05 (1H, m), 6.39-6.42 (2H, m), 6.57 (1H, s), 7.23 (1H, d, J = 7.3 Hz), 7.39 (1H, t, J = 8.2 Hz), 7.62 (2H, dd, J = 7.3, 2.4 Hz), 7.81 (1H, d, J = 7.9 Hz), 8.00 (1H, t, J = 6.1 Hz). |
| | | MS (m/z): 374 (M+H)⁺. |
| 40-1 | 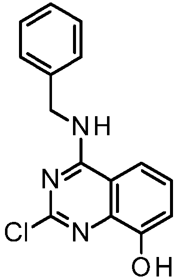 | ¹H-NMR (DMSO-D₆) δ: 4.75 (2H, d, *J* = 5.5 Hz), 7.15 (1H, d, *J* = 7.9 Hz), 7.26 (1H, t, *J* = 6.7 Hz), 7.31-7.41 (5H, m), 7.70 (1H, d, *J* = 7.9 Hz), 9.17 (1H, t, *J* = 5.8 Hz), 9.79 (1H, s). |
| 40-2 | 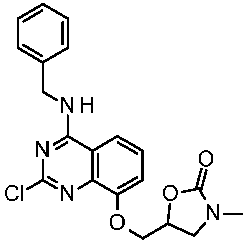 | ¹H-NMR (DMSO-D6) δ: 2.80 (3H, s), 3.45 (1H, t, J = 7.0 Hz), 3.73 (1H, t, J = 8.8 Hz), 4.25 (1H, dd, J = 10.6, 6.4 Hz), 4.34 (1H, dd, J = 11.5, 3.0 Hz), 4.75 (2H, d, J = 4.9 Hz), 4.89-4.99 (1H, m), 7.23-7.29 (1H, m), 7.30-7.41 (5H, m), 7.47 (1H, t, J = 7.9 Hz), 7.88 (1H, d, J = 8.5 Hz), 9.22 (1H, t, J = 5.8 Hz). |
| | | MS (m/z): 399 (M+H)⁺. |
| 41-1 | 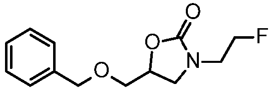 | ¹H-NMR (CDCl3) δ: 3.50-3.65 (3H, m), 3.68 (2H, d, J = 4.9 Hz), 3.78 (1H, t, J = 8.8 Hz), 4.55 (1H, dd, J = 5.2, 3.9 Hz), 4.62 (2H, s), 4.66-4.74 (2H, m), 7.32-7.41 (5H, m). |
| 41-2 | 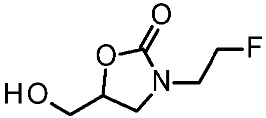 | ¹H-NMR (CDCl3) δ: 2.26 (1H, br t, J = 5.5 Hz), 3.47-3.71 (4H, m), 3.76 (1H, t, J = 8.8 Hz), 3.88-3.93 (1H, br m), 4.55 (1H, t, J = 4.6 Hz), 4.62-4.68 (2H, m). |
| 41-3 | 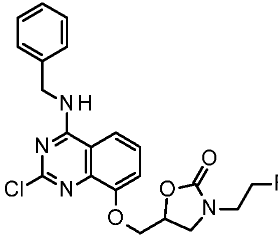 | 1H-NMR (DMSO-D6) δ: 3.48-3.51 (1H, m), 3.55-3.59 (2H, m), 3.83 (1H, t, J = 8.8 Hz), 4.25 (1H, dd, J = 10.9, 6.1 Hz), 4.36 (1H, dd, J = 10.9, 3.3 Hz), 4.52-4.76 (4H, m), 4.96-5.02 (1H, m), 7.24-7.29 (1H, m), 7.32-7.39 (5H, m), 7.47 (1H, t, J = 8.2 Hz), 7.88 (1H, dd, J = 8.5, 1.2 Hz), 9.22 (1H, t, J = 5.8 Hz). |
| | | MS (m/z): 431 (M+H)⁺. |
| 42-1 | 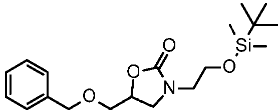 | 1H-NMR (CDCl3) δ: 0.05 (6H, s), 0.88 (9H, s), 3.30-3.42 (2H, m), 3.56 (1H, dd, J = 8.8, 6.4 Hz), 3.59-3.67 (2H, m), 3.72-3.77 (3H, m), 4.59 (2H, s), 4.60-4.67 (1H, m), 7.29-7.38 (5H, m). |
| 42-2 | 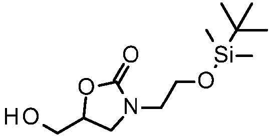 | 1H-NMR (CDCl3) δ: 0.07 (6H, s), 0.89 (9H, s), 2.05 (1H, br s), 3.30-3.44 (2H, m), 3.57 (1H, dd, J = 8.8, 7.0 Hz), 3.67 (1H, dd, J = 12.8, 4.9 Hz), 3.73-3.79 (3H, m), 3.86 (1H, dd, J = 12.5, 3.3 Hz), 4.57-4.63 (1H, m). |
| 42-3 | 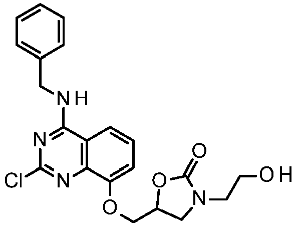 H | 1H-NMR (DMSO-D6) δ: 3.23-3.27 (2H, m), 3.51-3.59 (3H, m), 3.82 (1H, t, J = 9.2 Hz), 4.26 (1H, dd, J = 11.0, 6.1 Hz), 4.33 (1H, dd, J = 11.0, 3.7 Hz), 4.75 (2H, d, J = 5.5 Hz), 4.82 (1H, t, J = 5.5 Hz), 4.93-4.99 (1H, m), 7.24-7.28 (1H, m), 7.32-7.40 (5H, m), 7.45-7.49 (1H, m), 7.88 (1H, d, J = 8.5 Hz), 9.23 (1H, t, J = 5.8 Hz). |
| | | MS (m/z): 429 (M+H)⁺. |
| 43-1 | 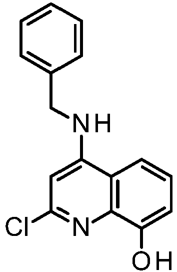 | ¹H-NMR (DMSO-D₆) δ: 4.57 (2H, d, *J* = 5.5 Hz), 6.29 (1H, s), 7.03 (1H, d, *J* = 7.9 Hz), 7.25 (1H, t, *J* = 7.0 Hz), 7.29-7.41 (5H, m), 7.70 (1H, d, *J* = 8.5 Hz), 8.19 (1H, t, *J =* 6.1 Hz), 9.32 (1H, s). |
| 43-1 | 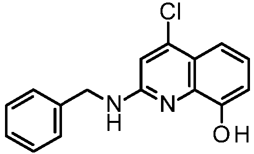 | ¹H-NMR (DMSO-D₆) δ: 4.77 (2H, d, *J* = 5.5 Hz), 6 6.98 (1H, d, *J* = 7.3 Hz), 7.04 (1H, s), 7.12 (1H, t, *J* = 7.9 Hz), 7.24 (1H, t, *J* = 6.7 Hz), 7.32 (3H, dd, *J* = 12.1, 6.1 Hz), 7.41 (2H, d, *J* = 7.9 Hz), 7.73 (1H, t, *J =* 6.1 Hz), 8.71 (1H, s). |
| 43-2 | 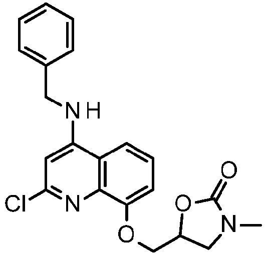 | 1H-NMR (DMSO-D6) δ: 2.80 (3H, s), 3.48 (1H, dd, J = 8.8, 6.4 Hz), 3.73 (1H, t, J = 8.8 Hz), 4.23 (1H, dd, J = 10.9, 6.1 Hz), 4.33 (1H, dd, J = 11.2, 3.3 Hz), 4.57 (2H, d, J = 6.1 Hz), 4.90-5.00 (1H, m), 6.32 (1H, s), 7.25 (2H, dd, J = 10.9, 7.3 Hz), 7.31-7.46 (5H, m), 7.88 (1H, d, J = 7.9 Hz), 8.19 (1H, t, J = 6.4 Hz). |
| | | MS (m/z): 398 (M+H)⁺. |

Compounds of Examples 44 to 103 shown below were produced in accordance with the steps described in Examples 18 to 43 above.

**[Table 4]**

| | | |
|---|---|---|
| 44 | 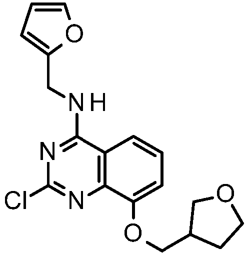 | 1H-NMR (DMSO-D6) δ: 1.67-1.75 (1H, m), 2.01-2.10 (1H, m), 2.73-2.80 (1H, m), 3.60 (1H, dd, J = 8.8, 5.2 Hz), 3.67 (1H, dd, J = 14.6, 7.9 Hz), 3.77-3.84 (2H, m), 3.99-4.09 (2H, m), 4.72 (2H, d, J = 5.5 Hz), 6.35 (1H, br d, J = 3.0 Hz), 6.42 (1H, dd, J = 3.6, 1.8 Hz), 7.35 (1H, d, J = 7.9 Hz), 7.44 (1H, t, J = 8.2 Hz), 7.60 (1H, s), 7.82 (1H, d, J = 7.3 Hz), 9.09 (1H, t, J = 5.5 Hz). |
| | | MS (m/z): 360 (M+H)⁺. |
| 45 | 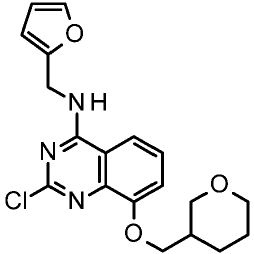 | 1H-NMR (DMSO-D6) δ: 1.39-1.65 (3H, m), 1.86-1.91 (1H, m), 2.08-2.14 (1H, br m), 3.29-3.41 (2H, m), 3.72-3.77 (1H, m), 3.92-4.03 (3H, m), 4.71 (2H, d, J = 5.5 Hz), 6.35 (1H, br d, J = 3.6 Hz), 6.42 (1H, dd, J = 3.0, 1.8 Hz), 7.33 (1H, d, J = 7.3 Hz), 7.43 (1H, t, J = 8.2 Hz), 7.61 (1H, br d, J = 1.8 Hz), 7.81 (1H, d, J = 7.9 Hz), 9.09 (1H, t, J = 5.5 Hz). |
| | | MS (m/z): 374 (M+H)⁺. |
| 46 | 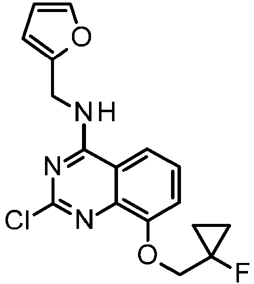 | 1H-NMR (DMSO-D6) δ: 0.88-0.94 (2H, m), 1.14-1.22 (2H, m), 4.43 (2H, d, J = 22.7 Hz), 4.72 (2H, d, J = 5.5 Hz), 6.36 (1H, d, J = 3.1 Hz), 6.42 (1H, dd, J = 3.1, 1.8 Hz), 7.35 (1H, d, J = 8.0 Hz), 7.45 (1H, t, J = 8.0 Hz), 7.61 (1H, d, J = 1.8 Hz), 7.84 (1H, dd, J = 8.6, 1.2 Hz), 9.12 (1H, t, J = 5.5 Hz). |
| | | MS (m/z): 348 (M+H)⁺. |
| 47 | 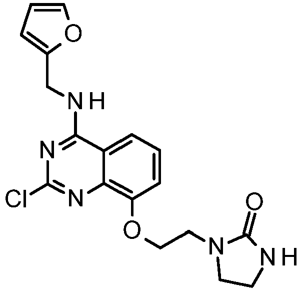 | 1H-NMR (DMSO-D6) δ: 3.23 (2H, t, J = 7.9 Hz), 3.49 (2H, t, J = 5.5 Hz), 3.60 (2H, dd, J = 8.8, 6.4 Hz), 4.20 (2H, t, J = 5.8 Hz), 4.72 (2H, br s), 6.35 (1H, d, J = 3.0 Hz), 6.38 (1H, br s), 6.42 (1H, dd, J = 3.0, 1.8 Hz), 7.35 (1H, d, J = 7.3 Hz), 7.45 (1H, t, J = 8.2 Hz), 7.61 (1H, d, J = 1.8 Hz), 7.83 (1H, d, J = 7.3 Hz), 9.12 (1H, br s). |
| | | MS (m/z): 388 (M+H)⁺. |
| 48 | 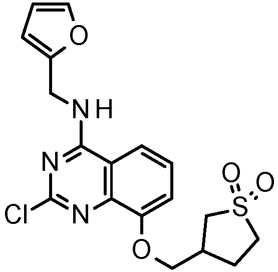 | 1H-NMR (DMSO-D6) δ: 1.97-2.07 (1H, m), 2.32-2.40 (1H, m), 2.91-3.04 (2H, m), 3.10-3.18 (1H, m), 3.28-3.36 (2H, m), 4.19 (2H, d, J = 6.1 Hz), 4.72 (2H, br s), 6.35 (1H, d, J = 3.6 Hz), 6.42 (1H, dd, J = 3.6, 2.1 Hz), 7.36 (1H, d, J = 7.3 Hz), 7.46 (1H, t, J = 8.2 Hz), 7.61 (1H, d, J = 1.8 Hz), 7.85 (1H, d, J = 8.5 Hz), 9.13 (1H, br s). |
| | | MS (m/z): 408 (M+H)⁺. |
| 49 | 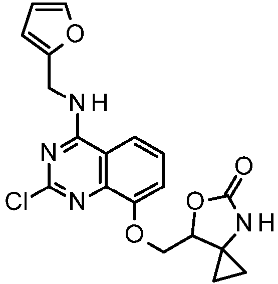 | 1H-NMR (DMSO-D6) δ: 0.67-0.73 (1H, m), 0.78-0.88 (2H, m), 1.09-1.15 (1H, m), 4.18 (1H, dd, J = 10.6, 4.3 Hz), 4.27 (1H, dd, J = 10.6, 7.0 Hz), 4.72 (2H, d, J = 6.1 Hz), 4.92 (1H, dd, J = 7.0, 3.9 Hz), 6.36 (1H, d, J = 3.0 Hz), 6.42 (1H, dd, J = 3.0, 1.8 Hz), 7.40-7.47 (2H, m), 7.61 (1H, br d, J = 1.8 Hz), 7.84-7.86 (2H, m), 9.13 (1H, t, J = 6.1 Hz). |
| | | MS (m/z): 401 (M+H)⁺. |
| 50 | 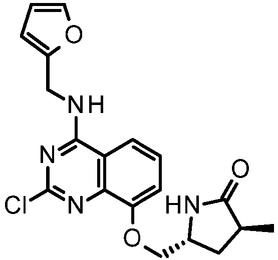 | 1H-NMR (DMSO-D6) δ: 1.13 (3H, d, J = 6.7 Hz), 1.52 (1H, dt, J = 14.6, 5.9 Hz), 2.32-2.48 (2H, m), 3.89-3.95 (1H, m), 4.03 (1H, dd, J = 10.3, 5.5 Hz), 4.11 (1H, dd, J = 10.3, 4.9 Hz), 4.72 (2H, s), 6.34-6.36 (1H, m), 6.41-6.43 (1H, m), 7.37 (1H, d, J = 7.3 Hz), 7.45 (1H, t, J = 8.2 Hz), 7.60-7.61 (1H, m), 7.81 (1H, s), 7.85 (1H, d, J = 9.1 Hz), 9.13 (1H, s). |
| | | MS (m/z): 387 (M+H)⁺. |
| 51 | 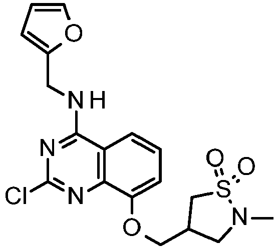 | 1H-NMR (DMSO-D6) δ: 2.58 (3H, s), 3.07-3.22 (3H, m), 3.37-3.39 (1H, m), 3.49-3.54 (1H, m), 4.19 (2H, d, J = 6.1 Hz), 4.72 (2H, d, J = 5.5 Hz), 6.35-6.36 (1H, m), 6.42-6.42 (1H, m), 7.38 (1H, d, J = 7.9 Hz), 7.46 (1H, t, J = 7.9 Hz), 7.61 (1H, br s), 7.87 (1H, d, J = 7.3 Hz), 9.14 (1H, t, J = 6.1 Hz). |
| | | MS (m/z): 423 (M+H)⁺. |
| 52 | 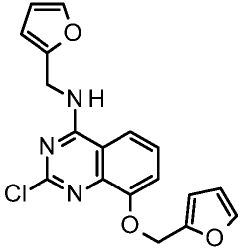 | 1H-NMR (DMSO-D6) δ: 4.71 (2H, br d, J = 4.3 Hz), 5.21 (2H, s), 6.35 (1H, br d, J= 3.0 Hz), 6.42 (1H, dd, J = 3.0, 1.8 Hz), 6.50 (1H, dd, J = 3.0, 1.8 Hz), 6.65 (1H, d, J = 3.0 Hz), 7.43-7.49 (2H, m), 7.61 (1H, br d, J = 1.8 Hz), 7.74 (1H, d, J = 1.2 Hz), 7.84 (1H, dd, J = 7.3, 1.8 Hz), 9.13 (1H, t, J = 4.3 Hz). |
| | | MS (m/z): 356 (M+H)⁺. |
| 53 | 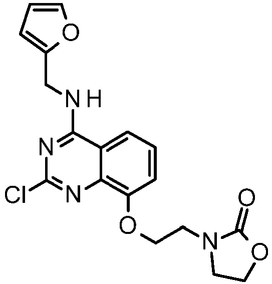 | 1H-NMR (DMSO-D6) δ: 3.62 (2H, t, J = 5.5 Hz), 3.83 (2H, dd, J = 8.5, 7.3 Hz), 4.25-4.29 (4H, m), 4.72 (2H, br s), 6.35 (1H, br d, J = 3.6 Hz), 6.42 (1H, dd, J = 3.6, 1.8 Hz), 7.37 (1H, d, J = 7.3 Hz), 7.45 (1H, t, J = 7.9 Hz), 7.60 (1H, br d, J = 1.2 Hz), 7.84 (1H, d, J = 7.9 Hz), 9.13 (1H, br s). |
| | | MS (m/z): 389 (M+H)⁺. |
| 54 | 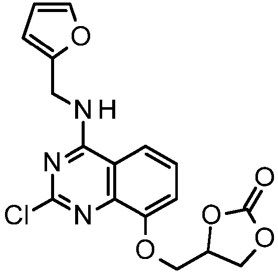 | 1H-NMR (DMSO-D6) δ: 4.36-4.52 (3H, m), 4.67 (1H, t, J = 8.5 Hz), 4.73 (2H, d, J = 5.5 Hz), 5.21-5.28 (1H, m), 6.35-6.36 (1H, m), 6.41-6.43 (1H, m), 7.38 (1H, d, J = 7.3 Hz), 7.47 (1H, t, J = 8.2 Hz), 7.61 (1H, d, J = 1.2 Hz), 7.89 (1H, d, J = 7.3 Hz), 9.15 (1H, t, J = 5.5 Hz). |
| | | MS (m/z): 376 (M+H)⁺. |
| 55 | 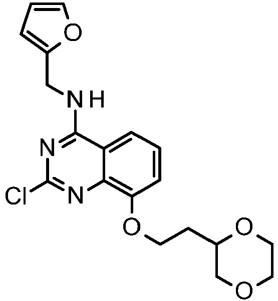 | 1H-NMR (DMSO-D6) δ: 1.78-1.96 (2H, m), 3.26 (1H, dd, J = 11.3, 10.1 Hz), 3.42-3.50 (1H, br m), 3.57 (1H, td, J = 11.0, 2.5 Hz), 3.64 (1H, dd, J = 11.0, 1.8 Hz), 3.69-3.75 (2H, m), 3.84 (1H, dd, J = 11.7, 2.5 Hz), 4.15-4.19 (2H, m), 4.72 (2H, d, J = 5.5 Hz), 6.35 (1H, d, J = 3.1 Hz), 6.42 (1H, dd, J = 3.1, 1.8 Hz), 7.32 (1H, d, J = 7.4 Hz), 7.44 (1H, t, J = 8.3 Hz), 7.61 (1H, d, J = 1.8 Hz), 7.81 (1H, d, J = 8.6 Hz), 9.10 (1H, t, J = 5.5 Hz). |
| | | MS (m/z): 390 (M+H)⁺. |
| 56 | 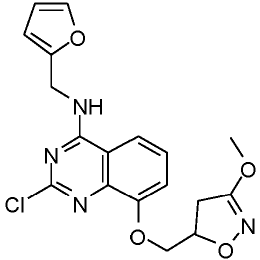 | 1H-NMR (DMSO-D6) δ: 2.98 (1H, dd, J = 16.4, 7.3 Hz), 3.23 (1H, dd, J = 16.4, 10.0 Hz), 3.79 (3H, s), 4.20-4.22 (2H, m), 4.72 (2H, d, J = 5.5 Hz), 4.98-5.05 (1H, m), 6.35 (1H, d, J = 3.0 Hz), 6.41-6.43 (1H, m), 7.36 (1H, d, J = 7.9 Hz), 7.45 (1H, t, J = 7.9 Hz), 7.61 (1H, d, J = 1.8 Hz), 7.83⁻ 7.85 (1H, m), 9.12 (1H, t, J = 5.8 Hz). |
| | | MS (m/z): 389 (M+H)⁺. |
| 57 | 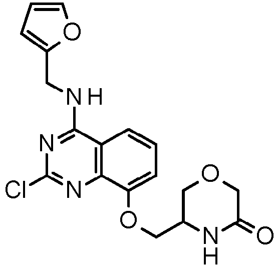 | 1H-NMR (DMSO-D6) δ: 3.76-3.82 (1H, m), 3.86 (1H, dd, J = 11.5, 3.3 Hz), 3.92 (1H, dd, J = 11.5, 3.6 Hz), 4.02 (2H, s), 4.08-4.20 (2H, m), 4.72 (2H, d, J = 5.5 Hz), 6.35 (1H, br d, J = 3.0 Hz), 6.42 (1H, dd, J = 3.0, 1.8 Hz), 7.41-7.48 (2H, m), 7.61 (1H, d, J = 1.2 Hz), 7.86 (1H, dd, J = 7.9, 1.2 Hz), 8.22 (1H, d, J = 3.0 Hz), 9.14 (1H, t, J = 5.5 Hz). |
| | | MS (m/z): 389 (M+H)⁺. |
| 58 | 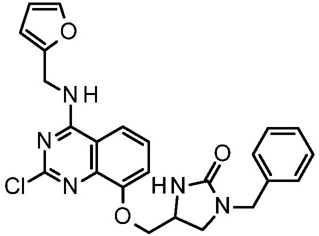 | 1H-NMR (DMSO-D6) δ: 3.17 (1H, dd, J = 8.8, 4.6 Hz), 3.44 (1H, t, J = 8.8 Hz), 3.99-4.07 (3H, m), 4.25 (1H, d, J = 15.2 Hz), 4.31 (1H, d, J = 15.2 Hz), 4.72 (2H, d, J = 5.5 Hz), 6.35 (1H, br d, J = 3.0 Hz), 6.42 (1H, dd, J = 3.0, 1.8 Hz), 6.81 (1H, s), 7.21-7.38 (6H, m), 7.44 (1H, t, J = 8.2 Hz), 7.61 (1H, br s), 7.84 (1H, d, J = 7.9 Hz), 9.12 (1H, t, J = 5.5 Hz). |
| | | MS (m/z): 464 (M+H)⁺. |
| 59 | 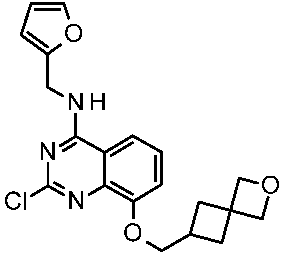 | 1H-NMR (DMSO-D6) δ: 2.07-2.12 (2H, m), 2.37-2.42 (2H, m), 2.52-2.61 (1H, m), 4.01 (2H, d, J = 6.7 Hz), 4.58 (4H, s), 4.71 (2H, d, J = 5.5 Hz), 6.34 (1H, d, J = 3.1 Hz), 6.42 (1H, dd, J = 3.1, 1.8 Hz), 7.30 (1H, d, J = 7.4 Hz), 7.43 (1H, t, J = 8.0 Hz), 7.60-7.61 (1H, m), 7.80 (1H, d, J = 7.4 Hz), 9.08 (1H, t, J = 5.8 Hz). |
| | | MS (m/z): 386 (M+H)⁺. |
| 60 | 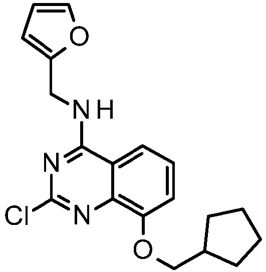 | 1H-NMR (DMSO-D6) δ: 1.35-1.41 (2H, m), 1.52-1.66 (4H, m), 1.77-1.83 (2H, m), 2.38-2.45 (1H, m), 3.97 (2H, d, J = 6.7 Hz), 4.71 (2H, br s), 6.35 (1H, d, J=3.1 Hz), 6.42 (1H, dd, J=3.1, 1.8 Hz), 7.32 (1H, d, J = 7.4 Hz), 7.42 (1H, t, J = 8.0 Hz), 7.60 (1H, d, J = 1.8 Hz), 7.79 (1H, dd, J = 8.6, 1.2 Hz), 9.07 (1H, br s). |
| | | MS (m/z): 358 (M+H)⁺. |
| 61 | 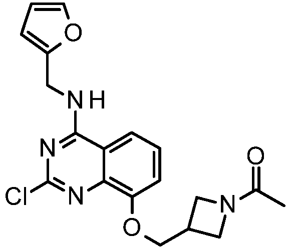 | 1H-NMR (DMSO-D6) δ: 1.76 (3H, s), 3.05-3.13 (1H, m), 3.67 (1H, dd, J = 9.4, 5.2 Hz), 3.95-4.01 (2H, m), 4.26-4.31 (3H, m), 4.72 (2H, d, J = 5.5 Hz), 6.35 (1H, br d, J = 2.4 Hz), 6.42 (1H, dd, J = 3.0, 1.8 Hz), 7.37 (1H, dd, J = 7.9, 1.2 Hz), 7.46 (1H, t, J = 8.2 Hz), 7.60-7.61 (1H, m), 7.84 (1H, dd, J = 8.5, 1.2 Hz), 9.12 (1H, t, J = 5.8 Hz). |
| | | MS (m/z): 387 (M+H)⁺. |
| 62 | 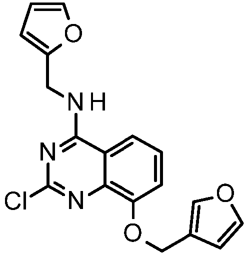 | 1H-NMR (DMSO-D6) δ: 4.71 (2H, br s), 5.11 (2H, s), 6.35 (1H, br d, J = 3.0 Hz), 6.42 (1H, dd, J = 3.0, 1.8 Hz), 6.63 (1H, br d, J = 1.8 Hz), 7.42-7.47 (2H, m), 7.60 (1H, d, J = 1.2 Hz), 7.69-7.70 (1H, m), 7.81-7.84 (2H, m), 9.11 (1H, br s). |
| | | MS (m/z): 356 (M+H)⁺. |
| 63 | 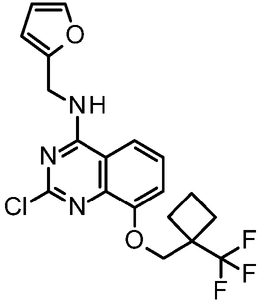 | 1H-NMR (DMSO-D6) δ: 1.90-1.99 (1H, m), 2.18-2.37 (5H, m), 4.34 (2H, s), 4.72 (2H, d, J = 5.5 Hz), 6.35 (1H, d, J = 2.5 Hz), 6.42 (1H, dd, J = 3.1, 1.8 Hz), 7.46-7.47 (2H, m), 7.61 (1H, br s), 7.88 (1H, dd, J = 6.1, 3.1 Hz), 9.13 (1H, t, J = 5.5 Hz). |
| | | MS (m/z): 412 (M+H)⁺. |
| 64 | 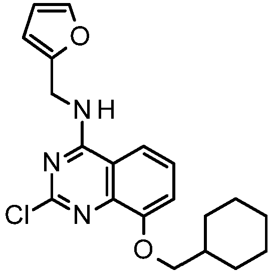 | 1H-NMR (DMSO-D6) δ: 1.01-1.10 (2H, m), 1.16-1.32 (3H, m), 1.65-1.74 (3H, br m), 1.83-1.90 (3H, br m), 3.90 (2H, d, J = 6.1 Hz), 4.71 (2H, br s), 6.35 (1H, d, J = 3.1 Hz), 6.42 (1H, dd, J = 3.1, 1.8 Hz), 7.30 (1H, dd, J = 8.0, 1.2 Hz), 7.42 (1H, t, J = 8.0 Hz), 7.61 (1H, d, J = 1.8 Hz), 7.79 (1H, dd, J = 8.6, 1.2 Hz), 9.08 (1H, br s). |
| | | MS (m/z): 372 (M+H)⁺. |
| 65 | 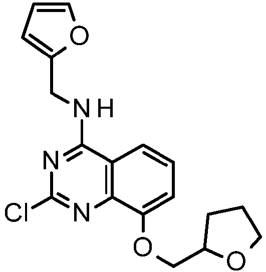 | 1H-NMR (DMSO-D6) δ: 1.67-1.76 (1H, m), 1.79-2.08 (3H, m), 3.67-3.72 (1H, m), 3.79-3.84 (1H, m), 4.02-4.11 (2H, m), 4.23-4.29 (1H, m), 4.71 (2H, br s), 6.35 (1H, dd, J = 3.1, 1.2 Hz), 6.42 (1H, dd, J = 3.1, 1.8 Hz), 7.33 (1H, d, J = 7.4 Hz), 7.43 (1H, t, J = 8.0 Hz), 7.60 (1H, d, J = 1.8 Hz), 7.81 (1H, dd, J = 8.6, 1.8 Hz), 9.09 (1H, br s). |
| | | MS (m/z): 360 (M+H)⁺. |
| 66 | 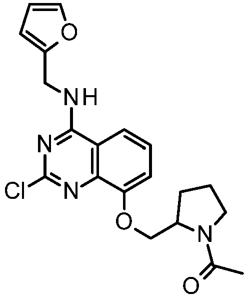 | 1H-NMR (DMSO-D6) δ: 1.91-2.04 (6H, m), 3.40-3.46 (1H, m), 3.52-3.57 (1H, m), 4.02-4.07 (2H, m), 4.19 (1H, dd, J = 9.7, 3.0 Hz), 4.28-4.32 (1H, br m), 4.72 (2H, br d, J = 4.6 Hz), 6.35 (1H, br d, J = 3.0 Hz), 6.42 (1H, dd, J = 3.0, 1.8 Hz), 7.42-7.46 (2H, m), 7.60 (1H, br d, J = 1.8 Hz), 7.81 (1H, dd, J = 10.0, 5.8 Hz), 9.10 (1H, t, J = 4.6 Hz). |
| | | MS (m/z): 401 (M+H)⁺. |
| 67 | 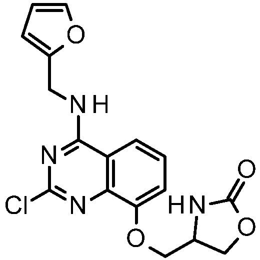 | 1H-NMR (DMSO-D6) δ: 4.11-4.17 (2H, m), 4.22-4.27 (2H, m), 4.49 (1H, t, J = 10.3 Hz), 4.72 (2H, br s), 6.35 (1H, br d, J = 3.2 Hz), 6.42 (1H, dd, J = 3.2, 1.8 Hz), 7.40 (1H, dd, J = 7.9, 1.2 Hz), 7.46 (1H, t, J = 7.9 Hz), 7.61 (1H, dd, J = 1.8, 1.2 Hz), 7.87 (1H, dd, J = 7.9, 1.2 Hz), 7.98 (1H, br s), 9.14 (1H, br s). |
| | | MS (m/z): 375 (M+H)⁺. |
| 68 | 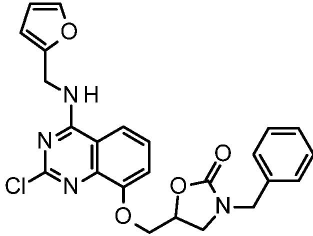 | 1H-NMR (DMSO-D6) δ: 3.36-3.40 (1H, m), 3.62 (1H, t, J = 8.8 Hz), 4.26 (1H, dd, J = 11.5, 5.5 Hz), 4.34 (1H, dd, J = 10.9, 3.0 Hz), 4.39 (1H, d, J = 15.2 Hz), 4.45 (1H, d, J = 15.2 Hz), 4.73 (2H, br s), 4.97-5.03 (1H, m), 6.36-6.37 (1H, m), 6.42-6.43 (1H, m), 7.27-7.37 (6H, m), 7.45 (1H, t, J = 7.9 Hz), 7.60-7.61 (1H, m), 7.86 (1H, d, J = 7.3 Hz), 9.14 (1H, br s). |
| | | MS (m/z): 465 (M+H)⁺. |
| 69 | 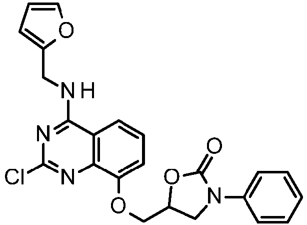 | 1H-NMR (DMSO-D6) δ: 4.01-4.05 (1H, m), 4.28 (1H, t, J = 9.1 Hz), 4.38-4.48 (2H, m), 4.72 (2H, br s), 5.13-5.19 (1H, m), 6.35-6.36 (1H, m), 6.41-6.43 (1H, m), 7.12-7.16 (1H, m), 7.38-7.43 (3H, m), 7.47 (1H, t, J = 7.9 Hz), 7.60-7.62 (3H, m), 7.87 (1H, dd, J = 8.5, 1.2 Hz), 9.15 (1H, br s). |
| | | MS (m/z): 451 (M+H)⁺. |
| 70 | 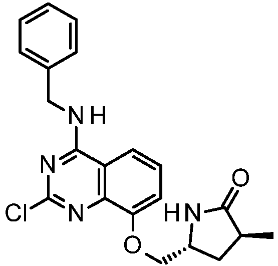 | 1H-NMR (DMSO-D6) δ: 1.13 (3H, d, J = 7.3 Hz), 1.48-1.56 (1H, m), 2.30-2.48 (2H, m), 3.89-3.96 (1H, m), 4.03 (1H, dd, J = 10.3, 5.5 Hz), 4.11 (1H, dd, J = 10.3, 4.9 Hz), 4.75 (2H, s), 7.24-7.28 (1H, m), 7.32-7.38 (5H, m), 7.46 (1H, t, J = 8.2 Hz), 7.81 (1H, s), 7.87 (1H, d, J = 7.9 Hz), 9.21 (1H, s). |
| | | MS (m/z): 397 (M+H)⁺. |
| 71 | 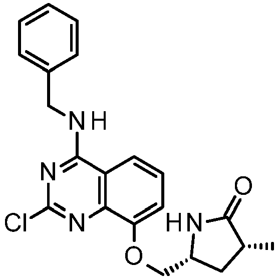 | 1H-NMR (DMSO-D6) δ: 1.06 (3H, d, J = 6.7Hz), 1.80-1.87 (1H, m), 2.21-2.27 (1H, m), 2.69-2.78 (1H, m), 3.85-3.90 (1H, br m), 4.03 (1H, dd, J = 10.0, 4.6 Hz), 4.09 (1H, dd, J = 10.0, 5.5 Hz), 4.75 (2H, s), 7.24-7.28 (1H, m), 7.32-7.38 (5H, m), 7.45 (1H, t, J = 8.2 Hz), 7.77 (1H, s), 7.86 (1H, d, J = 7.3 Hz), 9.20 (1H, br s). |
| | | MS (m/z): 397 (M+H)⁺. |
| 72 | 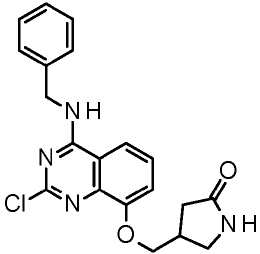 | 1H-NMR (DMSO-D6) δ: 2.08 (1H, dd, J = 16.8, 6.4 Hz), 2.36 (1H, dd, J = 16.8, 8.9 Hz), 2.90-3.00 (1H, m), 3.16 (1H, dd, J = 9.8, 4.9 Hz), 3.45 (1H, dd, J = 9.8, 7.6 Hz), 4.05-4.13 (2H, m), 4.74-4.76 (2H, m), 7.24-7.28 (1H, m), 7.32-7.38 (5H, m), 7.46 (1H, t, J = 8.2 Hz), 7.60 (1H, br s), 7.85 (1H, d, J = 7.3 Hz), 9.19 (1H, t, J = 5.8 Hz). |
| | | MS (m/z): 383 (M+H)⁺. |
| 73 | 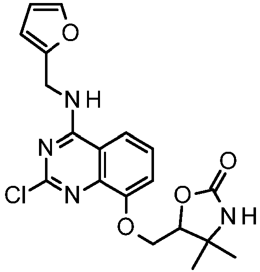 | 1H-NMR (DMSO-D6) δ: 1.21 (3H, s), 1.38 (3H, s), 4.34 (2H, d, J = 6.1 Hz), 4.60 (1H, t, J = 6.1 Hz), 4.72 (2H, d, J = 5.5 Hz), 6.36 (1H, dd, J = 3.0, 0.6 Hz), 6.42 (1H, dd, J = 3.0, 1.8 Hz), 7.41-7.49 (2H, m), 7.61-7.61 (1H, m), 7.80 (1H, s), 7.85 (1H, dd, J = 8.2, 1.5 Hz), 9.14 (1H, t, J = 5.5 Hz). |
| | | MS (m/z): 403 (M+H)⁺. |
| 74 | 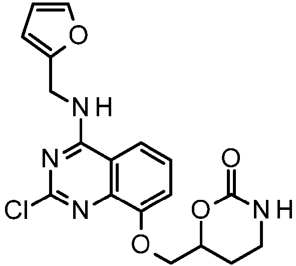 | 1H-NMR (DMSO-D6) δ: 1.80-1.91 (1H, m), 2.02-2.08 (1H, m), 3.24-3.30 (2H, m), 4.25 (2H, d, J = 5.5 Hz), 4.68-4.73 (3H, m), 6.36 (1H, d, J = 3.0 Hz), 6.42 (1H, dd, J = 3.0, 1.8 Hz), 7.31 (1H, br s), 7.38 (1H, d, J = 7.9 Hz), 7.46 (1H, t, J = 7.9 Hz), 7.61 (1H, br s), 7.85 (1H, d, J = 8.5 Hz), 9.12 (1H, t, J = 5.5 Hz). |
| | | MS (m/z): 389 (M+H)⁺. |
| 75 | 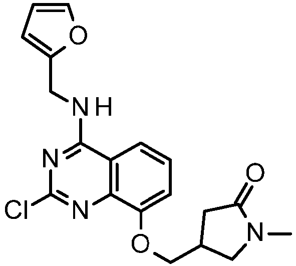 | 1H-NMR (DMSO-D6) δ: 2.17 (1H, dd, J = 16.4, 6.1 Hz), 2.44-2.49 (1H, m), 2.74 (3H, s), 2.84-2.93 (1H, br m), 3.26-3.30 (1H, m), 3.56 (1H, dd, J = 9.7, 8.5 Hz), 4.05-4.13 (2H, m), 4.72 (2H, d, J = 5.8 Hz), 6.35 (1H, d, J = 3.0 Hz), 6.42 (1H, dd, J = 3.0, 1.8 Hz), 7.36 (1H, dd, J = 7.9, 1.8 Hz), 7.45 (1H, t, J = 7.9 Hz), 7.61 (1H, d, J = 1.8 Hz), 7.83 (1H, dd, J = 8.5, 1.8 Hz), 9.11 (1H, t, J = 5.8 Hz). |
| | | MS (m/z): 387 (M+H)⁺. |
| 76 | 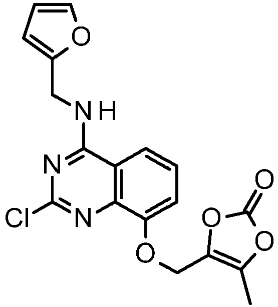 | 1H-NMR (DMSO-D6) δ: 2.21 (3H, s), 4.72 (2H, br s), 5.15 (2H, s), 6.36 (1H, d, J = 3.1 Hz), 6.41-6.44 (1H, m), 7.43-7.51 (2H, m), 7.61 (1H, br s), 7.90 (1H, d, J = 7.3 Hz), 9.18 (1H, br s). |
| | | MS (m/z): 388 (M+H)⁺. |
| 77 | 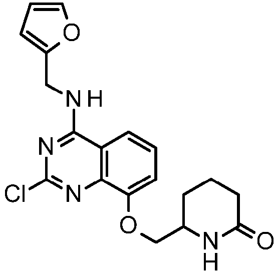 | 1H-NMR (DMSO-D6) δ: 1.61-1.71 (2H, m), 1.82-1.97 (2H, m), 2.13-2.20 (2H, br m), 3.71-3.79 (1H, br m), 4.02-4.13 (2H, m), 4.72 (2H, d, J = 5.5 Hz), 6.35 (1H, d, J = 3.7 Hz), 6.42 (1H, dd, J = 3.7, 1.8 Hz), 7.36-7.38 (2H, m), 7.45 (1H, t, J = 7.9 Hz), 7.60 (1H, d, J = 1.8 Hz), 7.84 (1H, dd, J = 8.5, 1.2 Hz), 9.11 (1H, t, J = 5.8 Hz). |
| | | MS (m/z): 387 (M+H)⁺. |
| 78 | 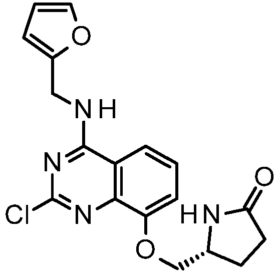 | 1H-NMR (DMSO-D6) δ: 1.89-2.00 (1H, m), 2.05-2.28 (2H, m), 2.42-2.57 (1H, m), 3.92-4.00 (1H, m), 4.03 (1H, dd, J = 10.0, 4.6 Hz), 4.10 (1H, dd, J = 9.7, 4.9 Hz), 4.72 (2H, d, J = 5.5 Hz), 6.35 (1H, d, J = 3.0 Hz), 6.41-6.43 (1H, m), 7.36 (1H, d, J = 7.9 Hz), 7.45 (1H, t, J = 8.2 Hz), 7.61 (1H, s), 7.78 (1H, s), 7.84 (1H, d, J = 8.5 Hz), 9.12 (1H, t, J = 5.8 Hz). |
| | | MS (m/z): 373 (M+H)⁺. |
| 79 | 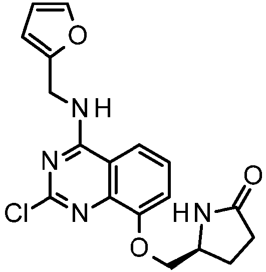 | 1H-NMR (DMSO-D6) δ: 1.89-2.00 (1H, m), 2.04-2.27 (2H, m), 2.43-2.52 (1H, m), 3.92-3.99 (1H, m), 4.03 (1H, dd, J = 10.0, 4.6 Hz), 4.10 (1H, dd, J = 9.7, 4.9 Hz), 4.72 (2H, d, J = 5.5 Hz), 6.35 (1H, d, J = 3.0 Hz), 6.42 (1H, dd, J = 3.0, 1.8 Hz), 7.36 (1H, d, J = 7.9 Hz), 7.45 (1H, t, J = 8.2 Hz), 7.61 (1H, s), 7.78 (1H, s), 7.84 (1H, d, J = 8.5 Hz), 9.11 (1H, t, J = 5.5 Hz). |
| | | MS (m/z): 373 (M+H)⁺. |
| 80 | 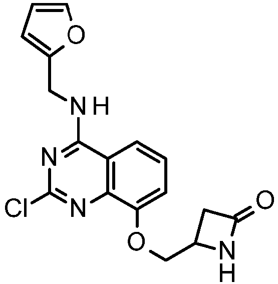 | 1H-NMR (DMSO-D6) δ: 2.63-2.78 (1H, m), 3.01-3.09 (1H, m), 3.95-4.04 (1H, m), 4.15 (1H, dd, J = 10.3, 7.3 Hz), 4.26 (1H, dd, J = 10.3, 4.3 Hz), 4.72 (2H, d, J = 5.5 Hz), 6.36 (1H, d, J = 3.0 Hz), 6.42 (1H, dd, J = 3.0, 1.8 Hz), 7.37 (1H, d, J = 7.9 Hz), 7.46 (1H, t, J = 8.2 Hz), 7.61 (1H, d, J = 1.8 Hz), 7.85 (1H, d, J = 7.3 Hz), 8.31 (1H, s), 9.12 (1H, t, J = 5.5 Hz). |
| | | MS (m/z): 359 (M+H)⁺. |
| 81 | 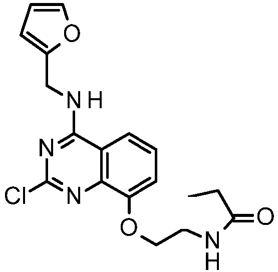 | 1H-NMR (DMSO-D6) δ: 1.30 (3H, s), 1.75-1.87 (1H, m), 2.08-2.27 (2H, m), 2.71-2.81 (1H, m), 3.89 (1H, d, J = 9.7 Hz), 4.02 (1H, t, J = 6.4 Hz), 4.72 (2H, d, J = 5.5 Hz), 6.35 (1H, d, J = 3.0 Hz), 6.42 (1H, dd, J = 3.3, 2.1 Hz), 7.34 (1H, d, J = 7.3 Hz), 7.44 (1H, t, J = 8.2 Hz), 7.60 (1H, d, J = 1.8 Hz), 7.69 (1H, s), 7.83 (1H, d, J = 7.3 Hz), 9.10 (1H, t, J = 5.5 Hz). |
| | | MS (m/z): 387 (M+H)⁺. |
| 82 | 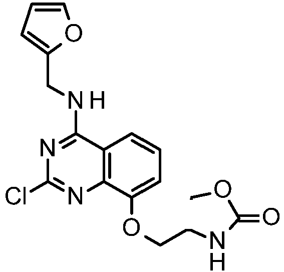 | 1H-NMR (DMSO-D6) δ: 1.38 (3H, s), 4.00 (1H, d, J = 10.4 Hz), 4.07 (2H, t, J = 9.2 Hz), 4.41 (1H, d, J = 9.2 Hz), 4.72 (2H, d, J = 5.5 Hz), 6.35 (1H, d, J = 3.1 Hz), 6.42 (1H, t, J = 2.7 Hz), 7.39-7.49 (2H, m), 7.61 (1H, s), 7.88 (1H, d, J = 6.7 Hz), 7.94 (1H, s), 9.14 (1H, t, J = 5.8 Hz). |
| | | MS (m/z): 389 (M+H)⁺. |
| 83 | 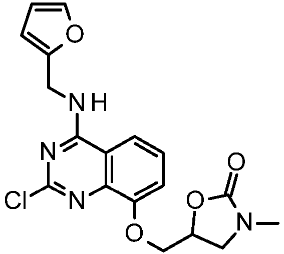 | 1H-NMR (DMSO-D6) δ: 1.38 (3H, s), 4.00 (1H, d, J = 10.4 Hz), 4.07 (2H, t, J = 9.2 Hz), 4.41 (1H, d, J = 9.2 Hz), 4.72 (2H, d, J = 5.5 Hz), 6.35 (1H, d, J = 3.1 Hz), 6.42 (1H, t, J = 2.7 Hz), 7.39-7.49 (2H, m), 7.61 (1H, s), 7.88 (1H, d, J = 6.7 Hz), 7.94 (1H, s), 9.14 (1H, t, J = 5.8 Hz). |
| | | MS (m/z): 389 (M+H)⁺. |
| 84 | 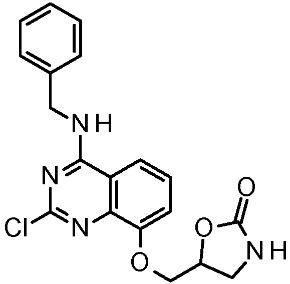 | 1H-NMR (DMSO-D6) δ: 3.36-3.44 (1H, m), 3.65 (1H, t, J = 8.8 Hz), 4.23-4.35 (2H, m), 4.75 (2H, d, J = 5.5 Hz), 4.97-5.06 (1H, m), 7.23-7.29 (1H, m), 7.31-7.42 (5H, m), 7.47 (1H, t, J = 8.2 Hz), 7.65 (1H, s), 7.88 (1H, d, J = 8.5 Hz), 9.22 (1H, t, J = 5.8 Hz). |
| | | MS (m/z): 385 (M+H)⁺. |
| 85 | 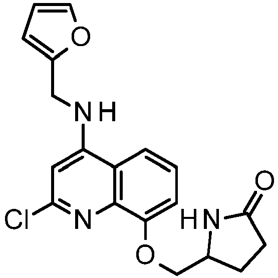 | 1H-NMR (DMSO-D6) δ: 1.92-1.99 (1H, m), 2.05-2.13 (1H, m), 2.16-2.26 (1H, m), 2.56 (1H, dd, J = 9.1, 7.3 Hz), 3.93-3.98 (1H, m), 4.02 (1H, dd, J = 10.0, 4.6 Hz), 4.10 (1H, dd, J = 9.7, 4.9 Hz), 4.54 (2H, d, J = 5.8 Hz), 6.39 (1H, d, J = 3.6 Hz), 6.41 (1H, dd, J = 3.0, 1.8 Hz), 6.56 (1H, s), 7.20 (1H, d, J = 7.3 Hz), 7.38 (1H, t, J = 8.2 Hz), 7.61 (1H, br s), 7.77-7.80 (2H, m), 7.99 (1H, t, J = 5.8 Hz). |
| | | MS (m/z): 372 (M+H)⁺. |
| 86 | 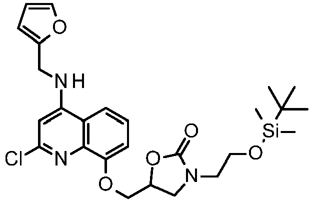 | 1H-NMR (DMSO-D6) δ: 0.08 (3H, s), 0.09 (3H, s), 0.89 (9H, s), 3.35-3.38 (2H, m), 3.61-3.67 (1H, m), 3.74-3.89 (3H, m), 4.28 (1H, dd, J = 10.9, 6.1 Hz), 4.37 (1H, dd, J = 10.9, 3.6 Hz), 4.60 (2H, d, J = 5.5 Hz), 4.99-5.05 (1H, m), 6.45-6.48 (2H, m), 6.63 (1H, s), 7.27 (1H, d, J = 7.3 Hz), 7.44 (1H, t, J = 8.2 Hz), 7.67 (1H, d, J = 1.8 Hz), 7.87 (1H, d, J = 7.9 Hz), 8.06 (1H, t, J = 5.5 Hz). |
| 87 | 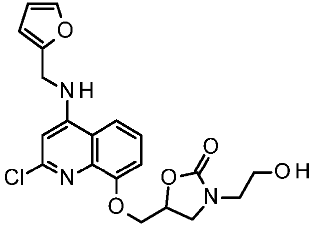 | 1H-NMR (DMSO-D6) δ: 3.21-3.28 (2H, m), 3.52-3.61 (3H, m), 3.82 (1H, t, J = 8.8 Hz), 4.24 (1H, dd, J = 10.9, 6.1 Hz), 4.32 (1H, dd, J = 10.9, 3.6 Hz), 4.54 (2H, d, J = 5.5 Hz), 4.82 (1H, t, J = 5.5 Hz), 4.93-4.99 (1H, m), 6.39-6.42 (2H, m), 6.57 (1H, s), 7.22 (1H, d, J = 6.7 Hz), 7.39 (1H, t, J = 7.9 Hz), 7.60-7.62 (1H, m), 7.81 (1H, d, J = 8.5 Hz), 8.01 (1H, t, J = 5.5 Hz). |
| | | MS (m/z): 418 (M+H)⁺. |
| 88 | 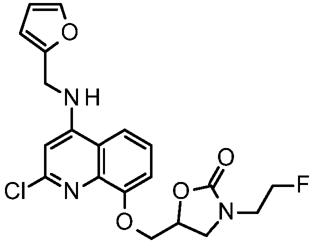 | 1H-NMR (DMSO-D6) δ: 3.49-3.52 (1H, m), 3.56-3.61 (2H, m), 3.83 (1H, t, J = 8.8 Hz), 4.24 (1H, dd, J = 10.9, 5.5 Hz), 4.34 (1H, dd, J = 11.2, 3.3 Hz), 4.53-4.76 (4H, m), 4.97-5.03 (1H, m), 6.39-6.42 (2H, m), 6.57 (1H, s), 7.22 (1H, d, J = 7.3 Hz), 7.39 (1H, t, J = 8.2 Hz), 7.61 (1H, d, J = 1.8 Hz), 7.81 (1H, d, J = 8.5 Hz), 8.01 (1H, t, J = 6.1 Hz). |
| | | MS (m/z): 420 (M+H)⁺. |
| 89 | 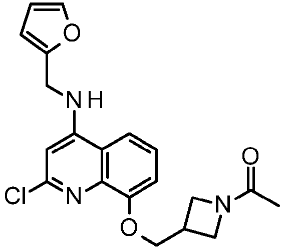 | 1H-NMR (DMSO-D6) δ: 1.76 (3H, s), 3.04-3.13 (1H, m), 3.68 (1H, dd, J = 9.7, 5.5 Hz), 3.95-4.02 (2H, m), 4.23-4.31 (3H, m), 4.54 (2H, d, J = 6.1 Hz), 6.39 (1H, br d, J = 3.6 Hz), 6.41 (1H, dd, J = 3.0, 1.8 Hz), 6.56 (1H, s), 7.22 (1H, d, J = 7.9 Hz), 7.39 (1H, t, J = 8.2 Hz), 7.61 (1H, br d, J = 1.8 Hz), 7.79 (1H, d, J = 8.5 Hz), 7.99 (1H, t, J = 6.1 Hz). |
| | | MS (m/z): 386 (M+H)⁺. |
| 90 | 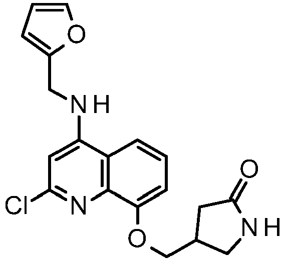 | 1H-NMR (DMSO-D6) δ: 2.10 (1H, dd, J = 17.0, 6.4 Hz), 2.36 (1H, dd, J = 17.0, 9.1 Hz), 2.89-3.00 (1H, m), 3.18 (1H, dd, J = 9.7, 4.9 Hz), 3.45 (1H, dd, J = 9.7, 7.9 Hz), 4.04-4.12 (2H, m), 4.54 (2H, d, J = 6.1 Hz), 6.39 (1H, br d, J = 3.6 Hz), 6.42 (1H, dd, J = 3.0, 1.8 Hz), 6.56 (1H, s), 7.14-7.28 (1H, m), 7.38 (1H, t, J = 8.2 Hz), 7.60 (2H, d, J = 7.9 Hz), 7.78 (1H, d, J = 8.5 Hz), 7.98 (1H, t, J = 6.1 Hz). |
| | | MS (m/z): 372 (M+H)⁺. |
| 91 | 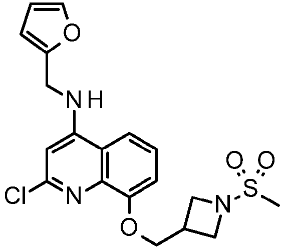 | 1H-NMR (DMSO-D6) δ: 3.13-3.20 (4H, m), 3.94-4.00 (4H, m), 4.19 (2H, d, J = 4.9 Hz), 4.55 (2H, d, J = 6.1 Hz), 6.39 (1H, br d, J = 3.0 Hz), 6.42 (1H, dd, J = 3.0, 1.8 Hz), 6.57 (1H, s), 7.21 (1H, d, J = 7.9 Hz), 7.40 (1H, t, J = 8.2 Hz), 7.61 (1H, d, J = 1.8 Hz), 7.80 (1H, d, J = 8.5 Hz), 8.01 (1H, t, J = 6.1 Hz). |
| | | MS (m/z): 422 (M+H)⁺. |
| 92 | 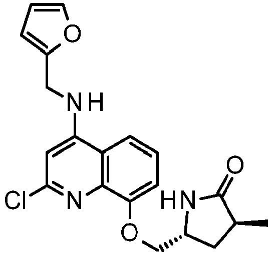 | 1H-NMR (DMSO-D6) δ: 1.13 (3H, d, J= 7.3 Hz), 1.51-1.58 (1H, m), 2.32-2.47 (2H, m), 3.88-3.96 (1H, m), 4.02 (1H, dd, J = 10.0, 5.2 Hz), 4.11 (1H, dd, J = 10.0, 4.6 Hz), 4.54 (2H, d, J = 6.1 Hz), 6.39-6.42 (2H, m), 6.56 (1H, s), 7.21 (1H, d, J = 7.3 Hz), 7.38 (1H, t, J = 8.2 Hz), 7.61 (1H, br d, J = 1.8 Hz), 7.79-7.81 (2H, m), 7.99 (1H, t, J = 6.1 Hz). |
| | | MS (m/z): 386 (M+H)⁺. |
| 93 | 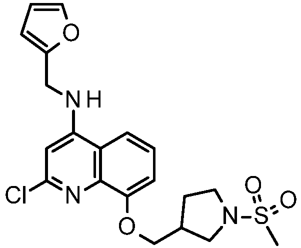 | 1H-NMR (DMSO-D6) δ: 1.81-1.89 (1H, m), 2.06-2.15 (1H, m), 2.78-2.86 (1H, m), 2.95 (3H, s), 3.21 (1H, dd, J = 10.1, 6.4 Hz), 3.25-3.32 (1H, m), 3.46-3.52 (2H, m), 4.06-4.15 (2H, m), 4.54 (2H, d, J = 6.1 Hz), 6.39 (1H, br d, J = 2.4 Hz), 6.42 (1H, dd, J = 3.1, 1.8 Hz), 6.56 (1H, s), 7.19 (1H, d, J = 7.3 Hz), 7.38 (1H, t, J = 7.9 Hz), 7.61 (1H, d, J = 1.8 Hz), 7.77 (1H, d, J = 8.5 Hz), 7.99 (1H, t, J = 6.1 Hz). |
| | | MS (m/z): 436 (M+H)⁺. |
| 94 | 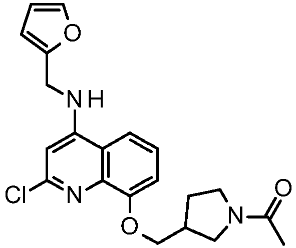 | 1H-NMR (DMSO-D6) δ: 1.70-2.17 (5H, m), 2.67-2.85 (1H, m), 3.20-3.32 (1H, m), 3.35-3.55 (2H, m), 3.61-3.70 (1H, m), 4.01-4.14 (2H, m), 4.53 (2H, d, J = 5.5 Hz), 6.38-6.41 (2H, m), 6.55 (1H, d, J = 1.2 Hz), 7.19 (1H, dd, J = 13.7, 7.6 Hz), 7.37 (1H, td, J = 8.0, 3.2 Hz), 7.60 (1H, s), 7.77 (1H, t, J = 7.6 Hz), 7.96-7.99 (1H, m). |
| | | MS (m/z): 400 (M+H)⁺. |
| 95 | 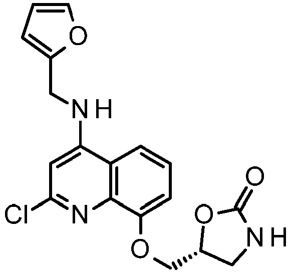 | 1H-NMR (DMSO-D6) δ: 3.41 (1H, t, J = 7.9 Hz), 3.65 (1H, t, J = 8.8 Hz), 4.23-4.32 (2H, m), 4.54 (2H, d, J = 5.5 Hz), 4.98-5.07 (1H, m), 6.38-6.43 (2H, m), 6.57 (1H, s), 7.23 (1H, d, J = 7.9 Hz), 7.39 (1H, t, J = 8.2 Hz), 7.60-7.65 (2H, m), 7.81 (1H, d, J = 7.9 Hz), 8.01 (1H, t, J = 5.8 Hz). |
| | | MS (m/z): 374 (M+H)⁺. |
| 96 | 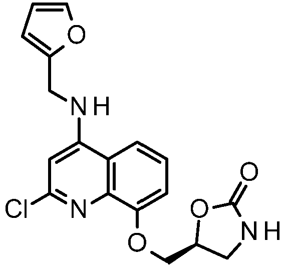 | 1H-NMR (DMSO-D6) δ: 3.41 (1H, t, J = 7.9 Hz), 3.65 (1H, t, J = 8.8 Hz), 4.22-4.33 (2H, m), 4.54 (2H, d, J = 5.5 Hz), 4.98-5.07 (1H, m), 6.38-6.43 (2H, m), 6.57 (1H, s), 7.23 (1H, d, J = 7.3 Hz), 7.39 (1H, t, J = 8.2 Hz), 7.62 (2H, d, J = 9.7 Hz), 7.81 (1H, d, J = 8.5 Hz), 8.01 (1H, t, J = 5.8 Hz). |
| | | MS (m/z): 374 (M+H)⁺. |
| 97 | 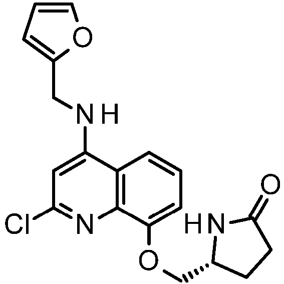 | 1H-NMR (DMSO-D6) δ: 1.89-2.03 (1H, m), 2.04-2.15 (1H, m), 2.15-2.29 (1H, m), 2.50-2.60 (1H, m), 3.92-3.99 (1H, m), 3.99-4.06 (1H, m), 4.10 (1H, dd, J = 9.7, 4.9 Hz), 4.54 (2H, d, J = 5.5 Hz), 6.39 (1H, d, J = 3.0 Hz), 6.41 (1H, dd, J = 3.0, 1.8 Hz), 6.56 (1H, s), 7.20 (1H, d, J = 7.3 Hz), 7.38 (1H, t, J = 8.2 Hz), 7.61 (1H, d, J = 1.2 Hz), 7.76 (1H, s), 7.79 (1H, d, J = 8.5 Hz), 7.99 (1H, t, J = 5.8 Hz). |
| | | MS (m/z): 372 (M+H)⁺. |
| 98 | 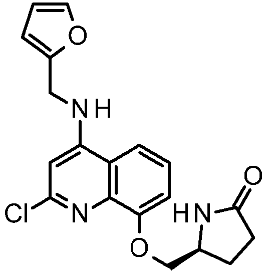 | 1H-NMR (DMSO-D6) δ: 1.90-2.01 (1H, m), 2.04-2.15 (1H, m), 2.15-2.28 (1H, m), 2.49-2.60 (1H, m), 3.92-3.99 (1H, m), 4.02 (1H, dd, J = 9.7, 4.9 Hz), 4.10 (1H, dd, J = 9.7, 4.9 Hz), 4.54 (2H, d, J = 5.5 Hz), 6.37-6.44 (2H, m), 6.57 (1H, s), 7.20 (1H, d, J = 7.3 Hz), 7.38 (1H, t, J = 8.2 Hz), 7.61 (1H, d, J = 1.2 Hz), 7.76 (1H, s), 7.79 (1H, d, J = 8.5 Hz), 8.00 (1H, t, J = 5.5 Hz). |
| | | MS (m/z): 372 (M+H)⁺. |
| 99 | 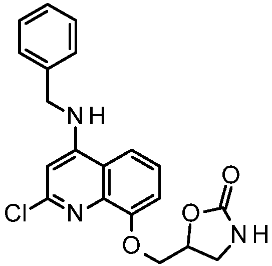 | 1H-NMR (DMSO-D6) δ: 3.36-3.45 (1H, m), 3.64 (1H, t, J = 8.8 Hz), 4.22-4.33 (2H, m), 4.57 (2H, d, J = 6.1 Hz), 4.97-5.07 (1H, m), 6.32 (1H, s), 7.25 (2H, t, J = 6.7 Hz), 7.31-7.45 (5H, m), 7.64 (1H, s), 7.88 (1H, d, J = 8.5 Hz), 8.19 (1H, t, J = 6.1 Hz). |
| | | MS (m/z): 384 (M+H)⁺. |
| 100 | 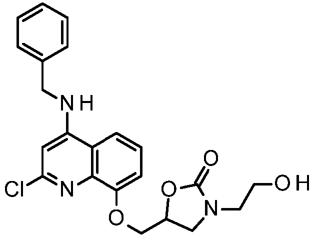 | 1H-NMR (DMSO-D6) δ: 3.21-3.27 (2H, m), 3.52-3.60 (3H, m), 3.81 (1H, t, J = 8.9 Hz), 4.24 (1H, dd, J = 11.0, 6.1 Hz), 4.32 (1H, dd, J = 11.0, 3.1 Hz), 4.57 (2H, d, J = 6.1 Hz), 4.82 (1H, t, J = 5.5 Hz), 4.92-4.98 (1H, m), 6.32 (1H, s), 7.23-7.28 (2H, m), 7.33-7.44 (5H, m), 7.88 (1H, d, J = 8.5 Hz), 8.20 (1H, t, J = 6.1 Hz). |
| | | MS (m/z): 428 (M+H)⁺. |
| 101 | 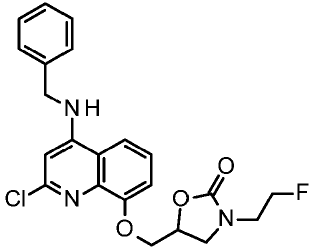 | 1H-NMR (DMSO-D6) δ: 3.48-3.51 (1H, m), 3.55-3.61 (2H, m), 3.82 (1H, t, J = 8.8 Hz), 4.24 (1H, dd, J = 10.9, 6.1 Hz), 4.35 (1H, dd, J = 10.9, 3.3 Hz), 4.52-4.75 (4H, m), 4.96-5.02 (1H, m), 6.32 (1H, s), 7.23-7.28 (2H, m), 7.32-7.44 (5H, m), 7.88 (1H, d, J = 8.5 Hz), 8.20 (1H, t, J = 6.1 Hz). |
| | | MS (m/z): 430 (M+H)⁺. |
| 102 | 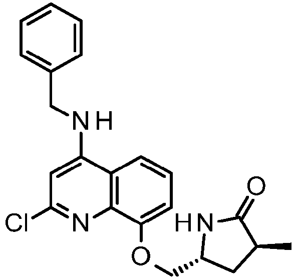 | 1H-NMR (DMSO-D6) δ: 1.13 (3H, d, J= 7.3 Hz), 1.50-1.57 (1H, m), 2.31-2.46 (2H, m), 3.89-3.95 (1H, m), 4.02 (1H, dd, J = 10.3, 5.5 Hz), 4.11 (1H, dd, J = 10.3, 4.9 Hz), 4.57 (2H, d, J = 6.1 Hz), 6.31 (1H, s), 7.22-7.27 (2H, m), 7.32-7.43 (5H, m), 7.80 (1H, s), 7.87 (1H, d, J = 7.9 Hz), 8.18 (1H, t, J = 6.1 Hz). |
| | | MS (m/z): 396 (M+H)⁺. |
| 103 | 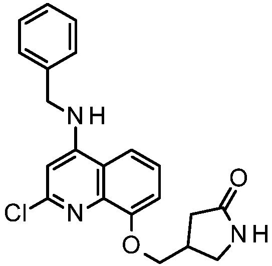 | 1H-NMR (DMSO-D6) δ: 2.09 (1H, dd, J = 16.7, 6.4 Hz), 2.36 (1H, dd, J = 16.7, 9.1 Hz), 2.89-2.99 (1H, m), 3.18 (1H, dd, J = 9.7, 5.5 Hz), 3.44 (1H, dd, J = 9.7, 8.5 Hz), 4.05-4.12 (2H, m), 4.57 (2H, d, J = 5.5 Hz), 6.31 (1H, s), 7.21-7.25 (2H, m), 7.33-7.43 (5H, m), 7.60 (1H, s), 7.85 (1H, d, J = 7.9 Hz), 8.17 (1H, t, J = 5.5 Hz). |
| | | MS (m/z): 382 (M+H)⁺. |

### (Example 104) 2-Chloro-8-(1,4-dioxan-2-ylmethoxy)-N-(furan-2-ylmethyl)-1,5-naphthyridin-4-amine

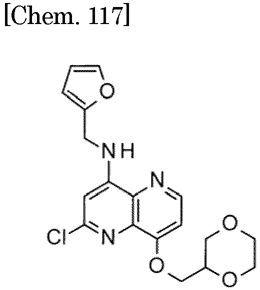

### (Example 104-1) 6-Chloro-8-[(furan-2-ylmethyl)amino]-1,5-naphthyridin-4-ol

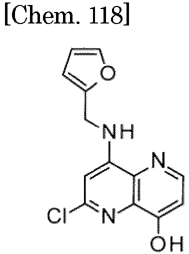

Furfurylamine (172 µL, 1.86 mmol) and N,N-diisopropylethylamine (410 µL, 2.35 mmol) were added to a solution of 6,8-dichloro-1,5-naphthyridin-4-ol (200 mg, 0.930 mmol) disclosed in WO 2017/055306 in NMP (3.7 mL), followed by stirring at 120°C for 17 hours. After the reaction solution was allowed to cool to room temperature, water was added, followed by extraction with ethyl acetate. The organic layer was washed with water and then with a saturated saline solution, dried over anhydrous magnesium sulfate, and then concentrated under reduced pressure. Ethyl acetate (5 mL) and methanol (2 mL) were added to the resulting residue, and the mixture was stirred at room temperature for 20 minutes. The precipitated solid was collected by filtration and dried under reduced pressure. Thus, the title compound (131 mg, 51% yield) was obtained as a gray solid.

### (Example 104-2) 2-Chloro-8-(1,4-dioxan-2-ylmethoxy)-N-(furan-2-ylmethyl)-1,5-naphthyridin-4-amine

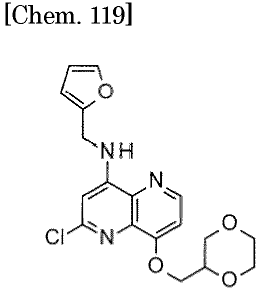

The same operation as in Example 18-3 was performed using 6-chloro-8-[(furan-2-ylmethyl)amino]-1,5-naphthyridin-4-ol (71.2 mg, 0.258 mmol) obtained in Example 104-1 and (1,4-dioxan-2-yl)methanol (45.0 µL, 0.420 mmol). Thus, the title compound (36.0 mg, 37% yield) was obtained as a white solid.

### (Example 105) 5-[({6-Chloro-8-[(furan-2-ylmethyl)amino]-1,5-naphthyridin-4-yl}oxy)methyl]-1,3-oxazolidin-2-one

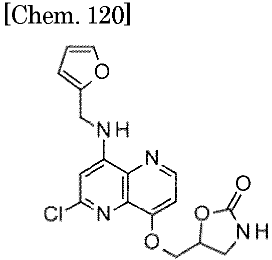

The same operation as in Example 18-3 was performed using 6-chloro-8-[(furan-2-ylmethyl)amino]-1,5-naphthyridin-4-ol (65.0 mg, 0.236 mmol) obtained in Example 104-1 and 5-(hydroxymethyl)-1,3-oxazolidin-2-one (45.6 mg, 0.389 mmol). Thus, the title compound (20.2 mg, 23% yield) was obtained as a white solid.

### (Example 106) 5-[({6-Chloro-8-[(furan-2-ylmethyl)amino]-1,5-naphthyridin-4-yl}oxy)methyl]-3-methyl-1,3-oxazolidin-2-one

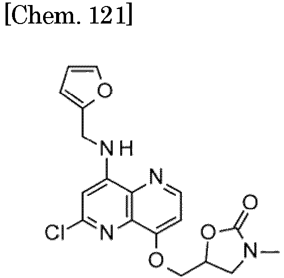

The same operation as in Example 18-3 was performed using 6-chloro-8-[(furan-2-ylmethyl)amino]-1,5-naphthyridin-4-ol (162 mg, 0.588 mmol) obtained in Example 104-1 and 5-(hydroxymethyl)-3-methyl-1,3-oxazolidin-2-one (125 mg, 0.953 mmol). Thus, the title compound (21.9 mg, 10% yield) was obtained as a white solid.

### (Example 107) (3S,5R)-5-[({6-Chloro-8-[(furan-2-ylmethyl)amino]-1,5-naphthyridin-4-yl}oxy)methyl]-3-methylpyrrolidin-2-one

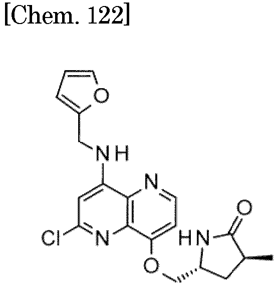

The same operation as in Example 18-3 was performed using 6-chloro-8-[(furan-2-ylmethyl)amino]-1,5-naphthyridin-4-ol (52.4 mg, 0.190 mmol) obtained in Example 104-1 and (3S,5R)-5-(hydroxymethyl)-3-methylpyrrolidin-2-one (36.8 mg, 0.285 mmol). Thus, the title compound (52.5 mg, 71% yield) was obtained as a light brown solid.

### (Example 108) 5-[({6-Chloro-8-[(furan-2-ylmethyl)amino]-1,5-naphthyridin-4-yl}oxy)methyl]-3-(2-hydroxyethyl)-1,3-oxazolidin-2-one

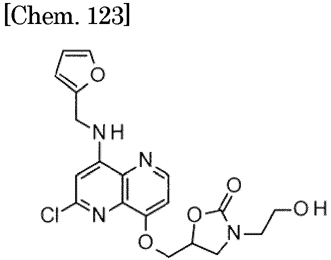

The same operation as in Example 18-3 was performed using 6-chloro-8-[(furan-2-ylmethyl)amino]-1,5-naphthyridin-4-ol (59.9 mg, 0.217 mmol) obtained in Example 104-1 and -3-(2-{[tert-butyl(dimethyl)silyl]oxy}ethyl)-5-(hydroxymethyl)-1,3 oxazolidin-2-one (91.1 mg, 0.331 mmol) obtained in Example 42-2. Thus, crude 3-(2-{[tert-butyl(dimethyl)silyl]oxy}ethyl)-5-[({6-chloro-8-[(furan-2-ylmethyl)amino]-1,5-naphthyridin-4-yl}oxy)methyl]-1,3-oxazolidin-2-one (164 mg) [MS (m/z): 533 (M)⁺] was obtained as a brown oily substance. The obtained 3-(2-{[tert-butyl(dimethyl)silyl]oxy}ethyl)-5-[({6-chloro-8-[(furan-2-ylmethyl)amino]-1,5-naphthyridin-4-yl}oxy)methyl]-1,3-oxazolidin-2-one was dissolved in THF (1.5 mL). 1M TBAF-THF (434 µL, 0.434 mmol) was added to the solution, and the same operation as in Example 42-3 was performed. Thus, the title compound (33.9 mg, 37% yield) was obtained as a white solid.

### (Example 109) 5-[({6-Chloro-8-[(furan-2-ylmethyl)amino]-1,5-naphthyridin-4-yl}oxy)methyl]-3-(2-fluoroethyl)-1,3-oxazolidin-2-one

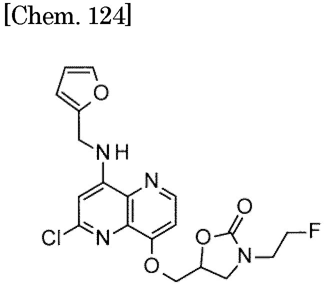

The same operation as in Example 18-3 was performed using 6-chloro-8-[(furan-2-ylmethyl)amino]-1,5-naphthyridin-4-ol (46.4 mg, 0.168 mmol) obtained in Example 104-1 and 3-(2-fluoroethyl)-5-(hydroxymethyl)-1,3-oxazolidin-2-one (49.7 mg, 0.305 mmol) obtained in Example 41-2. Thus, the title compound (54.3 mg, 77% yield) was obtained as a light brown solid.

### (Example 110) 5-({[8-(Benzylamino)-6-chloro-1,5-naphthyridin-4-yl]oxy}methyl)-1,3-oxazolidin-2-one

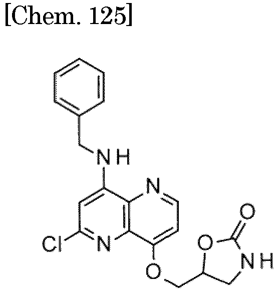

### (Example 110-1) 8-(Benzylamino)-6-chloro-1,5-naphthyridin-4-ol

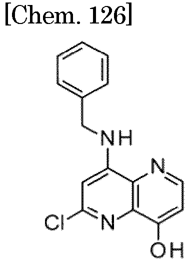

Benzylamine (591 µL, 5.49 mmol) and N,N-diisopropylethylamine (1.2 mL, 6.90 mmol) were added to a solution of 6,8-dichloro-1,5-naphthyridin-4-ol (582 mg, 2.71 mmol) disclosed in WO 2017/055306 in NMP (11 mL), followed by stirring at 120°C for 7 hours. After the reaction solution was allowed to cool to room temperature, water was added, followed by extraction with ethyl acetate. The precipitated solid in the extraction liquid was collected by filtration and dried under reduced pressure. Thus, the title compound (409 mg, 53% yield) was obtained as a white solid.

### (Example 110-2) 5-({[8-(Benzylamino)-6-chloro-1,5-naphthyridin-4-yl]oxy}methyl)-1,3-oxazolidin-2-one

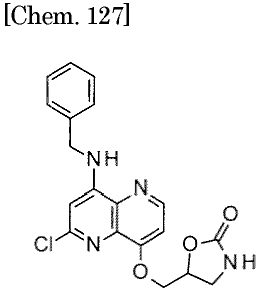

The same operation as in Example 18-3 was performed using 8-(benzylamino)-6-chloro-1,5-naphthyridin-4-ol (150 mg, 0.525 mmol) obtained in Example 110-1 and 5-(hydroxymethyl)-1,3-oxazolidin-2-one (104 mg, 0.888 mmol). Thus, the title compound (75.4 mg, 37% yield) was obtained as a white solid.

### (Example 111) 5-({[8-(Benzylamino)-6-chloro-1,5-naphthyridin-4-yl]oxy}methyl)-3-methyl-1,3-oxazolidin-2-one

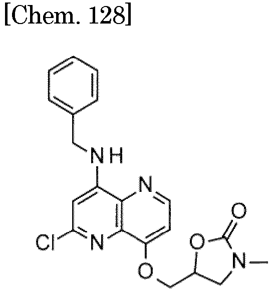

The same operation as in Example 18-3 was performed using 8-(benzylamino)-6-chloro-1,5-naphthyridin-4-ol (150 mg, 0.525 mmol) obtained in Example 110-1 and 5-(hydroxymethyl)-3-methyl-1,3-oxazolidin-2-one (107 mg, 0.816 mmol). Thus, the title compound (38.4 mg, 18% yield) was obtained as a white solid.

### (Example 112) (3S,5R)-5-({[8-(Benzylamino)-6-chloro-1,5-naphthyridin-4-yl]oxy}methyl)-3-methylpyrrolidin-2-one

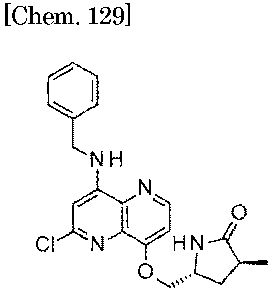

The same operation as in Example 18-3 was performed using 8-(benzylamino)-6-chloro-1,5-naphthyridin-4-ol (47.6 mg, 0.167 mmol) obtained in Example 110-1 and (3S,5R)-5-(hydroxymethyl)-3-methylpyrrolidin-2-one (32.3 mg, 0.250 mmol). Thus, the title compound (46.1 mg, 70% yield) was obtained as a light brown solid.

### (Example 113) 5-({[8-(Benzylamino)-6-chloro-1,5-naphthyridin-4-yl]oxy}methyl)-3-(2-hydroxyethyl)-1,3-oxazolidin-2-one

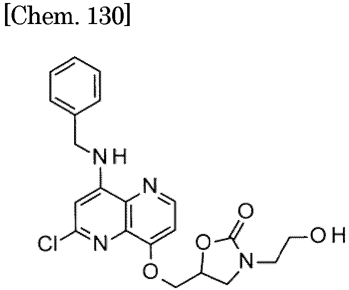

The same operation as in Example 18-3 was performed using 8-(benzylamino)-6-chloro-1,5-naphthyridin-4-ol (60.7 mg, 0.212 mmol) obtained in Example 110-1 and 3-(2-{[tert-butyl(dimethyl)silyl]oxy}ethyl)-5-(hydroxymethyl)-1,3-oxazolidin-2-one (92.2 mg, 0.335 mmol) obtained in Example 42-2. Thus, crude 5-({[8-(benzylamino)-6-chloro-1,5-naphthyridin-4-yl]oxy}methyl)-3-(2-{[tert-butyl(dimethyl)silyl]oxy}ethyl)-1,3-oxazolidin-2-one (47.6 mg) [MS (m/z): 543 (M)+] was obtained as a brown oily substance. The obtained 5-({[8-(benzylamino)-6-chloro-1,5-naphthyridin-4-yl]oxy}methyl)-3-(2-{[tert-butyl(dimethyl)silyl]oxy}ethyl)-1,3-oxazolidin-2-one was dissolved in THF (2 mL). 1M TBAF-THF (131 µL, 0.131 mmol) was added to the solution, and the same operation as in Example 42-3 was performed. Thus, the title compound (18.1 mg, 20% yield) was obtained as a white solid.

### (Example 114) 5-({[8-(Benzylamino)-6-chloro-1,5-naphthyridin-4-yl]oxy}methyl)-3-(2-fluoroethyl)-1,3-oxazolidin-2-one

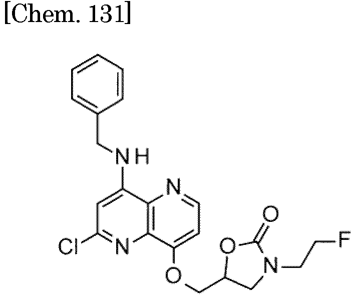

The same operation as in Example 18-3 was performed using 8-(benzylamino)-6-chloro-1,5-naphthyridin-4-ol (40.5 mg, 0.142 mmol) obtained in Example 110-1 and 3-(2-fluoroethyl)-5-(hydroxymethyl)-1,3-oxazolidin-2-one (40.1 mg, 0.246 mmol) obtained in Example 41-2. Thus, the title compound (49.5 mg, 81% yield) was obtained as a pale white solid.

### (Example 115) 5-({[4-(Benzylamino)-2-chloropyrido[3,2-d]pyrimidin-8-yl]oxy}methyl)-3-methyl-1,3-oxazolidin-2-one

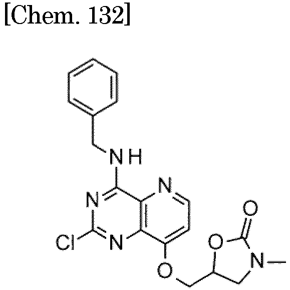

### (Example 115-1) 2,4-Dichloropyrido[3,2-d]pyrimidin-8-ol

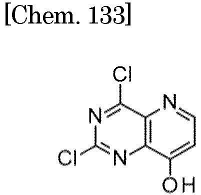

N,N-Diethylaniline (950 µL, 5.92 mmol) was added to a solution of 8-methoxy-1H-pyrido[3,2-d]pyrimidine-2,4-dione (286 mg, 1.48 mmol) disclosed in WO 2020/081636 in phosphoryl chloride (2.11 mL, 23.0 mmol), followed by stirring at 100°C for 7.5 hours. The reaction solution was allowed to cool to room temperature and then concentrated under reduced pressure. The resulting residue was purified by silica gel column chromatography (ethyl acetate/hexane = 0/100 to 100/0) to obtain crude 2,4-dichloro-8-methoxy-pyrido[3,2-d]pyrimidine (42.6 mg) [MS (m/z): 230 (M)+] as a white solid. Aluminum chloride (56.0 mg, 0.420 mmol) was added to a solution of the obtained 2,4-dichloro-8-methoxy-pyrido[3,2-d]pyrimidine (20.7 mg, 90.0 µmol) in dichloromethane (1.8 mL) under ice-cooling, followed by stirring at room temperature for 11 hours. Then, 1N hydrochloric acid was added to the reaction solution under ice-cooling, and the mixture was extracted with dichloromethane, followed by drying over anhydrous magnesium sulfate and filtration. The filtrate was then concentrated under reduced pressure to obtain the title compound in a crude form (8.10 mg, 42% yield) as a white solid.

### (Example 115-2) 5-({{4-(Benzylamino)-2-chloropyrido[3,2-d]pyrimidin-8-yl]oxy}methyl)-3-methyl-1,3-oxazolidin-2-one

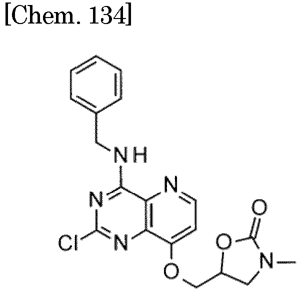

Benzylamine (29 µL, 0.25 mmol) and triethylamine (75 µL, 0.541 mmol) were added to a mixture of 2,4-dichloropyrido[3,2-d]pyrimidin-8-ol (46.5 mg, 0.215 mmol) obtained in Example 115-1 and acetonitrile (2 mL), followed by stirring at 80°C for 2.5 hours. The reaction solution was allowed to cool to room temperature and then concentrated under reduced pressure. Ethyl acetate (3.0 mL) was added to the resulting residue, and the mixture was stirred. The precipitated solid was collected by filtration and dried to obtain crude 4-(benzylamino)-2-chloropyrido[3,2-d]pyrimidin-8-ol (61.2 mg) [MS (m/z): 287 (M+H)+] as a yellow solid. 5-(Hydroxymethyl)-3-methyl-1,3-oxazolidin-2-one (37.5 mg, 0.286 mmol) and cyanomethylenetributylphosphorane (92 µL, 0.350 mmol) were added to a solution (1 mL) of the obtained 4-(benzylamino)-2-chloropyrido[3,2-d]pyrimidin-8-ol (50.0 mg, 0.174 mmol) in toluene, followed by stirring at 80°C for 2 hours. The reaction solution was allowed to cool to room temperature and then concentrated under reduced pressure. The residue was purified by silica gel column chromatography (ethyl acetate/hexane = 50/50 to 100/0) to obtain the title compound (17.2 mg, 25% yield) as a pale yellow solid.

Structural formulae and physical scientific data of the compounds described in Examples 104 to 115 are shown below.

**[Table 5]**

| Example | Structural formulae | Data |
|---|---|---|
| 104-1 | 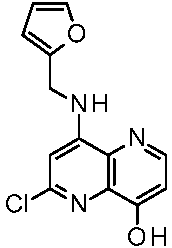 | ¹H-NMR (DMSO-D₆) δ: 4.57 (2H, d, *J* = 6.1 Hz), 6.35-6.45 (2H, m), 6.71 (1H, s), 7.01 (1H, br s), 7.60 (1H, s), 8.00 (1H, br s), 8.43 (1H, br s). |
| 104-2 | 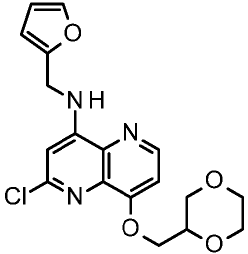 | 1H-NMR (DMSO-D6) δ: 3.39-3.47 (1H, m), 3.47-3.57 (1H, m), 3.61-3.73 (2H, m), 3.75-3.82 (1H, m), 3.84-3.91 (1H, m), 3.93-4.08 (1H, m), 4.20 (2H, d, J = 4.9 Hz), 4.58 (2H, d, J = 6.1 Hz), 6.37 (1H, d, J = 2.4 Hz), 6.41 (1H, dd, J = 3.1, 1.8 Hz), 6.73 (1H, s), 7.25 (1H, d, J = 5.5 Hz), 7.60 (1H, s), 8.06 (1H, t, J = 6.4 Hz), 8.59 (1H, d, J = 5.5 Hz). |
| | | MS (m/z): 376 (M+H)⁺. |
| 105 | 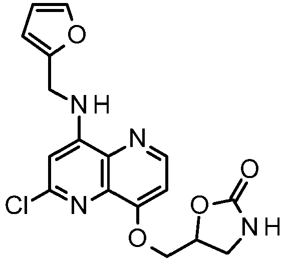 | 1H-NMR (DMSO-D6) δ: 3.34-3.41 (1H, m), 3.66 (1H, t, J = 9.1 Hz), 4.36-4.43 (2H, m), 4.58 (2H, d, J = 6.1 Hz), 5.02-5.13 (1H, m), 6.37 (1H, d, J = 2.4 Hz), 6.41 (1H, dd, J = 3.0, 1.8 Hz), 6.74 (1H, s), 7.29 (1H, d, J = 5.5 Hz), 7.60 (1H, t, J = 1.5 Hz), 7.68 (1H, s), 8.07 (1H, t, J = 6.4 Hz), 8.61 (1H, d, J = 4.9 Hz). |
| | | MS (m/z): 375 (M+H)⁺. |
| 106 | 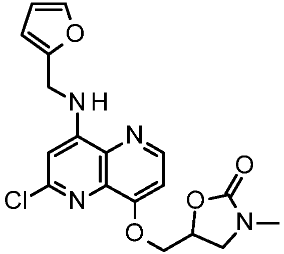 | 1H-NMR (DMSO-D6) δ: 2.81 (3H, s), 3.42 (1H, dd, J = 8.8, 6.4 Hz), 3.75 (1H, t, J = 8.8 Hz), 4.37 (1H, dd, J = 11.2, 6.7 Hz), 4.44 (1H, dd, J = 11.2, 3.3 Hz), 4.58 (2H, d, J = 6.1 Hz), 4.97-5.03 (1H, m), 6.37 (1H, br d, J = 3.6 Hz), 6.40-6.41 (1H, br m), 6.74 (1H, s), 7.28 (1H, d, J = 5.5 Hz), 7.61 (1H, d, J = 1.8 Hz), 8.08 (1H, t, J = 6.4 Hz), 8.61 (1H, d, J = 4.9 Hz). |
| | | MS (m/z): 389 (M+H)⁺. |
| 107 | 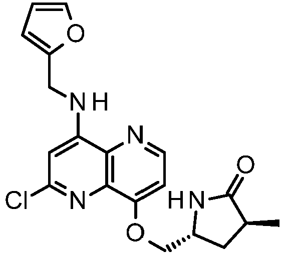 | 1H-NMR (DMSO-D6) δ: 1.14 (3H, d, J = 7.3 Hz), 1.50-1.58 (1H, m), 2.33-2.48 (2H, m), 3.96-4.01 (1H, br m), 4.13-4.22 (2H, m), 4.58 (2H, d, J = 6.7 Hz), 6.36-6.38 (1H, m), 6.40-6.41 (1H, m), 6.73 (1H, s), 7.26 (1H, d, J = 4.9 Hz), 7.60 (1H, s), 7.84 (1H, s), 8.05 (1H, t, J = 6.1 Hz), 8.59 (1H, d, J = 4.9 Hz). |
| | | MS (m/z): 387 (M+H)⁺. |
| 108 | 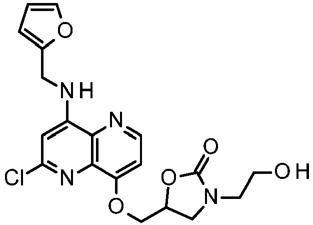 | 1H-NMR (DMSO-D6) δ: 3.24-3.27 (2H, m), 3.49-3.62 (3H, m), 3.83 (1H, t, J =9.1 Hz), 4.37 (1H, dd, J = 10.9, 6.7 Hz), 4.43 (1H, dd, J = 10.9, 3.6 Hz), 4.58 (2H, d, J = 6.7 Hz), 4.83 (1H, t, J= 5.5 Hz), 4.98-5.04 (1H, m), 6.37 (1H, br d, J = 3.0 Hz), 6.41 (1H, dd, J = 3.0, 1.8 Hz), 6.74 (1H, s), 7.28 (1H, d, J = 5.5 Hz), 7.60 (1H, d, J = 1.8 Hz), 8.08 (1H, t, J = 6.4 Hz), 8.61 (1H, d, J = 4.9 Hz). |
| | | MS (m/z): 419 (M+H)⁺. |
| 109 | 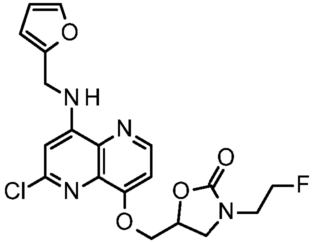 | 1H-NMR (DMSO-D6) δ: 3.48-3.58 (3H, m), 3.85 (1H, t, J = 9.1 Hz), 4.38 (1H, dd, J = 10.9, 6.1 Hz), 4.46 (1H, dd, J = 10.9, 3.0 Hz), 4.53-4.77 (4H, m), 5.02-5.09 (1H, m), 6.37 (1H, br d, J = 3.6 Hz), 6.41 (1H, dd, J = 3.6, 1.8 Hz), 6.74 (1H, s), 7.29 (1H, d, J = 4.9 Hz), 7.60 (1H, br s), 8.08 (1H, t, J = 6.1 Hz), 8.61 (1H, d, J = 5.5 Hz). |
| | | MS (m/z): 421 (M+H)⁺. |
| 110-1 | 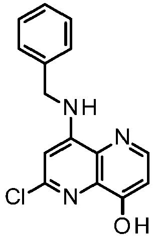 | ¹H-NMR (DMSO-D₆) δ: 4.54 (2H, d, *J* = 6.1 Hz), 6.34 (1H, br s), 6.54 (1H, br s), 7.25 (1H, t, *J* = 7.0 Hz), 7.30-7.43 (4H, m), 8.01 (1H, br s), 8.17 (1H, br s). |
| 110-2 | 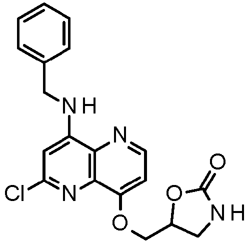 | ¹H-NMR (DMSO-D6) δ: 3.32-3.40 (1H, m), 3.66 (1H, t, J = 8.8 Hz), 4.40 (2H, d, J = 5.5 Hz), 4.58 (2H, d, J = 6.1 Hz), 5.07 (1H, br s), 6.51 (1H, s), 7.22-7.41 (6H, m), 7.69 (1H, s), 8.34 (1H, t, J = 6.7 Hz), 8.63 (1H, d, J = 6.1 Hz). |
| | | MS (m/z): 385 (M+H)⁺. |
| 111 | 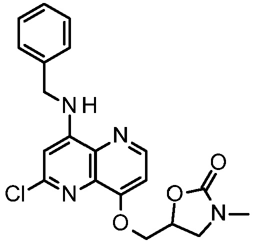 | 1H-NMR (DMSO-D6) δ: 2.80 (3H, s), 3.42 (1H, dd, J = 8.8, 6.4 Hz), 3.75 (1H, t, J = 8.8 Hz), 4.37 (1H, dd, J = 10.9, 6.7 Hz), 4.44 (1H, dd, J = 11.2, 3.3 Hz), 4.58 (2H, d, J = 6.7 Hz), 4.95-5.04 (1H, m), 6.51 (1H, s), 7.21-7.42 (6H, m), 8.34 (1H, t, J = 6.4 Hz), 8.63 (1H, d, J = 5.5 Hz). |
| | | MS (m/z): 399 (M+H)⁺. |
| 112 | 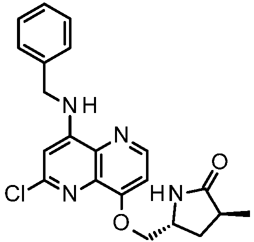 | 1H-NMR (DMSO-D6) δ: 1.14 (3H, d, J = 7.3 Hz), 1.50-1.57 (1H, m), 2.30-2.48 (2H, m), 3.95-4.02 (1H, m), 4.13-4.22 (2H, m), 4.58 (2H, d, J = 6.1 Hz), 6.50 (1H, s), 7.23-7.27 (2H, m), 7.32-7.39 (4H, m), 7.84 (1H, s), 8.32 (1H, t, J= 6.7 Hz), 8.62 (1H, d, J = 5.5 Hz). |
| | | MS (m/z): 397 (M+H)⁺. |
| 113 | 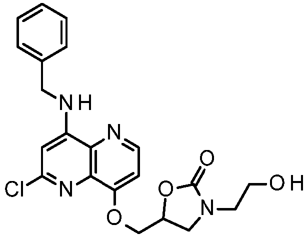 | 1H-NMR (DMSO-D6) δ: 3.23-3.26 (2H, m), 3.50-3.60 (3H, m), 3.83 (1H, t, J = 9.2 Hz), 4.37 (1H, dd, J = 11.0, 6.4 Hz), 4.44 (1H, dd, J = 11.0, 3.1 Hz), 4.59 (2H, br d, J = 6.7 Hz), 4.83 (1H, t, J = 5.5 Hz), 4.97-5.04 (1H, m), 6.51 (1H, s), 7.23-7.39 (6H, m), 8.35 (1H, t, J = 6.4 Hz), 8.63 (1H, d, J = 4.9 Hz). |
| | | MS (m/z): 429 (M+H)⁺. |
| 114 | 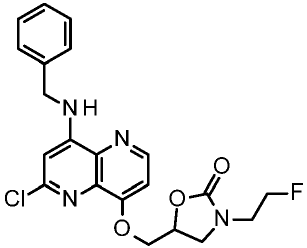 | 1H-NMR (DMSO-D6) δ: 3.48-3.58 (3H, m), 3.84 (1H, t, J = 9.2 Hz), 4.38 (1H, dd, J = 11.6, 6.1 Hz), 4.47 (1H, dd, J = 11.6, 3.1 Hz), 4.53-4.76 (4H, m), 5.02-5.08 (1H, m), 6.51 (1H, s), 7.23-7.39 (6H, m), 8.34 (1H, t, J = 6.7 Hz), 8.64 (1H, d, J = 4.9 Hz). |
| | | MS (m/z): 431 (M+H)⁺. |
| 115-1 | 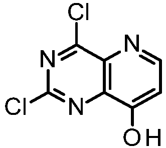 | 1H-NMR (CD3OD) δ: 6.68-6.83 (1H, m), 7.46-7.71 (1H, m), 8.14-8.29 (1H, m). |
| 115-2 | 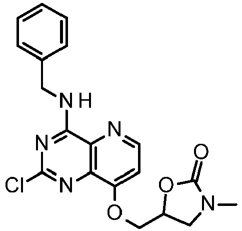 | 1H-NMR (DMSO-D6) 6: 2.80 (3H, s), 3.35-3.44 (1H, m), 3.75 (1H, t, J = 8.8 Hz), 4.39 (1H, dd, J = 10.9, 6.7 Hz), 4.45 (1H, dd, J = 11.2, 3.3 Hz), 4.70 (2H, d, J = 6.7 Hz), 4.94-5.03 (1H, m), 7.25 (1H, d, J = 6.7 Hz), 7.29-7.39 (4H, m), 7.41 (1H, d, J = 5.5 Hz), 8.65 (1H, d, J = 5.5 Hz), 9.47 (1H, t, J = 6.4 Hz). |
| | | MS (m/z): 400 (M+H)⁺. |

### (Example 116) (5R)-5-[({2-Chloro-4-[(furan-2-ylmethyl)amino]quinazolin-8-yl}oxy)methyl]-3-(methoxymethyl)pyrrolidin-2-one

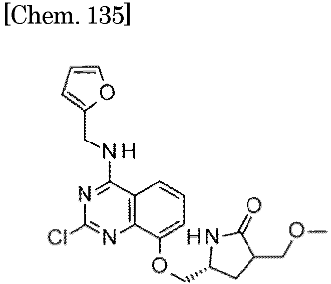

Triphenylphosphine (44.8 mg, 0.170 mmol) and diisopropyl azodicarboxylate (33.8 µL, 0.174 mmol) were added to a solution (1 mL) of 2-chloro-4-[(furan-2-ylmethyl)amino]quinazolin-8-ol (33.1 mg, 0.120 mmol) obtained in Example 18-2 and (5R)-5-(hydroxymethyl)-3-(methoxymethyl)pyrrolidin-2-one (21.6 mg, 0.136 mmol) in THF, followed by stirring at room temperature for 5.5 hours. Water was added to the reaction solution, followed by extraction with ethyl acetate. The organic layer was washed with a saturated saline solution and then concentrated under reduced pressure. The resulting residue was purified by silica gel column chromatography (ethyl acetate/hexane = 50/50 to 100/0, ethyl acetate/methanol = 100/0 to 95/5) to obtain the title compound (16.2 mg, 33% yield) as a yellow solid.

### (Example 117) 3-[({2-Chloro-4-[(furan-2-ylmethyl)amino]quinazolin-8-yl}oxy)methyl]pyrrolidin-2-one

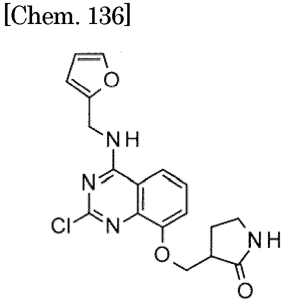

3-(Hydroxymethyl)pyrrolidin-2-one (55.7 mg, 0.484 mmol), triphenylphosphine (164 mg, 0.626 mmol) and 40% diethyl azodicarboxylate in toluene (250 µL, 0.550 mmol) were added to a solution (2.0 mL) of 2-chloro-4-[(furan-2-ylmethyl)amino]quinazolin-8-ol (67.6 mg, 0.245 mmol) obtained in Example 18-2 in THF, followed by stirring at room temperature for 5 hours. A saturated aqueous solution of sodium bicarbonate was added to the reaction solution, and the mixture was extracted with ethyl acetate, followed by drying over anhydrous magnesium sulfate and filtration. The filtrate was concentrated under reduced pressure, and the residue was purified by silica gel column chromatography (ethyl acetate/hexane = 5/95 to 100/0; methanol/dichloromethane = 2/98 to 5/95). Ethyl acetate/hexane = 2/1 (6 mL) was added to the resulting residue, and the resulting solid was then collected by filtration. Thus, the title compound (29.0 mg, 32% yield) was obtained as a white solid.

Structural formulae and physical scientific data of the compounds described in Examples 116 and 117 are shown below.

**[Table 6]**

| Example | Structural formulae | Data |
|---|---|---|
| 116 | 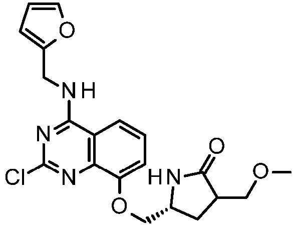 | 1H-NMR (CDCl3) δ: 1.76-1.85 (0.4H, m), 2.00-2.09 (0.6H, m), 231-253 (1H, m), 2.69-2.84 (1H, m), 3.38 (3H, d, J = 8.5 Hz), 3.58-3.72 (2H, m), 3.89-4.00 (1H, m), 4.18-4.28 (2H, m), 4.86 (2H, d, J = 4.9 Hz), 6.10 (1H, br s), 6.37-6.42 (2H, m), 6.77 (0.6H, br s), 6.81 (0.4H, br s), 7.15 (1H, t, J = 8.2 Hz), 7.22-7.29 (1H, m), 7.34-7.40 (1H, m), 7.42 (1H, s). |
| | | MS (m/z): 417 (M+H)⁺. |
| 117 | 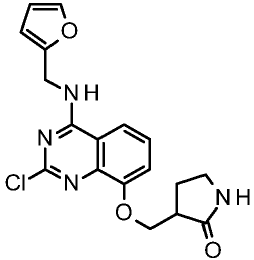 | 1H-NMR (DMSO-D6) δ: 2.05-2.17 (1H, m), 2.23-2.35 (1H, m), 2.73-2.82 (1H, m), 3.19-3.28 (1H, m), 3.31-3.43 (1H, m), 4.20-4.28 (2H, m), 4.72 (2H, d, J = 5.5 Hz), 6.35 (1H, d, J = 2.4 Hz), 6.42 (1H, dd, J = 3.6, 1.8 Hz), 7.35 (1H, d, J = 7.3 Hz), 7.44 (1H, t, J = 8.2 Hz), 7.61 (1H, d, J = 1.8 Hz), 7.78-7.85 (2H, m), 9.10 (1H, t, J = 5.8 Hz). |
| | | MS (m/z): 373 (M+H)⁺. |

Compounds of Examples 118 to 122 shown below were produced in accordance with the steps described in Examples 116 and 117 above.

**[Table 7]**

| Example | Structural formulae | Data |
|---|---|---|
| 118 | 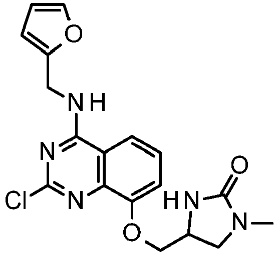 | 1H-NMR (CDCl3) δ: 2.82 (3H, s), 3.29 (1H, dd, J = 9.2, 4.9 Hz), 3.66 (1H, t, J = 8.9 Hz), 4.10 (1H, q, J = 7.9 Hz), 4.17 (1H, dd, J = 9.5, 5.2 Hz), 4.22-4.33 (1H, m), 4.85 (2H, d, J = 4.9 Hz), 5.19 (1H, s), 6.14 (1H, s), 6.36-6.42 (2H, m), 7.16 (1H, d, J = 7.9 Hz), 7.25-7.32 (1H, m), 7.36 (1H, t, J = 7.9 Hz), 7.42 (1H, d, J = 1.2 Hz). |
| | | MS (m/z): 388 (M+H)⁺. |
| 119 | 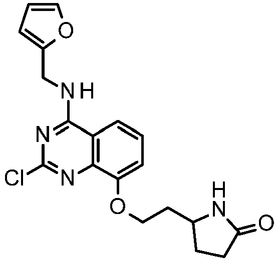 | 1H-NMR (DMSO-D6) δ: 1.67-1.80 (1H, m), 1.89-2.02 (2H, m), 2.08-2.28 (3H, m), 3.72-3.80 (1H, m), 4.10-4.28 (2H, m), 4.72 (2H, d, J = 5.5 Hz), 6.35 (1H, d, J = 3.0 Hz), 6.42 (1H, dd, J = 3.6, 1.8 Hz), 7.32 (1H, d, J = 7.3 Hz), 7.46 (1H, t, J = 7.9 Hz), 7.61 (1H, d, J = 1.8 Hz), 7.79-7.84 (2H, m), 9.11 (1H, t, J = 5.8 Hz). |
| | | MS (m/z): 387 (M+H)⁺. |
| 120 | 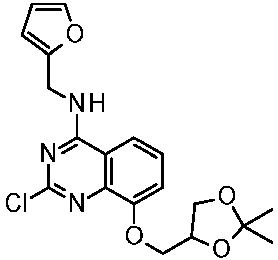 | 1H-NMR (DMSO-D6) δ: 1.33 (3H, s), 1.38 (3H, s), 3.81 (1H, dd, J = 8.5, 6.1 Hz), 4.08-4.20 (3H, m), 4.48-4.57 (1H, m), 4.72 (2H, d, J = 3.6 Hz), 6.35 (1H, d, J = 3.0 Hz), 6.42 (1H, dd, J = 3.0, 1.8 Hz), 7.36 (1H, d, J = 7.9 Hz), 7.44 (1H, t, J = 8.2 Hz), 7.61 (1H, d, J = 1.8 Hz), 7.83 (1H, d, J = 7.9 Hz), 9.11 (1H, s). |
| | | MS (m/z): 390 (M+H)⁺. |
| 121 | 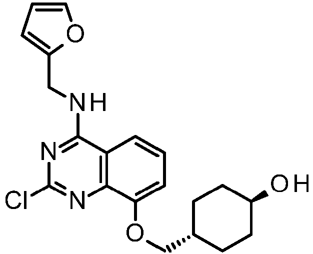 | 1H-NMR (DMSO-D6) δ: 0.92-1.30 (4H, m), 1.70-1.82 (1H, m), 1.82-1.92 (4H, m), 3.35-3.42 (1H, m), 3.89 (2H, d, J = 6.7 Hz), 4.55 (1H, d, J = 4.3 Hz), 4.71 (2H, d, J = 5.5 Hz), 6.35 (1H, d, J = 3.1 Hz), 6.42 (1H, dd, J = 3.1, 1.8 Hz), 7.29 (1H, d, J = 7.3 Hz), 7.42 (1H, t, J = 7.9 Hz), 7.60 (1H, d, J = 1.8 Hz), 7.78 (1H, d, J = 7.3 Hz), 9.08 (1H, t, J = 5.8 Hz). |
| | | MS (m/z): 388 (M+H)⁺. |
| 122 | 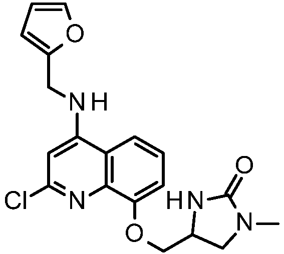 | 1H-NMR (CDCl3) δ: 2.82 (3H, s), 3.23-3.34 (1H, m), 3.60-3.70 (1H, m), 4.06-4.15 (1H, m), 4.15-4.37 (2H, m), 4.51 (2H, s), 5.29 (1H, s), 5.39 (1H, s), 6.39 (2H, d, J = 10.9 Hz), 6.56 (1H, d, J = 3.6 Hz), 7.09 (1H, d, J = 7.3 Hz), 7.25-7.41 (2H, m), 7.45 (1H, s). |
| | | MS (m/z): 387 (M+H)⁺. |

### (Example 123) 2-chloro-S-(cyclopropylmethoxy)-N-(furan-2-ylmethyl)quinazolin-4-amine

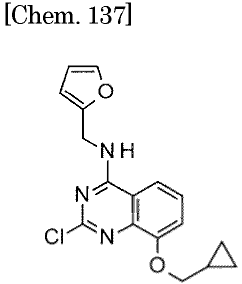

Potassium carbonate (64.7 mg, 0.468 mmol) and (bromomethyl)cyclopropane (27.2 µL, 0.281 mmol) were added to a solution (0.3 mL) of 2-chloro-4-[(furan-2-ylmethyl)amino]quinazolin-8-ol (64.5 mg, 0.234 mmol) obtained in Example 18-2 in DMF, followed by stirring at room temperature for 21 hours. Water (5 mL) was added to the reaction solution. The precipitated solid was collected by filtration, washed with water, and then dried to obtain the title compound (75.1 mg, 97% yield) as a light brown solid.

### (Example 124) 2-Chloro-N-(furan-2-ylmethyl)-8-(oxetan-3-ylmethoxy)quinazolin-4-amine

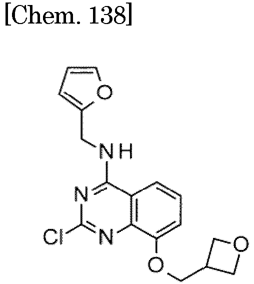

Potassium carbonate (59.3 mg, 0.429 mmol) and 3-(bromomethyl)oxetane (38.8 mg, 0.257 mmol) were added to a solution (0.3 mL) of 2-chloro-4-[(furan-2-ylmethyl)amino]quinazolin-8-ol (59.1 mg, 0.214 mmol) obtained in Example 18-2 in DMF, followed by stirring at room temperature for 23 hours. Water (5 mL) was added to the reaction solution. The precipitated solid was collected by filtration, washed with water, and then dried to obtain the title compound (73.0 mg, 99% yield) as a pale white solid.

### (Example 125) 2-Chloro-8-(1,3-dioxan-5-ylmethoxy)-N-(furan-2-ylmethyl)quinazolin-4-amine

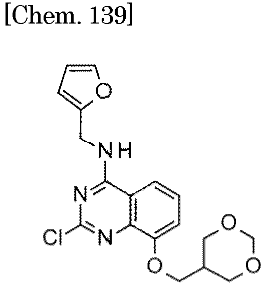

Potassium carbonate (21.8 mg, 0.158 mmol) and 3-(bromomethyl)-1,3-dioxane (18.1 mg, 0.100 mmol) were added to a solution (0.5 mL) of 2-chloro-4-[(furan-2-ylmethyl)amino]quinazolin-8-ol (20.8 mg, 0.0754 mmol) obtained in Example 18-2 in DMF, followed by stirring at 60°C for 4 hours. Water (5 mL) was added to the reaction solution, followed by extraction with ethyl acetate. The solvent was then concentrated under reduced pressure. The resulting residue was purified by silica gel column chromatography (ethyl acetate/hexane = 0/100 to 70/30) to obtain the title compound (23.7 mg, 84% yield) as a white solid.

### (Example 126) 2-chloro-8-(cyclobutylmethoxy)-N-(furan-2-ylmethyl)quinazolin-4-amine

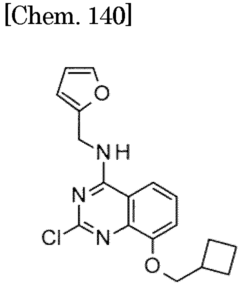

Potassium carbonate (50.0 mg, 0.363 mmol) and bromomethylcyclobutane (40.0 µL, 0.363 mmol) were added to a solution (0.5 mL) of 2-chloro-4-[(furan-2-ylmethyl)amino]quinazolin-8-ol (50.0 mg, 0.181 mmol) obtained in Example 18-2 in DMF, followed by stirring at 80°C for 1 hour. Water was added to the reaction solution, followed by extraction with ethyl acetate. Then, the organic layer was washed with a saturated saline solution, dried over anhydrous sodium sulfate, and then concentrated under reduced pressure. The resulting residue was purified by silica gel column chromatography (ethyl acetate/hexane = 0/100 to 40/60) to obtain the title compound (45.0 mg, 72% yield) as a white solid.

### (Example 127) 2-Chloro-8-[(2S)-1,4-dioxan-2-ylmethoxy]-N-(furan-2-ylmethyl) quinazolin-4-amine

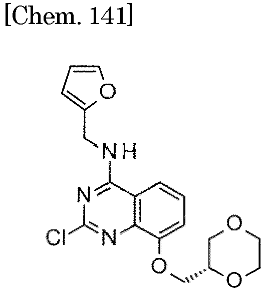

Potassium carbonate (70.0 mg, 0.506 mmol) was added to a solution (2 mL) of 2-chloro-4-[(furan-2-ylmethyl)amino]quinazolin-8-ol (70.0 mg, 0.253 mmol) obtained in Example 18-2 in DMF, followed by stirring at 90°C for 0.5 hours. The reaction solution was allowed to cool to room temperature. A solution (3 mL) of [(2S)-1,4-dioxan-2-yl]methyl 4-methylbenzenesulfonate (90.0 mg, 0.333 mmol) in DMF was then added, followed by stirring at 90°C for 12 hours. Water (20 mL) was added to the reaction solution, followed by extraction with ethyl acetate. Then, the organic layer was washed with a saturated saline solution, dried over anhydrous sodium sulfate, and then concentrated under reduced pressure. The resulting residue was purified by silica gel column chromatography (methanol/dichloromethane = 1/50) to obtain the title compound (39.5 mg, 29% yield) as a white solid.

### (Example 128) 2-Chloro-8-[(25)-1,4-dioxan-2-ylmethoxy]-7-fluoro-N-(furan-2-ylmethyl)quinazolin-4-amine

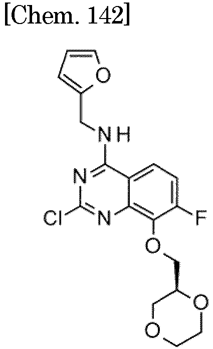

### (Example 128-1) 2,4-Dichloro-7-fluoroquinazolin-8-ol

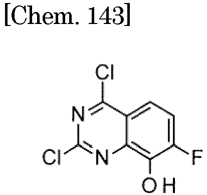

The same operation as in Example 18-1 was performed using 2,4-dichloro-7-fluoro-8-methoxyquinazoline (300 mg, 1.21 mmol) obtained in Example 3-2 and aluminum chloride (1.30 g, 9.75 mmol). Thus, the title compound (283 mg, 100% yield) was obtained as a white solid.

### (Example 128-2) 2-Chloro-7-fluoro-4-[(furan-2-ylmethyl)amino]quinazolin-8-ol

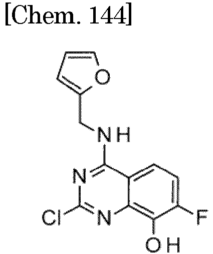

Diisopropylethylamine (500 µL, 2.87 mmol) and furfurylamine (132 µL, 1.44 mmol) were added to a solution (5 mL) of 2,4-dichloro-7-fluoroquinazolin-8-ol (300 mg, 1.29 mmol) obtained in Example 128-1 in THF, followed by stirring at room temperature for 2 hours. The reaction solution was concentrated under reduced pressure, water (50 mL) was added to the resulting residue, followed by extraction with ethyl acetate. Then, the organic layer was washed with a saturated saline solution, dried over anhydrous sodium sulfate, and then concentrated under reduced pressure. The resulting residue was purified by silica gel column chromatography (ethyl acetate/hexane = 17/83) to obtain the title compound (320 mg, 85% yield) as a yellow solid.

### (Example 128-3) 2-Chloro-8-[(2S)-1,4-dioxan-2-ylmethoxy]-7-fluoro-N-(furan-2-ylmethyl) quinazolin-4-amine

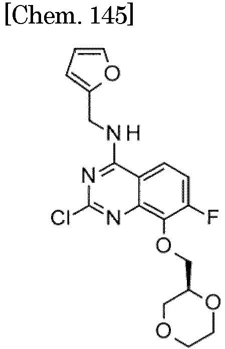

The same operation as in Example 127 was performed using 2-chloro-7-fluoro-4-[(furan-2-ylmethyl)amino]quinazolin-8-ol (150 mg, 0.511 mmol) obtained in Example 128-2 and [(2R)-1,4-dioxan-2-yl]methyl 4-methylbenzenesulfonate (150 mg, 0.551 mmol). Thus, the title compound (114 mg, 54% yield) was obtained as a white solid.

### (Example 129) 2-Chloro-N-(furan-2-ylmethyl)-8-(oxetan-3-ylmethoxy)quinolin-4-amine

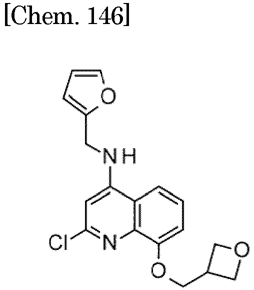

The same operation as in Example 125 was performed using 2-chloro-4-[(furan-2-ylmethyl)amino]quinolin-8-ol (140 mg, 0.510 mmol) obtained in Example 38-2 and 3-(bromomethyl)oxetane (58.9 µL, 0.612 mmol). Thus, the title compound (88.0 mg, 50% yield) was obtained as a white solid.

Structural formulae and physical scientific data of the compounds described in Examples 123 to 129 are shown below.

**[Table 8]**

| Example | Structural formulae | Data |
|---|---|---|
| 123 | 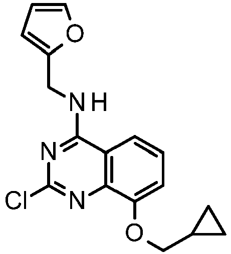 | 1H-NMR (DMSO-D6) δ: 0.35-0.39 (2H, m), 0.59-0.64 (2H, m), 1.30-1.38 (1H, m), 3.94 (2H, d, J = 6.7 Hz), 4.72 (2H, d, J = 5.5 Hz), 6.35 (1H, br d, J = 3.7 Hz), 6.42 (1H, dd, J = 3.1, 1.8 Hz), 7.27 (1H, d, J = 7.9 Hz), 7.42 (1H, t, J = 8.2 Hz), 7.61 (1H, br d, J = 1.8 Hz), 7.79 (1H, d, J = 7.3 Hz), 9.09 (1H, t, J = 5.5 Hz). |
| | | MS (m/z): 330 (M+H)⁺. |
| 124 | 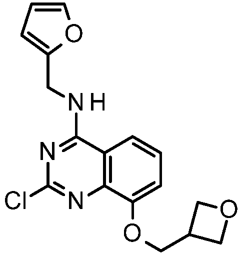 | 1H-NMR (DMSO-D6) δ: 3.45-3.52 (1H, m), 4.36 (2H, d, J = 6.7 Hz), 4.45 (2H, t, J = 6.1 Hz), 4.71-4.78 (4H, m), 6.35 (1H, br d, J = 3.0 Hz), 6.42 (1H, dd, J = 3.0, 1.8 Hz), 7.38 (1H, d, J = 8.2 Hz), 7.46 (1H, t, J = 8.2 Hz), 7.60 (1H, br s), 7.83 (1H, d, J = 8.2 Hz), 9.11 (1H, t, J = 5.5 Hz). |
| | | MS (m/z): 346 (M+H)⁺. |
| 125 | 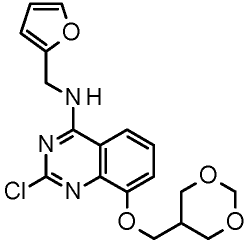 | 1H-NMR (DMSO-D6) δ: 2.24-2.28 (1H, m), 3.85 (2H, dd, J = 11.2, 5.8 Hz), 4.00-4.06 (2H, m), 4.19 (2H, d, J = 7.3 Hz), 4.72 (2H, br s), 4.78 (1H, d, J = 6.1 Hz), 4.82 (1H, d, J = 6.1 Hz), 6.35 (1H, d, J = 2.4 Hz), 6.42 (1H, dd, J = 3.0, 1.8 Hz), 7.37 (1H, d, J = 7.3 Hz), 7.45 (1H, t, J = 7.9 Hz), 7.61 (1H, d, J = 1.8 Hz), 7.83 (1H, dd, J = 7.9, 1.6 Hz), 9.11 (1H, br s). |
| | | MS (m/z): 376 (M+H)⁺. |
| 126 | 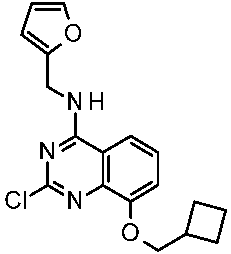 | 1H-NMR (DMSO-D6) δ: 1.81-1.97 (4H, m), 2.09-2.16 (2H, m), 2.77-2.85 (1H, m), 4.09 (2H, d, J = 6.7 Hz), 4.71 (2H, br s), 6.35 (1H, d, J = 3.1 Hz), 6.42 (1H, dd, J = 3.1, 1.8 Hz), 7.32 (1H, dd, J = 8.0, 1.2 Hz), 7.43 (1H, t, J = 8.0 Hz), 7.60 (1H, d, J = 1.8 Hz), 7.80 (1H, dd, J = 8.6, 1.2 Hz), 9.08 (1H, br s). |
| | | MS (m/z): 344 (M+H)⁺. |
| 127 | 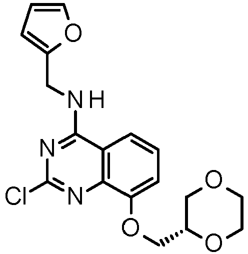 | 1H-NMR (DMSO-D6) δ: 3.38-3.54 (2H, m), 3.63-3.70 (2H, m), 3.76-3.81 (1H, m), 3.88 (1H, dd, J = 11.5, 2.4 Hz), 3.94-4.00 (1H, m), 4.05-4.13 (2H, m), 4.72 (2H, d, J = 5.8 Hz), 6.35 (1H, d, J = 3.0 Hz), 6.42 (1H, dd, J = 3.0, 1.8 Hz), 7.34 (1H, d, J = 7.9 Hz), 7.44 (1H, t, J = 7.9 Hz), 7.61 (1H, d, J = 1.8 Hz), 7.83 (1H, d, J = 7.9 Hz), 9.11 (1H, t, J = 5.8 Hz). |
| | | MS (m/z): 376 (M+H)⁺. |
| 128-1 | 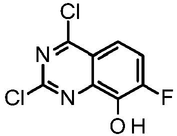 | 1H-NMR (CDCl3) δ: 7.25-7.27 (1H, m), 7.55 (1H, t, J = 9.2 Hz), 7.79 (1H, dd, J = 9.2, 4.8 Hz). |
| 128-2 | 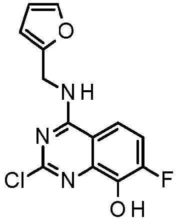 | 1H-NMR (CDCl3) δ: 4.87.(2H, d, J = 4.8 Hz), 6.08 (1H, br s), 6.39-6.41 (2H, m), 7.15 (1H, dd, J = 9.2, 4.4 Hz), 7.23 (1H, d, J = 9.2 Hz), 7.43 (1H, s). |
| 128-3 | 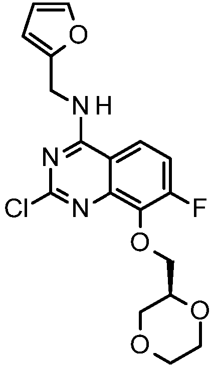 | 1H-NMR (DMSO-D6) δ: 3.47-3.73 (5H, m), 3.81-3.91 (2H, m), 4.20 (1H, dd, J = 10.9, 4.9 Hz), 4.26 (1H, dd, J = 10.9, 4.9 Hz), 4.72 (2H, d, J = 5.5 Hz), 6.37 (1H, d, J = 3.6 Hz), 6.42 (1H, dd, J = 3.6, 1.8 Hz), 7.51 (1H, dd, J = 10.6, 9.4 Hz), 7.61 (1H, d, J = 1.8 Hz), 8.08 (1H, dd, J = 9.4, 5.2 Hz), 9.30 (1H, t, J = 5.5 Hz). |
| | | MS (m/z): 394 (M+H)⁺. |
| 129 | 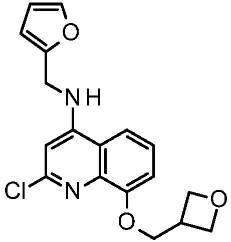 | 1H-NMR (DMSO-D6) δ: 3.44-3.54 (1H, m), 4.36 (2H, d, J = 6.8 Hz), 4.46 (2H, t, J = 6.0 Hz), 4.54 (2H, d, J = 5.6 Hz), 4.76 (2H, dd, J = 7.6, 6.0 Hz), 6.38 (1H, dd, J = 3.2, 0.4 Hz), 6.41 (1H, dd, J = 3.2, 1.6 Hz), 6.55 (1H, s), 7.23 (1H, d, J = 7.2 Hz), 7.39 (1H, t, J = 8.0 Hz), 7.61 (1H, dd, J = 1.6, 0.4 Hz), 7.78 (1H, dd, J = 4.8, 0.8 Hz), 7.97 (1H, t, J = 5.6 Hz). |
| | | MS (m/z): 345 (M+H)⁺. |

Compounds of Examples 130 to 143 shown below were produced in accordance with the steps described in Examples 123 to 129 above.

**[Table 9]**

| Example | Structural formulae | Data |
|---|---|---|
| 130 | 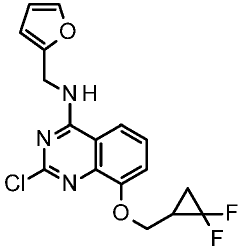 | 1H-NMR (DMSO-D6) δ: 1.48-1.56 (1H, m), 1.73-1.82 (1H, m), 2.30-2.42 (1H, m), 4.12-4.17 (1H, br m), 4.23-4.29 (1H, br m), 4.72 (2H, d, J = 4.9 Hz), 6.35 (1H, d, J = 3.1 Hz), 6.42 (1H, dd, J = 3.1, 1.8 Hz), 7.35 (1H, d, J = 8.0 Hz), 7.45 (1H, t, J = 8.3 Hz), 7.60-7.61 (1H, m), 7.84 (1H, dd, J = 8.6, 1.2 Hz), 9.12 (1H, t, J = 5.5 Hz). |
| | | MS (m/z): 366 (M+H)⁺. |
| 131 | 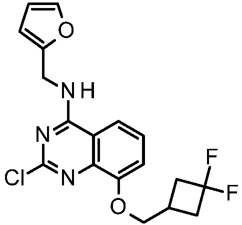 | 1H-NMR (DMSO-D6) δ: 2.52-2.56 (2H, m), 2.64-2.82 (3H, m), 4.18 (2H, d, J = 6.1 Hz), 4.72 (2H, d, J = 5.5 Hz), 6.35 (1H, d, J = 3.1 Hz), 6.42 (1H, dd, J = 3.1, 1.8 Hz), 7.35 (1H, dd, J = 8.0, 1.2 Hz), 7.45 (1H, t, J = 8.0 Hz), 7.60 (1H, d, J = 1.8 Hz), 7.83 (1H, dd, J = 8.6, 1.2 Hz), 9.11 (1H, t, J = 5.5 Hz). |
| | | MS (m/z): 380 (M+H)⁺. |
| 132 | 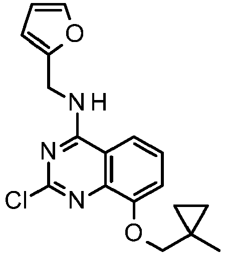 | 1H-NMR (DMSO-D6) δ: 0.42-0.45 (2H, br m), 0.55-0.57 (2H, br m), 1.23 (3H, s), 3.89 (2H, s), 4.72 (2H, d, J = 5.5 Hz), 6.35 (1H, dd, J = 3.1, 1.2 Hz), 6.42 (1H, dd, J = 3.1, 1.8 Hz), 7.26 (1H, d, J = 7.4 Hz), 7.42 (1H, t, J = 8.0 Hz), 7.61 (1H, dd, J = 1.8, 1.2 Hz), 7.79 (1H, d, J = 8.6 Hz), 9.07 (1H, t, J = 5.5 Hz). |
| | | MS (m/z): 344 (M+H)⁺. |
| 133 | 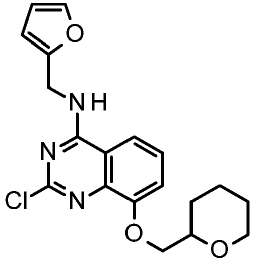 | 1H-NMR (DMSO-D6) δ: 1.32-1.39 (1H, m), 1.48-1.56 (3H, m), 1.68-1.73 (1H, br m), 1.82-1.86 (1H, br m), 3.39-3.46 (1H, m), 3.72-3.78 (1H, br m), 3.88-3.93 (1H, br m), 4.00 (1H, dd, J = 10.4, 3.7 Hz), 4.07 (1H, dd, J = 10.4, 6.7 Hz), 4.71 (2H, br s), 6.35 (1H, dd, J = 3.1, 1.2 Hz), 6.42 (1H, dd, J = 3.1, 1.8 Hz), 7.32 (1H, d, J = 7.4 Hz), 7.43 (1H, t, J = 8.0 Hz), 7.60-7.61 (1H, m), 7.80 (1H, dd, J = 8.0, 1.2 Hz), 9.09 (1H, br s). |
| | | MS (m/z): 374 (M+H)⁺. |
| 134 | 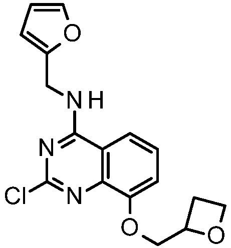 | 1H-NMR (DMSO-D6) δ: 2.53-2.61 (1H, m), 2.71-2.79 (1H, m), 4.23 (1H, dd, J = 11.0, 3.1 Hz), 4.33 (1H, dd, J = 11.0, 6.1 Hz), 4.51-4.59 (2H, m), 4.72 (2H, br s), 5.07-5.13 (1H, m), 6.35 (1H, dd, J = 3.1, 1.2 Hz), 6.42 (1H, dd, J = 3.1, 1.8 Hz), 7.38 (1H, dd, J = 8.3, 1.2 Hz), 7.45 (1H, t, J = 8.3 Hz), 7.61 (1H, d, J = 1.8 Hz), 7.83 (1H, dd, J = 8.3, 1.8 Hz), 9.11 (1H, br s). |
| | | MS (m/z): 346 (M+H)⁺. |
| 135 | 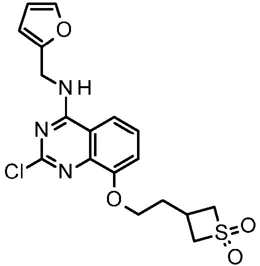 | 1H-NMR (DMSO-D6) δ: 2.15-2.20 (2H, m), 2.67-2.77 (1H, m), 4.03-4.08 (2H, m), 4.14 (2H, t, J = 6.1 Hz), 4.28-4.33 (2H, m), 4.72 (2H, d, J = 5.8 Hz), 6.35 (1H, d, J = 3.1 Hz), 6.42 (1H, dd, J = 3.1, 1.8 Hz), 7.31 (1H, d, J = 8.2 Hz), 7.45 (1H, t, J = 8.2 Hz), 7.61 (1H, br d, J = 1.8 Hz), 7.81 (1H, d, J = 7.9 Hz), 9.11 (1H, t, J = 5.8 Hz). |
| | | MS (m/z): 408 (M+H)⁺. |
| 136 | 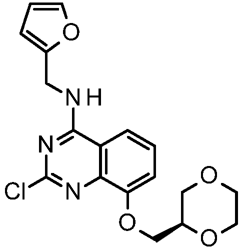 | 1H-NMR (DMSO-D6) δ: 3.41-3.54 (2H, m), 3.64-3.71 (2H, m), 3.76-3.79 (1H, m), 3.88 (1H, dd, J = 11.5, 2.4 Hz), 3.95-4.00 (1H, m), 4.04-4.12 (2H, m), 4.72 (2H, d, J = 5.5 Hz), 6.36 (1H, d, J = 3.0 Hz), 6.42 (1H, dd, J = 3.0, 1.8 Hz), 7.34 (1H, d, J = 7.9 Hz), 7.44 (1H, t, J = 7.9 Hz), 7.61 (1H, d, J = 1.8 Hz), 7.83 (1H, d, J = 7.9 Hz), 9.12 (1H, t, J = 5.5 Hz). |
| | | MS (m/z): 376 (M+H)⁺. |
| 137 | 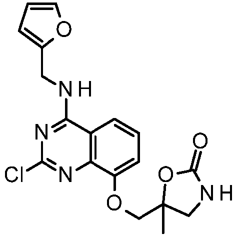 | 1H-NMR (DMSO-D6) δ: 1.50 (3H, s), 3.26-3.37 (1H, m), 3.61 (1H, d, J = 9.1 Hz), 4.14-4.24 (2H, m), 4.72 (2H, d, J = 6.1 Hz), 6.35 (1H, d, J = 3.0 Hz), 6.42 (1H, dd, J = 3.0, 1.8 Hz), 7.40 (1H, d, J = 7.3 Hz), 7.46 (1H, t, J = 7.9 Hz), 7.61 (2H, s), 7.87 (1H, d, J = 8.5 Hz), 9.13 (1H, t, J = 5.8 Hz). |
| | | MS (m/z): 389 (M+H)⁺. |
| 138 | 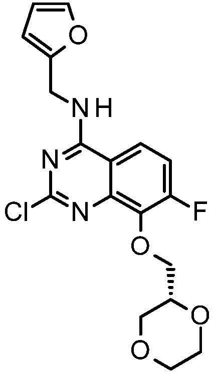 | 1H-NMR (DMSO-D6) δ: 3.44-3.73 (5H, m), 3.81-3.91 (2H, m), 4.20 (1H, dd, J = 10.9, 4.9 Hz), 4.26 (1H, dd, J = 10.9, 4.9 Hz), 4.72 (2H, d, J = 5.5 Hz), 6.37 (1H, d, J = 3.3 Hz), 6.42 (1H, dd, J = 3.3, 2.1 Hz), 7.51 (1H, dd, J = 10.6, 9.4 Hz), 7.61 (1H, d, J = 1.8 Hz), 8.08 (1H, dd, J = 9.1, 5.5 Hz), 9.30 (1H, t, J = 5.5 Hz). |
| | | MS (m/z): 394 (M+H)⁺. |
| 139 | 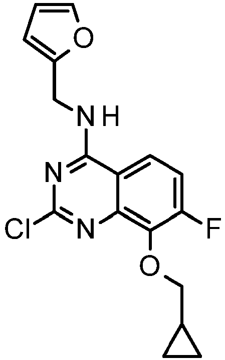 | 1H-NMR (DMSO-D6) δ: 0.22-0.26 (2H, m), 0.47-0.51 (2H, m), 1.15-1.25 (1H, m), 4.08 (2H, d, J = 6.7 Hz), 4.72 (2H, d, J = 5.5 Hz), 6.37 (1H, d, J = 4.3 Hz), 6.42 (1H, dd, J = 3.6, 1.8 Hz), 7.50 (1H, dd, J = 10.3, 9.1 Hz), 7.61 (1H, d, J = 1.2 Hz), 8.07 (1H, dd, J = 9.1, 5.2 Hz), 9.27 (1H, t, J = 5.5 Hz). |
| | | MS (m/z): 348 (M+H)⁺. |
| 140 | 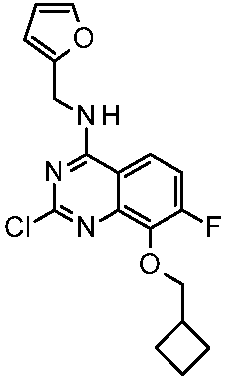 | 1H-NMR (DMSO-D6) δ: 1.84-1.90 (4H, m), 1.98-2.05 (2H, m), 2.64-2.71 (1H, m), 4.21 (2H, d, J = 6.7 Hz), 4.72 (2H, d, J = 5.5 Hz), 6.37 (1H, d, J = 3.6 Hz), 6.42 (1H, dd, J = 3.0, 1.8 Hz), 7.50 (1H, dd, J = 10.3, 9.1 Hz), 7.61 (1H, d, J = 1.2 Hz), 8.06 (1H, dd, J = 9.1, 4.9 Hz), 9.27 (1H, t, J = 5.5 Hz). |
| | | MS (m/z): 362 (M+H)⁺. |
| 141 | 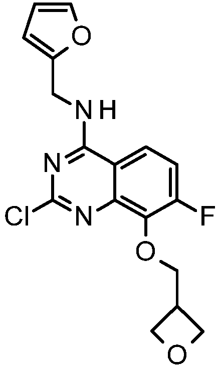 | 1H-NMR (DMSO-D6) δ: 3.31-3.38 (1H, m), 4.43 (2H, d, J = 6.7 Hz), 4.50 (2H, t, J = 6.1 Hz), 4.67-4.74 (4H, m), 6.38 (1H, d, J = 2.4 Hz), 6.43 (1H, dd, J = 3.0, 1.8 Hz), 7.53 (1H, t, J = 10.0 Hz), 7.62 (1H, d, J = 1.8 Hz), 8.11 (1H, dd, J = 9.4, 5.2 Hz), 9.31 (1H, t, J = 5.5 Hz). |
| | | MS (m/z): 364 (M+H)⁺. |
| 142 | 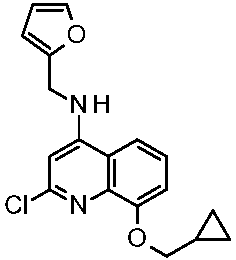 | 1H-NMR (DMSO-D6) δ: 0.35-0.39 (2H, m), 0.59-0.64 (2H, m), 1.29-1.39 (1H, m), 3.93 (2H, d, J = 6.8 Hz), 4.54 (2H, d, J = 5.6 Hz), 6.39 (1H, dd, J = 3.2, 0.8 Hz), 6.41 (1H, dd, J = 3.2, 2.0 Hz), 6.55 (1H, s), 7.11 (1H, d, J= 7.2 Hz), 7.35 (1H, t, J = 8.4 Hz), 7.61 (1H, dd, J = 2.0, 0.8 Hz), 7.74 (1H, dd, J = 8.4, 0.4 Hz), 7.94 (1H, t, J = 5.6 Hz). |
| | | MS (m/z): 329 (M+H)⁺. |
| 143 | 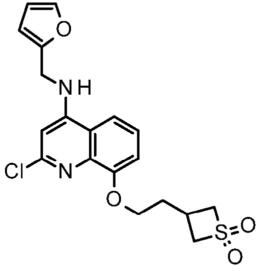 | 1H-NMR (DMSO-D6) δ: 2.18 (2H, dd, J = 13.6, 6.0 Hz), 2.67-2.79 (1H, m), 4.05-4.15 (4H, m), 4.28-4.34 (2H, m), 4.54 (2H, d, J = 6.0 H), 6.39 (1H, br d, J = 0.8 Hz), 6.41 (1H, dd, J = 3.2, 2.0 Hz), 6.56 (1H, s), 7.15 (1H, d, J = 7.2 Hz), 7.38 (1H, t, J = 8.0 Hz), 7.61 (1H, dd, J = 2.0, 0.8 Hz), 7.76 (1H, d, J = 8.0 Hz), 7.97 (1H, t, J = 6.0 Hz). |
| | | MS (m/z): 407 (M+H)⁺. |

### (Example 144) 2-Chloro-8-[(2,2-difluorocyclopropyl)methoxy]-N-(thiophen-2-ylmethyl)quinazolin-4-amine

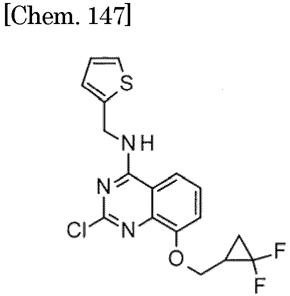

### (Example 144-1) 2-Chloro-4-[(thiophen-2-ylmethyl)amino]quinazolin-8-ol

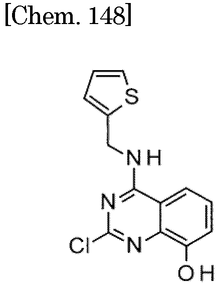

Triethylamine (0.154 mL, 1.12 mmol) and thiophene-2-methylamine (0.070 mL, 0.670 mmol) were added to a solution (4 mL) of 2,4-dichloroquinazolin-8-ol (120 mg, 0.558 mmol) obtained in Example 18-1 in acetonitrile, followed by stirring at 60°C for 1 hour. Water (10 mL) was added to the reaction solution, followed by extraction with ethyl acetate. Then, the organic layer was washed with a saturated saline solution, dried over anhydrous sodium sulfate, and then concentrated under reduced pressure. The residue was purified by silica gel column chromatography (ethyl acetate/hexane = 0/100 to 25/75) to obtain the title compound (89.0 mg, 55% yield) as a white solid.

### (Example 144-2) 2-chloro-8-[(2,2-difluorocyclopropyl)methoxy]-N-(thiophen-2-ylmethyl)quinazolin-4-amine

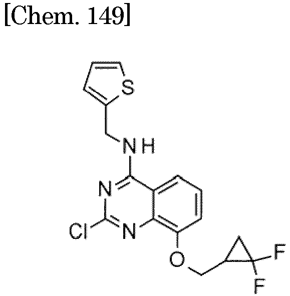

Potassium carbonate (30.0 mg, 0.206 mmol) and 2-(bromomethyl)-1,1-difluorocyclopropane (26.0 mg, 0.151 mmol) were added to a solution (2 mL) of 2-chloro-4-[(thiophen-2-ylmethyl)amino]quinazolin-8-ol (40.0 mg, 0.137 mmol) obtained in Example 144-1 in DMF, followed by stirring at 90°C for 1 hour. After the reaction solution was allowed to cool to room temperature, water (5 mL) was added to the reaction solution, followed by extraction with ethyl acetate. Then, the organic layer was washed with a saturated saline solution, dried over anhydrous sodium sulfate, and then concentrated under reduced pressure. The residue was purified by silica gel column chromatography (ethyl acetate/hexane = 0/100 to 40/60) to obtain the title compound (23.0 mg, 44% yield) as a pale yellow solid.

### (Example 145) 2-Chloro-8-(1,4-dioxan-2-ylmethoxy)-N-(thiophen-2-ylmethyl)quinazolin-4-amine

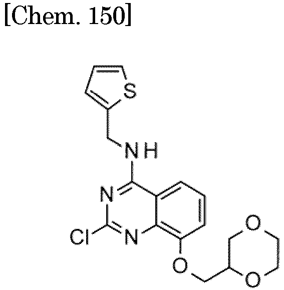

Potassium carbonate (50.0 mg, 0.355 mmol) and 1,4-dioxan-2-ylmethyl 4-methylbenzenesulfonate (77.0 mg, 0.284 mmol) were added to a solution (2 mL) of 2-chloro-4-[(thiophen-2-ylmethyl)amino]quinazolin-8-ol (69.0 mg, 0.237 mmol) obtained in Example 144-1 in DMF, followed by stirring at 90°C for 4 hours. After the reaction solution was allowed to cool to room temperature, water (10 mL) was added to the reaction solution, followed by extraction with ethyl acetate. Then, the organic layer was washed with a saturated saline solution, dried over anhydrous sodium sulfate, and then concentrated under reduced pressure. The residue was purified by silica gel column chromatography (ethyl acetate/hexane = 0/100 to 100/0) to obtain the title compound (67.0 mg, 72% yield) as a pale yellow solid.

### (Example 146) 2-Chloro-8-[(2,2-difluorocyclopropyl)methoxy]-N-(thiophen-3-ylmethyl)quinazolin-4-amine

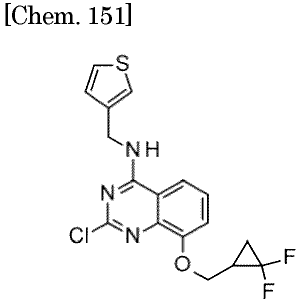

### (Example 146-1) 2-Chloro-4-[(thiophen-3-ylmethyl)amino]quinazolin-8-ol

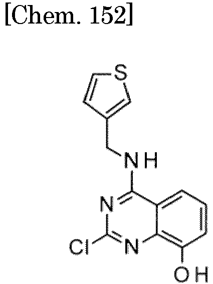

Triethylamine (0.154 mL, 1.12 mmol) and 3-thiophenemethylamine (60.0 µL, 0.614 mmol) were added to a solution (4 mL) of 2,4-dichloroquinazolin-8-ol (120 mg, 0.558 mmol) obtained in Example 18-1 in acetonitrile, followed by stirring at 60°C for 0.5 hours. After the reaction solution was allowed to cool to room temperature, water (10 mL) was added, followed by extraction with ethyl acetate. Then, the organic layer was washed with a saturated saline solution, dried over anhydrous sodium sulfate, and then concentrated under reduced pressure. The residue was purified by silica gel column chromatography (ethyl acetate/hexane = 0/100 to 15/85) to obtain the title compound (98.0 mg, 60% yield) as a white solid.

### (Example 146-2) 2-Chloro-8-[(2,2-difluorocyclopropyl)methoxy]-N-(thiophen-3-ylmethyl)quinazolin-4-amine

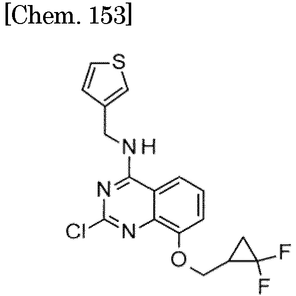

Potassium carbonate (30.0 mg, 0.206 mmol) and 2-(bromomethyl)-1,1-difluorocyclopropane (26.0 mg, 0.151 mmol) were added to a solution (2 mL) of 2-chloro-4-[(thiophen-3-ylmethyl)amino]quinazolin-8-ol (40.0 mg, 0.137 mmol) obtained in Example 146-1 in DMF, followed by stirring at 90°C for 2 hours. After the reaction solution was allowed to cool to room temperature, water (5 mL) was added, followed by extraction with ethyl acetate. Then, the organic layer was washed with a saturated saline solution, dried over anhydrous sodium sulfate, and then concentrated under reduced pressure. The residue was purified by silica gel column chromatography (ethyl acetate/hexane = 0/100 to 25/75) to obtain the title compound (46.0 mg, 88% yield) as a white solid.

### (Example 147) 2-Chloro-8-(1,4-dioxan-2-ylmethoxy)-N-(thiophen-3-ylmethyl)quinazolin-4-amine

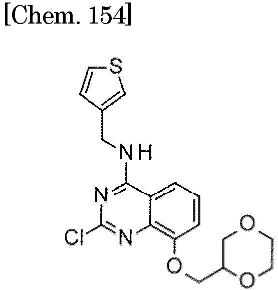

Potassium carbonate (58.0 mg, 0.416 mmol) and 1,4-dioxan-2-ylmethyl 4-methylbenzenesulfonate (91.0 mg, 0.333 mmol) were added to a solution of 2-chloro-4-[(thiophen-3-ylmethyl)amino]quinazolin-8-ol (81.0 mg, 0.278 mmol) obtained in Example 146-1 in DMF (2 mL), followed by stirring at 90°C for 3 hours. After the reaction solution was allowed to cool to room temperature, water (10 mL) was added, followed by extraction with ethyl acetate. Then, the organic layer was washed with a saturated saline solution, dried over anhydrous sodium sulfate, and then concentrated under reduced pressure. The resulting residue was purified by silica gel column chromatography (ethyl acetate/hexane = 0/100 to 100/0) to obtain the title compound (52.0 mg, 48% yield) as a pale brown solid.

### (Example 148) 5-{[(2-Chloro-4-{[(4-fluorofuran-2-yl)methyl]amino}quinazolin-8-yl)oxy]methyl}-1,3-oxazolidin-2-one

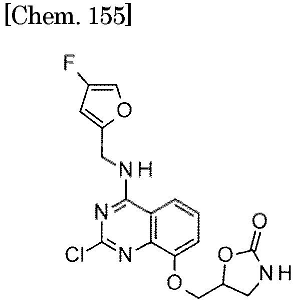

### (Example 148-1) 2-[(4-Fluorofuran-2-yl)methyl]-1H-isoindole-1,3(2H)-dione

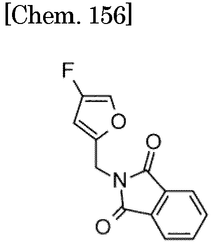

Triphenylphosphine (359 mg, 1.37 mmol) and 40% diethyl azodicarboxylate in toluene (580 µL, 1.30 mmol) were added to a solution (1.8 mL) of (4-fluoro-2-furyl)methanol (105 mg, 0.904 mmol) disclosed in US 2008/0058395 and phthalimide (163 mg, 1.11 mmol) in THF, followed by stirring at room temperature for 5 hours. Water was added to the reaction solution. The mixture was then concentrated under reduced pressure, and the residue was purified by silica gel column chromatography (ethyl acetate/hexane = 0/100 to 40/60) to obtain the title compound (110 mg, 50% yield) as a white solid.

### (Example 148-2) 2-chloro-4-{[(4-fluorofuran-2-yl)methyl]amino}quinazolin-8-ol

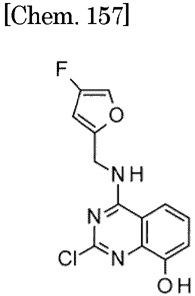

Hydrazine monohydrate (29.0 µL, 0.598 mmol) was added to a solution (4.0 mL) of 2-[(4-fluorofuran-2-yl)methyl]-1H-isoindole-1,3(2H)-dione (110 mg, 0.449 mmol) obtained in Example 148-1 in ethanol, followed by stirring at 80°C for 5.5 hours. The reaction solution was allowed to cool to room temperature and then diluted with dichloromethane. The insoluble material was separated by filtration, and the filter cake was washed with dichloromethane. The filtrate and washings were washed with a 1N aqueous sodium hydroxide solution, dried over anhydrous sodium sulfate, and then filtered. The filtrate was concentrated under reduced pressure to obtain crude 4-fluoro-2-furanmethanamine (39.2 mg, 76% yield) as a white solid. 2,4-Dichloroquinazolin-8-ol (75.0 mg, 0.349 mmol) obtained in Example 18-1 and triethylamine (90.0 µL, 0.649 mmol) were added to the mixture of the obtained 4-fluoro-2-furanmethanamine (39.2 mg, 0.341 mmol) and acetonitrile (25 mL), followed by stirring at 60°C for 2 hours. Triethylamine (45.0 µL, 0.325 mmol) was further added to the reaction solution, and the mixture was stirred at 60°C for 2 hours. The reaction solution was allowed to cool to room temperature and then concentrated under reduced pressure. The resulting residue was purified by silica gel column chromatography (ethyl acetate/hexane = 0/100 to 15/85) to obtain the title compound (21.0 mg, 21% yield) as a colorless oily substance.

### (Example 148-3) 5-{[(2-chloro-4-{[(4-fluorofuran-2-yl)methyl]amino}quinazolin-8-yl)oxy]methyl}-1,3-oxazolidin-2-one

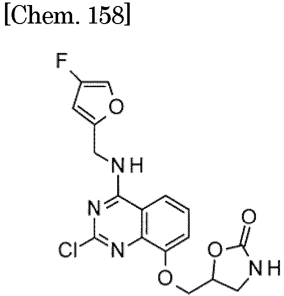

The same operation as in Example 18-3 was performed using 2-chloro-4-{[(4-fluorofuran-2-yl)methyl]amino}quinazolin-8-ol (21.0 mg, 0.0715 mmol) obtained in Example 148-2 and 5-(hydroxymethyl)-1,3-oxazolidin-2-one (25.6 mg, 0.219 mmol). Thus, the title compound (6.50 mg, 23% yield) was obtained as a white solid.

### (Example 149) 2-Chloro-8-[(2,2-difluorocyclopropyl)methoxy]-N-(1,2-thiazol-5-ylmethyl)quinazolin-4-amine

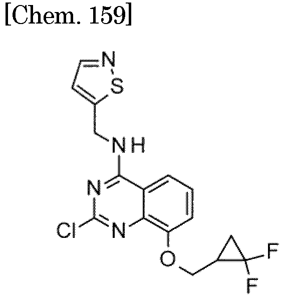

### (Example 149-1) 8-{[tert-Butyl(dimethyl)silyl]oxy}-2,4-dichloroquinazoline

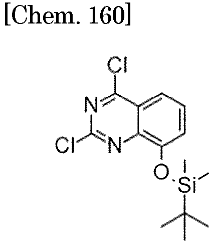

To a suspension (25 mL) of 2,4-dichloroquinazolin-8-ol (2.55 g, 11.9 mmol) obtained in Example 18-1 in DCM, 2,6-lutidine (2.76 mL, 23.7 mmol) and tertbutyldimethylsilyl trifluoromethanesulfonate (4.09 mL, 17.8 mmol) were added under ice-cooling, and the mixture was stirred under ice-cooling for 2 hours, followed by stirring at room temperature for 3 hours. The reaction solution was ice-cooled. Water (15 mL) was then added, followed by extraction with DCM and concentration under reduced pressure. The resulting residue was purified by silica gel column chromatography (ethyl acetate/hexane = 0/100 to 6/94) to obtain the title compound (3.54 g, 91% yield) as a pale yellow solid.

### (Example 149-2) 8-{[tert-Butyl(dimethyl)silyl]oxy}-2-chloro-N-(1,2-thiazol-5-ylmethyl)quinazolin-4-amine

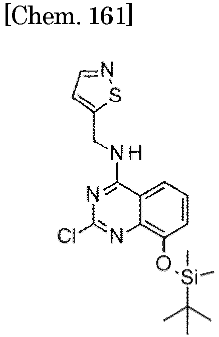

(1,2-Thiazol-5-yl)methanamine hydrochloride (90.0 mg, 0.601 mmol) and triethylamine (0.170 mL, 1.20 mmol) were added to a solution (1 mL) of 8-{[tert-butyl(dimethyDsilyl]oxy}-2,4-dichloroquinazolme (180 mg, 0.547 mmol) obtained in Example 149-1 in acetonitrile, followed by stirring at 60°C for 1 hour. After the reaction solution was allowed to cool to room temperature, water (5 mL) was added. The precipitated solid was collected by filtration and then dried to obtain the title compound (180 mg, 94% yield) as a pale yellow solid.

### (Example 149-3) 2-Chloro-4-[(1,2-thiazol-5-ylmethyl)amino]quinazolin-8-ol

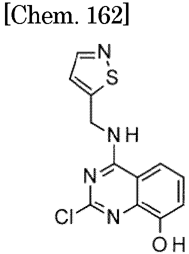

1M TBAF-THF (0.616 mL, 0.616 mmol) was added to a solution (1 mL) of 8-{[tert-butyl(dimethyl)silyl]oxy}-2-chloro-N-(1,2-thiazol-5-ylmethyl)quinazolin-4-amine (209 mg, 514 mmol) obtained in Example 149-2 in THF, followed by stirring at room temperature for 16 hours. Water (5 mL) was added to the reaction solution, followed by extraction with ethyl acetate and subsequent concentration under reduced pressure. The residue was purified by silica gel column chromatography (ethyl acetate/hexane = 0/100 to 100/0) to obtain the title compound (114 mg, 76% yield) as a white solid.

### (Example 149-4) 2-Chloro-8-[(2,2-difluorocyclopropyl)methoxy]-N-(1,2-thiazol-5-ylmethyl)quinazolin-4-amine

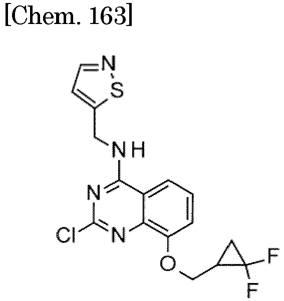

2-(Bromomethyl)-1,1-difluorocyclopropane (26.0 mg, 0.150 mmol) and potassium carbonate (28.0 mg, 0.205 mmol) were added to a solution (1 mL) of 2-chloro-4-[(1,2-thiazol-5-ylmethyl)amino]quinazolin-8-ol (40.0 mg, 0.137 mmol) obtained in Example 149-3 in DMF, followed by stirring at 90°C for 1 hour. The reaction solution was allowed to cool to room temperature. Then, water (5 mL) was added, followed by extraction with ethyl acetate and subsequent concentration under reduced pressure. The residue was purified by silica gel column chromatography (ethyl acetate/hexane = 0/100 to 100/0) to obtain the title compound (48.0 mg, 92% yield) as a pale yellow solid.

### (Example 150) 2-Chloro-8-[(2,2-difluorocydopropyl)methoxyl-N-(1,3-oxazol-4-ylmethyl)quinazolin-4-amine

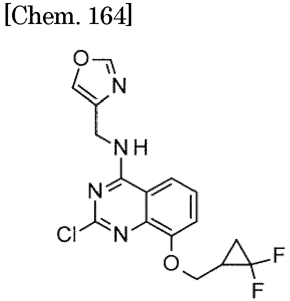

### (Example 150-1) 8-{[tert-Butyl(dimethyl)silyl]oxy}-2-chloro-N-(1,3-oxazol-4-ylmethyl)quinazolin-4-amine

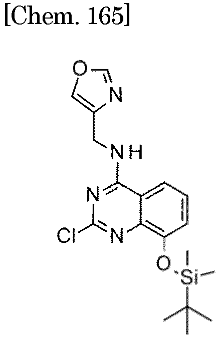

The same operation as in Example 149-2 was performed using 8-{[tert-butyl(dimethyDsilyl]oxy}-2,4-dichloroquinazolme (180 mg, 0.547 mmol) obtained in Example 149-1 and (1,3-oxazol-4-yl)methanamine hydrochloride (81.0 mg, 0.601 mmol). Thus, the title compound (184 mg, 86% yield) was obtained as a white solid.

### (Example 150-2) 2-Chloro-4-[(1,3-oxazol-4-ylmethyl)amino]quinazolin-8-ol

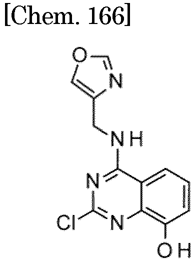

The same operation as in Example 149-3 was performed using 8-{[tert-butyl(dimethyl)silyl]oxy}-2-chloro-N-(1,3-oxazol-4-ylmethyl)quinazolin-4-amine (184 mg, 0.471 mmol) obtained in Example 150-1. Thus, the title compound (110 mg, 84% yield) was obtained as a white solid.

### (Example 150-3) 2-Chloro-8-[(2,2-difluorocyclopropyl)methoxy]-N-(1,3-oxazol-4-ylmethyl) quinazolin-4-amine

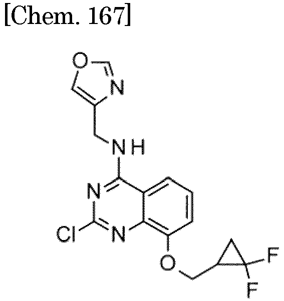

The same operation as in Example 149-4 was performed using 2-chloro-4-[(1,3-oxazol-4-ylmethyl)amino]quinazolin-8-ol (40.0 mg, 0.145 mmol) obtained in Example 150-2 and 2-(bromomethyl)-1,1-difluorocyclopropane (33.0 mg, 0.160 mmol). Thus, the title compound (33.0 mg, 62% yield) was obtained as a white solid.

### (Example 151) 2-Chloro-8-(1,4-dioxan-2-ylmethoxy)-N-(1,2-oxazol-3-ylmethyl)quinazolin-4-amine

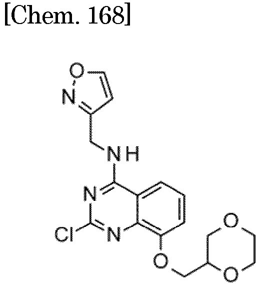

### (Example 151-1) 8-{[tert-Butyl(dimethyl)silyl]oxy}-2-chloro-N-(1,2-oxazol-3-ylmethyl)quinazolin-4-amine

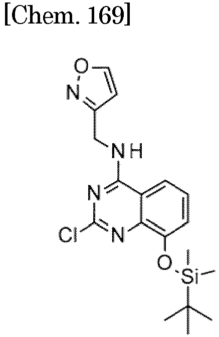

The same operation as in Example 149-2 was performed using 8-{[tert-butyl(dimethyDsilyl]oxy}-2,4-dichloroquinazolme (180 mg, 0.547 mmol) obtained in Example 149-1 and isoxazol-3-ylmethanamine (60.0 mg, 0.601 mmol). Thus, the title compound (195 mg, 91% yield) was obtained as a white solid.

### (Example 151-2) 2-Chloro-4-[(1,2-oxazol-3-ylmethyl)amino]quinazolin-8-ol

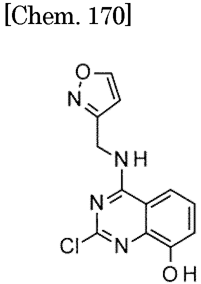

The same operation as in Example 149-3 was performed using 8-{[tert-butyl(dimethyl)silyl]oxy}-2-chloro-N-(1,2-oxazol-3-ylmethyl)quinazolin-4-amine (195 mg, 0.499 mmol) obtained in Example 151-1. Thus, the title compound (87.0 mg, 63% yield) was obtained as a white solid.

### (Example 151-3) 2-Chloro-8-(1,4-dioxan-2-ylmethoxy)-N-(1,2-oxazol-3-ylmethyl) quinazolin-4-amine

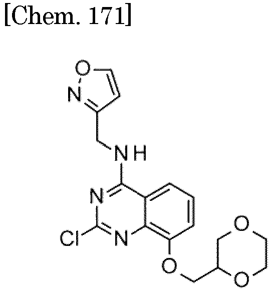

The same operation as in Example 149-4 was performed using 2-chloro-4-[(1,2-oxazol-3-ylmethyl)amino]quinazolin-8-ol (40.0 mg, 0.145 mmol) obtained in Example 151-2 and 2-(bromomethyl)-1,4-dioxane (27.0 mg, 0.159 mmol). Thus, the title compound (12.0 mg, 22% yield) was obtained as a pale yellow solid.

### (Example 152) 5-[({2-Chloro-4-[(1,3-oxazol-2-ylmethyl)amino]quinazolin-8-yl}oxy)methyl]-1,3-oxazolidin-2-one

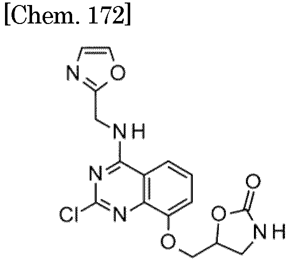

### (Example 152-1) 8-{[tert-Butyl(dimethyl)silyl]oxyl-2-chloro-N-(1,3-oxazol-2-ylmethyl)quinazolin-4-amine

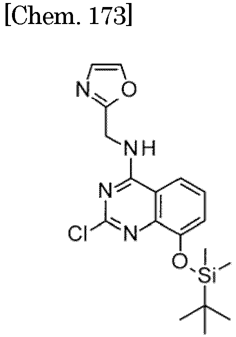

Oxazol-2-yl-methylamine hydrochloride (52.1 mg, 0.387 mmol) and N,N-diisopropylethylamine (106 µL, 0.609 mmol) were added to a solution (1.5 mL) of 8-{[tert-butyl(dimethyl)silyl]oxy}-2,4-dichloroquinazoline (99.4 mg, 0.302 mmol) obtained in Example 149-1 in NMP, followed by stirring at 120°C for 1 hour. After the reaction solution was allowed to cool to room temperature, water was added, followed by extraction with ethyl acetate. The organic layer was washed with a saturated saline solution, dried over anhydrous magnesium sulfate, and then filtered. The filtrate was then concentrated under reduced pressure. The residue was purified by silica gel column chromatography (ethyl acetate/hexane = 0/100 to 100/0) to obtain the title compound (94.3 mg, 80% yield) as a white solid.

### (Example 152-2) 2-Chloro-4-[(1,3-oxazol-2-ylmethyl)amino]quinazolin-8-ol

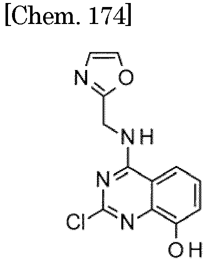

The same operation as in Example 149-3 was performed using 8-{[tert-butyl(dimethylsilylloxy}-2-chloro-N-(1,3-oxazol-2-ylmethyl)quinazolin-4-amine (94.3 mg, 0.241 mmol) obtained in Example 152-1. Thus, the title compound (59.6 mg, 89% yield) was obtained as a white solid.

### (Example 152-3) 5-[({2-Chloro-4-[(1,3-oxazol-2-ylmethyl)amino]quinazolin-8-yl}oxy)methyl]-1,3-oxazolidin-2-one

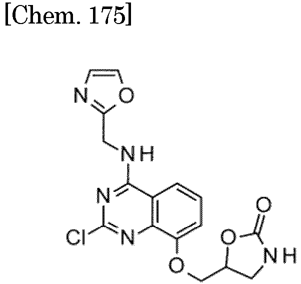

The same operation as in Example 18-3 was performed using 2-chloro-4-[(1,3-oxazol-2-ylmethyl)amino]quinazolin-8-ol (75.6 mg, 0.273 mmol) obtained in Example 152-2 and 5-(hydroxymethyl)-1,3-oxazolidin-2-one (46.7 mg, 0.399 mmol). Thus, the title compound (20.4 mg, 20% yield) was obtained as a white solid.

### (Example 153) 2-Chloro-8-(1,4-dioxan-2-ylmethoxy)-N-(1,3-oxazol-2-ylmethyl)quinazolin-4-amine

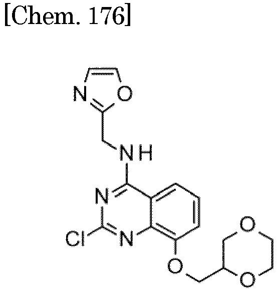

### (Example 153-1) 2,4-Dichloro-8-(1,4-dioxan-2-ylmethoxy)quinazoline

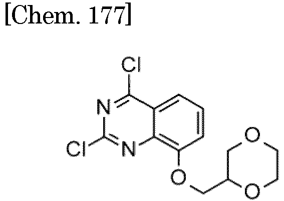

(1,4-Dioxan-2-yl)methanol (41.0 mg, 0.349 mmol), bis(2-methoxymethyl)azodicarboxylate (110 mg, 0.465 mmol), and triphenylphosphine (120 mg, 0.465 mmol) were added to a solution (2 mL) of 2,4-dichloroquinazolin-8-ol (50.0 mg, 0.233 mmol) obtained in Example 18-1 in toluene, followed by stirring at 90°C for 40 minutes. The reaction solution was allowed to cool to room temperature and then concentrated under reduced pressure. The residue was purified by silica gel column chromatography (ethyl acetate/hexane = 0/100 to 50/50) to obtain the title compound (19.0 mg, 26% yield).

### (Example 153-2) 2-Chloro-8-(1,4-dioxan-2-ylmethoxy)-N-(1,3-oxazol-2-ylmethyl)quinazolin-4-amine

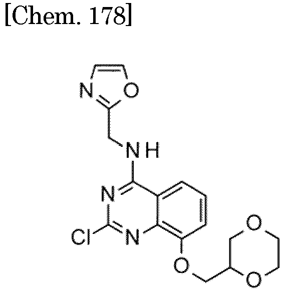

Triethylamine (0.060 mL, 0.413 mmol) and oxazol-2-ylmethanamine hydrochloride (25.0 mg, 0.182 mmol) were added to a solution (1 mL) of 2,4-dichloro-8-(1,4-dioxan-2-ylmethoxy)quinazoline (52.0 mg, 0.165 mmol) obtained in Example 153-1 in acetonitrile, followed by stirring at 60°C for 4 hours. The reaction solution was allowed to cool to room temperature and then concentrated under reduced pressure. The residue was purified by silica gel column chromatography (ethyl acetate/hexane = 0/100 to 100/0) to obtain the title compound (32.0 mg, 51% yield) as a white solid.

Structural formulae and physical scientific data of the compounds described in Examples 144 to 153 are shown below.

**[Table 10]**

| Example | Structural formulae | Data |
|---|---|---|
| 144-1 | 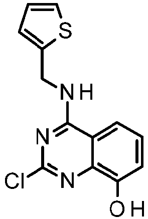 | 1H-NMR (DMSO-D6) δ: 4.88 (2H, d, J = 5.5 Hz), 6.98 (1H, dd, J = 5.2, 3.4 Hz), 7.11 (1H, dd, J = 3.7, 1.2 Hz), 7.16 (1H, dd, J = 8.0, 1.2 Hz), 7.36 (1H, t, J = 8.0 Hz), 7.40 (1H, dd, J = 4.9, 1.2 Hz), 7.65 (1H, dd, J = 8.6, 1.2 Hz), 9.26 (1H, t, J = 5.5 Hz), 9.81 (1H, s). |
| 144-2 | 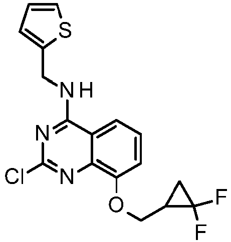 | 1H-NMR (DMSO-D6) δ: 1.48-1.56 (1H, m), 1.73-1.82 (1H, m), 2.30-2.40 (1H, m), 4.12-4.17 (1H, br m), 4.24-4.29 (1H, br m), 4.88 (2H, d, J = 6.1 Hz), 6.98 (1H, dd, J = 5.2, 3.1 Hz), 7.10 (1H, dd, J = 3.1, 1.2 Hz), 7.35 (1H, d, J = 7.4 Hz), 7.40 (1H, dd, J = 4.9, 1.2 Hz), 7.45 (1H, t, J = 8.0 Hz), 7.80 (1H, dd, J = 8.6, 1.2 Hz), 9.29 (1H, t, J = 6.1 Hz). |
| | | MS (m/z): 382 (M+H)⁺. |
| 145 | 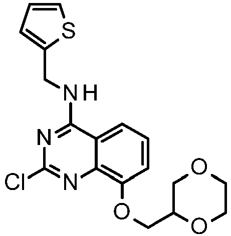 | 1H-NMR (DMSO-D6) δ: 3.43 (1H, dd, J = 11.7, 9.8 Hz), 3.48-3.54 (1H, m), 3.63-3.71 (2H, m), 3.76-3.80 (1H, m), 3.88 (1H, dd, J = 11.7, 2.5 Hz), 3.94-4.00 (1H, m), 4.05-4.13 (2H, m), 4.87 (2H, d, J = 5.5 Hz), 6.98 (1H, dd, J = 5.2, 3.4 Hz), 7.10 (1H, dd, J = 3.4, 1.2 Hz), 7.34 (1H, d, J = 7.4 Hz), 7.40 (1H, dd, J = 4.9, 1.2 Hz), 7.44 (1H, t, J = 8.0 Hz), 7.78 (1H, d, J = 8.6 Hz), 9.27 (1H, t, J = 5.5 Hz). |
| | | MS (m/z): 392 (M+H)⁺. |
| 146-1 | 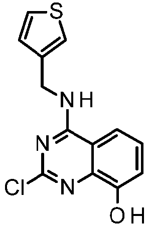 | Y 82637-010 1H-NMR (DMSO-D6) δ: 4.73 (2H, d, J = 5.5 Hz), 7.13-7.16 (2H, m), 7.32-7.38 (2H, m), 7.50 (1H, dd, J = 4.9, 3.1 Hz), 7.69 (1H, d, J = 8.6 Hz), 9.10 (1H, t, J = 5.5 Hz), 9.75 (1H, s). |
| 146-2 | 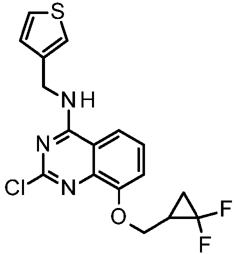 | 1H-NMR (DMSO-D6) δ: 1.48-1.56 (1H, m), 1.73-1.82 (1H, m), 2.32-2.40 (1H, m), 4.12-4.16 (1H, br m), 4.23-4.29 (1H, br m), 4.72 (2H, d, J = 5.5 Hz), 7.14 (1H, dd, J = 4.9, 1.2 Hz), 7.34 (1H, dd, J = 8.0, 1.2 Hz), 7.38 (1H, dd, J = 3.1, 1.2 Hz), 7.45 (1H, t, J = 8.0 Hz), 7.51 (1H, dd, J = 4.9, 2.5 Hz), 7.84 (1H, dd, J = 8.6, 1.2 Hz), 9.14 (1H, t, J = 6.1 Hz). |
| | | MS (m/z): 382 (M+H)⁺. |
| 147 | 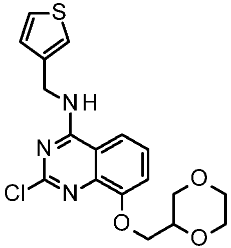 | 1H-NMR (DMSO-D6) δ: 3.43 (1H, dd, J = 11.7, 9.8 Hz), 3.48-3.54 (1H, m), 3.63-3.71 (2H, br m), 3.76-3.80 (1H, br m), 3.88 (1H, dd, J = 11.0, 2.5 Hz), 3.94-4.00 (1H, m), 4.05-4.13 (2H, m), 4.72 (2H, d, J = 5.5 Hz), 7.14 (1H, dd, J = 4.9, 1.2 Hz), 7.34 (1H, dd, J = 8.0, 1.2 Hz), 7.38-7.39 (1H, br m), 7.44 (1H, t, J = 8.3 Hz), 7.51 (1H, dd, J = 4.9, 3.1 Hz), 7.83 (1H, d, J = 8.6 Hz), 9.13 (1H, t, J = 5.5 Hz). |
| | | MS (m/z): 392 (M+H)⁺. |
| 148-1 | 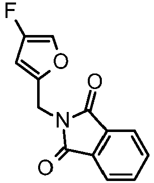 | ¹H-NMR (CDCl₃) δ: 4.77 (2H, s), 6.34 (1H, s), 7.27 (1H, s), 7.74 (2H, dd, *J* = 5.5, 3.0 Hz), 7.88 (2H, dd, *J* = 5.5, 3.0 Hz). |
| 148-2 | 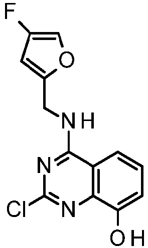 | ¹H-NMR (CDCl₃) δ: 4.79 (2H, d, *J* = 5.5 Hz), 6.09 (1H, br s), 6.38 (1H, s), 7.15 (1H, d, *J* = 9.1 Hz), 7.24 (1H, dd, *J* = 7.9, 1.2 Hz), 7.34-7.39 (2H, m), 7.48 (1H, s). |
| 148-3 | 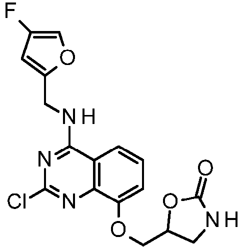 | 1H-NMR (DMSO-D6) δ: 3.35-3.42 (1H, m), 3.65 (1H, t, J = 9.1 Hz), 4.20-4.35 (2H, m), 4.65 (2H, s), 4.96-5.08 (1H, m), 6.52 (1H, s), 7.39 (1H, d, J = 7.9 Hz), 7.47 (1H, t, J = 8.2 Hz), 7.65 (1H, s), 7.83 (2H, dd, J = 14.0, 6.1 Hz), 9.14 (1H, s). |
| | | MS (m/z): 393 (M+H)⁺. |
| 149-1 | 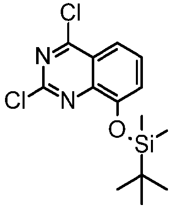 | 1H-NMR (CDCl3) δ: 0.29 (6H, s), 1.06 (9H, s), 7.41 (1H, dd, J = 7.9, 1.2 Hz), 7.59 (1H, dd, J = 8.5, 7.9 Hz), 7.83 (1H, dd, J = 8.5, 1.2 Hz). |
| 149-2 | 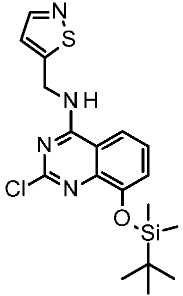 | 1H-NMR (DMSO-D6) δ: 0.23 (6H, s), 1.00 (9H, s), 4.98 (2H, d, J = 5.5 Hz), 7.29 (1H, dd, J = 7.4, 1.2 Hz), 7.41-7.46 (2H, m), 7.80 (1H, dd, J = 8.6, 1.2 Hz), 8.46 (1H, d, J = 1.8 Hz), 9.46 (1H, t, J = 5.5 Hz). |
| 149-3 | 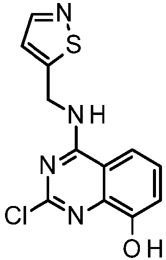 | 1H-NMR (DMSO-D6) δ: 4.98 (2H, d, J = 5.5 Hz), 7.18 (1H, dd, J = 8.0, 1.2 Hz), 7.36-7.40 (2H, m), 7.63 (1H, d, J = 8.0 Hz), 8.46 (1H, d, J = 1.8 Hz), 9.40 (1H, t, J = 5.5 Hz), 9.87 (1H, s). |
| 149-4 | 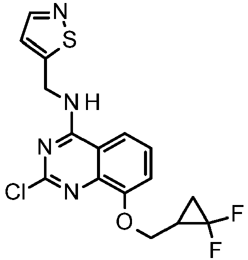 | 1H-NMR (DMSO-D6) δ: 1.48-1.57 (1H, m), 1.73-1.82 (1H, m), 2.32-2.40 (1H, m), 4.13-4.18 (1H, m), 4.25-4.30 (1H, m), 4.98 (2H, d, J = 5.5 Hz), 7.37-7.41 (2H, m), 7.48 (1H, t, J = 8.0 Hz), 7.78 (1H, d, J = 8.6 Hz), 8.46 (1H, d, J = 1.2 Hz), 9.44 (1H, t, J = 5.5 Hz). |
| | | MS (m/z): 383 (M+H)⁺. |
| 150-1 | 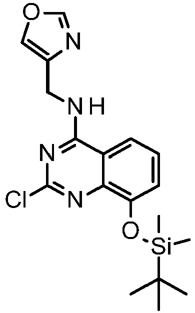 | 1H-NMR (DMSO-D6) δ: 0.22 (6H, s), 1.00 (9H, s), 4.63 (2H, d, J = 5.5 Hz), 7.26 (1H, dd, J = 8.0, 1.2 Hz), 7.40 (1H, t, J = 8.0 Hz), 7.86 (1H, dd, J = 8.6, 1.2 Hz), 8.03 (1H, d, J = 1.2 Hz), 8.34 (1H, s), 9.10 (1H, t, J = 5.5 Hz). |
| 150-2 | 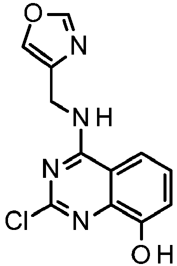 | 1H-NMR (DMSO-D6) δ: 4.62 (2H, d, J = 5.5 Hz), 7.15 (1H, dd, J = 8.0, 1.2 Hz), 7.35 (1H, t, J = 8.0 Hz), 7.69 (1H, dd, J = 8.0, 1.2 Hz), 8.01 (1H, d, J = 1.2 Hz), 8.34 (1H, d, J = 1.2 Hz), 9.04 (1H, t, J = 5.5 Hz), 9.74 (1H, s). |
| 150-3 | 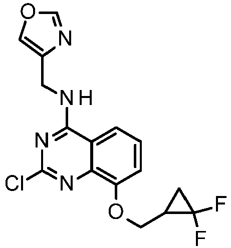 | 1H-NMR (DMSO-D6) δ: 1.47-1.56 (1H, m), 1.72-1.82 (1H, m), 2.29-2.41 (1H, m), 4.12-4.17 (1H, br m), 4.24-4.29 (1H, br m), 4.62 (2H, d, J = 5.5 Hz), 7.34 (1H, d, J = 7.4 Hz), 7.45 (1H, t, J = 8.0 Hz), 7.84 (1H, d, J = 8.6 Hz), 8.01 (1H, d, J = 1.2 Hz), 8.34 (1H, s), 9.08 (1H, t, J = 5.5 Hz). |
| | | MS (m/z): 367 (M+H)⁺ |
| 151-1 | 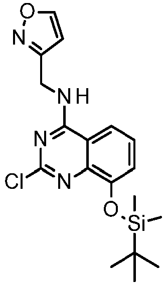 | 1H-NMR (DMSO-D6) δ: 0.22 (6H, s), 1.00 (9H, s), 4.82 (2H, d, J = 5.5 Hz), 6.59 (1H, d, J = 1.8 Hz), 7.28 (1H, dd, J = 7.4, 1.2 Hz), 7.43 (1H, t, J = 8.3 Hz), 7.85 (1H, dd, J = 8.3, 1.2 Hz), 8.87 (1H, d, J = 1.8 Hz), 9.25 (1H, t, J = 5.5 Hz). |
| 151-2 | 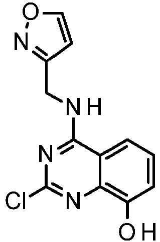 | 1H-NMR (DMSO-D6) δ: 4.81 (2H, d, J = 6.1 Hz), 6.57 (1H, d, J = 1.2 Hz), 7.17 (1H, dd, J = 8.0, 1.2 Hz), 7.37 (1H, t, J = 8.0 Hz), 7.66 (1H, dd, J = 8.6, 1.2 Hz), 8.86 (1H, d, J = 1.8 Hz), 9.18 (1H, t, J = 6.1 Hz), 9.81 (1H, s). |
| 151-3 | 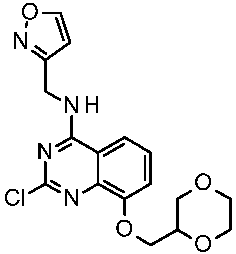 | 1H-NMR (DMSO-D6) δ: 3.41-3.54 (2H, m), 3.63-3.71 (2H, m), 3.76-3.80 (1H, m), 3.88 (1H, dd, J = 11.3, 2.8 Hz), 3.94-4.00 (1H, m), 4.06-4.14 (2H, m), 4.81 (2H, d, J = 5.5 Hz), 6.57 (1H, d, J = 1.2 Hz), 7.36 (1H, d, J = 7.4 Hz), 7.47 (1H, t, J = 8.0 Hz), 7.81 (1H, dd, J = 8.6, 1.2 Hz), 8.86 (1H, d, J = 1.2 Hz), 9.21 (1H, t, J = 5.5 Hz). |
| | | MS (m/z): 377 (M+H)⁺ |
| 152-1 | 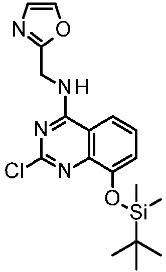 | ¹H-NMR (CDCl₃) δ: 0.28 (3H, s), 0.29 (3H, s), 1.04 (9H, s), 4.94-4.99 (2H, m), 6.72 (1H, br s), 7.13 (1H, d, *J=* 1.2 Hz), 7.23 (1H, d, *J* = 7.3 Hz), 7.29-7.40 (2H, m), 7.70 (1H, d, *J* = 1.8 Hz). |
| 152-2 | 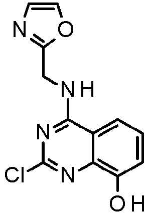 | ¹H-NMR (DMSO-D₆) δ: 4.84 (2H, d, *J* = 6.1 Hz), 7.18 (2H, d, *J* = 7.3 Hz), 7.39 (1H, t, *J* = 8.2 Hz), 7.69 (1H, d, *J* = 8.5 Hz), 8.07 (1H, s), 9.25 (1H, t, *J* = 5.8 Hz), 9.86 (1H, s). |
| 152-3 | 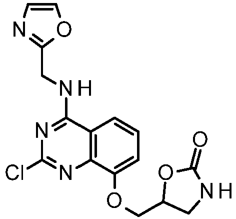 | 1H-NMR (DMSO-D6) δ: 3.35-3.43 (1H, m), 3.65 (1H, t, J = 8.8 Hz), 4.24-4.35 (2H, m), 4.85 (2H, s), 4.98-5.06 (1H, m), 7.17 (1H, s), 7.41 (1H, d, J = 7.3 Hz), 7.50 (1H, t, J = 8.2 Hz), 7.65 (1H, s), 7.86 (1H, d, J = 8.5 Hz), 8.07 (1H, s), 9.32 (1H, s). |
| | | MS (m/z): 376 (M+H)⁺. |
| 153-1 | 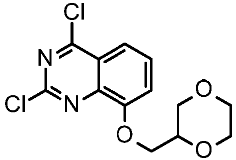 | 1H-NMR (DMSO-D6) δ: 3.44-3.55 (2H, m), 3.64-3.72 (2H, m), 3.78-3.82 (1H, m), 3.90 (1H, dd, J = 11.0, 2.5 Hz), 4.00-4.05 (1H, m), 4.21-4.22 (2H, m), 7.66 (1H, dd, J = 7.4, 1.8 Hz), 7.78-7.85 (2H, m). |
| 153-2 | 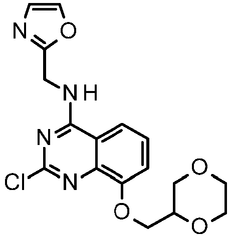 | 1H-NMR (DMSO-D6) δ: 3.41-3.54 (2H, m), 3.63-3.71 (2H, m), 3.76-3.80 (1H, m), 3.88 (1H, dd, J = 11.3, 2.8 Hz), 3.95-4.00 (1H, m), 4.06-4.13 (2H, m), 4.84 (2H, d, J = 5.8 Hz), 7.17 (1H, s), 7.37 (1H, dd, J = 8.0, 1.2 Hz), 7.48 (1H, t, J = 8.3 Hz), 7.83 (1H, dd, J = 8.3, 0.9 Hz), 8.07 (1H, s), 9.28 (1H, t, J = 5.8 Hz). |
| | | MS (m/z): 377 (M+H)⁺ |

Compounds of Examples 154 to 169 shown below were produced in accordance with the steps described in Examples 144 to 153 above.

**[Table 11]**

| Example | Structural formulae | Data |
|---|---|---|
| 154 | 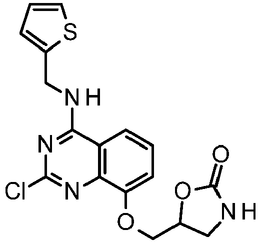 | 1H-NMR (DMSO-D6) δ: 3.35-3.43 (1H, m), 3.65 (1H, t, J = 9.1 Hz), 4.24-4.34 (2H, m), 4.88 (2H, d, J = 5.5 Hz), 4.97-5.06 (1H, m), 6.98 (1H, dd, J = 4.9, 3.6 Hz), 7.10 (1H, d, J = 2.4 Hz), 7.36-7.42 (2H, m), 7.47 (1H, t, J = 7.9 Hz), 7.65 (1H, s), 7.82 (1H, d, J = 7.3 Hz), 9.30 (1H, t, J = 6.1 Hz). |
| | | MS (m/z): 391 (M+H)⁺. |
| 155 | 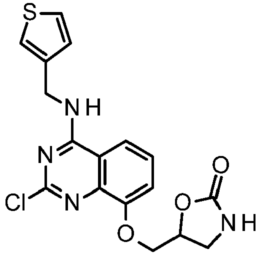 | 1H-NMR (DMSO-D6) δ: 3.39 (1H, t, J = 7.9 Hz), 3.65 (1H, t, J = 8.8 Hz), 4.23-4.34 (2H, m), 4.73 (2H, d, J = 5.5 Hz), 4.95-5.07 (1H, m), 7.14 (1H, d, J = 4.9 Hz), 7.38 (2H, d, J = 7.9 Hz), 7.46 (1H, t, J = 7.9 Hz), 7.51 (1H, dd, J = 4.9, 3.0 Hz), 7.65 (1H, s), 7.86 (1H, d, J = 8.5 Hz), 9.15 (1H, t, J = 5.8 Hz). |
| | | MS (m/z): 391 (M+H)⁺. |
| 156-1 | 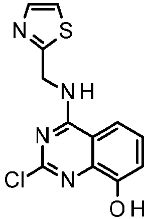 | 1H-NMR (DMSO-D6) δ: 5.01 (3H, d, J = 6.1 Hz), 7.19 (2H, dd, J = 8.0, 1.2 Hz), 7.40 (2H, t, J = 8.0 Hz), 7.64 (1H, d, J = 3.1 Hz), 7.69 (1H, dd, J = 8.0, 1.2 Hz), 7.76 (1H, d, J = 3.7 Hz), 9.48 (1H, t, J = 6.1 Hz), 9.89 (1H, s). |
| 156-2 | 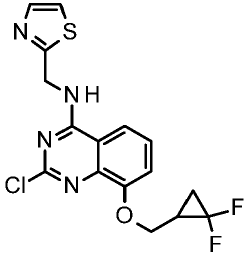 | 1H-NMR (DMSO-D6) δ: 1.48-1.57 (1H, m), 1.73-1.82 (1H, m), 2.32-2.41 (1H, m), 4.13-4.18 (1H, br m), 4.25-4.30 (1H, br m), 5.01 (2H, d, J = 6.1 Hz), 7.38 (1H, d, J = 7.4 Hz), 7.50 (1H, t, J = 8.0 Hz), 7.65 (1H, d, J = 3.1 Hz), 7.76 (1H, d, J = 3.1 Hz), 7.84 (1H, d, J = 8.6 Hz), 9.52 (1H, t, J = 6.1 Hz). |
| | | MS (m/z): 383 (M+H)⁺. |
| 157 | 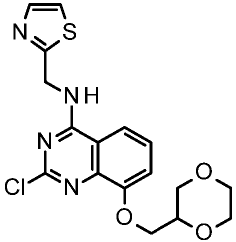 | 1H-NMR (DMSO-D6) δ: 3.41-3.54 (2H, m), 3.63-3.70 (2H, m), 3.76-3.81 (1H, m), 3.88 (1H, dd, J = 11.0, 2.5 Hz), 3.95-4.00 (1H, m), 4.06-4.14 (2H, m), 5.01 (2H, d, J = 5.5 Hz), 7.38 (1H, d, J = 8.0 Hz), 7.49 (1H, t, J = 8.0 Hz), 7.64 (1H, d, J = 3.1 Hz), 7.76 (1H, d, J = 3.1 Hz), 7.82 (1H, d, J = 8.6 Hz), 9.50 (1H, t, J = 5.5 Hz). |
| | | MS (m/z): 393 (M+H)⁺. |
| 158 | 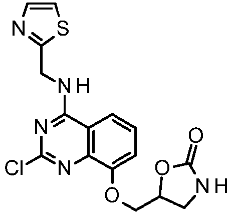 | 1H-NMR (DMSO-D6) δ: 3.35-3.43 (1H, m), 3.65 (1H, t, J = 9.1 Hz), 4.23-4.37 (2H, m), 4.94-5.09 (3H, m), 7.42 (1H, d, J = 7.9 Hz), 7.51 (1H, t, J = 7.9 Hz), 7.65 (2H, t, J = 2.4 Hz), 7.76 (1H, d, J = 3.0 Hz), 7.86 (1H, d, J = 7.9 Hz), 9.53 (1H, br s). |
| | | MS (m/z): 392 (M+H)⁺. |
| 159 | 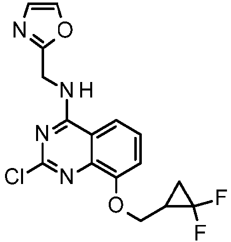 | 1H-NMR (DMSO-D6) δ: 1.48-1.57 (1H, m), 1.73-1.82 (1H, m), 2.30-2.40 (1H, m), 4.13-4.18 (1H, br m), 4.24-4.29 (1H, br m), 4.85 (2H, d, J = 6.1 Hz), 7.17 (1H, br s), 7.38 (1H, d, J = 7.4 Hz), 7.49 (1H, t, J = 8.3 Hz), 7.85 (1H, dd, J = 8.6, 1.2 Hz), 8.07 (1H, s), 9.30 (1H, t, J = 6.1 Hz). |
| | | MS (m/z): 367 (M+H)⁺. |
| 160-1 | 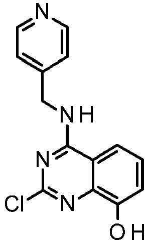 | Y82795-046 1H-NMR (DMSO-D6) δ: 4.77 (2H, d, J = 6.1 Hz), 7.18 (1H, dd, J = 8.0, 1.2 Hz), 7.34-7.35 (2H, m), 7.39 (1H, t, J = 8.0Hz), 7.71 (1H, dd, J=8.0, 1.2 Hz), 8.51-8.52 (2H, m), 9.22 (1H, t, J = 6.1 Hz), 9.82 (1H, s). |
| 160-2 | 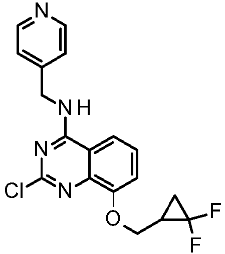 | 1H-NMR (DMSO-D6) δ: 1.48-1.57 (1H, m), 1.73-1.82 (1H, m), 2.32-2.40 (1H, m), 4.13-4.18 (1H, br m), 4.25-4.30 (1H, br m), 4.77 (2H, d, J = 5.5 Hz), 7.34-7.39 (3H, m), 7.49 (1H, t, J = 8.0 Hz), 7.87 (1H, d, J = 8.0 Hz), 8.51-8.52 (2H, m), 9.26 (1H, t, J = 5.5 Hz). |
| | | MS (m/z): 377 (M+H)⁺. |
| 161 | 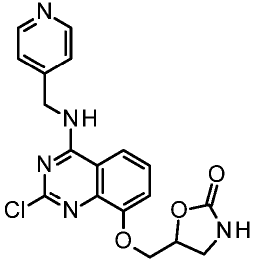 | 1H-NMR (DMSO-D6) δ: 3.35-3.43 (1H, m), 3.65 (1H, t, J = 8.8 Hz), 4.24-4.35 (2H, m), 4.77 (2H, d, J = 4.3 Hz), 4.97-5.06 (1H, m), 7.34 (2H, d, J = 5.5 Hz), 7.41 (1H, d, J = 7.9 Hz), 7.50 (1H, t, J = 8.2 Hz), 7.65 (1H, s), 7.89 (1H, d, J = 8.5 Hz), 8.51 (2H, d, J = 5.5 Hz), 9.27 (1H, br s). |
| | | MS (m/z): 386 (M+H)⁺. |
| 162-1 | 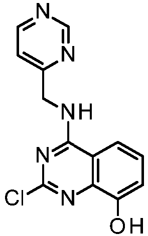 | ¹H-NMR (DMSO-D₆) δ: 4.81 (2H, d, *J* = 6.1 Hz), 7.19 (1H, dd, *J* = 7.9, 1.2 Hz), 7.41 (1H, t, *J* = 7.9 Hz), 7.46 (1H, dd, *J* = 5.5, 1.2 Hz), 7.71-7.77 (1H, m), 8.72 (1H, d, *J* = 4.9 Hz), 9.13 (1H, d, *J* = 1.2 Hz), 9.28 (1H, t, *J* = 5.8 Hz), 9.84 (1H, s). |
| 162-2 | 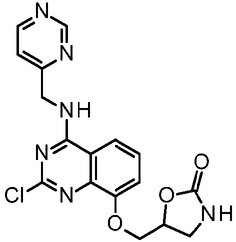 | 1H-NMR (DMSO-D6) δ: 3.34-3.44 (1H, m), 3.65 (1H, t, J = 8.8 Hz), 4.25-4.36 (2H, m), 4.82 (2H, s), 4.97-5.07 (1H, m), 7.42 (1H, d, J = 7.3 Hz), 7.47 (1H, dd, J = 5.5, 1.2 Hz), 7.52 (1H, t, J = 8.2 Hz), 7.65 (1H, s), 7.91 (1H, d, J = 8.5 Hz), 8.73 (1H, d, J = 4.9 Hz), 9.13 (1H, s), 9.34 (1H, br s). |
| | | MS (m/z): 387 (M+H)⁺. |
| 163-1 | 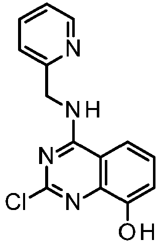 | ¹H-NMR (DMSO-D₆) δ: 4.83 (2H, d, *J* = 6.1 Hz), 7.17 (1H, d, *J* = 6.7 Hz), 7.29 (1H, dd, *J* = 7.0, 5.2 Hz), 7.37 (2H, dd, *J* = 15.8, 7.9 Hz), 7.71-7.79 (2H, m), 8.53 (1H, d, *J* = 4.3 Hz), 9.24 (1H, t, *J* = 6.1 Hz), 9.79 (1H, s). |
| 163-2 | 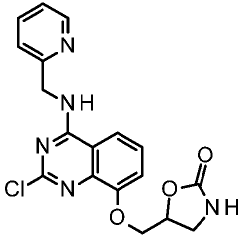 | 1H-NMR (DMSO-D6) δ: 3.39 (1H, t, J = 7.3 Hz), 3.65 (1H, t, J = 8.8 Hz), 4.22-4.35 (2H, m), 4.83 (2H, d, J = 5.5 Hz), 4.97-5.08 (1H, m), 7.29 (1H, dd, J = 7.3, 4.9 Hz), 7.35 (1H, d, J = 7.9 Hz), 7.40 (1H, d, J = 7.3 Hz), 7.49 (1H, t, J = 7.9 Hz), 7.65 (1H, s), 7.76 (1H, td, J = 7.6, 1.8 Hz), 7.91 (1H, d, J = 7.3 Hz), 8.53 (1H, d, J = 4.9 Hz), 9.29 (1H, t, J = 5.5 Hz). |
| | | MS (m/z): 386 (M+H)⁺. |
| 164-1 | 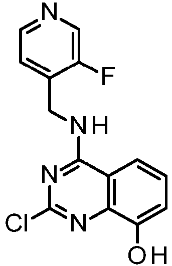 | ¹H-NMR (DMSO-D₆) δ: 4.81 (2H, d, *J* = 6.1 Hz), 7.18 (1H, dd, *J* = 7.9, 1.2 Hz), 7.36-7.43 (2H, m), 7.72 (1H, dd, *J* = 8.5, 1.2 Hz), 8.37 (1H, dd, *J* = 4.9, 1.2 Hz), 8.56 (1H, d, *J* = 1.8 Hz), 9.22 (1H, t, *J* = 5.8 Hz), 9.86 (1H, s). |
| 164-2 | 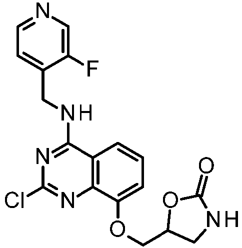 | 1H-NMR (DMSO-D6) δ: 3.33-3.44 (1H, m), 3.65 (1H, t, J = 9.1 Hz), 4.24-4.37 (2H, m), 4.81 (2H, d, J = 5.5 Hz), 4.95-5.09 (1H, m), 7.40 (2H, dd, J = 14.3, 6.4 Hz), 7.51 (1H, t, J = 8.2 Hz), 7.65 (1H, s), 7.89 (1H, d, J = 7.9 Hz), 8.37 (1H, d, J = 4.9 Hz), 8.56 (1H, d, J = 1.8 Hz), 9.26 (1H, t, J = 5.8 Hz). |
| | | MS (m/z): 404 (M+H)⁺. |
| 165-1 | 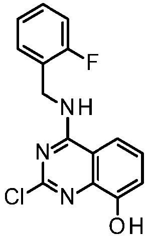 | ¹H-NMR (CDCl₃) δ: 4.93 (2H, d, *J* = 5.5 Hz), 6.17 (1H, s), 7.05-7.19 (3H, m), 7.22 (1H, d, *J* = 7.3 Hz), 7.28-7.39 (2H, m), 7.50 (2H, t, *J* = 7.3 Hz). |
| 165-2 | 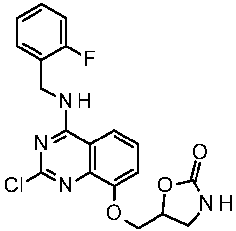 | 1H-NMR (DMSO-D6) δ: 3.34-3.43 (1H, m), 3.65 (1H, t, J = 9.1 Hz), 4.21-4.37 (2H, m), 4.77 (2H, d, J = 5.5 Hz), 4.96-5.07 (1H, m), 7.10-7.30 (2H, m), 7.30-7.43 (3H, m), 7.48 (1H, t, J = 7.9 Hz), 7.65 (1H, s), 7.90 (1H, d, J = 7.9 Hz), 9.17 (1H, t, J = 5.5 Hz). |
| | | MS (m/z): 389 (M+H)⁺. |
| 166-1 | 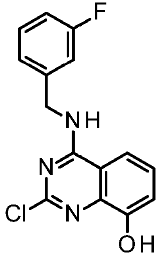 | ¹H-NMR (CDCl₃) δ: 4.88 (2H, d, *J* = 5.5 Hz), 6.08 (1H, s), 6.95-7.09 (1H, m), 7.12 (2H, t, *J* = 9.4 Hz), 7.19 (1H, d, *J* = 7.9 Hz), 7.21-7.28 (1H, m), 7.29-7.42 (2H, m), 7.47-7.59 (1H, m). |
| 166-2 | 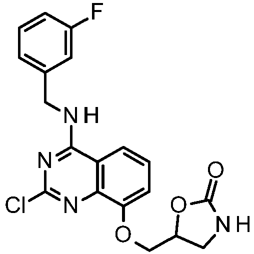 | 1H-NMR (DMSO-D6) δ: 3.36-3.41 (1H, m), 3.65 (1H, t, J = 8.8 Hz), 4.23-4.35 (2H, m), 4.76 (2H, d, J = 5.5 Hz), 4.97-5.07 (1H, m), 7.10 (1H, td, J = 8.7, 2.2 Hz), 7.19 (2H, t, J = 7.6 Hz), 7.34-7.43 (2H, m), 7.48 (1H, t, J = 7.9 Hz), 7.65 (1H, s), 7.88 (1H, d, J = 8.5 Hz), 9.23 (1H, t, J = 5.8 Hz). |
| | | MS (m/z): 403 (M+H)⁺. |
| 167-1 | 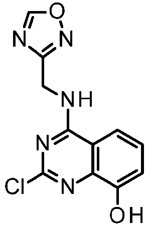 | ¹H-NMR (CD₃OD) δ: 4.98 (2H, s), 7.18 (1H, dd, *J* = 7.9, 1.2 Hz), 7.37 (1H, t, *J* = 8.2 Hz), 7.55 (1H, dd, *J* = 8.5, 1.2 Hz), 9.20 (1H, s). |
| 167-2 | 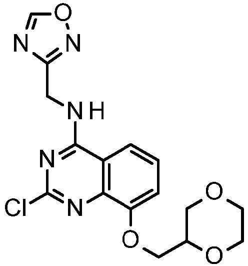 | 1H-NMR (DMSO-D6) δ: 3.39-3.57 (2H, m), 3.61-3.72 (2H, m), 3.78 (1H, d, J = 11.0 Hz), 3.88 (1H, dd, J = 11.6, 2.4 Hz), 3.93-4.01 (1H, m), 4.04-4.16 (2H, m), 4.90 (2H, s), 7.37 (1H, d, J = 7.9 Hz), 7.49 (1H, t, J = 8.2 Hz), 7.83 (1H, d, J = 7.3 Hz), 9.32 (1H, s), 9.58 (1H, s). |
| | | MS (m/z): 378 (M+H)⁺. |
| 168 | 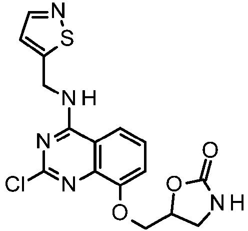 | 1H-NMR (DMSO-D6) δ: 3.39 (1H, t, J = 7.6 Hz), 3.65 (1H, t, J = 8.8 Hz), 4.25-4.35 (2H, m), 4.93-5.09 (3H, m), 7.42 (2H, dd, J = 6.4, 1.5 Hz), 7.50 (1H, t, J = 8.2 Hz), 7.65 (1H, s), 7.80 (1H, d, J = 8.5 Hz), 8.46 (1H, d, J = 1.8 Hz), 9.46 (1H, t, J = 5.8 Hz). |
| | | MS (m/z): 392 (M+H)⁺. |
| 169-1 | 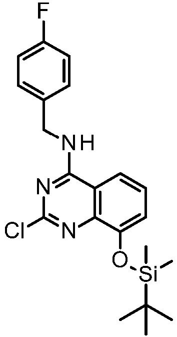 | ¹H-NMR (CDCl₃) δ: 0.29 (6H, s), 1.04 (9H, s), 4.81 (2H, d, *J* = 5.5 Hz), 5.93 (1H, br s), 7.07 (2H, t, *J* = 8.8 Hz), 7.20 (2H, dd, *J* = 11.2, 8.2 Hz), 7.30 (1H, t, *J* = 7.9 Hz), 7.39 (2H, dd, *J*= 8.2, 5.2 Hz). |
| 169-2 | 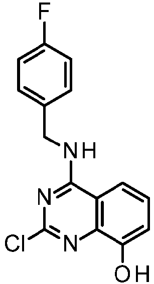 | ¹H-NMR (CDCl₃) δ: 4.84 (2H, d, *J* = 5.5 Hz), 5.11 (2H, br s), 7.03-7.14 (3H, m), 7.21-7.25 (1H, m), 7.33 (1H, d, *J* = 7.9 Hz), 7.36-7.43 (2H, m). |
| 169-3 | 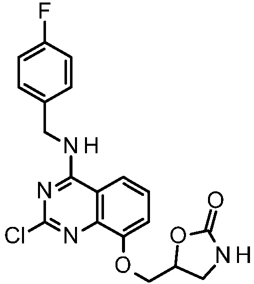 | 1H-NMR (DMSO-D6) δ: 3.34-3.43 (1H, m), 3.65 (1H, t, J = 9.1 Hz), 4.23-4.34 (2H, m), 4.72 (2H, d, J = 4.9 Hz), 4.94-5.07 (1H, m), 7.17 (2H, t, J = 8.8 Hz), 7.40 (3H, dt, J = 10.1, 3.9 Hz), 7.47 (1H, t, J = 7.9 Hz), 7.65 (1H, s), 7.86 (1H, d, J = 8.5 Hz), 9.20 (1H, t, J = 5.5 Hz). |
| | | MS (m/z): 403 (M+H)⁺. |

### (Example 170) 5-[({2-Chloro-4-[(furan-2-ylmethyl)amino]-1,7-naphthyridin-8-yl}oxy)methyl]-3-methyl-1,3-oxazolidin-2-one

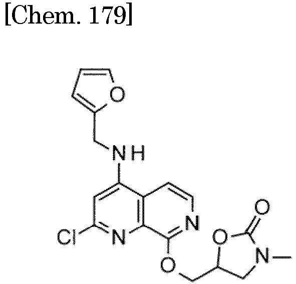

### (Example 170-1) 8-Methoxy-2H-pyrido[3,4-d][1,3]oxazine-2,4(1H)-dione

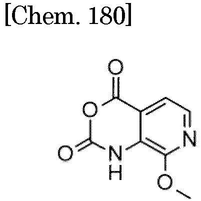

Triphosgene (1.73 g, 5.84 mmol) was slowly added to a suspension (30 mL) of 3-amino-2-methoxy-isonicotinic acid (1.00 g, 5.96 mmol) in THF, followed by stirring at room temperature overnight. The reaction solution was concentrated under reduced pressure. Diethyl ether (30 mL) was added to the resulting residue, and the insoluble material was collected by filtration. Thus, the title compound (1.09 g, 94% yield) was obtained as a cream-colored solid.

### (Example 170-2) Ethyl 4-hydroxy-8-methoxy-2-oxo-1,2-dihydro-1,7-naphthyridine-3-carboxylate

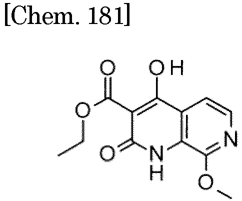

A mixed solution (25 mL) of 8-methoxy-2H-pyrido[3,4-d][1,3]oxazine-2,4(1H)-dione (1.09 g, 5.61 mmol) obtained in Example 170-1 and diethyl malonate (8.51 mL, 56.0 mmol) in DMF was added to a suspension (25 mL) of 55% sodium hydride (dispersed in liquid paraffin) (320 mg, 7.30 mmol) in DMF at room temperature, followed by stirring at 160°C for 4 hours. After the reaction solution was allowed to cool to room temperature, 1M hydrochloric acid (13 mL) was added to acidify the reaction solution, followed by extraction with ethyl acetate. The organic layer was washed with a saturated saline solution, dried over anhydrous magnesium sulfate, filtered, and then concentrated under reduced pressure. Diethyl ether (200 mL) was added to the resulting residue, and the insoluble material was collected by filtration. Thus, the title compound (902 mg, 61% yield) was obtained as a cream-colored solid.

### (Example 170-3) 4-Hydroxy-8-methoxy-1,7-naphthyridin-2(1H)-one

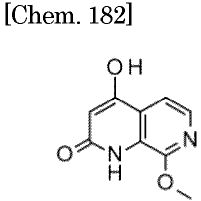

An aqueous solution (19 mL) of ethyl 4-hydroxy-8-methoxy-2-oxo-1,2-dihydro-1,7-naphthyridine-3-carboxylate (900 mg, 3.41 mmol) obtained in Example 170-2 and potassium hydroxide (712 mg, 12.7 mmol) was stirred at 120°C for 16 hours. After the reaction solution was allowed to cool to room temperature, 1M hydrochloric acid (14 mL) was added to acidify the reaction solution, followed by stirring at 0°C for 30 minutes. The precipitated solid was collected by filtration to obtain the title compound (595 mg, 91% yield) as a white solid.

### (Example 170-4) 8-Methoxy-2-oxo-1,2-dihydro-1,7-naphthyridin-4-yl trifluoromethanesulfonate

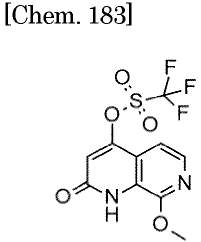

N-Phenylbis(trifluoromethanesulfonimide) (445 mg, 1.24 mmol) was added to a solution (2 mL) of 4-hydroxy-8-methoxy-1,7-naphthyridin-2(1H)-one (199 mg, 1.04 mmol) obtained in Example 170-3 and triethylamine (0.430 mL, 3.11 mmol) in THF, followed by stirring at room temperature overnight. The reaction solution was concentrated under reduced pressure. Chloroform (2 mL) was added to the resulting residue, and the insoluble material was collected by filtration. Thus, the title compound (174 mg) was obtained as a white solid. In addition, the filtrate was concentrated under reduced pressure. The resulting residue was then purified by silica gel column chromatography (ethyl acetate/hexane = 25/75 to 50/50) to obtain the title compound (129 mg, 90% overall yield) as a white solid.

### (Example 170-5) 4-[(Furan-2-ylmethyl)amino]-8-methoxy-1,7-naphthyridin-2(1H)-one

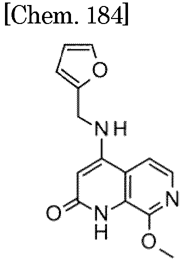

Furfurylamine (820 µL, 8.86 mmol) was added to a suspension (15 mL) of 8-methoxy-2-oxo-1,2-dihydro-1,7-naphthyridin-4-yl trifluoromethanesulfonate (958 mg, 2.95 mmol) obtained in Example 170-4 in acetonitrile, followed by stirring at 60°C for 6 hours. The reaction solution was allowed to cool to room temperature and then concentrated under reduced pressure until its volume was reduced to approximately half. The solid was then collected by filtration. The resulting crudely purified product was washed with ice-cooled acetonitrile and then dried to obtain the title compound (547 mg, 68% yield) as a light brown solid.

### (Example 170-6) 2,8-Dichloro-N-(furan-2-ylmethyl)-1,7-naphthyridin-4-amine

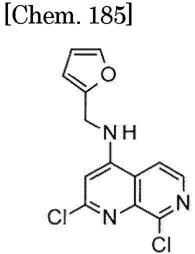

N,N-Diethylaniline (635 µL, 3.96 mmol) was added to a mixture of 4-[(furan-2-ylmethyl)amino]-8-methoxy-1,7-naphthyridin-2(1H)-one (537 mg, 1.98 mmol) obtained in Example 170-5 and phosphoryl chloride (16.8 g, 109 mmol). The mixture was then stirred at 90°C for 3 hours, followed by stirring at 100°C for 9 hours. The reaction solution was allowed to cool to room temperature and then concentrated under reduced pressure. Then, the operation of adding toluene to the resulting residue and concentrating the mixture was repeated twice. Chloroform (10 mL) was added to the resulting residue, and a saturated aqueous solution of sodium bicarbonate was added under vigorous stirring for neutralization. The neutralized mixture was then extracted with chloroform, followed by concentration under reduced pressure. The resulting residue was purified by silica gel column chromatography (NH, ethyl acetate/hexane = 0/100 to 40/60) to obtain the title compound (401 mg, 69% yield) as a light brown solid.

### (Example 170-7) 5-[({2-Chloro-4-[(furan-2-ylmethyl)amino]-1,7-naphthyridin-8-yl}oxy)methyl]-3-methyl-1,3-oxazolidin-2-one

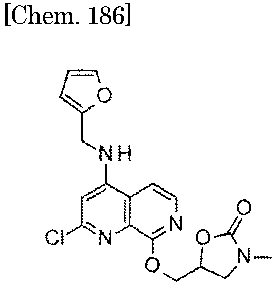

55% Sodium hydride (dispersed in liquid paraffin) (47.0 mg, 1.08 mmol) was added to a solution (5 mL) of 5-(hydroxymethyl)-3-methyl-1,3-oxazolidin-2-one (106 mg, 0.808 mmol) in THF at room temperature, and the mixture was stirred for 1 hour. To this mixture, a solution (3 mL) of 2,8-dichloro-N-(furan-2-ylmethyl)-1,7-naphthyridin-4-amine (198 mg, 0.673 mmol) obtained in Example 170-6 in THF was added, followed by stirring at 60°C for 7 hours. To this reaction solution, a mixture of 5-(hydroxymethyl)-3-methyl-1,3-oxazolidin-2-one (98.2 mg, 0.749 mmol) and 55% sodium hydride (dispersed in liquid paraffin) (34.0 mg, 0.779 mmol) in THF (3 mL), which had been prepared beforehand by stirring for 1 hour, was added. The mixture was then stirred at 60°C for an additional 7 hours. Water (5 mL) was added to the reaction solution under ice-cooling, followed by extraction with ethyl acetate and subsequent concentration under reduced pressure. The resulting residue was purified by silica gel column chromatography (ethyl acetate/hexane = 10/90 to 100/0) to obtain the title compound (131 mg, 50% yield) as a white solid.

### (Example 171) (5S)-5-[({2-Chloro-4-[(furan-2-ylmethyl)amino]-1,7-naphthyridin-8-yl}oxy)methyl]-3-methyl-1,3-oxazolidin-2-one

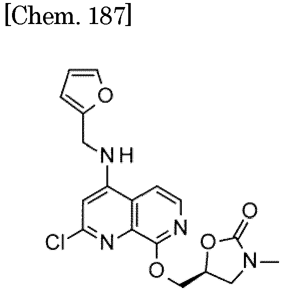

(5S)-5-(Hydroxymethyl)-3-methyl-1,3-oxazolidin-2-one (123 mg, 0.941 mmol) and cesium carbonate (472 mg, 1.45 mmol) were added to a solution (7.2 mL) of 2,8-dichloro-N-(furan-2-ylmethyl)- 1,7-naphthyridin-4-amine (213 mg, 0.724 mmol) obtained in Example 170-6 in acetonitrile, followed by treatment in a microwave reactor at 110°C for 2.5 hours. After the reaction solution was allowed to cool to room temperature, ethyl acetate (8 mL) was added to the reaction solution. The insoluble material was filtered, followed by concentration under reduced pressure. The resulting residue was crudely purified by silica gel column chromatography (NH, ethyl acetate/hexane = 20/80 to 100/0), and then purified again by silica gel column chromatography (ethyl acetate/hexane = 20/80 to 100/0) to obtain the title compound (184 mg, 65% yield) as a white solid. Note that the title compound can also be obtained by dividing 5-[(12-chloro-4-[(furan-2-ylmethyl)amino]- 1,7-naphthyridin-8-yl}oxy)methyl]-3-methyl-1,3-oxazolidin-2-one obtained in Example 170-7, by chiral HPLC. (Analytical Conditions) Column: DAICEL CHIRALPAK IC (4.6 x 150 mm), Elution solvent: hexane/ethanol = 55/45, Flow rate: 1.00 mL/min, Retention time: 4.92 min.

### (Example 172) (5R)-5-[({2-Chloro-4-[(furan-2-ylmethyl)amino]-1,7-naphthyridin-8-yl}oxy)methyl]-3-methyl-1,3-oxazolidin-2-one

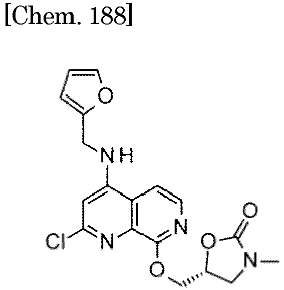

The same operation as in Example 171 was performed using 2,8-dichloro-N-(furan-2-ylmethyl)-1,7-naphthyridin-4-amine (210 mg, 0.714 mmol) obtained in Example 170-6 and (5R)-5-(hydroxymethyl)-3-methyl-1,3-oxazolidin-2-one (122 mg, 0.928 mmol). Thus, the title compound (171 mg, 62% yield) was obtained as a white solid. Note that the title compound can also be obtained by dividing 5-[({2-chloro-4-[(furan-2-yhnethyl)amino]-1,7-naphthyridin-8-yl}oxy)methyl]-3-methyl-1,3-oxazolidin-2-one obtained in Example 170-7, by chiral HPLC. (Analytical Conditions) Column: DAICEL CHIRALPAK IC (4.6 x 150 mm), Elution solvent: hexane/ethanol = 55/45, Flow rate: 1.00 mL/min, Retention time: 6.36 min.

### (Example 173) 5-({[4-(Benzylamino)-2-chloro-1,7-naphthyridin-8-yl]oxy}methyl)-1,3-oxazolidin-2-one

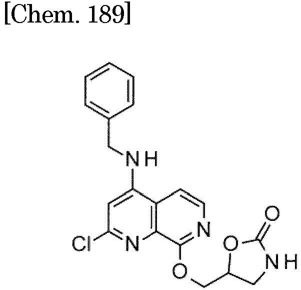

### (Example 173-1) 4-(Benzylamino)-8-methoxy-1,7-naphthyridin-2(1H)-one

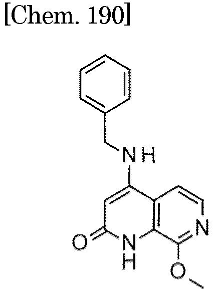

The same operation as in Example 170-5 was performed using 8-methoxy-2-oxo-1,2-dihydro-1,7-naphthyridin-4-yl trifluoromethanesulfonate (1.09 g, 3.36 mmol) obtained in Example 170-4 and benzylamine (1.10 mL, 10.1 mmol). Thus, the title compound (0.610 g, 65% yield) was obtained as a white solid.

### (Example 173-2) N-Benzyl-2,8-dichloro-1,7-naphthyridin-4-amine

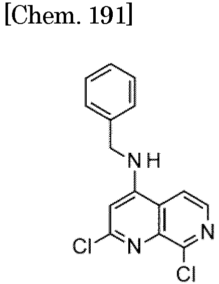

The same operation as in Example 170-6 was performed using 4-(benzylamino)-8-methoxy-1,7-naphthyridin-2(1H)-one (610 mg, 2.17 mmol) obtained in Example 173-1. Thus, the title compound (610 mg, 93% yield) was obtained as a white solid.

### (Example 173-3) 5-({[4-(Benzylamino)-2-chloro-1,7-naphthyridin-8-yl]oxy}methyl)-1,3-oxazolidin-2-one

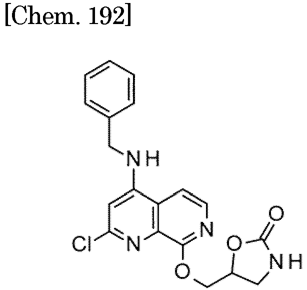

The same operation as in Example 170-7 was performed using N-benzyl-2,8-dichloro-1,7-naphthyridin-4-amine (151 mg, 0.496 mmol) obtained in Example 173-2 and 5-(hydroxymethyl)-3-[(4-methoxyphenyl)methyl]oxazolidin-2-one (142 mg, 0.596 mmol) disclosed in WO 2014/085413. Thus, crude 5-({[4-(benzylamino)-2-chloro-1,7-naphthyridin -8-yl]oxy}methyl)-3-(4-methoxybenzyl)-1,3-oxazolidin-2-one (58.4 mg) [MS (m/z): 505 (M+H)⁺] was obtained as a colorless solid. A solution (1 mL) of the obtained 5-({[4-(benzylamino)-2-chloro-1,7-naphthyridin-8-yl]oxy}methyl)-3-(4-methoxybenzyl)-1,3-oxazolidin-2-one (50.2 mg, 0.0994 mmol) in trifluoroacetic acid was stirred at 60°C for 46 hours. Then, trifluoroacetic acid (0.5 mL) was further added, and the mixture was stirred for an additional 18 hours. The reaction solution was concentrated under reduced pressure, dissolved in dichloromethane to form a solution, and a saturated aqueous solution of sodium bicarbonate was added for neutralization. The mixture was then extracted with dichloromethane, followed by ethyl acetate. All organic layers were concentrated under reduced pressure. The resulting residue was crudely purified by silica gel column chromatography (ethyl acetate/hexane = 20/80 to 100/0), and then purified again by silica gel column chromatography (NH, ethyl acetate/hexane = 50/50 to 100/0) to obtain the title compound (13.5 mg, 35% yield) as a colorless solid.

### (Example 174) 5-[({2-Chloro-4-[(furan-2-ylmethyl)amino]-1,7-naphthyridin-8-yl}oxy)methyl]-3-2-fluoroethyl)-1,3-oxazolidin-2-one

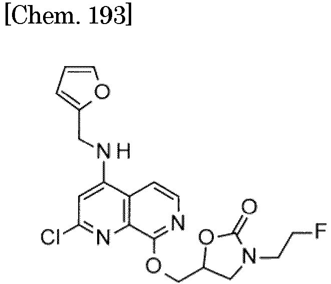

The same operation as in Example 171 was performed using 2,8-dichloro-N-(furan-2-ylmethyl)-1,7-naphthyridin-4-amine (85.0 mg, 0.289 mmol) obtained in Example 170-6 and 3-(2-fluoroethyl)-5-(hydroxymethyl)-1,3-oxazolidin-2-one (70.7 mg, 0.433 mmol) obtained in Example 41-2. Thus, the title compound (85.1 mg, 70% yield) was obtained as a colorless solid.

### (Example 175) 5-[({2-Chloro-4-[(furan-2-ylmethyl)amino]-1,7-naphthyridin-8-yl}oxy)methyl]-3-(2-hydroxyethyl)-1,3-oxazolidin-2-one

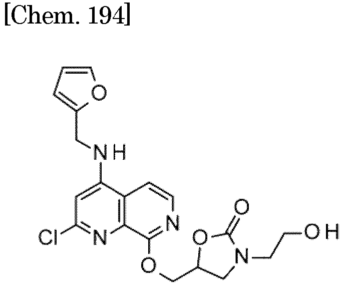

### (Example 175-1) 3-(2-{[tert-Butyl(dimethyl)silyl]oxy}ethyl)-5-[({2-chloro-4-[(furan-2-ylmethyl)amino]-1,7-naphthyridin-8-yl}oxy)methyl]-1,3-oxazolidin-2-one

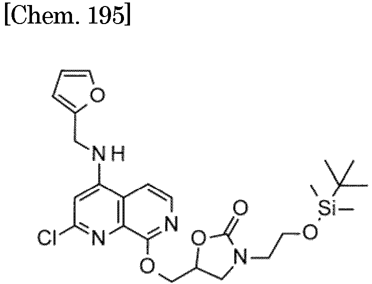

The same operation as in Example 170-7 was performed using 2,8-dichloro-N-(furan-2-ylmethyl)-1,7-naphthyridin-4-amine (46.3 mg, 0.157 mmol) obtained in Example 170-6 and 3-(2-{[tert-butyl(dimethyl)silyl]oxy}ethyl)-5-(hydroxymethyl)-1,3-oxazolidin-2-one (60.6 mg, 0.220 mmol) obtained in Example 42-2. Thus, the title compound (12.9 mg, 15% yield) was obtained as a colorless solid.

### (Example 175-2) 5-[({2-Chloro-4-[(furan-2-ylmethyl)amino]-1,7-naphthyridin-8-yl}oxy)methyl]-3-(2-hydroxyethyl)-1,3-oxazolidin-2-one

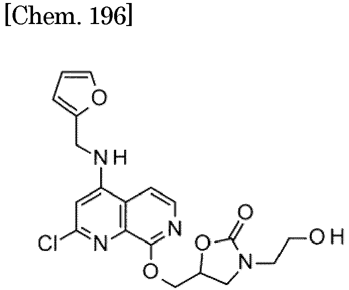

1M TBAF-THF (36.0 µL, 0.0360 mmol) was added to a solution (0.6 mL) of 3-(2-{[tert-butyl(dimethyl)silyl]oxy}ethyl)-5-[({2-chloro-4-[(furan-2-ylmethyl)amino]-1,7-naphthyridin-S-yl}oxy)methyl]-1,3-oxazolidin-2-one (12.5 mg, 0.0234 mmol) obtained in Example 175-1 in THF, followed by stirring at room temperature for 1 hour. The reaction solution was concentrated under reduced pressure. The resulting residue was purified by silica gel column chromatography (NH, methanol/ethyl acetate = 0/100 to 7/93) to obtain the title compound (7.10 mg, 72% yield) as a white solid.

### (Example 176) 5-({[4-(Benzylamino)-2-chloro-1,7-naphthyridin-8-yl]oxy}methyl)-3-(2-fluoroethyl)-1,3-oxazolidin-2-one

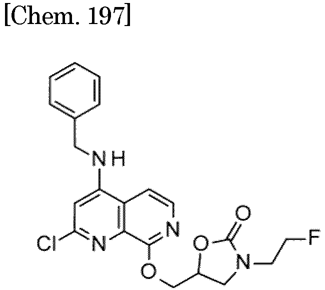

The same operation as in Example 170-7 was performed using N-benzyl-2,8-dichloro-1,7-naphthyridin-4-amine (94.6 mg, 0.311 mmol) obtained in Example 173-2 and 3-(2-fluoroethyl)-5-(hydroxymethyl)-1,3-oxazolidin -2-one (76.1 mg, 0.467 mmol) obtained in Example 41-2. Thus, the title compound (58.6 mg, 44% yield) was obtained as a white solid.

### (Example 177) 5-({[4-(Benzylamino)-2-chloro-1,7-naphthyridin-8-yl]oxy}methyl)-3-methyl-1,3-oxazolidin-2-one

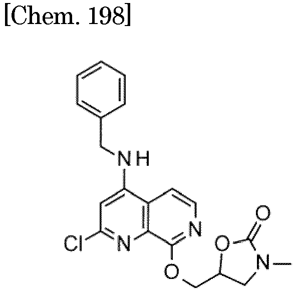

The same operation as in Example 170-7 was performed using N-benzyl-2,8-dichloro-1,7-naphthyridin-4-amine (201 mg, 0.661 mmol) obtained in Example 173-2 and 5-(hydroxymethyl)-3-methyl-1,3-oxazolidin-2-one (104 mg, 0.793 mmol). Thus, the title compound (167 mg, 63% yield) was obtained as a white solid.

### (Example 178) 5-({[4-(Benzylamino)-2-chloro-1,7-naphthyridin-8-yl]oxy}methyl)-3-(2-hydroxyethyl)-1,3-oxazolidin-2-one

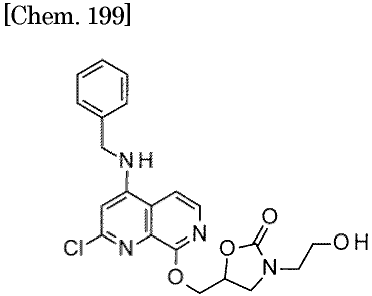

### (Example 178-1) 5-({[4-(Benzylamino)-2-chloro-1,7-naphthyridin-8-yl]oxy}methyl)-3-(2-{[tert-butyl(dimethyl)silyl]oxy}ethyl)-1,3-oxazolidin-2-one

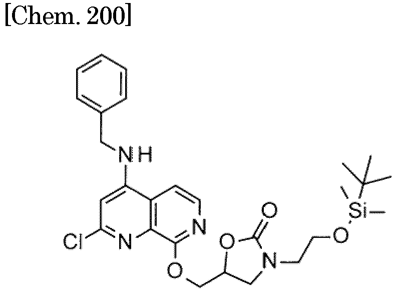

The same operation as in Example 170-7 was performed using N-benzyl-2,8-dichloro-1,7-naphthyridin-4-amine (109 mg, 0.358 mmol) obtained in Example 173-2 and 3-(2-{[tert-butyl(dimethyl)silyl]oxy}ethyl)-5-(hydroxymethyl)-1,3-oxazolidin-2-one (148 mg, 0.538 mmol) obtained in Example 42-2. Thus, the title compound (59.9 mg, 31% yield) was obtained as a white solid.

### (Example 178-2) 5-({[4-(Benzylamino)-2-chloro-1,7-naphthyridin-8-yl]oxy}methyl)-3-(2-hydroxyethyl)-1,3-oxazolidin-2-one

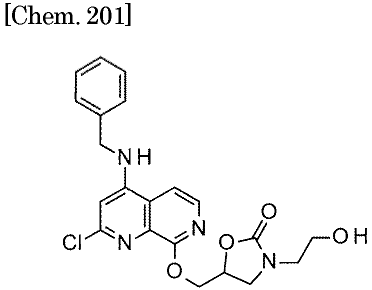

The same operation as in Example 175-2 was performed using 5-({[4-(benzylamino)-2-chloro-1, 7-naphthyridin-8-yl]oxy}methyl)-3-(2-{[tert-butyl(dimethyl)silyl]oxy}ethyl)-1,3-oxazolidin-2-one (58.9 mg, 0.108 mmol) obtained in Example 178-1. Thus, the title compound (25.6 mg, 55% yield) was obtained as a white solid.

### (Example 179) 2-Chloro-8-(1,4-dioxan-2-ylmethoxy)-N-(furan-2-ylmethyl)pyrido[3,2-d]pyrimidin-4-amine

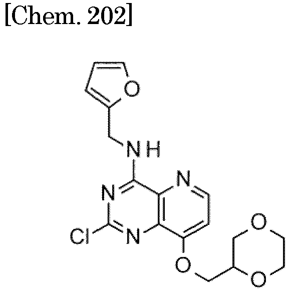

### (Example 179-1) 2,8-Dichloro-N-(furan-2-ylmethyl)pyrido[3,2-d]pyrimidin-4-amine

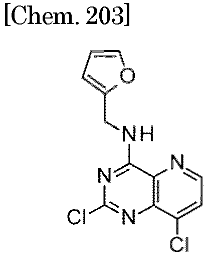

N,N-Diisopropylethylamine (384 µL, 2.21 mmol) was added to a solution of 8-methoxy-1H-pyrido[3,2-d]pyrimidine-2,4-dione (182 mg, 0.512 mmol) disclosed in WO 2020/081636 in phosphoryl chloride (2.72 mL, 29.7 mmol), followed by stirring at 110°C for 3 hours. N,N-Diisopropylethylamine (178 µL, 1.02 mmol) was added to the reaction solution, and the mixture was stirred at 110°C for 1 hour. The reaction solution was allowed to cool to room temperature and then concentrated under reduced pressure. The resulting residue was purified by silica gel column chromatography (ethyl acetate/hexane = 0/100 to 40/60) to obtain crude 2,4,8-trichloropyrido[3,2-d]pyrimidine (34.8 mg) [MS (m/z): 234 (M)⁺] as a white solid. Furfurylamine (14 µL, 0.151 mmol) and triethylamine (39.0 µL, 0.281 mmol) were added to a mixture of the obtained 2,4,8-trichloropyrido[3,2-d]pyrimidine and acetonitrile (2 mL), followed by stirring at 60°C for 1 hour. The reaction solution was allowed to cool to room temperature and then concentrated under reduced pressure. The resulting residue was purified by silica gel column chromatography (ethyl acetate/hexane = 0/100 to 30/70) to obtain the title compound (39.5 mg, 90% yield) as a white solid.

### (Example 179-2) 2-Chloro-8-(1,4-dioxan-2-ylmethoxy)-N-(furan-2-ylmethyl)pyrido[3,2-d]pyrimidin-4-amine

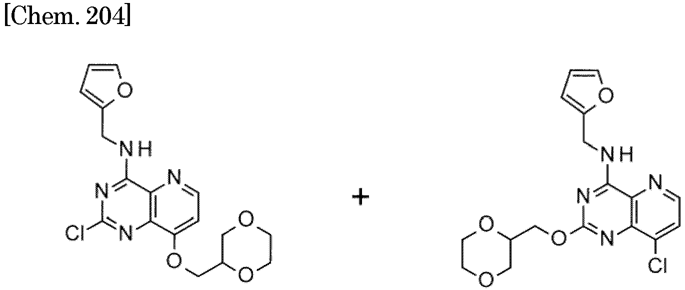

(1,4-Dioxan-2-yl)methanol (14.0 µL, 0.127 mmol) and 55% sodium hydride (dispersed in liquid paraffin) (5.3 mg, 0.120 mmol) were added to a solution of 2,8-dichloro-N-(furan-2-ylmethyl)pyrido[3,2-d]pyrimidin-4-amine (35.5 mg, 0.120 mmol) obtained in Example 179-1 in THF (1 mL), followed by stirring at 65°C for 4 hours. (1,4-Dioxan-2-yl)methanol (14.0 µL, 0.127 mmol) and 55% sodium hydride (dispersed in liquid paraffin) (4.40 mg, 0.100 mmol) were further added to the reaction solution, and the mixture was stirred at 65°C for 4 hours. The reaction solution was allowed to cool to room temperature and then concentrated under reduced pressure. The resulting residue was purified by silica gel column chromatography (ethyl acetate/hexane = 10/90 to 50/50) to obtain the title compound (3.90 mg, 9% yield) as a white solid. In addition, an isomer, 8-chloro-2-(1,4-dioxan-2-ylmethoxy)-N-(furan-2-ylmethyl)pyrido[3,2-d]pyrimidin-4-amine (5.40 mg, 12% yield), of the title compound was obtained as a white solid.

### (Example 180) 2-Chloro-8-(1,4-dioxan-2-ylmethoxy)-N-(furan-2-ylmethyl)pyrido[3,4-d]pyrimidin-4-amine

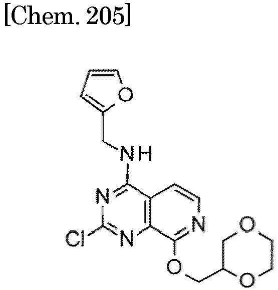

### (Example 180-1) 2,4,8-Trichloropyrido[3,4-d]pyrimidine

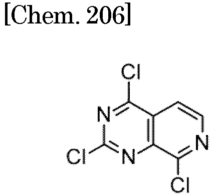

N,N-Diethylaniline (259 µL, 1.61 mmol) was added to a mixture of 8-methoxypyrido[3,4-d]pyrimidine-2,4(1H,3H)-dione (156 mg, 0.806 mmol) disclosed in Bioorg. Med. Chem. Lett., 2013, 23, 5923-5930 and phosphoryl chloride (0.92 g, 6.00 mmol), followed by stirring at 100°C for 7 hours. The reaction solution was allowed to cool to room temperature. Then, the solvent was distilled off under reduced pressure, and the resulting residue was azeotroped with toluene. The residue was dissolved in chloroform. The resulting solution was neutralized with a saturated aqueous solution of sodium bicarbonate at 0°C and then extracted with chloroform. After concentration under reduced pressure, the resulting residue was purified by silica gel column chromatography (ethyl acetate/hexane = 0/100 to 20/80) to obtain the title compound (101 mg, 53% yield) as a dark brown solid.

### (Example 180-2) 2,8-Dichloro-N-(furan-2-ylmethyl)pyrido[3,4-d]pyrimidin-4-amine

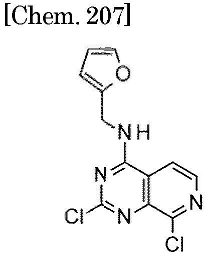

Furfurylamine (21.2 µL, 0.238 mmol) and triethylamine (58.0 µL, 0.418 mmol) were added to a solution of 2,4,8-trichloropyrido[3,4-d]pyrimidine (45.1 mg, 0.192 mmol) obtained in Example 180-1 in acetonitrile (1.5 mL), followed by stirring at 60°C for 2 hours. The reaction solution was allowed to cool to room temperature and then concentrated under reduced pressure. The resulting residue was purified by silica gel column chromatography (ethyl acetate/hexane = 10/90 to 50/50) to obtain the title compound (55.9 mg, 98% yield) as a yellow solid.

### (Example 180-3) 2-Chloro-8-(1,4-dioxan-2-ylmethoxy)-N-(furan-2-ylmethyl)pyrido[3,4-d]pyrimidin-4-amine

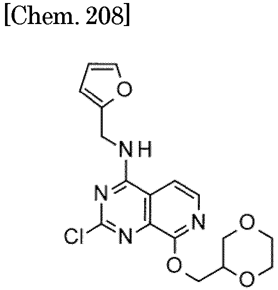

55% Sodium hydride (dispersed in liquid paraffin) (4.90 mg, 0.112 mmol) was added to a solution (1 mL) of (1,4-dioxan-2-yl)methanol (15.7 mg, 0.133 mmol) in THF, followed by stirring at room temperature for 1 hour. 2,8-Dichloro-N-(furan-2-ylmethyl)pyrido[3,4-d]pyrimidin-4-amine (29.6 mg, 0.100 mmol) obtained in Example 180-2 was added to the reaction solution, followed by stirring at 65°C for 18 hours. Water was added to the reaction solution, followed by extraction with chloroform. The organic layer was washed with a saturated saline solution and then concentrated under reduced pressure. The resulting residue was purified by silica gel column chromatography (ethyl acetate/hexane = 20/80 to 50/50) to obtain the title compound (6.00 mg, 16% yield) as a pale yellow solid.

### (Example 181) 5-[({2-Chloro-4-[(furan-2-ylmethyl)amino]pyrido[3,4-d]pyrimidin-8-yl}oxy)methyl]-3-methyl-1,3-oxazolidin-2-one

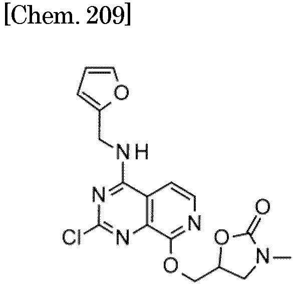

The same operation as in Example 180-3 was performed using 2,8-dichloro-N-(furan-2-ylmethyl)pyrido[3,4-d]pyrimidin-4-amine (108 mg, 0.366 mmol) obtained in Example 180-2 and 5-(hydroxymethyl)-3-methyl-1,3-oxazolidin-2-one (144 mg, 1.10 mmol). Thus, the title compound (20.1 mg, 14% yield) was obtained as a white solid.

### (Example 182) 5-({[4-(Benzylamino)-2-chloropyrido[3,4-d]pyrimidin-8-yl]oxy}methyl)-3-methyl-1,3-oxazolidin-2-one

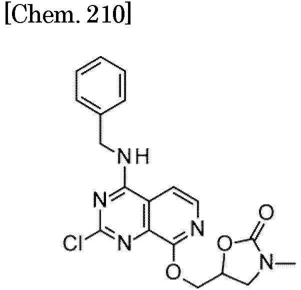

### (Example 182-1) N-Benzyl-2,8-dichloropyrido[3,4-d]pyrimidin-4-amine

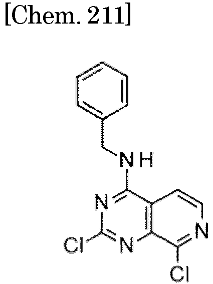

The same operation as in Example 180-2 was performed using 2,4,8-trichloropyridol3,4-dlpyrimidine (47.1 mg, 0.201 mmol) obtained in Example 180-1 and benzylamine (24.0 µL, 0.221 mmol). Thus, the title compound (60.9 mg, 99% yield) was obtained as a white solid.

### (Example 182-2) 5-({[4-(Benzylamino)-2-chloropyrido[3,4-d]pyrimidin-8-yl]oxy}methyl)-3-methyl-1,3-oxazolidin-2-one

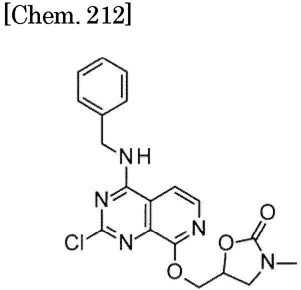

The same operation as in Example 180-3 was performed using N-benzyl-2,8-dichloropyrido[3,4-d]pyrimidin-4-amine (56.8 mg, 0.186 mmol) obtained in Example 182-1 and 5-(hydroxymethyl)-3-methyl-1,3-oxazolidin-2-one (29.3 mg, 0.223 mmol). Thus, the title compound (11.2 mg, 15% yield) was obtained as a white solid.

Structural formulae and physical scientific data of the compounds described in Examples 170 to 182 are shown below.

**[Table 12]**

| Example | Structural formulae | Data |
|---|---|---|
| 170-1 | 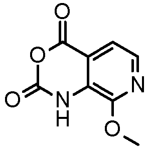 | 1H-NMR (DMSO-D6) δ: 3.99 (3H, s), 7.36 (1H, d, J = 5.5 Hz), 7.96 (1H, d, J = 5.5 Hz), 11.67 (1H, s). |
| 170-2 | 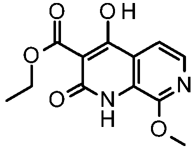 | 1H-NMR (DMSO-D6) δ: 1.29 (3H, t, J = 7.3 Hz), 3.98 (3H, s), 4.30 (2H, q, J = 7.1 Hz), 7.40 (1H, d, J = 5.5 Hz), 7.88 (1H, d, J = 5.5 Hz), 11.22 (1H, br s), 12.67 (1H, br s). |
| 170-3 | 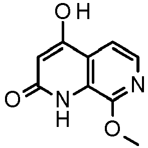 | 1H-NMR (DMSO-D6) δ: 3.96 (3H, s), 5.90 (1H, s), 7.26 (1H, d, J = 5.5 Hz), 7.83 (1H, d, J = 5.5 Hz), 10.81 (1H, br s), 11.64 (1H, br s). |
| 170-4 | 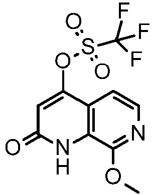 | 1H-NMR (DMSO-D6) δ: 4.02 (3H, s), 7.06 (1H, s), 7.15 (1H, d, J = 5.5 Hz), 8.01 (1H, d, J = 5.5 Hz), 12.14 (1H, br s). |
| 170-5 | 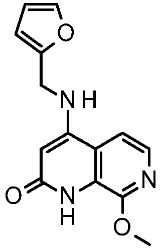 | 1H-NMR (DMSO-D6) δ: 3.95 (3H, s), 4.42 (2H, d, J = 5.5 Hz), 5.54 (1H, s), 6.38 (1H, d, J = 2.4 Hz), 6.42 (1H, dd, J = 3.0, 1.8 Hz), 7.51 (1H, d, J = 6.1 Hz), 7.58-7.62 (2H, m), 7.81 (1H, d, J = 6.1 Hz), 10.22 (1H, br s). |
| 170-6 | 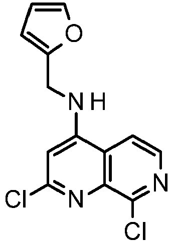 | 1H-NMR (DMSO-D6) δ: 4.61 (2H, d, J = 5.5 Hz), 6.43 (1H, dd, J = 3.3, 2.4 Hz), 6.47 (1H, d, J = 2.4 Hz), 6.87 (1H, s), 7.63-7.64 (1H, m), 8.20 (1H, d, J = 6.1 Hz), 8.31 (1H, d, J = 6.1 Hz), 8.52 (1H, t, J = 5.5 Hz). |
| 170-7 | 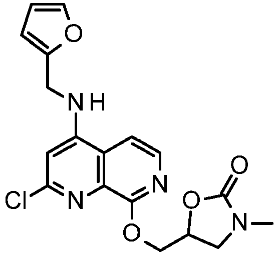 | 1H-NMR (DMSO-D6) δ: 2.79 (3H, s), 3.38-3.44 (1H, m), 3.72 (1H, t, J = 9.1 Hz), 4.50-4.62 (4H, m), 4.93-5.00 (1H, m), 6.41-6.44 (2H, m), 6.77 (1H, s), 7.63 (1H, br s), 7.72 (1H, d, J = 6.1 Hz), 8.01 (1H, d, J = 5.5 Hz), 8.20 (1H, t, J = 6.1 Hz). |
| | | MS (m/z): 389 (M+H)⁺. |
| 171 | 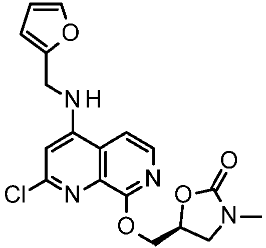 | 1H-NMR (DMSO-D6) δ: 2.79 (3H, s), 3.37-3.44 (1H, m), 3.72 (1H, t, J = 8.8 Hz), 4.51-4.62 (4H, m), 4.93-5.00 (1H, m), 6.42-6.44 (2H, m), 6.77 (1H, s), 7.62 (1H, d, J = 1.8 Hz), 7.72 (1H, d, J = 6.1 Hz), 8.01 (1H, d, J = 6.1 Hz), 8.20 (1H, t, J = 6.1 Hz). |
| | | MS (m/z): 389 (M+H)⁺. |
| 172 | 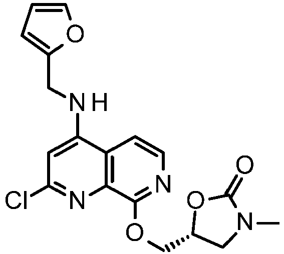 | 1H-NMR (DMSO-D6) δ: 2.79 (3H, s), 3.38-3.44 (1H, m), 3.72 (1H, t, J = 8.8 Hz), 4.50-4.62 (4H, m), 4.93-5.00 (1H, m), 6.41-6.44 (2H, m), 6.77 (1H, s), 7.62 (1H, d, J = 1.8 Hz), 7.72 (1H, d, J = 6.1 Hz), 8.01 (1H, d, J = 6.1 Hz), 8.20 (1H, t, J = 5.8 Hz). |
| | | MS (m/z): 389 (M+H)⁺. |
| 173-1 | 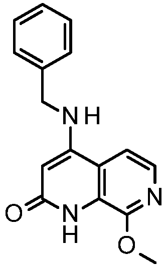 | 1H-NMR (DMSO-D6) δ: 3.95 (3H, s), 4.45 (2H, d, J = 6.1 Hz), 5.28 (1H, s), 7.23-7.28 (1H, m), 7.32-7.37 (4H, m), 7.57 (1H, d, J = 6.1 Hz), 7.79 (1H, t, J = 6.1 Hz), 7.85 (1H, d, J = 6.1 Hz), 10.17 (1H, br s). |
| 173-2 | 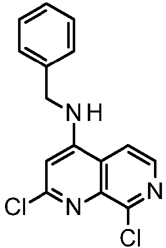 | 1H-NMR (DMSO-D6) δ: 4.63 (2H, br s), 6.62 (1H, s), 7.26-7.30 (1H, m), 7.34-7.41 (4H, m), 8.26 (1H, d, J = 5.5 Hz), 8.34 (1H, d, J = 5.5 Hz), 8.69 (1H, br s). |
| 173-3 | 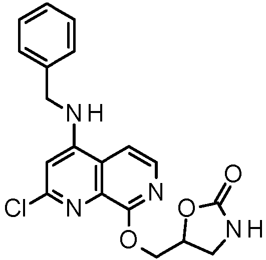 | 1H-NMR (DMSO-D6) δ: 3.36-3.39 (1H, m), 3.63 (1H, t, J =8.8 Hz), 4.52-4.62 (4H, m), 5.00-5.06 (1H, m), 6.52 (1H, s), 7.24-7.29 (1H, m), 7.33-7.40 (4H, m), 7.64 (1H, s), 7.79 (1H, d, J = 5.5 Hz), 8.03 (1H, d, J = 5.5 Hz), 8.37 (1H, t, J = 6.1 Hz). |
| | | MS (m/z): 385 (M+H)⁺. |
| 174 | 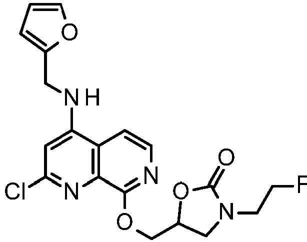 | 1H-NMR (DMSO-D6) δ: 3.47-3.56 (3H, m), 3.82 (1H, t, J= 8.8 Hz), 4.52-4.74 (6H, m), 4.98-5.05 (1H, m), 6.41-6.44 (2H, m), 6.77 (1H, s), 7.63 (1H, br d, J = 1.8 Hz), 7.73 (1H, d, J = 6.1 Hz), 8.01 (1H, d, J = 5.5 Hz), 8.20 (1H, t, J = 5.8 Hz). |
| | | MS (m/z): 421 (M+H)⁺. |
| 175-1 | 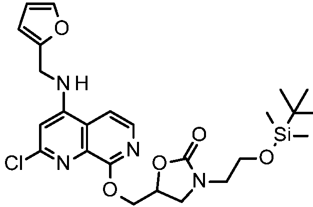 | 1H-NMR (DMSO-D6) δ: 0.01 (3H, s), 0.02 (3H, s), 0.82 (9H, s), 3.23-3.32 (2H, m), 3.51 (1H, dd, J = 8.8, 5.8 Hz), 3.69-3.75 (2H, m), 3.79 (1H, t, J = 9.1 Hz), 4.55-4.57 (4H, m), 4.95-5.01 (1H, m), 6.41-6.43 (2H, m), 6.77 (1H, s), 7.62 (1H, d, J = 1.2 Hz), 7.72 (1H, d, J = 6.1 Hz), 8.00 (1H, d, J = 6.1 Hz), 8.19 (1H, t, J = 6.1 Hz). |
| 175-2 | 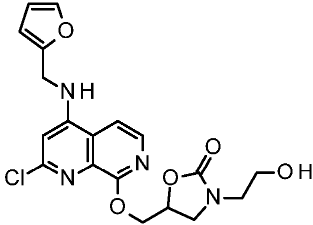 | 1H-NMR (DMSO-D6) δ: 3.23 (2H, t, J = 5.2 Hz), 3.49-3.57 (3H, m), 3.80 (1H, t, J = 8.8 Hz), 4.52-4.60 (4H, m), 4.82 (1H, t, J= 5.5 Hz), 4.94-5.01 (1H, m), 6.42-6.44 (2H, m), 6.77 (1H, s), 7.62-7.63 (1H, m), 7.72 (1H, d, J =6.1 Hz), 8.01 (1H, d, J = 5.5 Hz), 8.20 (1H, t, J = 5.8 Hz). |
| | | MS (m/z): 419 (M+H)⁺. |
| 176 | 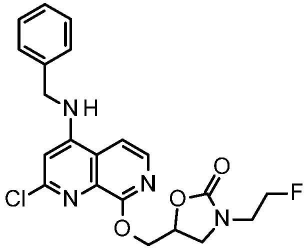 | 1H-NMR (DMSO-D6) δ: 3.47 (1H, t, J = 4.6 Hz), 3.52-3.57 (2H, m), 3.82 (1H, t, J = 8.8 Hz), 4.51-4.73 (6H, m), 4.99-5.04 (1H, m), 6.52 (1H, s), 7.25-7.29 (1H, m), 7.33-7.39 (4H, m), 7.79 (1H, d, J = 6.1 Hz), 8.03 (1H, d, J = 6.1 Hz), 8.36 (1H, t, J = 6.4 Hz). |
| | | MS (m/z): 431 (M+H)⁺. |
| 177 | 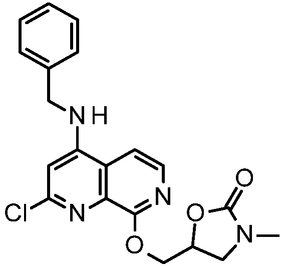 | 1H-NMR (DMSO-D6) δ: 2.78 (3H, s), 3.42 (1H, dd, J = 9.1, 6.7 Hz), 3.72 (1H, t, J = 8.8 Hz), 4.51-4.62 (4H, m), 4.94-4.99 (1H, m), 6.52 (1H, s), 7.25-7.28 (1H, m), 7.34-7.40 (4H, m), 7.79 (1H, d, J = 5.5 Hz), 8.03 (1H, d, J = 5.5 Hz), 8.37 (1H, t, J = 6.1 Hz). |
| | | MS (m/z): 399 (M+H)⁺. |
| 178-1 | 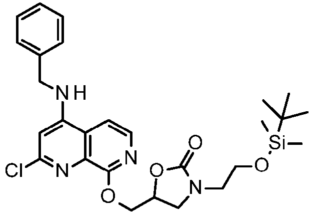 | 1H-NMR (DMSO-D6) δ: 0.00 (6H, s), 0.82 (9H, s), 3.21-3.30 (2H, m), 3.51 (1H, dd, J = 9.1, 6.1 Hz), 3.67-3.75 (2H, m), 3.79 (1H, t, J = 9.1 Hz), 4.55-4.60 (4H, m), 4.95-5.01 (1H, m), 6.52 (1H, s), 7.24-7.28 (1H, m), 7.32-7.40 (4H, m), 7.78 (1H, d, J = 6.1 Hz), 8.02 (1H, d, J = 5.5 Hz), 8.35 (1H, t, J = 6.1 Hz). |
| 178-2 | 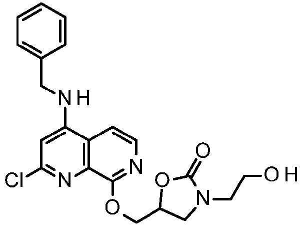 | 1H-NMR (DMSO-D6) δ: 3.23 (2H, t, J = 5.5 Hz), 3.49-3.59 (3H, m), 3.80 (1H, t, J = 8.8 Hz), 4.52-4.61 (4H, m), 4.81 (1H, t, J = 5.5 Hz), 4.94-5.00 (1H, m), 6.52 (1H, s), 7.24-7.29 (1H, m), 7.34-7.40 (4H, m), 7.79 (1H, d, J =6.1 Hz), 8.04 (1H, d, J = 6.1 Hz), 8.37 (1H, t, J = 6.1 Hz). MS (m/z): 429 (M+H)⁺. |
| 179-1 | 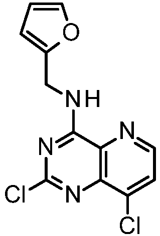 | ¹H-NMR (CDCl₃) δ: 4.86 (2H, d, *J*= 6.1 Hz), 6.36-6.41 (2H, m), 7.42 (1H, d, *J*= 1.2 Hz), 7.61 (1H, br s), 7.74 (1H, d, *J*= 4.9 Hz), 8.53 (1H, d, *J*= 4.9 Hz). |
| 179-2 | 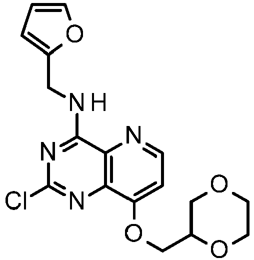 | 1H-NMR (CDCl3) δ: 3.47-3.60 (1H, m), 3.62-3.71 (1H, m), 3.76 (1H, d, J = 12.1 Hz), 3.79-3.88 (2H, m), 4.00 (1H, d, J = 11.5 Hz), 4.18 (2H, dd, J = 12.5, 4.6 Hz), 4.27 (1H, dd, J = 12.1, 7.3 Hz), 4.83 (2H, d, J = 5.5 Hz), 6.34-6.39 (2H, m), 7.08 (1H, d, J = 4.9 Hz), 7.41 (1H, s), 7.52 (1H, br s), 8.49 (1H, d, J = 4.9 Hz). |
| | | MS (m/z): 377 (M+H)⁺. |
| 179-2 | 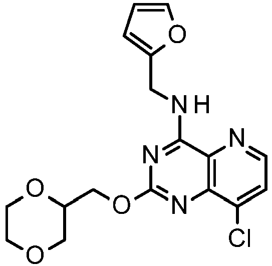 | ¹H-NMR (CDCl₃) δ: 3.56 (1H, t, *J*= 10.9 Hz), 3.63-3.90 (4H, m), 3.99 (1H, dd, *J*= 11.5, 2.4 Hz), 4.08-4.17 (1H, m), 4.46 (1H, dd, *J*= 11.5, 4.9 Hz), 4.54 (1H, dd, *J*= 11.5, 6.1 Hz), 4.84 (2H, d, *J*= 5.5 Hz), 6.35 (2H, br s), 7.40 (1H, s), 7.46 (1H, br s), 7.64 (1H, d, *J*= 4.3 Hz), 8.36 (1H, d, *J*= 4.3 Hz). |
| | | MS (m/z): 377 (M+H)⁺. |
| 180-1 | 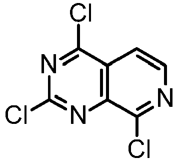 | 1H-NMR (DMSO-D6) δ: 7.95 (1H, d, J = 5.5 Hz), 8.46 (1H, d, J = 5.5 Hz). |
| | | MS (m/z): 234 (M+H)⁺. |
| 180-2 | 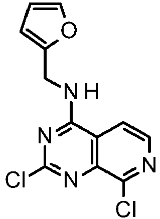 | 1H-NMR (DMSO-D6) δ: 4.75 (2H, s), 6.40-6.43 (1H, m), 6.43-6.45 (1H, m), 7.63 (1H, s), 8.20 (1H, d, J = 5.5 Hz), 8.41 (1H, d, J = 5.5 Hz), 9.74 (1H, br s). |
| 180-3 | 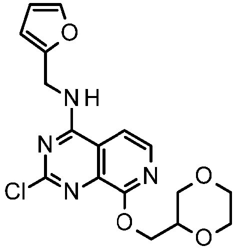 | 1H-NMR (DMSO-D6) δ: 3.36-3.44 (1H, m), 3.46-3.55 (1H, m), 3.60-3.72 (2H, m), 3.75-3.86 (2H, m), 3.94-4.01 (1H, m), 4.32-4.43 (2H, m), 4.73 (2H, d, J = 5.5 Hz), 6.39 (1H, d, J = 3.1 Hz), 6.42-6.44 (1H, m), 7.63 (1H, d, J= 1.8 Hz), 7.72 (1H, d, J = 6.1 Hz), 8.10 (1H, d, J = 6.1 Hz), 9.37 (1H, t, J = 5.5 Hz). |
| | | MS (m/z): 377 (M+H)⁺. |
| 181 | 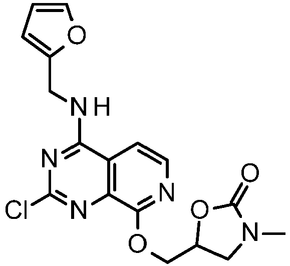 | 1H-NMR (DMSO-D6) δ: 2.78 (3H, s), 3.42 (1H, dd, J = 8.8, 7.0 Hz), 3.72 (1H, t, J = 8.8 Hz), 4.53 (1H, dd, J = 12.1, 7.0 Hz), 4.60 (1H, dd, J = 12.1, 3.6 Hz), 4.73 (2H, d, J = 5.5 Hz), 4.92-4.99 (1H, m), 6.39 (1H, d, J = 2.4 Hz), 6.43 (1H, dd, J = 3.0, 1.8 Hz), 7.62-7.63 (1H, m), 7.62 (1H, t, J = 1.5 Hz), 7.75 (1H, d, J= 6.1 Hz), 8.12 (1H, d, J = 6.1 Hz), 9.39 (1H, t, J = 5.5 Hz). |
| | | MS (m/z): 390 (M+H)⁺. |
| 182-1 | 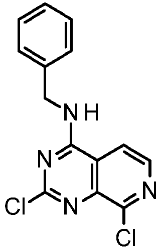 | 1H-NMR (DMSO-D6) δ: 4.78 (2H, s), 7.26-7.32 (1H, m), 7.32-7.42 (4H, m), 8.22 (1H, d, J = 5.5 Hz), 8.44 (1H, d, J = 5.5 Hz), 9.79 (1H, br s). |
| 182-2 | 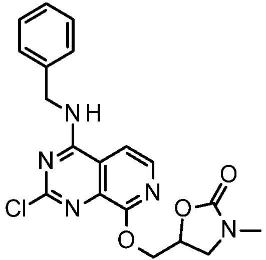 | 1H-NMR (DMSO-D6) δ: 2.78 (3H, s), 3.37-3.46 (1H, m), 3.72 (1H, t, J = 8.9 Hz), 4.50-4.64 (2H, m), 4.75 (2H, d, J= 5.5 Hz), 4.91-5.00 (1H, m), 7.24-7.41 (5H, m), 7.77 (1H, d, J = 5.5 Hz), 8.13 (1H, d, J= 5.5 Hz), 9.46 (1H, t, J = 6.1 Hz). |
| | | MS (m/z): 400 (M+H)⁺. |

### (Example 183) 5-[({2-Chloro-4-[(furan-2-ylmethyl)amino]pyrido[4,3-d]pyrimidin-8-yl}oxy)methyl]-3-methyl-1,3-oxazolidin-2-one

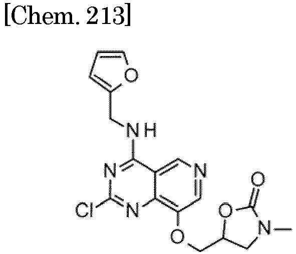

### (Example 183-1) Methyl 4-[(3-methyl-2-oxo-1,3-oxazolidin-5-yl)methoxy]-3-oxobutanoate

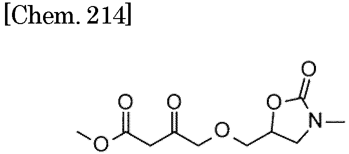

55% Sodium hydride (dispersed in liquid paraffin) (421 mg, 9.65 mmol) was added to a solution of 5-(hydroxymethyl)-3-methyl-1,3-oxazolidin-2-one (671 mg, 5.12 mmol) in THF (17 mL) under ice-cooling, and the mixture was stirred at room temperature for 30 minutes. A solution of methyl 4-chloroacetoacetate (500 µL, 4.33 mmol) in THF (17 mL) was added to the reaction mixture, followed by stirring at room temperature for 7 hours. A saturated aqueous solution of ammonium chloride was added to the reaction solution, followed by extraction with ethyl acetate. The organic layer was washed with a saturated saline solution, dried over anhydrous magnesium sulfate, and then concentrated under reduced pressure. The resulting residue was then purified by silica gel column chromatography (100% ethyl acetate) to obtain the title compound (407 mg, 38% yield) as a yellow oily substance.

### (Example 183-2) Methyl 4-amino-5-[(3-methyl-2-oxo-1,3-oxazolidin-5-yl)methoxy]pyridine-3-carboxylate

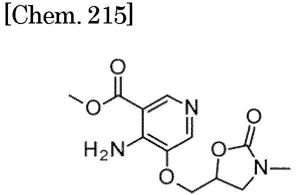

Methyl 4-[(3-methyl-2-oxo-1,3-oxazolidin-5-yl)methoxy]-3-oxobutanoate (44.8 mg, 0.183 mmol) obtained in Example 183-1 and ammonium acetate (30.0 mg, 0.183 mmol) were added to a solution of 3-methyl-5-nitro-pyrimidin-4-one (21.3 mg, 0.137 mmol) disclosed in WO 2018/125961 in methanol (1 mL), followed by stirring at 70°C for 8.5 hours. The reaction solution was allowed to cool to room temperature and then concentrated under reduced pressure. The resulting residue was purified by preparative TLC (100% ethyl acetate) to obtain the title compound (30.3 mg, 78% yield) as a yellow oily substance.

### (Example 183-3) 8-[(3-Methyl-2-oxo-1,3-oxazolidin-5-yl)methoxy]pyrido[4,3-d]pyrimidine-2,4(1H,3H)-dione

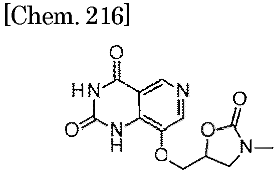

Trichloroacetyl isocyanate (20.0 µL, 0.169 mmol) was added to a solution of methyl 4-amino-5-[(3-methyl-2-oxo-1,3-oxazolidin-5-ylmethoxylpyridine-3-carboxylate (30.3 mg, 0.108 mmol) obtained in Example 183-2 in THF (1 mL), followed by stirring at room temperature for 1 hour. Water was added to the reaction solution, followed by extraction with ethyl acetate. The organic layer was washed with a saturated saline solution, dried over anhydrous magnesium sulfate, and then concentrated under reduced pressure. 7M aqueous ammonia (2 mL) was added to the resulting residue, followed by stirring at room temperature for 1.5 hours. After concentration under reduced pressure, ethyl acetate (2 mL) was added, followed by stirring. The precipitated solid was then collected by filtration to obtain the title compound (20.6 mg, 65% yield) as a white solid.

### (Example 183-4) 5-[({2-Chloro-4-[(furan-2-ylmethyl)amino]pyrido[4,3-d]pyrimidin-8-yl}oxy)methyl]-3-methyl-1,3-oxazolidin-2-one

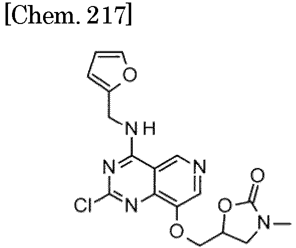

N,N-diisopropylethylamine (330 µL, 1.90 mmol) was added to a mixture of 8-[(3-methyl-2-oxo-1,3-oxazolidin-5-yl)methoxy]pyrido[4,3-d]pyrimidine-2,4(1H,3H)-dione (55.4 mg, 0.190 mmol) obtained in Example 183-3 with phosphoryl chloride (1.64 mL, 17.9 mmol), followed by stirring at 115°C for 1 hour. The solvent was distilled off under reduced pressure, and NMP (1.5 mL) was added to the resulting residue. Then, furfurylamine (70.0 µL, 0.757 mmol) and N,N-diisopropylethylamine (66.0 µL, 0.379 mmol) were added under ice-cooling, and the mixture was stirred for 10 minutes under ice-cooling. Ethyl acetate was added to the reaction solution, and the mixture was washed with a saturated saline solution. The organic layer was dried over anhydrous magnesium sulfate and then concentrated under reduced pressure. The resulting residue was purified by silica gel column chromatography (methanol/ethyl acetate = 0/100 to 90/10) to obtain the title compound (4.00 mg, 5% yield) as a pale yellow solid.

### (Example 184) 5-({[4-(Benzylamino)-2-chloropyrido[4,3-d]pyrimidin-8-yl]oxy}methyl)-3-methyl-1,3-oxazolidin-2-one

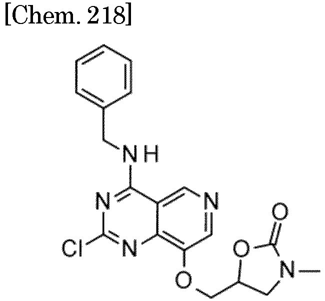

The same operation as in Example 183-4 was performed using 8-[(3-methyl-2-oxo-1,3-oxazolidin-5-ylmethoxy]pyrido[4,3-d]pyrimidine-2,4(1H,3H)-dione (178 mg, 0.609 mmol) obtained in Example 183-3 and benzylamine (270 µL, 2.50 mmol). Thus, the title compound (9.80 mg, 4% yield) was obtained as a white solid.

Structural formulae and physical scientific data of the compounds described in Examples 183 and 184 are shown below.

**[Table 13]**

| Example | Structural formulae | Data |
|---|---|---|
| 183-1 | 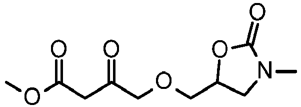 | ¹H-NMR (CDCl₃) δ: 2.90 (3H, s), 3.50 (2H, s), 3.56-3.73 (4H, m), 3.75 (3H, s), 4.27 (2H, s), 4.59-4.70 (1H, m). |
| 183-2 | 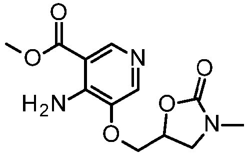 | ¹H-NMR (CD₃OD) δ: 2.89 (3H, s), 3.56 (1H, dd, *J*= 8.8, 6.4 Hz), 3.80 (1H, t, *J*= 9.1 Hz), 3.90 (3H, s), 4.22-4.36 (2H, m), 4.90-5.01 (1H, m), 7.94 (1H, s), 8.48 (1H, s). |
| 183-3 | 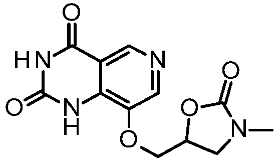 | ¹H-NMR (DMSO-D₆) δ: 2.78 (3H, s), 3.50 (1H, dd, *J*= 8.8, 6.4 Hz), 3.70 (1H, t, *J*= 8.8 Hz), 4.36 (2H, d, *J*= 4.3 Hz), 4.84-4.96 (1H, m), 8.44 (1H, s), 8.62 (1H, s), 11.22 (1H, s), 11.56 (1H, s). |
| 183-4 | 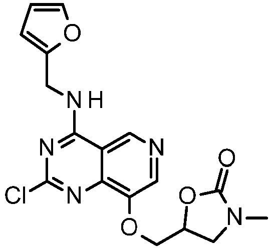 | 1H-NMR (DMSO-D6) δ: 2.80 (3H, s), 3.40-3.52 (1H, m), 3.73 (1H, t, J = 9.1 Hz), 4.36 (1H, dd, J = 11.2, 6.4 Hz), 4.45 (1H, dd, J = 11.2, 3.3 Hz), 4.76 (2H, d, J = 5.5 Hz), 4.89-4.99 (1H, m), 6.41 (1H, d, J = 4.3 Hz), 6.44 (1H, dd, J = 3.0, 1.8 Hz), 7.64 (1H, d, J = 1.8 Hz), 8.53 (1H, s), 9.24 (1H, s), 9.67 (1H, t, J = 5.8 Hz). |
| | | MS (m/z): 390 (M+H)⁺. |
| 184 | 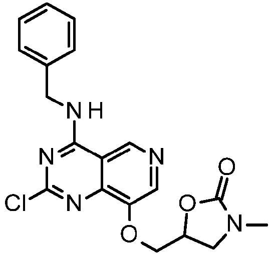 | 1H-NMR (DMSO-D6) δ: 2.80 (3H, s), 3.41-3.48 (1H, m), 3.73 (1H, t, J = 8.8 Hz), 4.36 (1H, dd, J = 11.2, 6.4 Hz), 4.45 (1H, dd, J = 11.5, 3.0 Hz), 4.78 (2H, s), 4.89-4.99 (1H, m), 7.25-7.32 (1H, m), 7.32-7.42 (4H, m), 8.53 (1H, s), 9.25 (1H, s), 9.74 (1H, s). |
| | | MS (m/z): 400 (M+H)⁺. |

### (Example 185) 5-({[4-(Benzylamino)-2-chloro-1,6-naphthyridin-8-yl]oxy}methyl)-1,3-oxazolidin-2-one

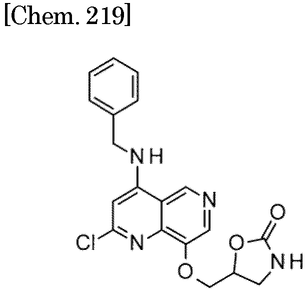

### (Example 185-1) Methyl 4-{[3-(4-methoxybenzyl)-2-oxo-1,3-oxazolidin-5-yl]methoxy}-3-oxobutanoate

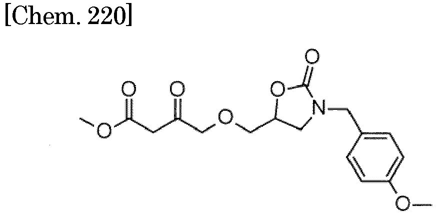

The same operation as in Example 183-1 was performed using 5-(hydroxymethyl)-3-[(4-methoxyphenyl)methyl]oxazolidin-2-one (434 mg, 1.83 mmol) disclosed in WO 2014/085413 and methyl 4-chloroacetoacetate (211 µL, 1.83 mmol). Thus, the title compound (203 mg, 32% yield) was obtained as a colorless oily substance.

### (Example 185-2) Methyl 4-amino-5-{[3-(4-methoxybenzyl)-2-oxo-1,3-oxazolidin-5-yl]methoxy}pyridine-3-carboxylate

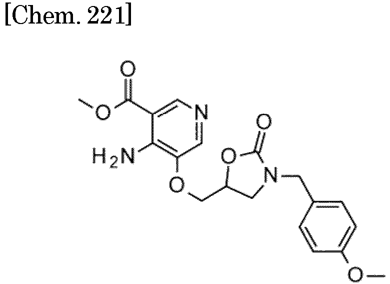

The same operation as in Example 183-2 was performed using methyl 4-{[3-(4-methoxybenzyl)-2-oxo-1,3-oxazolidin-5-yl]methoxy}-3-oxobutanoate (203 mg, 0.578 mmol) obtained in Example 185-1 and 3-methyl-5-nitro-pyrimidin-4-one (80.0 mg, 0.516 mmol). Thus, the title compound (115 mg, 58% yield) was obtained as a yellow oily substance.

### (Example 185-3) Methyl 4-(diacetylamino)-5-{[3-(4-methoxybenzyl)-2-oxo-1,3-oxazolidin-5-yl]methoxy}pyridine-3-carboxylate

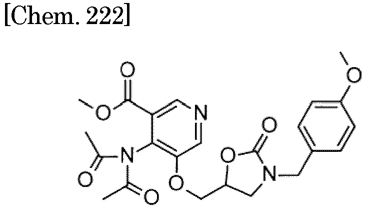

A mixture of methyl 4-amino-5-{[3-(4-methoxybenzyl)-2-oxo-1,3-oxazolidin-5-yl]methoxy}pyridine-3-carboxylate (115 mg, 0.297 mmol) obtained in Example 185-2, pyridine (24.0 µL, 0.297 mmol), and acetic anhydride (1 mL) was stirred at 100°C for 9 hours. The reaction solution was allowed to cool to room temperature and then concentrated under reduced pressure. The resulting residue was purified by silica gel column chromatography (ethyl acetate/methanol = 100/0 to 97/3) to obtain the title compound (125 mg, 89% yield) as a pale yellow oily substance.

### (Example 185-4) 4-Hydroxy-8-{[3-(4-methoxybenzyl)-2-oxo-1,3-oxazolidin-5-yl]methoxy}-1,6-naphthyridin-2(1H)-one

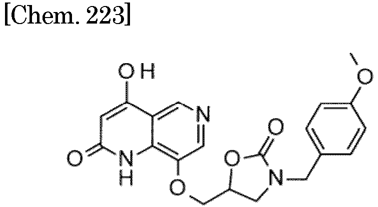

1M potassium bis(trimethylsilyl)amide-THF (0.530 mL, 0.530 mmol) was added dropwise to a solution of methyl 4-(diacetylamino)-5-{[3-(4-methoxybenzyl)-2-oxo-1,3-oxazolidin-5-yl]methoxy}pyridine-3-carboxylate (123 mg, 0.261 mmol) obtained in Example 185-3 in THF (2 mL) at -78°C, and the mixture was stirred for 2.5 hours while gradually raising the temperature to room temperature. Methanol was added to the reaction solution, followed by concentration under reduced pressure. The resulting residue was purified by silica gel column chromatography (ethyl acetate/methanol = 100/0 to 75/25) to obtain the title compound (37.4 mg, 36% yield) as a pale brown solid.

### (Example 185-5) 8-{[3-(4-Methoxybenzyl)-2-oxo-1,3-oxazolidin-5-yl]methoxy}-2-oxo-1,2-dihydro-1,6-naphthyridin-4-yl trifluoromethanesulfonate

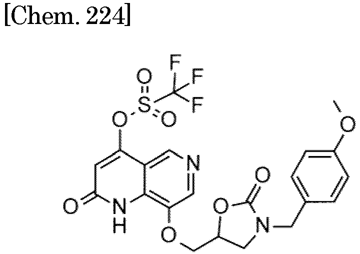

N-Phenylbis(trifluoromethanesulfonimide) (40.2 mg, 0.113 mmol) and a small amount of DMF were added to a solution of 4-hydroxy-8-{[3-(4-methoxybenzyl)-2-oxo-1,3-oxazolidin-5-yl]methoxy}-1,6-naphthyridin-2(1H)-one (35.8 mg, 0.0901 mmol) obtained in Example 185-4 and triethylamine (37.5 µL, 0.270 mmol) in THF (1 mL), followed by stirring at room temperature overnight. The reaction solution was concentrated under reduced pressure. The resulting residue was purified by silica gel column chromatography (ethyl acetate/hexane = 0/100 to 100/0) to obtain the title compound in a crude form (54.5 mg, 114% yield) containing a trace amount of DMF as a pale yellow solid.

### (Example 185-6) 4-(Benzylamino)-8-{[3-(4-methoxybenzyl)-2-oxo-1,3-oxazolidin-5-yl]methoxy}-1,6-naphthyridin-2(1H)-one

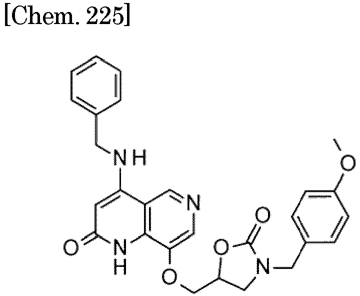

Benzylamine (87.6 µL, 0.801 mmol) was added to a solution (1 mL) of 8-{[3-(4-methoxybenzyl)-2-oxo-1,3-oxazolidin-5-yl]methoxy}-2-oxo-1,2-dihydro-1,6-naphthyridin-4-yl trifluoromethanesulfonate (42.2 mg, 0.0801 mmol) obtained in Example 185-5 in 1,4-dioxane, followed by stirring at 80°C for 2 hours. The reaction solution was allowed to cool to room temperature and then concentrated under reduced pressure. The resulting residue was purified by silica gel column chromatography (ethyl acetate/methanol = 100/0 to 90/10) to obtain the title compound in a crude form (14.2 mg, 36% yield) as a pale yellow solid.

### (Example 185-7) 5-({[4-(Benzylamino)-2-chloro-1,6-naphthyridm-8-yl]oxy}methyl)-1,3-oxazolidin-2-one

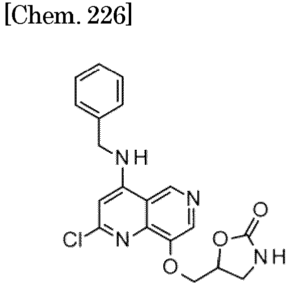

Phosphoryl chloride (24.5 mg, 0.159 mmol) and N,N-diethylaniline (8.0 µL, 0.0448 mmol) were added to a solution (1 mL) of 4-(benzylamino)-8-{[3-(4-methoxybenzyl)-2-oxo-1,3-oxazolidin-5-yl]methoxy}- 1,6-naphthyridin-2(1H)-one (10.9 mg, 0.0224 mmol) obtained in Example 185-6 in 1,4-dioxane, followed by stirring at 80°C for 1.5 hours. Phosphoryl chloride (43.0 mg, 0.280 mmol) was added, and the mixture was stirred at 80°C for an additional 2.5 hours. The reaction solution was allowed to cool to room temperature, and the solvent was then distilled off under reduced pressure. The resulting residue was purified by silica gel column chromatography (ethyl acetate/methanol = 100/0 to 85/15) to obtain crude 5-({[4-(benzylamino)-2-chloro-1,6-naphthyridin-8-yl]oxy}methyl)-3-(4-methoxybenzyl)-1,3-oxazolidin-2-one (15.2 mg) [MS (m/z): 505 (M+H)⁺]. The obtained 5-({[4-(benzylamino)-2-chloro-1,6-naphthyridin-8-yl]oxy}methyl)-3-(4-methoxybenzyl)-1,3-oxazolidin-2-one was stirred in trifluoroacetic acid (0.8 mL) at 80°C for 1.5 hours. After stirring at a reaction temperature of 100°C for an additional 7 hours, the reaction solution was allowed to cool to room temperature, and the solvent was distilled off under reduced pressure. The resulting residue was purified by preparative HPLC (column: Develosil Combi-RP-5, 100 × 28 mm × 5 µm; elution solvent: 0.1% aqueous formic acid/acetonitrile = 77/23 to 50/50) to obtain the title compound (1.64 mg, 14% yield) as a white solid.

### (Example 186) 5-({[4-(Benzylamino)-2-chloro-1,6-naphthyridin-8-yl]oxy}methyl)-3-methyl-1,3-oxazolidin-2-one

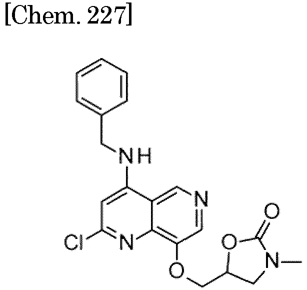

### (Example 186-1) Methyl 4-(diacetylamino)-5-[(3-methyl-2-oxo-1,3-oxazolidin-5-yl)methoxy]pyridine-3-carboxylate

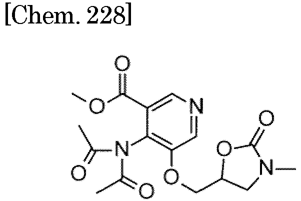

A mixture of methyl 4-amino-5-[(3-methyl-2-oxo-1,3-oxazolidin-5-yl)methoxy]pyridine-3-carboxylate (42.5 mg, 0.151 mmol) obtained in Example 183-2, pyridine (12.0 µL, 0.151 mmol), and acetic anhydride (1 mL) was stirred at 100°C for 8.5 hours. The reaction solution was allowed to cool to room temperature and then concentrated under reduced pressure. The resulting residue was purified by silica gel column chromatography (ethyl acetate/methanol = 100/0 to 91/9) to obtain the title compound (56.4 mg, 100% yield) as a pale yellow oily substance.

### (Example 186-2) 8-[(3-Methyl-2-oxo-1,3-oxazolidin-5-yl)methoxy]-2-oxo-1,2-dihydro-1,6-naphthyridin-4-yl trifluoromethanesulfonate

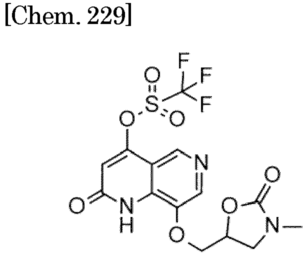

1M potassium bis(trimethylsilyl)amide-THF (0.270 mL, 0.270 mmol) was added dropwise to a solution of methyl 4-(diacetylamino)-5-[(3-methyl-2-oxo-1,3-oxazolidin-5-yl)methoxy]pyridine-3-carboxylate (49.3 mg, 0.135 mmol) obtained in Example 186-1 in THF (1 mL) at -78°C, and the mixture was stirred overnight while gradually raising the temperature to room temperature. 1M Hydrochloric acid (290 µL, 0.290 mmol) was added to the reaction solution, followed by concentration under reduced pressure. The resulting residue was purified by preparative HPLC (column: Develosil Combi-RP-5, 100 × 28 mm × 5 µm; elution solvent: 0.1% aqueous formic acid/acetonitrile = 100/0 to 79/21) to obtain crude 4-hydroxy-8-[(3-methyl-2-oxo-1,3-oxazolidin-5-yl)methoxy]-1,6-naphthyridin-2(1H)-one (9.10 mg) [MS (m/z): 293 (M+H)⁺] as a pale brown solid. N-Phenylbis(trifluoromethanesulfonimide) (13.3 mg, 0.0372 mmol) and a small amount of DMF were added to a solution of the obtained 4-hydroxy-8-[(3-methyl-2-oxo-1,3-oxazolidin-5-yl)methoxy]-1,6-naphthyridin-2(1H)-one and triethylamine (13.0 µL, 0.0938 mmol) in THF (3 mL), followed by stirring at room temperature overnight. The reaction solution was concentrated under reduced pressure. The resulting residue was purified by silica gel column chromatography (ethyl acetate/methanol = 100/0 to 90/10) to obtain the title compound in a crude form (14.4 mg, 110% yield) containing a trace amount of DMF as a white solid.

### (Example 186-3) 5-({[4-(Benzylamino)-2-chloro-1,6-naphthyridin-8-yl]oxy}methyl)-3-methyl-1,3-oxazolidin-2-one

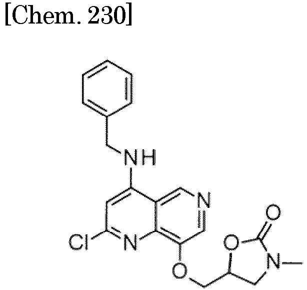

Benzylamine (16.0 µL, 0.143 mmol) and N,N-diisopropylethylamine (7.50 µL, 0.0429 mmol) were added to a solution of 8-[(3-methyl-2-oxo-1,3-oxazolidin-5-yl)methoxy]-2-oxo-1,2-dihydro-1,6-naphthyridin-4-yl trifluoromethanesulfonate (12.1 mg, 0.0286 mmol) obtained in Example 186-2 in NMP (0.5 mL), followed by stirring at 50°C for 40 minutes. After the reaction solution was allowed to cool to room temperature, water was added, followed by extraction with ethyl acetate. The organic layer was washed with a saturated saline solution and then concentrated under reduced pressure. The resulting residue was purified by silica gel column chromatography (ethyl acetate/methanol = 100/0 to 75/25) to obtain crude 4-(benzylamino)-8-[(3-methyl-2-oxo-1,3-oxazolidin-5-yl)methoxy]-1,6-naphthyridin-2(1H)-one (8.50 mg) [MS (m/z): 381 (M+H)⁺] as a white solid. Phosphoryl chloride (35.0 mg, 0.228 mmol) and N,N-diethylaniline (13.6 µL, 0.0783 mmol) were added to a solution (2 mL) of the obtained 4-(benzylamino)-8-[(3-methyl-2-oxo-1,3-oxazolidin-5-yl)methoxy]-1,6-naphthyridin-2(1H)-one (14.9 mg, 0.0392 mmol) in 1,4-dioxane, followed by stirring at 80°C for 1 hour. Phosphoryl chloride (52.0 mg, 0.339 mmol) was added, and the mixture was stirred at 80°C for an additional 3 hours. The reaction solution was allowed to cool to room temperature, and the solvent was distilled off under reduced pressure. The resulting residue was purified by preparative HPLC (column: Develosil Combi-RP-5, 100 × 28 mm × 5 µm; elution solvent: 0.1% aqueous formic acid/acetonitrile = 75/25 to 47/53) to obtain the title compound (3.30 mg, 21% yield) as a pale yellow solid.

Structural formulae and physical scientific data of the compounds described in Examples 185 and 186 are shown below.

**[Table 14]**

| Example | Structural formulae | Data |
|---|---|---|
| 185-1 | 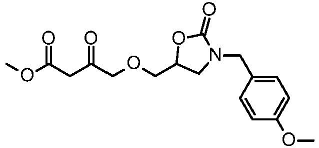 | ¹H-NMR (CDCl₃) δ: 3.22-3.37 (2H, m), 3.39-3.49 (3H, m), 3.50-3.90 (7H, m), 4.16-4.28 (2H, m), 4.28-4.45 (2H, m), 4.54-4.67 (1H, m), 6.81-6.91 (2H, m), 7.14-7.24 (2H, m). |
| 185-2 | 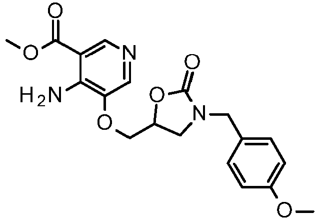 | ¹H-NMR (CDCl₃) δ: 3.37 (1H, t, *J*= 7.6 Hz), 3.60 (1H, t, *J*= 9.1 Hz), 3.82 (3H, s), 3.90 (3H, s), 4.11 (1H, dd, *J* = 10.6, 5.2 Hz), 4.21 (1H, d, *J*= 7.3 Hz), 4.35 (1H, d, *J*= 14.6 Hz), 4.46 (1H, d, *J*= 14.6 Hz), 4.77-4.93 (1H, m), 6.89 (2H, d, *J*= 8.5 Hz), 7.20-7.30 (2H, m), 7.90 (1H, s), 8.63 (1H, s). |
| 185-3 | 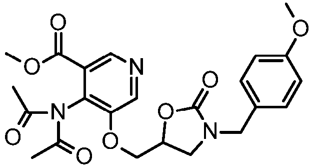 | 1H-NMR (CDCl₃) δ: 2.23 (3H, s), 2.29 (3H, s), 3.26 (1H, dd, J = 8.9, 6.4 Hz), 3.51 (1H, t, J = 9.2 Hz), 3.81 (3H, s), 3.91 (3H, s), 4.21 (1H, d, J = 14.6 Hz), 4.24-4.30 (2H, m), 4.55 (1H, d, J = 14.6 Hz), 4.73-4.80 (1H, m), 6.89 (2H, d, J = 8.5 Hz), 7.21 (2H, d, J = 8.5 Hz), 8.58 (1H, s), 8.97 (1H, s). |
| 185-4 | 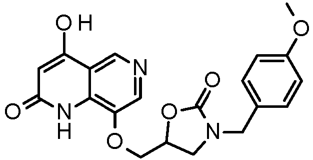 | 1H-NMR (CD3OD) δ: 3.46 (1H, dd, J = 9.2, 6.7 Hz), 3.67 (1H, t, J = 9.2 Hz), 3.76 (3H, s), 4.37 (2H, s), 4.38-4.48 (2H, m), 4.97-5.05 (1H, m), 5.90 (1H, s), 6.86 (2H, d, J = 8.5 Hz), 7.21 (2H, d, J = 8.5 Hz), 8.29 (1H, s), 8.70 (1H, s). |
| 185-5 | 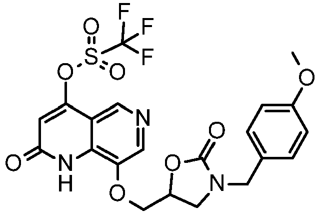 | 1H-NMR (CDCl₃) δ: 3.31 (1H, dd, J = 9.2, 6.1 Hz), 3.64 (1H, t, J = 9.2 Hz), 3.81 (3H, s), 4.34 (2H, d, J = 4.9 Hz), 4.39 (1H, d, J = 14.6 Hz), 4.45 (1H, d, J = 14.6 Hz), 4.88-4.96 (1H, m), 6.74 (1H, s), 6.90 (2H, d, J= 8.5 Hz), 7.24 (2H, d, J = 8.5 Hz), 8.31 (1H, s), 8.69 (1H, s). |
| 185-6 | 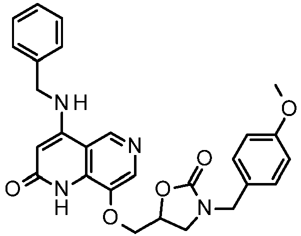 | 1H-NMR (CD3OD) δ: 3.45 (1H, dd, J = 9.2, 6.1 Hz), 3.57-3.69 (1H, m), 3.73 (3H, s), 4.32-4.51 (4H, m), 4.55 (2H, s), 4.96-5.04 (1H, m), 5.47 (1H, s), 6.85 (2H, d, J = 9.2 Hz), 7.18-7.41 (7H, m), 8.29 (1H, s), 8.85 (1H, s). |
| | | MS (m/z): 487 (M+H)⁺. |
| 185-7 | 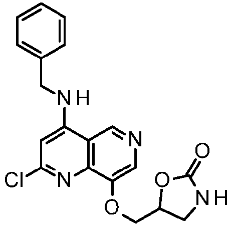 | 1H-NMR (CD3OD) δ: 3.68 (1H, dd, J = 9.2, 6.7 Hz), 3.81 (1H, t, J = 9.2 Hz), 4.46 (2H, d, J = 4.9 Hz), 4.62 (2H, s), 5.12-5.20 (1H, m), 6.52 (1H, s), 7.25-7.31 (1H, m), 7.33-7.42 (4H, m), 8.35 (1H, s), 9.17 (1H, s). |
| | | MS (m/z): 385 (M+H)⁺. |
| 186-1 | 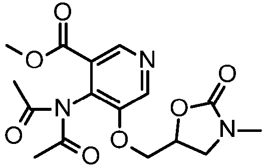 | 1H-NMR (CDCl₃) δ: 2.23 (3H, s), 2.33 (3H, s), 2.91 (3H, s), 3.45 (1H, dd, J = 8.9, 5.8 Hz), 3.69 (1H, t, J = 8.9 Hz), 3.91 (3H, s), 4.28 (1H, dd, J = 10.1, 4.0 Hz), 4.36 (1H, dd, J = 10.1, 4.0 Hz), 4.78-4.85 (1H, m), 8.61 (1H, s), 8.97 (1H, s). |
| 186-2 | 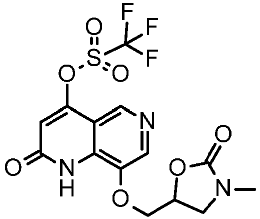 | 1H-NMR (CD3OD) δ: 2.89 (3H, s), 3.60 (1H, dd, J = 9.2, 6.7 Hz), 3.81 (1H, t, J = 9.2 Hz), 4.46-4.56 (2H, m), 5.00-5.08 (1H, m), 6.80 (1H, s), 8.46 (1H, s), 8.57 (1H, s). |
| 186-3 | 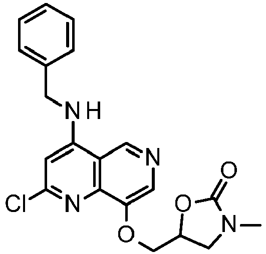 | 1H-NMR (CD3OD) δ: 2.90 (3H, s), 3.72 (1H, dd, J = 8.9, 6.4 Hz), 3.82 (1H, t, J = 8.9 Hz), 4.42 (1H, dd, J = 11.0, 4.9 Hz), 4.48 (1H, dd, J = 11.0, 4.3 Hz), 4.62 (2H, s), 4.98-5.07 (1H, m), 6.52 (1H, s), 7.25-7.32 (1H, m), 7.32-7.43 (4H, m), 8.34 (1H, s), 9.17 (1H, s). |
| | | MS (m/z): 399 (M+H)⁺. |

### (Example 187) 5-[({2-Chloro-4-[(furan-2-ylmethyl)amino] quinazolin-8-yl}oxy)methyl]-3-ethyl-1,3-oxazolidin-2-one

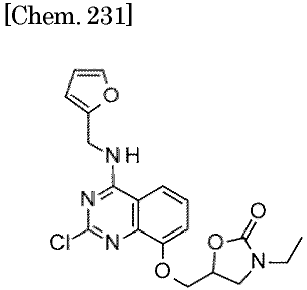

tert-Butoxypotassium (9.10 mg, 0.0811 mmol) and iodoethane (10.0 µL, 0.125 mmol) were added to a mixed solution of 5-[({2-chloro-4-[(furan-2-ylmethyl)amino]quinazolin-8-yl}oxy)methyl]-1,3-oxazolidin-2-one (13.4 mg, 0.0358 mmol) obtained in Example 19 in THF (0.3 mL)/DMF (0.1 mL) under ice-cooling, followed by stirring at the same temperature for 5.5 hours. Water (5 mL) was added to the reaction solution, followed by extraction with ethyl acetate and subsequent concentration under reduced pressure. The resulting residue was purified by silica gel column chromatography (ethyl acetate/hexane = 20/80 to 85/15) to obtain the title compound (8.30 mg, 58% yield) as a white solid.

### (Example 188) 5-[({2-Chloro-4-[(furan-2-ylmethyl)amino]quinazolin-8-yl}oxy)methyl]-3-(propan-2-yl)-1,3-oxazolidin-2-one

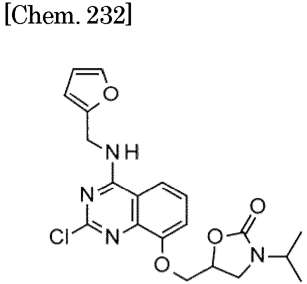

tert-Butoxypotassium (22.8 mg, 0.203 mmol) and 2-iodopropane (19.0 µL, 0.183 mmol) were added to a mixed solution of 5-[({2-chloro-4-[(furan-2-ylmethyl)amino]quinazolin-8-yl}oxy)methyl]-1,3-oxazolidin-2-one (34.3 mg, 0.0915 mmol) obtained in Example 19 in THF (0.6 mL)/DMF (0.2 mL) under ice-cooling, and the mixture was stirred at the same temperature for 3 hours, followed by stirring at room temperature for 4 hours. tert-Butoxypotassium (22.8 mg, 0.203 mmol) and 2-iodopropane (19.0 µL, 0.183 mmol) were added again to the reaction solution, followed by stirring at 60°C for 4 hours. The reaction solution was allowed to cool to room temperature. Then, water (5 mL) was added to the reaction solution, followed by extraction with ethyl acetate and subsequent concentration under reduced pressure. The resulting residue was purified by silica gel column chromatography (ethyl acetate/hexane = 0/100 to 70/30) to obtain the title compound (15.8 mg, 41% yield) as a white solid.

### (Example 189) 5-[({2-Chloro-4-[(furan-2-ylmethyl)amino]quinazolin-8-yl}oxy)methyl]-3-2-fluoroethyl)-1,3-oxazolidin-2-one

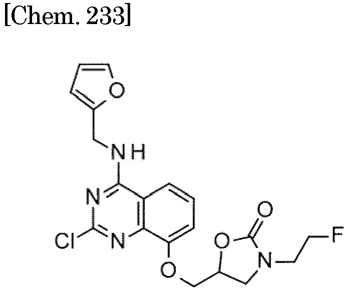

tert-Butoxypotassium (19.1 mg, 0.170 mmol) and 1-bromo-2-fluoromethane (18.0 µL, 0.157 mmol) were added to a solution (0.4 mL) of 5-[({2-chloro-4-[(furan-2-ylmethyl)amino]quinazolin-8-yl}oxy)methyl]-1,3-oxazolidin-2-one (29.4 mg, 0.0784 mmol) obtained in Example 19 and TBAI (14.7 mg, 0.0398 mmol) in DMF, followed by stirring at room temperature for 1 hour. Water (6 mL) was added to the reaction solution, followed by extraction with ethyl acetate and subsequent concentration under reduced pressure. The resulting residue was purified by silica gel column chromatography (ethyl acetate/hexane = 20/80 to 80/20) to obtain the title compound (26.9 mg, 82% yield) as a white solid.

### (Example 190) 5-[({2-Chloro-4-[(furan-2-ylmethyl)amino]quinazolin-8-yl}oxy)methyl]-3-(cyclopropylmethyl)-1,3-oxazolidin-2-one

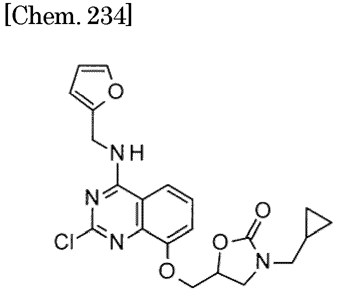

tert-Butoxypotassium (21.2 mg, 0.189 mmol) and (bromomethylcyclopropane (16.0 µL, 0.168 mmol) were added to a solution (0.4 mL) of 5-[({2-chloro-4-[(furan-2-ylmethyl)amino]quinazolin-8-yl}oxy)methyl]-1,3-oxazolidin-2-one (31.5 mg, 0.0840 mmol) obtained in Example 19 and TBAI (15.8 mg, 0.0428 mmol) in DMF, followed by stirring at room temperature for 1 hour. Water (6 mL) was added to the reaction solution, followed by extraction with ethyl acetate and subsequent concentration under reduced pressure. The resulting residue was purified by silica gel column chromatography (ethyl acetate/hexane = 20/80 to 70/30) to obtain the title compound (28.0 mg, 78% yield) as a white solid.

### (Example 191) 5-[({2-Chloro-4-[(furan-2-ylmethyl)amino]quinazolin-8-yl}oxy)methyl]-3-(2,2-difluoroethyl)-1,3-oxazolidin-2-one

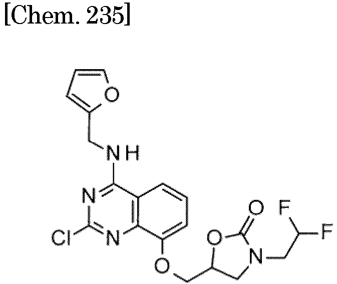

tert-Butoxypotassium (22.4 mg, 0.200 mmol) and 2-iodo-1,1-difluoroethane (35.3 mg, 0.184 mmol) were added to a solution (0.5 mL) of 5-[({2-chloro-4-[(furan-2-ylmethyl)amino]quinazolin-8-yl}oxy)methyl]-1,3-oxazolidin-2-one (31.2 mg, 0.0832 mmol) obtained in Example 19 in DMF, followed by stirring at room temperature for 3.5 hours. Water (6 mL) was added to the reaction solution, followed by extraction with ethyl acetate and subsequent concentration under reduced pressure. The resulting residue was purified by silica gel column chromatography (ethyl acetate/hexane = 20/80 to 80/20) to obtain the title compound (19.1 mg, 52% yield) as a white solid.

### (Example 192) 5-[({2-Chloro-4-[(furan-2-ylmethyl)amino]quinazolin-8-yl}oxy)methyl]-3-(2-hydroxyethyl)-1,3-oxazolidin-2-one

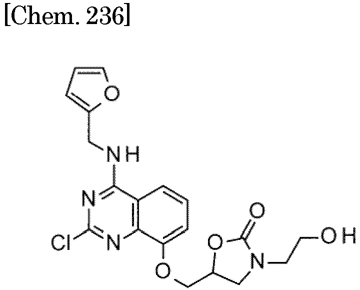

### (Example 192-1) 3-(2-{[tert-Butyl(dimethyl)silyl]oxy}ethyl)-5-[({2-chloro-4-[(furan-2-ylmethyl)amino]quinazolin-8-yl}oxy)methyl]-1,3-oxazolidin-2-one

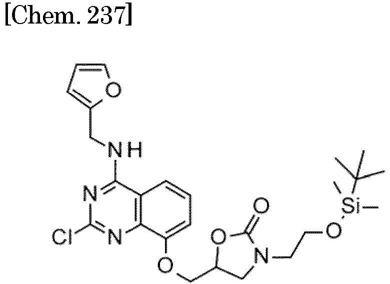

tert-Butoxypotassium (31.3 mg, 0.279 mmol) and (2-bromoethoxy)-tert-butyldimethylsilane (39.0 µL, 0.179 mmol) were added to a mixed solution of 5-[({2-chloro-4-[(furan-2-ylmethyl)amino]quinazolin-8-yl}oxy)methyl]-1,3-oxazolidin-2-one (55.9 mg, 0.149 mmol) obtained in Example 19 in THF (0.8 mL)/DMF (0.2 mL) under ice-cooling, followed by stirring at the same temperature for 3 hours. TBAI (27.6 mg, 0.0746 mmol) was added to the reaction solution, followed by stirring for 2 hours and then at room temperature for 2 hours. tert-Butoxypotassium (31.3 mg, 0.279 mmol) and (2-bromoethoxy)-tert-butyldimethylsilane (39.0 µL, 0.179 mmol) were added to the reaction solution, followed by stirring for an additional 37 hours. Then, water (5 mL) was added to the reaction solution, followed by extraction with ethyl acetate and subsequent concentration under reduced pressure. The resulting residue was purified by silica gel column chromatography (ethyl acetate/hexane = 0/100 to 70/30) to obtain the title compound (35.8 mg, 45% yield) as a pale yellow solid.

### (Example 192-2) 5-[({2-Chloro-4-[(furan-2-ylmethyl)amino]quinazolin-8-yl}oxy)methyl]-3-(2-hydroxyethyl)-1,3-oxazolidin-2-one

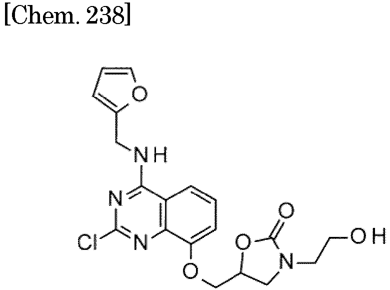

1M TBAF-THF (98.0 µL, 0.0980 mmol) was added to a solution (1 mL) of 3-(2-{[tert-butyl(dimethyl)silyl]oxy}ethyl)-5-[({2-chloro-4-[(furan-2-ylmethyl)amino]quinazolin-8-yl}oxy)methyl]-1,3-oxazolidin-2-one (34.8 mg, 0.0653 mmol) obtained in Example 192-1 in THF, followed by stirring at room temperature for 2.5 hours. Water (3 mL) was added to the reaction solution, followed by extraction with ethyl acetate and subsequent concentration under reduced pressure. The resulting residue was purified by silica gel column chromatography (methanol/ethyl acetate = 0/100 to 10/90) to obtain the title compound (21.1 mg, 77% yield) as a white solid.

### (Example 193) 5-[({2-Chloro-4-[(furan-2-ylmethyl)amino]quinazolin-8-yl}oxy)methyl]-3-(oxetan-3-yl)- 1,3-oxazolidin-2-one

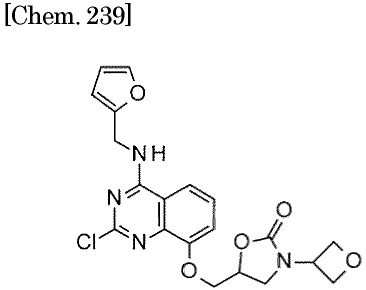

Cesium carbonate (67.5 mg, 0.207 mmol) and 3-iodooxetane (32.1 mg, 0.174 mmol) were added to a solution (0.4 mL) of 5-[({2-chloro-4-[(furan-2-ylmethyl)amino]quinazolin-8-yl}oxy)methyl]-1,3-oxazolidin-2-one (40.1 mg, 0.107 mmol) obtained in Example 19 in DMF, followed by stirring at room temperature for 3 hours and then at 60°C for 17.5 hours. The reaction solution was allowed to cool to room temperature. Then, water (6 mL) was added, followed by extraction with ethyl acetate and subsequent concentration under reduced pressure. The resulting residue was purified by silica gel column chromatography (ethyl acetate/hexane = 50/50 to 100/0) to obtain the title compound (7.10 mg, 15% yield) as a white solid.

### (Example 194) 5-[({2-Chloro-4-[(furan-2-ylmethyl)amino]quinazolin-8-yl}oxy)methyl]-3-(2-hydroxy-2-methylpropyl)-1,3-oxazolidin-2-one

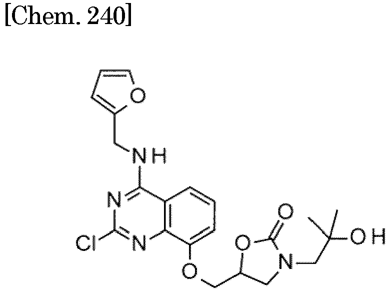

tert-Butoxypotassium (19.1 mg, 0.170 mmol) and isobutylene oxide (15.0 µL, 0.158 mmol) were added to a mixed solution of 5-[({2-chloro-4-[(furan-2-ylmethyl)amino]quinazolin-8-yl}oxy)methyl]-1,3-oxazolidin-2-one (29.6 mg, 0.0790 mmol) obtained in Example 19 in DMF (0.5 mL), followed by stirring at room temperature for 3 hours. Isobutylene oxide (57.0 µL, 0.632 mmol) was added to the reaction solution, and the mixture was further stirred at room temperature for 17 hours. Water (6 mL) was added to the reaction solution, followed by extraction with ethyl acetate and subsequent concentration under reduced pressure. The resulting residue was crudely purified by silica gel column chromatography (ethyl acetate/hexane = 50/50 to 90/10), and then purified by silica gel column chromatography (NH, methanol/ethyl acetate = 0/100 to 2/98) to obtain the title compound (12.1 mg, 34% yield) as a white solid.

### (Example 195) 5-[({2-Chloro-4-[(furan-2-ylmethyl)amino]quinazolin-8-yl}oxy)methyl]-N-ethyl-2-oxo-1,3-oxazolidine-3-carboxamide

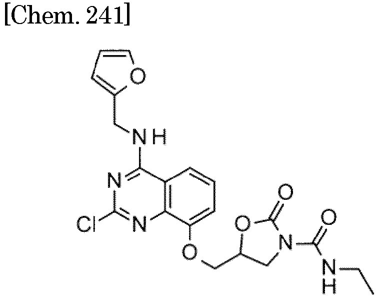

1,4-Diazabicyclo[2.2.2]octane (23.2 mg, 0.207 mmol) and ethyl isocyanate (17.0 µL, 0.207 mmol) were added to a solution (1 mL) of 5-[({2-chloro-4-[(furan-2-ylmethyl)amino]quinazolin-8-yl}oxy)methyl]-1,3-oxazolidin-2-one (38.7 mg, 0.103 mmol) obtained in Example 19 in DMF, followed by stirring at room temperature for 9 hours. Water (9 mL) was added to the reaction solution, followed by extraction with ethyl acetate and subsequent concentration under reduced pressure. The resulting residue was purified by silica gel column chromatography (NH, ethyl acetate/hexane = 30/70 to 90/10) to obtain the title compound (18.7 mg, 41% yield) as a colorless solid.

### (Example 196) 4-[({2-Chloro-4-[(furan-2-ylmethyl)amino]quinazolin-8-yl}oxy)methyl]-1-(2-hydroxyethyl)pyrrolidin-2-one

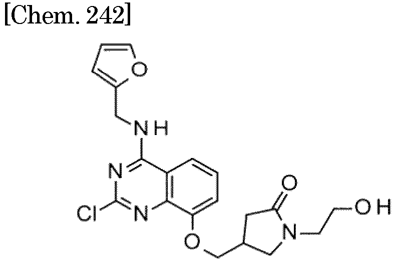

The same operation as in Example 192-1 was performed using 4-[({2-chloro-4-[(furan-2-ylmethyl)amino]quinazolin-8-yl}oxy)methyl]pyrrolidin-2-one (73.9 mg, 0.198 mmol) obtained in Example 31. Thus, crude 1-(2-{[tert-butyl(dimethyl)silyl]oxy}ethyl)-4-[({2-chloro-4-[(furan-2-ylmethyl)amino]quinazolin-8-yl}oxy)methyl]pyrrolidin-2-one (51.8 mg) [MS (m/z): 531 (M)⁺] was obtained as a colorless solid. 1M TBAF-THF (147 µL, 0.147 mmol) was added to a solution (1.5 mL) of the obtained 1-(2-{[tert-butyl(dimethyl)silyl]oxy}ethyl)-4-[({2-chloro-4-[(furan-2-ylmethyl)amino]quinazolin-8-yl}oxy)methyl]pyrrolidin-2-one (51.8 mg, 0.0975 mmol) in THF, followed by stirring at room temperature for 1 hour. Water (7 mL) was added to the reaction solution, followed by extraction with ethyl acetate and subsequent concentration under reduced pressure. A mixed solvent of diethyl ether/hexane (3/1) was added to the resulting residue, and the solid was collected by filtration. Thus, the title compound (31.2 mg, 77% yield) was obtained as a white solid.

### (Example 197) 4-[({2-Chloro-4-[(furan-2-ylmethyl)amino]quinazolin-8-yl}oxy)methyl]-1-(2-fluoroethyl)pyrrolidin-2-one

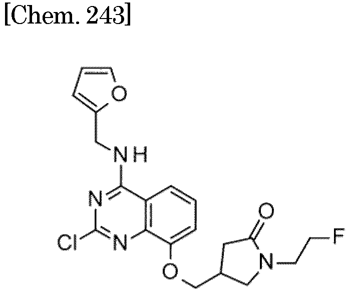

The same operation as in Example 189 was performed using 4-[({2-chloro-4-[(furan-2-ylmethyl)amino]quinazolin-8-yl}oxy)methyl]pyrrolidin-2-one (40.0 mg, 0.107 mmol) obtained in Example 31. Thus, the title compound (20.4 mg, 45% yield) was obtained as a white solid.

Structural formulae and physical scientific data of the compounds described in Examples 187 to 197 are shown below.

**[Table 15]**

| Example | Structural formulae | Data |
|---|---|---|
| 187 | 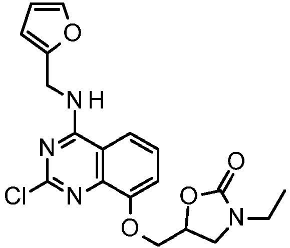 | 1H-NMR (DMSO-D₆) δ: 1.12 (3H, t, J = 7.3 Hz), 3.18-3.29 (2H, m), 3.51 (1H, dd, J = 8.8, 6.4 Hz), 3.73 (1H, t, J = 8.8 Hz), 4.24 (1H, dd, J = 10.9, 6.1 Hz), 4.35 (1H, dd, J = 10.9, 3.0 Hz), 4.72 (2H, br s), 4.92-4.98 (1H, m), 6.34-6.36 (1H, m), 6.41-6.43 (1H, m), 7.38 (1H, d, J = 7.3 Hz), 7.46 (1H, t, J = 8.2 Hz), 7.60-7.62 (1H, m), 7.86 (1H, d, J = 9.1 Hz), 9.14 (1H, br s). |
| | | MS (m/z): 403 (M+H)⁺. |
| 188 | 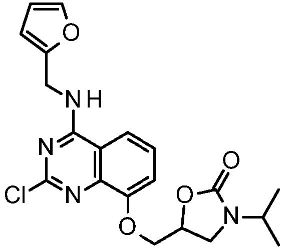 | 1H-NMR (DMSO-D₆) δ: 1.14 (3H, d, J = 6.7 Hz), 1.19 (3H, d, J = 6.7 Hz), 3.49 (1H, dd, J = 8.8, 6.4 Hz), 3.67 (1H, t, J = 8.8 Hz), 3.88-3.95 (1H, m), 4.22 (1H, dd, J = 11.2, 5.2 Hz), 4.36 (1H, dd, J = 11.2, 3.3 Hz), 4.72 (2H, d, J = 3.6 Hz), 4.92-4.99 (1H, m), 6.34-6.36 (1H, m), 6.41-6.43 (1H, m), 7.38 (1H, d, J = 7.3 Hz), 7.46 (1H, t, J = 8.2 Hz), 7.61 (1H, brd, J = 1.8 Hz), 7.86 (1H, d, J = 6.1 Hz), 9.14 (1H, brt, J = 4.6 Hz). |
| | | MS (m/z): 417 (M+H)⁺. |
| 189 | 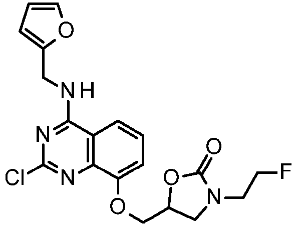 | 1H-NMR (DMSO-D₆) δ: 3.49-3.52 (1H, m), 3.55-3.59 (2H, m), 3.83 (1H, t, J= 8.8 Hz), 4.25 (1H, dd, J = 11.2, 5.8 Hz), 4.36 (1H, dd, J = 10.9, 3.0 Hz), 4.53-4.76 (4H, m), 4.96-5.03 (1H, m), 6.35-6.36 (1H, m), 6.41-6.43 (1H, m), 7.38 (1H, br d, J = 7.9 Hz), 7.46 (1H, t, J = 7.9 Hz), 7.60-7.61 (1H, m), 7.86 (1H, br d, J = 7.3 Hz), 9.14 (1H, br s). |
| | | MS (m/z): 421 (M+H)⁺. |
| 190 | 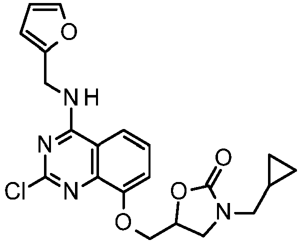 | 1H-NMR (DMSO-D₆) δ: 0.19-0.23 (2H, m), 0.48-0.52 (2H, m), 0.97-1.06 (1H, m), 3.02 (1H, dd, J = 14.0, 7.3 Hz), 3.12 (1H, dd, J = 14.3, 7.0 Hz), 3.62 (1H, dd, J = 8.8, 6.4 Hz), 3.81 (1H, t, J = 9.1 Hz), 4.25 (1H, dd, J = 10.9, 6.1 Hz), 4.38 (1H, dd, J = 10.9, 3.0 Hz), 4.72 (2H, br s), 4.95-5.01 (1H, m), 6.35 (1H, br d, J = 3.6 Hz), 6.41-6.42 (1H, m), 7.39 (1H, d, J = 7.9 Hz), 7.46 (1H, t, J = 8.2 Hz), 7.60-7.61 (1H, m), 7.86 (1H, d, J = 7.9 Hz), 9.14 (1H, s). |
| | | MS (m/z): 429 (M+H)⁺. |
| 191 | 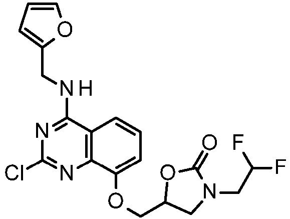 | 1H-NMR (DMSO-D₆) δ: 3.58-3.72 (3H, m), 3.86 (1H, t, J = 8.8 Hz), 4.25 (1H, dd, J = 10.9, 5.5 Hz), 4.37 (1H, dd, J = 10.9, 3.0 Hz), 4.72 (2H, d, J = 5.5 Hz), 5.00-5.06 (1H, m), 6.18-6.47 (3H, m), 7.38 (1H, d, J = 7.3 Hz), 7.46 (1H, t, J= 8.2 Hz), 7.61 (1H, d, J = 1.8 Hz), 7.86 (1H, d, J = 8.2 Hz), 9.15 (1H, t, J = 5.5 Hz). |
| | | MS (m/z): 439 (M+H)⁺. |
| 192-1 | 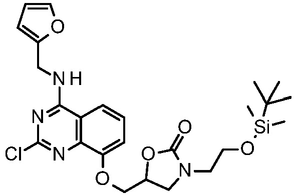 | 1H-NMR (DMSO-D6) δ: 0.02 (3H, s), 0.03 (3H, s), 0.83 (9H, s), 3.30 (2H, t, J = 5.5 Hz), 3.55 (1H, dd, J = 9.1, 6.1 Hz), 3.70-3.77 (2H, m), 3.81 (1H, t, J = 9.1 Hz), 4.24 (1H, dd, J = 10.9, 6.1 Hz), 4.32 (1H, dd, J = 10.9, 3.6 Hz), 4.72 (2H, d, J= 4.9 Hz), 4.93-4.99 (1H, m), 6.35 (1H, d, J = 4.3 Hz), 6.42 (1H, dd, J = 3.0, 1.8 Hz), 7.36 (1H, d, J = 7.3 Hz), 7.45 (1H, t, J = 7.9 Hz), 7.61 (1H, d, J = 1.8 Hz), 7.86 (1H, d, J = 7.9 Hz), 9.14 (1H, t, J = 5.5 Hz). |
| 192-2 | 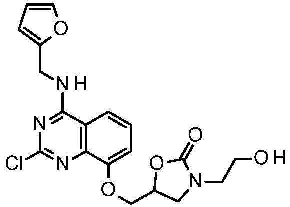 | 1H-NMR (DMSO-D₆) δ: 3.23-3.27 (2H, m), 3.52-3.58 (3H, m), 3.82 (1H, t, J = 8.8 Hz), 4.25 (1H, dd, J = 10.9, 6.4 Hz), 4.33 (1H, dd, J = 10.9, 3.6 Hz), 4.72 (2H, br s), 4.82 (1H, t, J = 5.8 Hz), 4.92-4.98 (1H, m), 6.36 (1H, d, J = 3.6 Hz), 6.42 (1H, dd, J = 3.6, 1.8 Hz), 7.38 (1H, d, J = 7.9 Hz), 7.46 (1H, t, J = 8.2 Hz), 7.61 (1H, d, J = 1.8 Hz), 7.86 (1H, d, J = 7.3 Hz), 9.15 (1H, br s). |
| | | MS (m/z): 419 (M+H)⁺. |
| 193 | 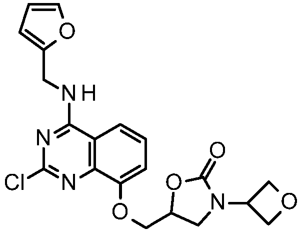 | 1H-NMR (DMSO-D₆) δ: 3.84 (1H, dd, J = 8.5, 6.1 Hz), 4.02 (1H, t, J = 8.8 Hz), 4.28 (1H, dd, J= 11.2, 5.2 Hz), 4.41 (1H, dd, J= 11.2, 2.7 Hz), 4.70-4.79 (5H, m), 4.85 (1H, t, J= 6.4 Hz), 4.92-4.99 (1H, m), 5.02-5.07 (1H, m), 6.35 (1H, br d, J = 2.4 Hz), 6.42 (1H, dd, J = 3.0, 1.8 Hz), 7.38 (1H, d, J = 7.9 Hz), 7.47 (1H, t, J = 7.9 Hz), 7.60-7.62 (1H, m), 7.86 (1H, d, J = 8.5 Hz), 9.14 (1H, br t, J = 4.9 Hz). |
| | | MS (m/z): 431 (M+H)⁺. |
| 194 | 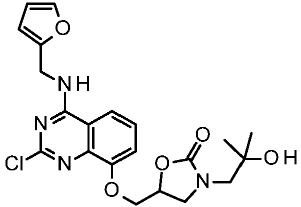 | 1H-NMR (DMSO-D₆) δ: 1.36 (6H, s), 3.27-3.33 (2H, m), 3.45 (2H, s), 4.03-4.05 (2H, m), 4.09-4.14 (1H, m), 4.72 (2H, d, J= 5.5 Hz), 5.43 (1H, d, J = 5.5 Hz), 6.36 (1H, d, J = 3.6 Hz), 6.42 (1H, dd, J = 3.6, 1.8 Hz), 7.34 (1H, d, J = 7.9 Hz), 7.45 (1H, t, J = 8.2 Hz), 7.61 (1H, d, J = 1.8 Hz), 7.83 (1H, d, J = 7.9 Hz), 9.12 (1H, t, J = 5.8 Hz). |
| | | MS (m/z): 447 (M+H)⁺. |
| 195 | 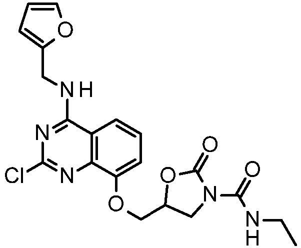 | 1H-NMR (DMSO-D₆) δ: 1.08 (3H, t, J = 7.0 Hz), 3.18-3.25 (2H, m), 3.81 (1H, dd, J = 9.7, 6.7 Hz), 4.07 (1H, t, J = 9.7 Hz), 4.39 (2H, d, J = 4.3 Hz), 4.72 (2H, d, J = 5.5 Hz), 5.06-5.13 (1H, m), 6.35 (1H, d, J = 3.0 Hz), 6.42 (1H, dd, J = 3.0, 1.8 Hz), 7.37 (1H, d, J = 7.9 Hz), 7.46 (1H, t, J = 8.2 Hz), 7.60-7.62 (1H, m), 7.80 (1H, t, J = 5.8 Hz), 7.87 (1H, d, J = 7.3 Hz), 9.14 (1H, t, J = 5.5 Hz). |
| | | MS (m/z): 446 (M+H)⁺. |
| 196 | 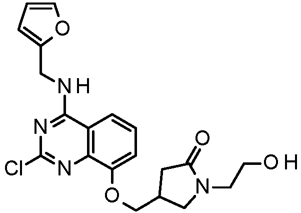 | 1H-NMR (DMSO-D₆) 6: 2.19 (1H, dd, J= 16.5, 6.1 Hz), 2.46-2.52 (1H, m), 2.83-2.93 (1H, br m), 3.22-3.28 (2H, m), 3.35-3.40 (1H, m), 3.47-3.51 (2H, m), 3.66 (1H, dd, J = 9.8, 8.5 Hz), 4.09 (2H, d, J= 6.7 Hz), 4.71-4.74 (3H, m), 6.35 (1H, d, J = 3.1 Hz), 6.41-6.43 (1H, m), 7.36 (1H, d, J= 7.9 Hz), 7.45 (1H, t, J = 7.9 Hz), 7.61 (1H, br s), 7.83 (1H, d, J = 8.5 Hz), 9.11 (1H, t, J = 5.5 Hz). |
| | | MS (m/z): 417 (M+H)⁺. |
| 197 | 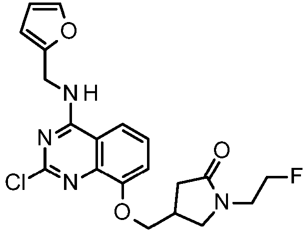 | 1H-NMR (DMSO-D6) 6: 2.22 (1H, dd, J = 17.1, 6.1 Hz), 2.50-2.56 (1H, m), 2.88-2.95 (1H, m), 3.37 (1H, dd, J = 9.8, 5.5 Hz), 3.49 (1H, t, J = 4.9 Hz), 3.56 (1H, t, J = 4.9 Hz), 3.67 (1H, dd, J = 10.4, 8.5 Hz), 4.11 (2H, d, J = 7.3 Hz), 4.48-4.52 (1H, m), 4.60-4.63 (1H, m), 4.72 (2H, d, J = 5.5 Hz), 6.36 (1H, br d, J = 2.4 Hz), 6.43 (1H, dd, J = 3.1, 1.8 Hz), 7.37 (1H, d, J = 6.7 Hz), 7.46 (1H, t, J = 7.9 Hz), 7.61-7.62 (1H, m), 7.84 (1H, d, J = 7.3 Hz), 9.12 (1H, t, J = 5.2 Hz). |
| | | MS (m/z): 419 (M+H)⁺. |

Compounds of Examples 198 to 204 shown below were produced in accordance with the steps described in Examples 189 to 197 above.

**[Table 16]**

| Example | Structural formulae | Data |
|---|---|---|
| 198 | 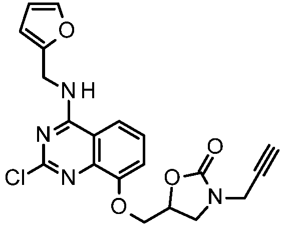 | 1H-NMR (DMSO-D₆) δ: 3.37 (1H, br s), 3.51 (1H, t, J= 7.6 Hz), 3.79 (1H, t, J= 8.8 Hz), 4.02-4.14 (2H, m), 4.28 (1H, dd, J = 11.5, 6.7 Hz), 4.38 (1H, dd, J = 11.5, 3.6 Hz), 4.72 (2H, d, J = 5.5 Hz), 5.01-5.07 (1H, br m), 6.36 (1H, br s), 6.42 (1H, br s), 7.39 (1H, d, J = 7.9 Hz), 7.46 (1H, t, J = 7.9 Hz), 7.61 (1H, s), 7.87 (1H, d, J = 8.5 Hz), 9.14 (1H, t, J = 5.5 Hz). |
| | | MS (m/z): 413 (M+H)⁺. |
| 199 | 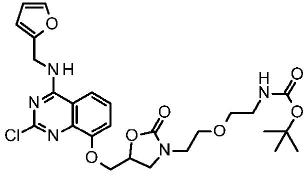 | 1H-NMR (DMSO-D₆) δ: 1.35 (9H, s), 3.05-3.09 (2H, m), 3.33-3.41 (4H, m), 3.49-3.61 (3H, m), 3.81 (1H, t, J = 9.1 Hz), 4.24 (1H, dd, J = 10.9, 6.1 Hz), 4.33 (1H, dd, J = 10.9, 3.0 Hz), 4.72 (2H, d, J = 5.5 Hz), 4.93-4.98 (1H, m), 6.36 (1H, br d, J = 3.0 Hz), 6.42 (1H, dd, J = 3.0, 1.8 Hz), 6.81 (1H, t, J = 5.5 Hz), 7.38 (1H, d, J = 7.3 Hz), 7.46 (1H, t, J = 8.2 Hz), 7.61 (1H, br d, J = 1.5 Hz), 7.86 (1H, d, J = 7.9 Hz), 9.14 (1H, t, J = 5.5 Hz). |
| | | MS (m/z): 562 (M)⁺. |
| 200 | 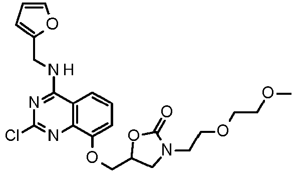 | 1H-NMR (DMSO-D6) δ: 3.21 (3H, s), 3.36-3.42 (4H, m), 3.52-3.62 (5H, m), 3.80 (1H, t, J = 8.8 Hz), 4.24 (1H, dd, J = 10.9, 6.1 Hz), 4.33 (1H, dd, J = 11.2, 3.3 Hz), 4.72 (2H, d, J = 5.5 Hz), 4.93-4.99 (1H, m), 6.36 (1H, d, J = 3.0 Hz), 6.42 (1H, dd, J = 3.0, 1.8 Hz), 7.38 (1H, d, J = 7.3 Hz), 7.46 (1H, t, J = 8.2 Hz), 7.61 (1H, d, J = 1.2 Hz), 7.86 (1H, d, J = 7.9 Hz), 9.14 (1H, t, J = 5.5 Hz). |
| | | MS (m/z): 477 (M+H)⁺. |
| 201 | 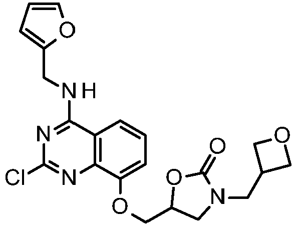 | 1H-NMR (DMSO-D₆) δ: 3.24-3.32 (1H, m), 3.44-3.58 (3H, m), 3.70 (1H, t, J = 8.8 Hz), 4.22 (1H, dd, J = 10.9, 5.5 Hz), 4.31-4.36 (3H, m), 4.65-4.69 (2H, m), 4.72 (2H, br s), 4.92-4.97 (1H, m), 6.36 (1H, br d, J = 2.4 Hz), 6.41-6.43 (1H, m), 7.37 (1H, d, J = 7.3 Hz), 7.46 (1H, t, J = 7.9 Hz), 7.61 (1H, br d, J = 1.2 Hz), 7.86 (1H, d, J = 7.3 Hz), 9.15 (1H, br s). |
| | | MS (m/z): 445 (M+H)⁺. |
| 202 | 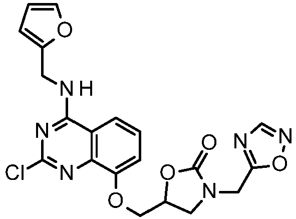 | 1H-NMR (DMSO-D₆) δ: 3.60 (1H, dd, J = 8.8, 6.4 Hz), 3.91 (1H, t, J = 8.8 Hz), 4.30 (1H, dd, J = 10.9, 6.1 Hz), 4.39 (1H, dd, J = 10.9, 3.3 Hz), 4.72 (2H, br d, J = 5.5 Hz), 4.83 (1H, d, J = 17.0 Hz), 4.90 (1H, d, J = 17.0 Hz), 5.06-5.12 (1H, m), 6.36 (1H, br d, J = 3.0 Hz), 6.42 (1H, dd, J = 3.0, 1.8 Hz), 7.40 (1H, br d, J = 7.9 Hz), 7.46 (1H, t, J = 7.9 Hz), 7.60-7.61 (1H, m), 7.87 (1H, br d, J = 7.9 Hz), 9.03 (1H, s), 9.15 (1H, br t, J = 5.5 Hz). |
| | | MS (m/z): 457 (M+H)⁺. |
| 203 | 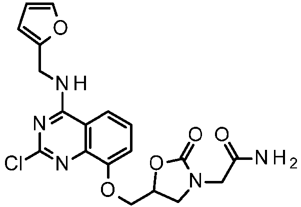 | 1H-NMR (DMSO-D₆) δ: 3.47 (1H, dd, J = 8.5, 6.1 Hz), 3.78-3.84 (3H, m), 4.27-4.35 (2H, m), 4.72 (2H, d, J = 5.5 Hz), 4.97-5.04 (1H, m), 6.36 (1H, d, J = 3.0 Hz), 6.42 (1H, dd, J = 3.0, 1.8 Hz), 7.20 (1H, br s), 7.39 (1H, d, J = 7.3 Hz), 7.46 (1H, t, J = 7.9 Hz), 7.54 (1H, br s), 7.61 (1H, d, J = 1.8 Hz), 7.86 (1H, d, J = 7.3 Hz), 9.14 (1H, t, J = 5.8 Hz). |
| | | MS (m/z): 432 (M+H)⁺. |
| 204 | 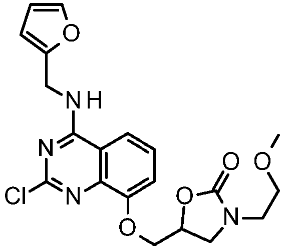 | 1H-NMR (DMSO-D₆) δ: 3.26 (3H, s), 3.35-3.38 (2H, m), 3.46-3.56 (3H, m), 3.78 (1H, t, J = 9.1 Hz), 4.24 (1H, dd, J = 11.5, 6.1 Hz), 4.34 (1H, dd, J = 11.2, 3.3 Hz), 4.72 (2H, br s), 4.92-4.99 (1H, m), 6.36 (1H, br d, J = 4.3 Hz), 6.42 (1H, dd, J = 3.0, 1.8 Hz), 7.38 (1H, br d, J = 7.9 Hz), 7.46 (1H, t, J = 8.2 Hz), 7.61 (1H, br d, J = 1.8 Hz), 7.86 (1H, dd, J = 8.5, 1.2 Hz), 9.14 (1H, br s). |
| | | MS (m/z): 433 (M+H)⁺. |

### Measurement of Activity Value

A HeLa cell line was seeded (2 × 10⁵ cells/well) on a 24-well plate (Corning) with DMEM medium containing 10% fetal bovine serum and 1% penicillin-streptomycin (Nacalai Tesque, Inc.) and the next day transfected with a GLA splicing reporter vector (FIG. 1) using Lipofectamine 2000 (Thermo Fisher Scientific). Five hours after transfection, the medium was replaced, and the evaluation compound prepared in each example above and dissolved in DMSO at 10 mM was added in a volume of 0.1% (final concentrations: 10 µM evaluation compound and 0.1% DMSO). In addition, a group in which only DMSO containing no evaluation compound was added was prepared as a negative control, and a group in which a known splicing control compound, 2-chloro-6-(2-furylmethyl)purine, was added at 10 µM was prepared as a positive control. These groups were used as indices for evaluating the activity. Sixteen hours after the addition of the compound, the cells on the plate were fixed through treatment with 4% paraformaldehyde at room temperature for 15 minutes. The fixing solution was then replaced with phosphate-buffered saline. Subsequently, the fluorescence intensities of GFP (GLA splicing: exon 4 + exon 5) and RFP (GLA splicing: exon 4 + pseudoexon (Ψ) + exon 5) were measured using a BZ-X700 fluorescence microscope (Keyence Corp.), and their ratio (GFP signal/RFP signal) was measured. Then, the activity value (GLA_Signal-REC 10_ 2) of each evaluation compound was quantified using the fluorescence ratio obtained from the treatment with the 10 µM known compound (positive control) as 1.

The results obtained are shown in the table below.

**[Table 17]**

| Example | GLA_Signal_ REC_10_2 |
|---|---|
| 1 | 1.48 |
| 2 | 2.91 |
| 3 | 2.15 |
| 4 | 1.63 |
| 5 | 1.8 |
| 6 | 3.92 |
| 7 | 3.13 |
| 8 | 2.6 |
| 9 | 1.24 |
| 10 | 1.16 |
| 11 | 1.04 |
| 12 | 1.75 |
| 13 | 1.08 |
| 14 | 2.24 |
| 15 | 1.81 |
| 16 | 0.97 |
| 17 | 1.13 |
| 18 | 4.32 |
| 19 | 6.87 |
| 20 | 4.5 |
| 21 | 3.73 |
| 22 | 2.81 |
| 23 | 1.26 |
| 24 | 1.24 |
| 25 | 10.1 |
| 26 | 5.83 |
| 27 | 3.93 |
| 28 | 6.54 |
| 30 | 1.81 |
| 31 | 4.98 |
| 32 | 6.66 |
| 33 | 2.79 |
| 34 | 6.51 |
| 35 | 1.5 |
| 36 | 4.59 |
| 37 | 1.11 |
| 38 | 7.48 |
| 39 | 8.94 |
| 40 | 3.23 |
| 41 | 1.86 |
| 42 | 1.39 |
| 43 | 6.76 |
| 44 | 5.46 |
| 45 | 5.29 |
| 46 | 3.79 |
| 47 | 3.68 |
| 48 | 3.64 |
| 49 | 3.13 |
| 50 | 2.96 |
| 51 | 2.78 |
| 52 | 2.72 |
| 53 | 2.69 |
| 54 | 2.54 |
| 55 | 2.3 |
| 56 | 2.15 |
| 57 | 2.06 |
| 58 | 1.84 |
| 59 | 1.81 |
| 60 | 1.37 |
| 61 | 1.3 |
| 62 | 1.26 |
| 63 | 1.12 |
| 64 | 1.09 |
| 65 | 0.96 |
| 66 | 0.92 |
| 67 | 3.52 |
| 68 | 3.91 |
| 69 | 3.61 |
| 70 | 2.11 |
| 71 | 1.06 |
| 72 | 1.15 |
| 73 | 6.27 |
| 74 | 3.18 |
| 75 | 2.5 |
| 76 | 1.3 |
| 77 | 1.68 |
| 78 | 9.48 |
| 79 | 5.79 |
| 80 | 3.57 |
| 81 | 2.55 |
| 82 | 2.01 |
| 83 | 6.67 |
| 84 | 3.39 |
| 85 | 4.6 |
| 87 | 3.93 |
| 88 | 3.44 |
| 89 | 2.3 |
| 90 | 2.05 |
| 91 | 1.96 |
| 92 | 1.8 |
| 93 | 1.71 |
| 94 | 3.01 |
| 95 | 9.52 |
| 96 | 5.93 |
| 97 | 8.37 |
| 98 | 7.68 |
| 99 | 5.93 |
| 100 | 4.41 |
| 101 | 2.52 |
| 102 | 2.05 |
| 103 | 2.02 |
| 104 | 2.92 |
| 105 | 9.37 |
| 106 | 4.75 |
| 107 | 4.43 |
| 108 | 4.16 |
| 109 | 3.79 |
| 110 | 2.88 |
| 111 | 4.46 |
| 112 | 2.53 |
| 113 | 5.23 |
| 114 | 3.12 |
| 115 | 1.34 |
| 116 | 4.12 |
| 117 | 2.3 |
| 118 | 3.8 |
| 119 | 1.45 |
| 120 | 1.44 |
| 121 | 1.75 |
| 122 | 2.88 |
| 123 | 2.28 |
| 124 | 1.49 |
| 125 | 4.24 |
| 126 | 1.76 |
| 127 | 2.75 |
| 128 | 1.66 |
| 129 | 2.67 |
| 130 | 4.85 |
| 131 | 2.29 |
| 132 | 1.31 |
| 133 | 1.19 |
| 134 | 1.18 |
| 135 | 0.94 |
| 136 | 3.83 |
| 137 | 1.85 |
| 138 | 1.54 |
| 139 | 1.48 |
| 140 | 1.41 |
| 141 | 0.98 |
| 142 | 2.46 |
| 143 | 2.14 |
| 144 | 2.49 |
| 145 | 5.17 |
| 146 | 2.25 |
| 147 | 2.57 |
| 148 | 2.05 |
| 149 | 1.55 |
| 150 | 0.9 |
| 151 | 1 |
| 152 | 2.33 |
| 153 | 1.35 |
| 154 | 4.65 |
| 155 | 4.39 |
| 156 | 3.07 |
| 157 | 2.56 |
| 158 | 2.69 |
| 159 | 1.85 |
| 160 | 1.62 |
| 161 | 3.22 |
| 162 | 3.13 |
| 163 | 1.63 |
| 164 | 1.24 |
| 165 | 1.55 |
| 166 | 1.22 |
| 167 | 1.16 |
| 168 | 1.07 |
| 169 | 1.09 |
| 170 | 3.14 |
| 171 | 3.38 |
| 172 | 3.96 |
| 173 | 6.96 |
| 174 | 3.44 |
| 175 | 4.39 |
| 176 | 3.16 |
| 177 | 6.05 |
| 178 | 5.21 |
| 179 | 1.7 |
| 180 | 2.53 |
| 181 | 7.43 |
| 182 | 3.84 |
| 183 | 1.57 |
| 184 | 1.93 |
| 185 | 2.25 |
| 186 | 2.16 |
| 187 | 2.68 |
| 188 | 4.04 |
| 189 | 4.97 |
| 190 | 3.79 |
| 191 | 3.01 |
| 192 | 5.26 |
| 193 | 3.38 |
| 194 | 0.9 |
| 195 | 1.34 |
| 196 | 3.2 |
| 197 | 1.69 |
| 198 | 2.24 |
| 199 | 2.04 |
| 200 | 1.43 |
| 201 | 2.23 |
| 202 | 1.65 |
| 203 | 1.54 |
| 204 | 3.65 |

### System for Evaluating Aberrant Splicing Caused by GLAIVS4+919G>A Mutation in Fabry Disease

A region spanning from exon 4 to exon 5 of GLA (nt 7272-9215 in the GLA gene sequence) and containing either the normal IVS4 or the IVS4+919G>A mutation was cloned downstream of a cytomegalovirus (CMV) early gene promoter, disclosed in WO 2018/151326 and to be used for investigating the effects of a splicing manipulation compound on aberrant splicing caused by the GLA IVS4+919G>A mutation, to prepare vectors pAM1 (normal type) and pAM2 (919 G>A mutant type) serving as evaluation systems. By introducing these vectors into cultured cells, it is possible to reproduce aberrant splicing (pseudoexon inclusion) in cells from patients with Fabry disease and determine the effects of the compounds.

## Claims

1. A compound represented by Formula (I) below or a pharmaceutically acceptable salt thereof: 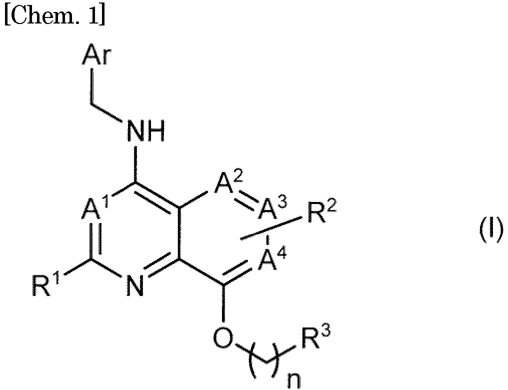 wherein, in Formula (I),
A¹, A², A³, and A⁴ each independently represent CH or N,
Ar represents a 6- to 12-membered aryl group, or a 5- or 6-membered heteroaryl group having 1 to 3 heteroatoms selected from the group consisting of nitrogen, oxygen, and sulfur atoms in a ring, wherein the 6- to 12-membered aryl group and the 5- or 6-membered heteroaryl group may be substituted with one or more halogen atoms,
n is 1 or 2,
R¹ represents a halogen atom,
R² represents a hydrogen atom, a halogen atom, or a C₁-C₄ alkoxy group, and
R³ represents a hydrogen atom, a C₃-C₆ cycloalkyl group, or a 4- to 6-membered heterocyclyl group having 1 to 3 heteroatoms selected from the group consisting of nitrogen, oxygen, and sulfur atoms in a ring,
wherein the C₃-C₆ cycloalkyl group and the 4- to 6-membered heterocyclyl group of R³ may have one or two oxo groups (=O) on the ring and/or may be substituted with one or two substituents selected from Group A below:
Group A:
a halogen atom,
a hydroxy group,
a C₁-C₄ alkyl group that may be substituted with a halogen atom,
a C₁-C₆ hydroxyalkyl group that may be substituted with a halogen atom,
a C₁-C₂ alkoxy group that may be substituted with a halogen atom,
a C₂-C₆ alkoxyalkyl group that may be substituted with a halogen atom,
a C₃-C₆ alkoxyalkoxyalkyl group that may be substituted with a halogen atom,
a C₁-C₂ alkylsulfonyl group that may be substituted with a halogen atom,
an acetyl group (-C(O)CH₃),
a cyclopropylmethyl group,
a phenyl group that may be substituted with a halogen atom,
a phenyl C₁-C₃ alkyl group that may be substituted with a halogen atom,
a 3- to 5-membered heterocyclyl group having one or two oxygen atoms,
a 3- to 5-membered heterocyclyl-C₁-C₃ alkyl group having one or two oxygen atoms,
an amino-oxo C₁-C₃ alkyl group,
a C₂-C₄ alkynyl group that may be substituted with a halogen atom,
an amino C₂-C₆ (alkoxyalkyl group) that may be protected,
a C₁-C₃ alkylaminocarbonyl group,
a 5-membered heteroaryl C₁-C₃ alkyl group,
a C₂-C₄ alkylene group (linked to the same carbon),
a C₁-C₄ alkylenedioxy group (linked to the same carbon), and
a C₁-C₄ alkyleneoxy-C₁-C₄ alkyl group (linked to the same carbon).

2. The compound according to claim 1 or pharmaceutically acceptable salt thereof,
wherein the substituent in the C₃-C₆ cycloalkyl group and the 4- to 6-membered heterocyclyl group of R³ is selected from Group B below:
Group B:
a fluorine atom;
a hydroxy group;
a methyl group, an ethyl group, and an isopropyl group;
a fluoroethyl group, a difluoroethyl group, and a trifluoromethyl group;
a hydroxyethyl group and a 2-hydroxy-2-methylpropyl group;
a methoxy group (-OCH₃) and a difluoromethoxy group (-OCHF₂);
a methoxymethyl group (-CH₂OCH₃) and a methoxyethyl group (-C₂H₄OCH₃);
a methoxyethoxyethyl group (-C₂H₄OC₂H₄OCH₃);
a methylsulfonyl group (-S(O)₂CH₃);
an acetyl group (-C(O)CH₃);
a cyclopropylmethyl group;
a phenyl group;
a benzyl group;
an oxetanyl group;
an oxetanylmethyl group;
a 2-amino-2-oxoethyl group (-CH₂C(O)NH₂);
a propargyl group;
a tert-butoxycarbonyl-2-aminoethoxyethyl group (-C₂H₄OC₂H₄NHC(O)O(CH₃)₃);
an ethylaminocarbonyl group (-C(O)NHCH₂CH₃);
an oxadiazolylmethyl group;
an ethylene group (linked to the same carbon);
a methylenedioxy group (linked to the same carbon) and an ethylenedioxy group (linked to the same carbon); and
a methyleneoxymethyl group (linked to the same carbon).

3. The compound according to claim 1 or pharmaceutically acceptable salt thereof,
wherein A¹ represents N or CH, and one or none of A², A³, and A⁴ represents N.

4. The compound according to claim 1 or pharmaceutically acceptable salt thereof,
wherein Ar represents a phenyl group that may be substituted with one halogen atom or a 5- or 6-membered heteroaryl group that may be substituted with one halogen atom,
the 5-membered heteroaryl group is selected from the group consisting of a furyl group, a thienyl group, a pyrrolyl group, an oxazolyl group, a thiazolyl group, an isothiazolyl group, and an oxadiazolyl group, and
the 6-membered heteroaryl group is a pyridyl group or a pyrimidyl group.

5. The compound according to claim 1 or pharmaceutically acceptable salt thereof,
wherein the halogen atom is fluorine or chlorine.

6. The compound according to claim 1 or pharmaceutically acceptable salt thereof,
wherein R¹ represents a chlorine atom or a bromine atom.

7. The compound according to claim 1 or pharmaceutically acceptable salt thereof,
wherein R² is selected from the group consisting of a hydrogen atom, a fluorine atom, a chlorine atom, a bromine atom, and a C₁-C₂ alkoxy group.

8. The compound according to claim 1 or pharmaceutically acceptable salt thereof,
wherein the heterocyclyl group of R³ has one oxygen atom, one sulfur atom, one nitrogen atom, one oxygen atom and one nitrogen atom, one sulfur atom and one nitrogen atom, two oxygen atoms, or two nitrogen atoms.

9. The compound according to claim 1 or pharmaceutically acceptable salt thereof,
wherein the heterocyclyl group of R³ is an oxetanyl group, a tetrahydrofuryl group, a furyl group, a tetrahydropyranyl group, a dioxolanyl group, and a dioxanyl group.

10. The compound according to claim 1 or pharmaceutically acceptable salt thereof,
wherein the heterocyclyl group having an oxo group on the ring, of R³ is selected from the group consisting of a 2-oxooxazolidinyl group, a 2-oxooxazinyl group, a 3-oxomorpholinyl group, a 2-oxoazetidinyl group, a 2-oxopyrrolidinyl group, a 2-oxopiperidinyl group, a 2-oxoimidazolidinyl group, a 2-oxo-1,3-dioxolanyl group, a 2-oxo-1,3-dioxolyl group, a 1,1-dioxo-thietanyl group, a 1,1-dioxo-tetrahydrothiophenyl group, and a 1,1-dioxo-isothizolidinyl group.

11. The compound according to claim 1 or pharmaceutically acceptable salt thereof,
wherein the heterocyclyl group having no oxo group as a substituent on the ring, of R³ is selected from the group consisting of an oxazolyl group, an azetidinyl group, a pyrrolidinyl group, a dioxolanyl group, a dioxanyl group, a tetrahydrofuryl group, a tetrahydropyranyl group, a furyl group, and an oxetanyl group.

12. A compound represented by Formula (II) below or a pharmaceutically acceptable salt thereof: 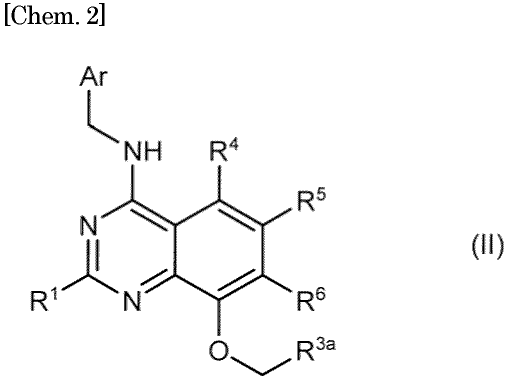 wherein, in Formula (II),
Ar represents a furyl group, a phenyl group, a thienyl group, a pyridyl group, a pyrimidinyl group, a thiazolyl group, an oxazolyl group, an isoxazolyl group, an isothiazolyl group, or an oxadiazolyl group, wherein the furyl group, the phenyl group, and the pyridyl group may be substituted with one fluorine atom,
R¹ represents a halogen atom,
R⁴, R⁵, and R⁶ each independently represent a hydrogen atom, a halogen atom, or a methoxy group, wherein at least two of R⁴, R⁵, and R⁶ represent hydrogen atoms, and
R^{3a} represents a hydrogen atom or any group selected from Group C below:
Group C: 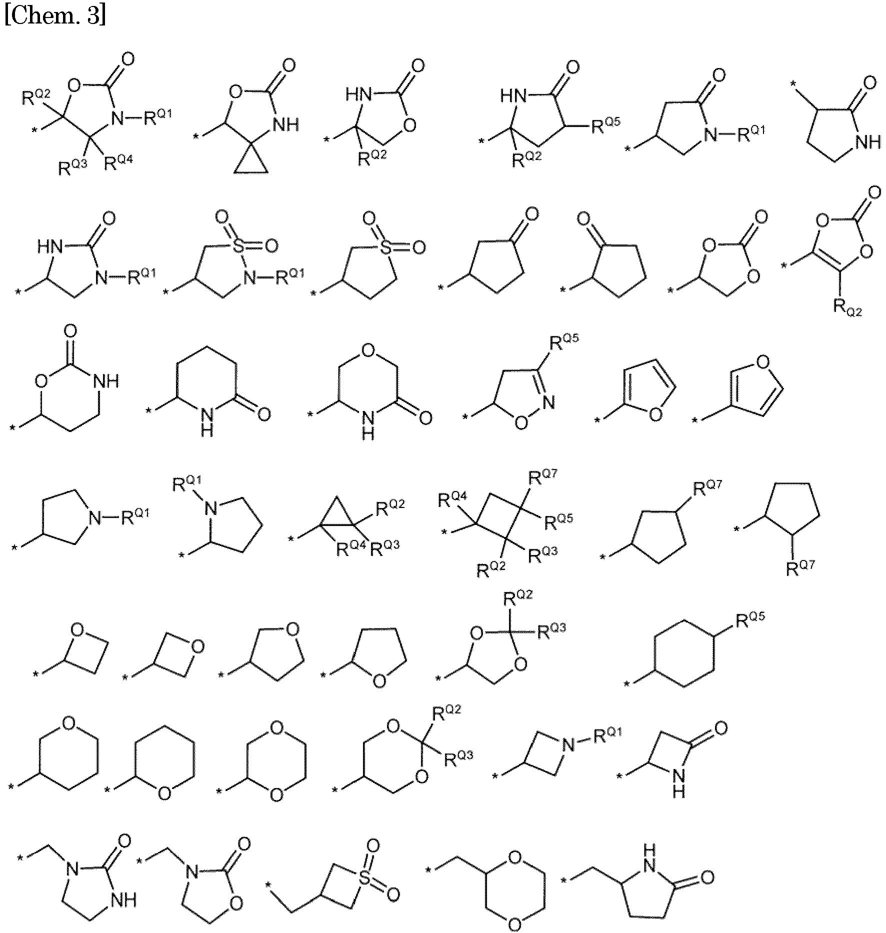
wherein R^{Q1} represents a hydrogen atom, a fluorine atom, a methyl group, an ethyl group, an isopropyl group, a fluoroethyl group, a difluoroethyl group, a hydroxyethyl group, a 2-hydroxy-2-methylpropyl group, a methoxyethyl group (-C₂H₄OCH₃), a methoxyethoxyethyl group (-C₂H₄OC₂H₄OCH₃), a methylsulfonyl group (-S(O)₂CH₃), an acetyl group (-C(O)CH₃), a cyclopropylmethyl group, a phenyl group, a benzyl group, an oxetanyl group, an oxetanylmethyl group, a 2-amino-2-oxoethyl group (-CH₂C(O)NH₂), a propargyl group (-CH₂C≡CH), a tert-butoxycarbonyl-2-aminoethoxyethyl group (-C₂H₄OC₂H₄NHC(O)O(CH₃)₃), an ethylaminocarbonyl group (-C(O)NHCH₂CH₃), or an oxadiazolylmethyl group,
R^{Q2}, R^{Q3}, and R^{Q4} each independently represent a hydrogen atom, a fluorine atom, a hydroxy group, a methyl group, or a trifluoromethyl group,
R^{Q5} and R^{Q6} each independently represent a hydrogen atom, a fluorine atom, a hydroxy group, a methyl group, a methoxy group (-OCH₃), a difluoromethoxy group (-OCHF₂), or a methoxymethyl group, and
R^{Q7} represents a hydrogen atom, a fluorine atom, a methyl group, or an ethyl group, or alternatively an ethylenedioxy group (linked to the same carbon) or a methyleneoxymethyl group (linked to the same carbon).

13. A compound represented by Formula (III) below or a pharmaceutically acceptable salt thereof: 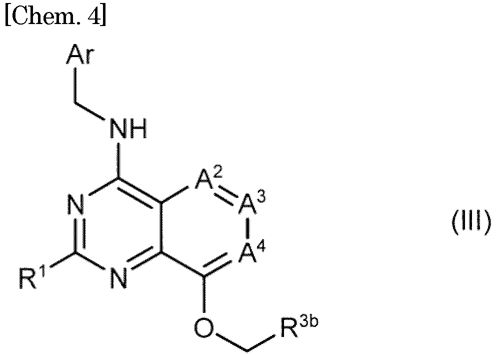 wherein, in Formula (III),
Ar represents a furyl group or a phenyl group,
R¹ represents a chlorine atom,
A², A³, and A⁴ each independently represent CH or N and any one of A², A³, and A⁴ represents N, and
R^{3b} represents a hydrogen atom or any group selected from Group D below:
Group D:

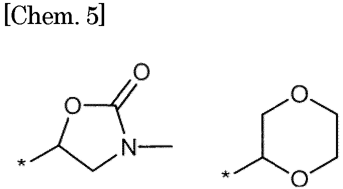

14. A compound represented by Formula (IV) below or a pharmaceutically acceptable salt thereof: 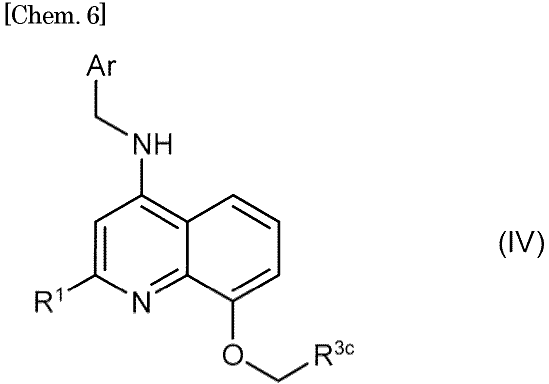 wherein, in Formula (IV),
Ar represents a furyl group or a phenyl group,
R¹ represents a chlorine atom,
R^{3c} represents a hydrogen atom or any group selected from Group E below:
Group E: 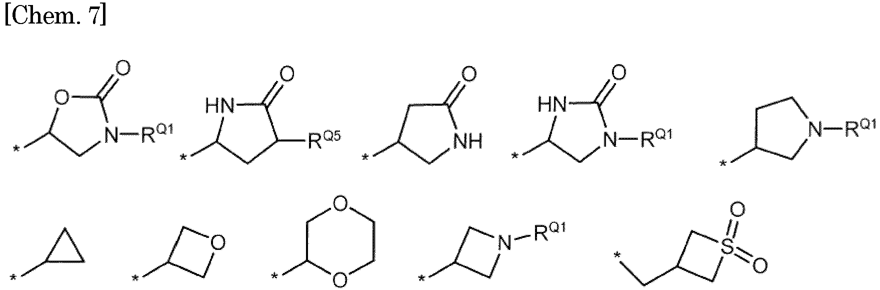
wherein R^{Q1} represents a hydrogen atom, a methyl group, a methylsulfonyl group (-S(O)₂CH₃), an acetyl group (-C(O)CH₃), a fluoroethyl group, or a hydroxyethyl group, and
R^{Q5} represents a hydrogen atom or a methyl group.

15. A compound represented by Formula (V) below or a pharmaceutically acceptable salt thereof: 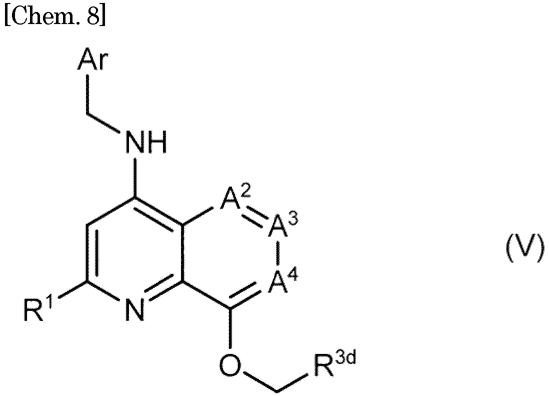 wherein, in Formula (V),
Ar represents a furyl group or a phenyl group,
R¹ represents a chlorine atom,
A², A³, and A⁴ each independently represent CH or N and any one of A², A³, and A⁴ represents N, and
R^{3d} represents a hydrogen atom or any group selected from Group F below:
Group F: 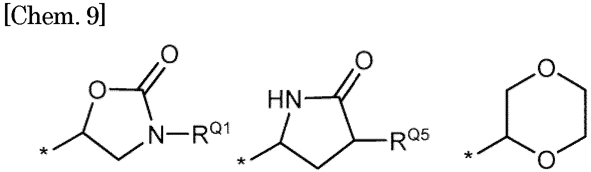
wherein R^{Q1} represents a hydrogen atom, a methyl group, a fluoroethyl group, or a hydroxyethyl group, and
R^{Q5} represents a hydrogen atom or a methyl group.

16. A compound or a pharmaceutically acceptable salt thereof, the compound being any one compound selected from the group consisting of:
2-chloro-N-(furan-2-ylmethyl)-8-methoxyquinazolin-4-amine,
2-chloro-N-(furan-2-ylmethyl)-8-methoxyquinolin-4-amine,
2-chloro-7-fluoro-N-(furan-2-ylmethyl)-8-methoxyquinazolin-4-amine,
2-chloro-5-fluoro-N-(furan-2-ylmethyl)-8-methoxyquinazolin-4-amine,
2-bromo-N-(furan-2-ylmethyl)-8-methoxyquinazolin-4-amine,
2-chloro-N-(furan-2-ylmethyl)-8-methoxypyrido[3,4-d]pyrimidin-4-amine,
2-chloro-N-(furan-2-ylmethyl)-8-methoxypyrido[4,3-d]pyrimidin-4-amine,
2-chloro-N-(furan-2-ylmethyl)-8-methoxypyrido[3,2-d]pyrimidin-4-amine,
2-chloro-6-fluoro-N-(furan-2-ylmethyl)-8-methoxyquinazolin-4-amine,
2-chloro-N-(furan-2-ylmethyl)-6,8-dimethoxyquinazolin-4-amine,
2-chloro-N-(furan-2-ylmethyl)-7,8-dimethoxyquinazolin-4-amine,
2-chloro-8-methoxy-N-(thiophen-2-ylmethyl) quinazolin-4-amine,
2-chloro-8-methoxy-N-(1,3-thiazol-2-ylmethyl)quinazolin-4-amine,
2-chloro-8-methoxy-N-(1,3-oxazol-2-ylmethyl)quinazolin-4-amine,
2-chloro-8-methoxy-N-(thiophen-3-ylmethyl) quinazolin-4-amine,
2-chloro-8-methoxy-N-(pyridin-4-ylmethyl)quinazolin-4-amine,
2-chloro-N-(2-fluorobenzyl)-8-methoxyquinazolin-4-amine,
2-chloro-8-(1,4-dioxan-2-ylmethoxy)-N-(furan-2-ylmethyl)quinazolin-4-amine,
5-[({2-chloro-4-[(furan-2-ylmethyl)amino]quinazolin-8-yl}oxy)methyl]-1,3-oxazolidin-2-one,
(5R)-5-[({2-chloro-4-[(furan-2-ylmethyl)amino]quinazolin-8-yl}oxy)methyl]-1,3-oxazolidin-2-one,
(5S)-5-[({2-chloro-4-[(furan-2-ylmethyl)amino]quinazolin-8-yl}oxy)methyl]-1,3-oxazolidin-2-one,
1-{3-[({2-chloro-4-[(furan-2-ylmethyl)amino]quinazolin-8-yl}oxy)methyl]pyrrolidin-1-yl}ethanone,
2-chloro-N-(furan-2-ylmethyl)-8-{[1-(methylsulfonyl)pyrrolidin-3-yl]methoxy}quinazolin-4-amine,
2-chloro-8-[(2,2-dimethyl-1,3-dioxan-5-yl)methoxy]-N-(furan-2-ylmethyl)quinazolin-4-amine,
5-[({2-chloro-4-[(furan-2-ylmethyl)amino]quinazolin-8-yl}oxy)methyl]pyrrolidin-2-one,
2-chloro-8-[(2,2-difluorocyclobutyl)methoxy]-N-(furan-2-ylmethyl)quinazolin-4-amine,
2-chloro-8-(1,4-dioxaspiro[4.4]nonan-7-ylmethoxy)-N-(furan-2-ylmethyl)quinazolin-4-amine,
3-[({2-chloro-4-[(furan-2-ylmethyl)amino]quinazolin-8-yl}oxy)methyl]cyclopentanone,
2-chloro-8-(1,4-dioxaspiro[4.4]nonan-6-ylmethoxy)-N-(furan-2-ylmethyl)quinazolin-4-amine,
2-[({2-chloro-4-[(furan-2-ylmethyl)amino]quinazolin-8-yl}oxy)methyl]cyclopentanone,
4-[({2-chloro-4-[(furan-2-ylmethyl)amino]quinazolin-8-yl}oxy)methyl]pyrrolidin-2-one,
3-[({2-chloro-4-[(furan-2-ylmethyl)amino]quinazolin-8-yl}oxy)methyl]cyclobutanol,
2-chloro-8-{[3-(difluoromethoxy)cyclobutyl]methoxy}-N-(furan-2-ylmethyl)quinazolin-4-amine,
5-[({2,6-dichloro-4-[(furan-2-ylmethyl)amino]quinazolin-8-yl}oxy)methyl]-3-methyl-1,3-oxazolidin-2-one,
(3S,5R)-5-[({2,6-dichloro-4-[(furan-2-ylmethyl)amino]quinazolin-8-yl}oxy)methyl]-3-methylpyrrolidin-2-one,
5-({[4-(benzylamino)-2,6-dichloroquinazolin-8-yl]oxy}methyl)-3-methyl-1,3-oxazolidin-2-one,
5-[({2,7-dichloro-4-[(furan-2-ylmethyl)amino]quinazolin-8-yl}oxy)methyl]-3-methyl-1,3-oxazolidin-2-one,
2-chloro-8-(1,4-dioxan-2-ylmethoxy)-N-(furan-2-ylmethyl) quinolin-4-amine,
5-[({2-chloro-4-[(furan-2-ylmethyl)amino]quinolin-8-yl}oxy)methyl]-1,3-oxazolidin-2-one,
5-({[4-(benzylamino)-2-chloroquinazolin-8-yl]oxy}methyl)-3-methyl-1,3-oxazolidin-2-one,
5-({[4-(benzylamino)-2-chloroquinazolin-8-yl]oxy}methyl)-3-(2-fluoroethyl)-1,3-oxazolidin-2-one,
5-({[4-(benzylamino)-2-chloroquinazolin-8-yl]oxy}methyl)-3-(2-hydroxyethyl)-1,3-oxazolidin-2-one,
5-({[4-(benzylamino)-2-chloroquinolin-8-yl]oxy}methyl)-3-methyl-1,3-oxazolidin-2-one,
2-chloro-N-(furan-2-ylmethyl)-8-(tetrahydrofuran-3-ylmethoxy)quinazolin-4-amine,
2-chloro-N-(furan-2-ylmethyl)-8-(tetrahydro-2H-pyran-3-ylmethoxy)quinazolin-4-amine,
2-chloro-8-[(1-fluorocyclopropyl)methoxy]-N-(furan-2-ylmethyl)quinazolin-4-amine,
1-[2-({2-chloro-4-[(furan-2-ylmethyl)amino]quinazolin-8-yl}oxy)ethyl]imidazolidin-2-one,
2-chloro-8-[(1,1-dioxidotetrahydrothiophen-3-yl)methoxy]-N-(furan-2-ylmethyl)quinazolin-4-amine,
7-[({2-chloro-4-[(furan-2-ylmethyl) amino] quinazolin-8-yl}oxy)methyl]-6-oxa-4-azaspiro[2.4]heptan-5-one,
(3S,5R)-5-[({2-chloro-4-[(furan-2-ylmethyl)amino]quinazolin-8-yl}oxy)methyl]-3-methylpyrrolidin-2-one,
2-chloro-N-(furan-2-ylmethyl)-8-[(2-methyl-1,1-dioxido-1,2-thiazolidin-4-yl)methoxy] quinazolin-4-amine,
2-chloro-8-(furan-2-ylmethoxy)-N-(furan-2-ylmethyl)quinazolin-4-amine,
3-[2-({2-chloro-4-[(furan-2-ylmethyl)amino]quinazolin-8-yl}oxy)ethyl]-1,3-oxazolidin-2-one,
4-[({2-chloro-4-[(furan-2-ylmethyl) amino] quinazolin-8-yl}oxy)methyl]-1,3-dioxolan-2-one,
2-chloro-8-[2-(1,4-dioxan-2-yl)ethoxy]-N-(furan-2-ylmethyl)quinazolin-4-amine,
2-chloro-N-(furan-2-ylmethyl)-8-[(3-methoxy-4,5-dihydro-1,2-oxazol-5-yl)methoxy] quinazolin-4-amine,
5-[({2-chloro-4-[(furan-2-ylmethyl)amino]quinazolin-8-yl}oxy)methyl]morpholin-3-one,
1-benzyl-4-[({2-chloro-4-[(furan-2-ylmethyl)amino]quinazolin-8-yl}oxy)methyl]imidazolidin-2-one,
2-chloro-N-(furan-2-ylmethyl)-8-(2-oxaspiro[3.3]heptan-6-ylmethoxy)quinazolin-4-amine,
2-chloro-8-(cyclopentylmethoxy)-N-(furan-2-ylmethyl)quinazolin-4-amine,
1-{3-[({2-chloro-4-[(furan-2-ylmethyl)amino]quinazolin-8-yl}oxy)methyl]azetidin-1-yl}ethanone,
2-chloro-8-(furan-3-ylmethoxy)-N-(furan-2-ylmethyl)quinazolin-4-amine,
2-chloro-N-(furan-2-ylmethyl)-8-{[1-(trifluoromethyl)cyclobutyl]methoxy}quinazolin-4-amine,
2-chloro-8-(cyclohexylmethoxy)-N-(furan-2-ylmethyl)quinazolin-4-amine,
2-chloro-N-(furan-2-ylmethyl)-8-(tetrahydrofuran-2-ylmethoxy)quinazolin-4-amine,
1-{2-[({2-chloro-4-[(furan-2-ylmethyl)amino]quinazolin-8-yl}oxy)methyl]pyrrolidin-1-yl}ethanone,
4-[({2-chloro-4-[(furan-2-ylmethyl) amino] quinazolin-8-yl}oxy)methyl]-1,3-oxazolidin-2-one,
3-benzyl-5-[({2-chloro-4-[(furan-2-ylmethyl)amino]quinazolin-8-yl}oxy)methyl]-1,3-oxazolidin-2-one,
5-[({2-chloro-4-[(furan-2-ylmethyl)amino]quinazolin-8-yl}oxy)methyl]-3-phenyl-1,3-oxazolidin-2-one,
(3S,5R)-5-({[4-(benzylamino)-2-chloroquinazolin-8-yl]oxy}methyl)-3-methylpyrrolidin-2-one,
(3R,5R)-5-({[4-(benzylamino)-2-chloroquinazolin-8-yl]oxy}methyl)-3-methylpyrrolidin-2-one,
4-({[4-(benzylamino)-2-chloroquinazolin-8-yl]oxy}methyl)pyrrolidin-2-one,
5-[({2-chloro-4-[(furan-2-ylmethyl)amino]quinazolin-8-yl}oxy)methyl]-4,4-dimethyl-1,3-oxazolidin-2-one,
6-[({2-chloro-4-[(furan-2-ylmethyl) amino] quinazolin-8-yl}oxy)methyl]-1,3-oxazinan-2-one,
4-[({2-chloro-4-[(furan-2-ylmethyl)amino]quinazolin-8-yl}oxy)methyl]-1-methylpyrrolidin-2-one,
4-[({2-chloro-4-[(furan-2-ylmethyl)aminolquinazolin-8-yloxy)methyl]-5-methyl-1,3-dioxol-2-one,
6-[({2-chloro-4-[(furan-2-ylmethyl)amino]quinazolin-8-yl}oxy)methyl]piperidin-2-one,
(5R)-5-[({2-chloro-4-[(furan-2-ylmethyl)amino]quinazolin-8-yl}oxy)methyl]pyrrolidin-2-one,
(5S)-5-[({2-chloro-4-[(furan-2-ylmethyl)amino]quinazolin-8-yl}oxy)methyl]pyrrolidin-2-one,
4-[({2-chloro-4-[(furan-2-ylmethyl)amino]quinazolin-8-yl}oxy)methyl]azetidin-2-one,
5-[({2-chloro-4-[(furan-2-ylmethyl)amino]quinazolin-8-yl}oxy)methyl]-5-methylpyrrolidin-2-one,
4-[({2-chloro-4-[(furan-2-ylmethyl)amino]quinazolin-8-yl}oxy)methyl]-4-methyl-1,3-oxazolidin-2-one,
5-[({2-chloro-4-[(furan-2-ylmethyl)amino]quinazolin-8-yl}oxy)methyl]-3-methyl-1,3-oxazolidin-2-one,
5-({[4-(benzylamino)-2-chloroquinazolin-8-yl]oxy}methyl)-1,3-oxazolidin-2-one,
5-[({2-chloro-4-[(furan-2-ylmethyl)amino]quinolin-8-yl}oxy)methyl]pyrrolidin-2-one,
5-[({2-chloro-4-[(furan-2-ylmethyl) amino] quinolin-8-yl} oxy)methyl]-3-(2-hydroxyethyl)-1,3-oxazolidin-2-one,
5-[({2-chloro-4-[(furan-2-ylmethyl)amino]quinolin-8-yl}oxy)methyl]-3-(2-fluoroethyl)-1,3-oxazolidin-2-one,
1-{3-[({2-chloro-4-[(furan-2-ylmethyl)amino]quinolin-8-yl}oxy)methyl]azetidin-1-yl}ethanone,
4-[({2-chloro-4-[(furan-2-ylmethyl)aminolquinolin-8-yl}oxy)methyl]pyrrolidin-2-one,
2-chloro-N-(furan-2-ylmethyl)-8-{[1-(methylsulfonyl)azetidin-3-yl]methoxy}quinolin-4-amine,
(3S,5R)-5-[({2-chloro-4-[(furan-2-ylmethyl)amino]quinolin-8-yl}oxy)methyl]-3-methylpyrrolidin-2-one,
2-chloro-N-(furan-2-ylmethyl)-8-{[1-(methylsulfonyl)pyrrolidin-3-yl]methoxy}quinolin-4-amine,
1-{3-[({2-chloro-4-[(furan-2-ylmethyl)amino]quinolin-8-yl}oxy)methyl]pyrrolidin-1-yl}ethanone,
(5R)-5-[({2-chloro-4-[(furan-2-ylmethyl)amino] quinolin-8-yl}oxy)methyl]-1,3-oxazolidin-2-one,
(5S)-5-[({2-chloro-4-[(furan-2-ylmethyl)amino]quinolin-8-yl}oxy)methyl]-1,3-oxazolidin-2-one,
(5R)-5-[({2-chloro-4-[(furan-2-ylmethyl)amino] quinolin-8-yl}oxy)methyl]pyrrolidin-2-one,
(5S)-5-[({2-chloro-4-[(furan-2-ylmethyl)amino]quinolin-8-yl}oxy)methyl]pyrrolidin-2-one,
5-({{4-(benzylamino)-2-chloroquinolin-8-yl]oxy}methyl)-1,3-oxazolidin-2-one,
5-({[4-(benzylamino)-2-chloroquinolin-8-yl]oxy}methyl)-3-(2-hydroxyethyl)-1,3-oxazolidin-2-one,
5-({[4-(benzylamino)-2-chloroquinolin-8-yl]oxy}methyl)-3-(2-fluoroethyl)-1,3-oxazolidin-2-one,
(3S,5R)-5-({[4-(benzylamino)-2-chloroquinolin-8-yl]oxy}methyl)-3-methylpyrrolidin-2-one,
4-({[4-(benzylamino)-2-chloroquinolin-8-yl]oxy}methyl)pyrrolidin-2-one,
2-chloro-8-(1,4-dioxan-2-ylmethoxy)-N-(furan-2-ylmethyl)- 1,5-naphthyridin-4-amine,
5-[({6-chloro-8-[(furan-2-ylmethyl)amino]-1,5-naphthyridin-4-yl}oxy)methyl]-1,3-oxazolidin-2-one,
5-[({6-chloro-8-[(furan-2-ylmethyl)amino]-1,5-naphthyridin-4-yl}oxy)methyl]-3-methyl-1,3-oxazolidin-2-one,
(3S,5R)-5-[({6-chloro-8-[(furan-2-ylmethyl)amino]-1,5-naphthyridin-4-yl}oxy)methyl]-3-methylpyrrolidin-2-one,
5-[({6-chloro-8-[(furan-2-ylmethyl)amino]-1,5-naphthyridin-4-yl}oxy)methyl]-3-(2-hydroxyethyl)-1,3-oxazolidin-2-one,
5-[({6-chloro-8-[(furan-2-ylmethyl)amino]-1,5-naphthyridin-4-yl}oxy)methyl]-3-(2-fluoroethyl)-1,3-oxazolidin-2-one,
5-({[8-(benzylamino)-6-chloro-1,5-naphthyridin-4-yl]oxy}methyl)-1,3-oxazolidin-2-one,
5-({[8-(benzylamino)-6-chloro-1,5-naphthyridin-4-yl]oxy}methyl)-3-methyl-1,3-oxazolidin-2-one,
(3S,5R)-5-({[8-(benzylamino)-6-chloro-1,5-naphthyridin-4-yl]oxy}methyl)-3-methylpyrrolidin-2-one,
5-({[8-(benzylamino)-6-chloro-1,5-naphthyridin-4-yl]oxy}methyl)-3-(2-hydroxyethyl)-1,3-oxazolidin-2-one,
5-({[8-(benzylamino)-6-chloro-1,5-naphthyridin-4-yl]oxy}methyl)-3-(2-fluoroethyl)-1,3-oxazolidin-2-one,
5-({[4-(benzylamino)-2-chloropyrido[3,2-d]pyrimidin-8-yl]oxy}methyl)-3-methyl-1,3-oxazolidin-2-one,
(5R)-5-[({2-chloro-4-[(furan-2-ylmethyl)amino]quinazolin-8-yl}oxy)methyl]-3-(methoxymethylpyrrolidin-2-one,
3-[({2-chloro-4-[(furan-2-ylmethyl)amino]quinazolin-8-yl}oxy)methyl]pyrrolidin-2-one,
4-[({2-chloro-4-[(furan-2-ylmethyl)amino]quinazolin-8-yl}oxy)methyl]-1-methylimidazolidin-2-one,
5-[2-({2-chloro-4-[(furan-2-ylmethyl)amino]quinazolin-8-yl}oxy)ethyl]pyrrolidin-2-one,
2-chloro-8-[(2,2-dimethyl-1,3-dioxolan-4-yl)methoxy]-N-(furan-2-ylmethyl)quinazolin-4-amine,
trans-4-[({2-chloro-4-[(furan-2-ylmethyl)amino]quinazolin-8-yl}oxy)methyl]cyclohexanol,
4-[({2-chloro-4-[(furan-2-ylmethyl)amino]quinolin-8-yl}oxy)methyl]- 1-methylimidazolidin-2-one,
2-chloro-8-(cyclopropylmethoxy)-N-(furan-2-ylmethyl)quinazolin-4-amine,
2-chloro-N-(furan-2-ylmethyl)-8-(oxetan-3-ylmethoxy)quinazolin-4-amine,
2-chloro-8-(1,3-dioxan-5-ylmethoxy)-N-(furan-2-ylmethyl)quinazolin-4-amine,
2-chloro-8-(cyclobutylmethoxy)-N-(furan-2-ylmethyl)quinazolin-4-amine,
2-chloro-8-[(2S)-1,4-dioxan-2-ylmethoxy]-N-(furan-2-ylmethyl)quinazolin-4-amine,
2-chloro-8-[(2S)-1,4-dioxan-2-ylmethoxy]-7-fluoro-N-(furan-2-ylmethyl)quinazolin-4-amine,
2-chloro-N-(furan-2-ylmethyl)-8-(oxetan-3-ylmethoxy)quinolin-4-amine,
2-chloro-8-[(2,2-difluorocyclopropyl)methoxyl-N-(furan-2-ylmethyl)quinazolin-4-amine,
2-chloro-8-[(3,3-difluorocyclobutyl)methoxy]-N-(furan-2-ylmethyl)quinazolin-4-amine,
2-chloro-N-(furan-2-ylmethyl)-8-[(1-methylcyclopropyl)methoxy]quinazolin-4-amine,
2-chloro-N-(furan-2-ylmethyl)-8-(tetrahydro-2H-pyran-2-ylmethoxy)quinazolin-4-amine,
2-chloro-N-(furan-2-ylmethyl)-8-(oxetan-2-ylmethoxy)quinazolin-4-amine,
2-chloro-8-[2-(1,1-dioxidothiethan-3-yl)ethoxy]-N-(furan-2-ylmethyl)quinazolin-4-amine,
2-chloro-8-[(2R)-1,4-dioxan-2-ylmethoxy]-N-(furan-2-ylmethyl) quinazolin-4-amine,
5-[({2-chloro-4-[(furan-2-ylmethyl)amino]quinazolin-8-yl}oxy)methyl]-5-methyl-1,3-oxazolidin-2-one,
2-chloro-8-[(2R)-1,4-dioxan-2-ylmethoxy]-7-fluoro-N-(furan-2-ylmethyl)quinazolin-4-amine,
2-chloro-8-(cyclopropylmethoxy)-7-fluoro-N-(furan-2-ylmethyl) quinazolin-4-amine,
2-chloro-8-(cyclobutylmethoxy)-7-fluoro-N-(furan-2-ylmethylquinazolin-4-amine,
2-chloro-7-fluoro-N-(furan-2-ylmethyl)-8-(oxetan-3-ylmethoxy)quinazolin-4-amine,
2-chloro-8-(cyclopropylmethoxy)-N-(furan-2-ylmethyl)quinolin-4-amine,
2-chloro-8-[2-(1,1-dioxidothiethan-3-yl)ethoxyl]-N-(furan-2-ylmethyl)quinolin-4-amine,
2-chloro-8-[(2,2-difluorocyclopropyl)methoxy]-N-(thiophen-2-ylmethyl)quinazolin-4-amine,
2-chloro-8-(1,4-dioxan-2-ylmethoxy)-N-(thiophen-2-ylmethyl)quinazolin-4-amine,
2-chloro-8-[(2,2-difluorocyclopropyl)methoxy]-N-(thiophen-3-ylmethyl)quinazolin-4-amine,
2-chloro-8-(1,4-dioxan-2-ylmethoxy)-N-(thiophen-3-ylmethyl)quinazolin-4-amine,
5-{[(2-chloro-4-{[(4-fluorofuran-2-yl)methyl]amino}quinazolin-8-yl)oxy]methyl}-1,3-oxazolidin-2-one,
2-chloro-8-[(2,2-difluorocyclopropyl)methoxy]-N-(1,2-thiazol-5-ylmethyl)quinazolin-4-amine,
2-chloro-8-[(2,2-difluorocyclopropyl)methoxy]-N-(1,3-oxazol-4-ylmethyl)quinazolin-4-amine,
2-chloro-8-(1,4-dioxan-2-ylmethoxy)-N-(1,2-oxazol-3-ylmethyl)quinazolin-4-amine,
5-[({2-chloro-4-[(1,3-oxazol-2-ylmethyl)amino]quinazolin-8-yl}oxy)methyl]-1,3-oxazolidin-2-one,
2-chloro-8-(1,4-dioxan-2-ylmethoxy)-N-(1,3-oxazol-2-ylmethyl)quinazolin-4-amine,
5-[({2-chloro-4-[(thiophen-2-ylmethyl)amino]quinazolin-8-yl}oxy)methyl]-1,3-oxazolidin-2-one,
5-[({2-chloro-4-[(thiophen-3-ylmethyl)amino]quinazolin-8-yl}oxy)methyl]-1,3-oxazolidin-2-one,
2-chloro-8-[(2,2-difluorocyclopropyl)methoxy]-N-(1,3-thiazol-2-ylmethyl)quinazolin-4-amine,
2-chloro-8-(1,4-dioxan-2-ylmethoxy)-N-(1,3-thiazol-2-ylmethyl)quinazolin-4-amine,
5-[({2-chloro-4-[(1,3-thiazol-2-ylmethyl)amino]quinazolin-8-yl}oxy)methyl]-1,3-oxazolidin-2-one,
2-chloro-8-[(2,2-difluorocyclopropyl)methoxy]-N-(1,3-oxazol-2-ylmethyl)quinazolin-4-amine,
2-chloro-8-[(2,2-difluorocyclopropyl)methoxy]-N-(pyridin-4-ylmethyl)quinazolin-4-amine,
5-[({2-chloro-4-[(pyridin-4-ylmethyl)amino]quinazolin-8-yl}oxy)methyl]-1,3-oxazolidin-2-one,
5-[({2-chloro-4-[(pyrimidin-4-ylmethyl)amino]quinazolin-8-yl}oxy)methyl]-1,3-oxazolidin-2-one,
5-[({2-chloro-4-[(pyridin-2-ylmethyl)amino]quinazolin-8-yl}oxy)methyl]-1,3-oxazolidin-2-one,
5-{[(2-chloro-4-{[(3-fluoropyridin-4-yl)methyl]amino}quinazolin-8-yl)oxy]methyl}-1,3-oxazolidin-2-one,
5-[({2-chloro-4-[(2-fluorobenzyl)amino]quinazolin-8-yl}oxy)methyl]-1,3-oxazolidin-2-one,
5-[({2-chloro-4-[(3-fluorobenzyl)amino]quinazolin-8-yl}oxy)methyl]-1,3-oxazolidin-2-one,
2-chloro-8-(1,4-dioxan-2-ylmethoxy)-N-(1,2,4-oxadiazol-3-ylmethyl) quinazolin-4-amine,
5-[({2-chloro-4-[(1,2-thiazol-5-ylmethyl)amino]quinazolin-8-yl}oxy)methyl]-1,3-oxazolidin-2-one,
5-[({2-chloro-4-[(4-fluorobenzyl)amino]quinazolin-8-yl}oxy)methyl]-1,3-oxazolidin-2-one,
5-[({2-chloro-4-[(furan-2-ylmethyl)amino]-1,7-naphthyridin-8-yl}oxy)methyl]-3-methyl-1,3-oxazolidin-2-one,
(5S)-5-[({2-chloro-4-[(furan-2-ylmethyl)amino]-1,7-naphthyridin-8-yl}oxy)methyl]-3-methyl-1,3-oxazolidin-2-one,
(5R)-5-[({2-chloro-4-[(furan-2-ylmethyl)amino]- 1, 7-naphthyridin-8-yl}oxy)methyl]-3-methyl- 1,3-oxazolidin-2-one,
5-({[4-(benzylamino)-2-chloro-1,7-naphthyridin-8-yl]oxy}methyl)-1,3-oxazolidin-2-one,
5-[({2-chloro-4-[(furan-2-ylmethyl)amino]-1,7-naphthyridin-8-yl}oxy)methyl]-3-(2-fluoroethyl)-1,3-oxazolidin-2-one,
5-[({2-chloro-4-[(furan-2-ylmethyl)amino]-1,7-naphthyridin-8-yl}oxy)methyl]-3-(2-hydroxyethyl)-1,3-oxazolidin-2-one,
5-({[4-(benzylamino)-2-chloro-1,7-naphthyridin-8-yl]oxy}methyl)-3-(2-fluoroethyl)- 1,3-oxazolidin-2-one,
5-({[4-(benzylamino)-2-chloro-1,7-naphthyridin-8-yl]oxy}methyl)-3-methyl-1,3-oxazolidin-2-one,
5-({[4-(benzylamino)-2-chloro-1,7-naphthyridin-8-yl]oxy}methyl)-3-(2-hydroxyethyl)-1,3-oxazolidin-2-one,
2-chloro-8-(1,4-dioxan-2-ylmethoxy)-N-(furan-2-ylmethyl)pyrido[3,2-d]pyrimidin-4-amine,
2-chloro-8-(1,4-dioxan-2-ylmethoxy)-N-(furan-2-ylmethyl)pyrido[3,4-d]pyrimidin-4-amine,
5-[({2-chloro-4-[(furan-2-ylmethyl)amino]pyrido[3,4-d]pyrimidin-8-yl}oxy)methyl]-3-methyl-1,3-oxazolidin-2-one,
5-({[4-(benzylamino)-2-chloropyrido[3,4-d]pyrimidin-8-yl]oxy}methyl)-3-methyl-1,3-oxazolidin-2-one,
5-[({2-chloro-4-[(furan-2-ylmethyl)amino]pyrido[4,3-d]pyrimidin-8-yl}oxy)methyl]-3-methyl-1,3-oxazolidin-2-one,
5-({[4-(benzylamino)-2-chloropyrido[4,3-d]pyrimidin-8-yl]oxy}methyl)-3-methyl-1,3-oxazolidin-2-one,
5-({[4-(benzylamino)-2-chloro-1,6-naphthyridin-8-yl]oxy}methyl)-1,3-oxazolidin-2-one,
5-({[4-(benzylamino)-2-chloro-1,6-naphthyridin-8-yl]oxy}methyl)-3-methyl-1,3-oxazolidin-2-one,
5-[({2-chloro-4-[(furan-2-ylmethyl)amino]quinazolin-8-yl}oxy)methyl]-3-ethyl-1,3-oxazolidin-2-one,
5-[({2-chloro-4-[(furan-2-ylmethyl)amino]quinazolin-8-yl}oxy)methyl]-3-(propan-2-yl)-1,3-oxazolidin-2-one,
5-[({2-chloro-4-[(furan-2-ylmethyl)amino]quinazolin-8-yl}oxy)methyl]-3-(2-fluoroethyl)- 1,3-oxazolidin-2-one,
5-[({2-chloro-4-[(furan-2-ylmethyl)amino]quinazolin-8-yl}oxy)methyl]-3-(cyclopropylmethyl)-1,3-oxazolidin-2-one,
5-[({2-chloro-4-[(furan-2-ylmethyl)amino]quinazolin-8-yl}oxy)methyl]-3-(2,2-difluoroethyl)-1,3-oxazolidin-2-one,
5-[({2-chloro-4-[(furan-2-ylmethyl)amino]quinazolin-8-yl}oxy)methyl]-3-(2-hydroxyethyl)-1,3-oxazolidin-2-one,
5-[({2-chloro-4-[(furan-2-ylmethyl)amino]quinazolin-8-yl}oxy)methyl]-3-(oxetan-3-yl)-1,3-oxazolidin-2-one,
5-[({2-chloro-4-[(furan-2-ylmethyl)amino]quinazolin-8-yl}oxy)methyl]-3-(2-hydroxy-2-methylpropyl)-1,3-oxazolidin-2-one,
5-[({2-chloro-4-[(furan-2-ylmethyl)amino]quinazolin-8-yl}oxy)methyl]-N-ethyl-2-oxo-1,3-oxazolidine-3-carboxamide,
4-[({2-chloro-4-[(furan-2-ylmethyl)amino]quinazolin-8-yl}oxy)methyl]-1-(2-hydroxyethylpyrrolidin-2-one,
4-[({2-chloro-4-[(furan-2-ylmethyl) amino] quinazolin-8-yl}oxy)methyl]-1-(2-fluoroethyl)pyrrolidin-2-one,
5-[({2-chloro-4-[(furan-2-ylmethyl)amino]quinazolin-8-yl}oxy)methyl]-3-(prop-2-yn-1-yl)-1,3-oxazolidin-2-one,
tert-butyl[2-(2-{5-[({2-chloro-4-[(furan-2-ylmethyl)amino]quinazolin-8-yl}oxy)methyl]-2-oxo-1,3-oxazolidin-3-yl}ethoxy)ethyl]carbamate,
5-[({2-chloro-4-[(furan-2-ylmethyl)amino]quinazolin-8-yl}oxy)methyl]-3-[2-(2-methoxyethoxy)ethyl]-1,3-oxazolidin-2-one,
5-[({2-chloro-4-[(furan-2-ylmethyl)amino]quinazolin-8-yl}oxy)methyl]-3-(oxetan-3-ylmethyl)-1,3-oxazolidin-2-one,
5-[({2-chloro-4-[(furan-2-ylmethyl)amino]quinazolin-8-yl}oxy)methyl]-3-(1,2,4-oxadiazol-5-ylmethyl)- 1,3-oxazolidin-2-one,
2-{5-[({2-chloro-4-[(furan-2-ylmethyl)amino]quinazolin-8-yl}oxy)methyl]-2-oxo-1,3-oxazolidin-3-yl}acetamide, and
5-[({2-chloro-4-[(furan-2-ylmethyl)amino]quinazolin-8-yl}oxy)methyl]-3-(2-methoxyethyl)-1,3-oxazolidin-2-one.

17. A pharmaceutical composition containing, as an active ingredient, the compound according to any one of claims 1 to 16 or pharmaceutically acceptable salt thereof.

18. A pharmaceutical composition for treating a genetic disease caused by aberrant splicing, the pharmaceutical composition containing, as an active ingredient, the compound according to any one of claims 1 to 16 or pharmaceutically acceptable salt thereof.

19. The pharmaceutical composition according to claim 18,
wherein the aberrant splicing is caused by an IVS4+9 19G>A mutation in the α-galactosidase gene.

20. The pharmaceutical composition according to claim 18,
wherein the genetic disease caused by aberrant splicing is Fabry disease.

21. A method for treating a genetic disease caused by aberrant splicing, the method comprising:
administering the compound according to any one of claims 1 to 16 or pharmaceutically acceptable salt thereof to a subject.

22. The method according to claim 21,
wherein the genetic disease caused by aberrant splicing is Fabry disease.

23. Use of the compound according to any one of claims 1 to 16 or pharmaceutically acceptable salt thereof in production of a pharmaceutical composition for use in treatment of a genetic disease caused by aberrant splicing.

24. The use according to claim 23,
wherein the genetic disease caused by aberrant splicing is Fabry disease.
